# EUROPEAN PATENT APPLICATION

(11) **EP 2 302 069 A1**
(43) Date of publication of application: **30.03.2011**
(21) Application number: 10008955.6
(22) Date of filing: 11.12.2008
(51) Int. Cl.: C12Q 1/68

(54) **Methods and nucleic acids for analyses of cell proliferative disorders**

(30) Priority: 11.12.2007 EP 07122844; 23.01.2008 EP 08150557
(62) Divisional of application: 08860719.7
(71) Applicant: Epigenomics AG, 10178 Berlin (DE)
(72) Inventor: Dietrich, Dimo, 10437 Berlin (DE); Liebenberg, Volker, 14050 Berlin (DE); Tetzner, Reimo, 10439 Berlin (DE); Distler, Jürgen, 12163 Berlin (DE)
(74) Representative: Krauss, Jan

(57) **Abstract**

The invention provides methods, nucleic acids and kits for detecting lung carcinoma. The invention discloses genomic FOXL2 sequences the methylation patterns of which have utility for the improved detection of said disorder, thereby enabling the improved diagnosis and treatment of patients.

## Description

### FIELD OF THE INVENTION

The present invention relates to genomic DNA sequences that exhibit altered expression patterns in disease states relative to normal. Particular embodiments provide methods, nucleic acids, nucleic acid arrays and kits useful for detecting, or for diagnosing cell proliferative disorders.

### BACKGROUND

*CpG island methylation.* Apart from mutations aberrant methylation of CpG islands has been shown to lead to the transcriptional silencing of certain genes that have been previously linked to the pathogenesis of various cell proliferative disorders, including cancer. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, *i.e*., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, *i.e*., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cell proliferative disorders, often involve a plurality of genes and proteins. Additionally, certain proteins may be elevated for reasons unrelated to a disease state. Specificity is important when the cost or risk associated with further diagnostic procedures or further medical intervention are very high.

### SUMMARY OF THE INVENTION

The present invention provides a method for detecting or differentiating cell proliferative disorders, preferably those according to Table 2, and most preferably lung carcinomas, in a subject comprising determining the expression levels wherein determining expression levels also includes determining methylation levels and patterns of at least one gene or genomic sequence selected from the group consisting of FOXL-2, ONECUT1, TFAP2E, EN2-2, EN2-3, SHOX2-2, and BARHL2 in a biological sample isolated from said subject wherein hyper-methylation and /or under-expression is indicative of the presence of said disorder. Various aspects of the present invention provide an efficient and unique genetic marker, whereby expression analysis of said marker enables the detection of cell proliferative disorders, preferably those according to Table 2 with a particularly high sensitivity, specificity and/or predictive value. Preferred is that the lung cancer is selected from the group consisting of Lung adenocarcinoma; Large cell lung cancer; Squamous cell lung carcinoma and Small cell lung carcinoma.

In one embodiment the invention provides a method for detecting cell proliferative disorders, preferably those according to Table 2 (most preferably lung carcinoma), in a subject comprising determining the expression levels of at least one gene or genomic sequence selected from the group consisting of FOXL-2, ONECUT1, TFAP2E, EN2-2, EN2-3, SHOX2-2 and BARHL2 in a biological sample isolated from said subject wherein under-expression and/or CpG methylation is indicative of the presence of said disorder. In one embodiment said expression level is determined by detecting the presence, absence or level of mRNA transcribed from said gene. In a further embodiment said expression level is determined by detecting the presence, absence or level of a polypeptide encoded by said gene or sequence thereof.

In a further preferred embodiment said expression is determined by detecting the presence or absence or level of CpG methylation within said gene, wherein under-expression, which is understood as indicated by presence of CpG methylation, or by presence of a certain level of methylation, indicates the presence of cell proliferative disorders, preferably those according to Table 2 (most preferably lung carcinoma).

Said method comprises the following steps: i) contacting genomic DNA isolated from a biological sample (preferably selected from the group consisting of cells or cell lines, histological slides, biopsies, paraffin-embedded tissue, body fluids, ejaculate, urine, blood plasma, blood serum, whole blood, isolated blood cells, sputum and biological matter derived from bronchoscopy (including, but not limited to, bronchial lavage, bronchial alveolar lavage, bronchial brushing, and bronchial abrasion) obtained from the subject, preferably a human subject, with at least one reagent, or series of reagents that distinguishes between methylated and non-methylated CpG dinucleotides within at least one target region of the genomic DNA, wherein the target region is the region which is investigated and wherein the nucleotide sequence of said target region comprises at least one CpG dinucleotide sequence of at least one gene or genomic sequence selected from the group consisting of FOXL-2, ONECUT1, TFAP2E (including promoter or regulatory elements thereof) and EN2-2, EN2-3, SHOX2-2 and BARHL2- and ii) detecting cell proliferative disorders, preferably those according to (most preferably lung carcinoma), at least in part. Preferably the target region is located within a genomic sequences selected from the group mentioned above. It is preferred that the target region comprises, or hybridizes under stringent conditions to a sequence of at least 16 contiguous nucleotides of SEQ ID NO: 1 to SEQ ID NO: 7.

Preferably, the sensitivity of said detection is from about 75% to about 96%, or from about 80% to about 90%, or from about 80% to about 85%. Preferably, the specificity is from about 75% to about 96%, or from about 80% to about 90%, or from about 80% to about 85%.

Said use of the gene may be enabled by means of any analysis of the expression of the gene, by means of mRNA expression analysis or protein expression analysis. However, in the most preferred embodiment of the invention the detection of cell proliferative disorders, preferably those according to (most preferably lung carcinoma), is enabled by means of analysis of the *methylation status* of at least one gene or genomic sequence selected from the group consisting of FOXL-2; ONECUT1; TFAP2E (including promoter or regulatory elements thereof) and EN2-2, EN2-3, SHOX2-2, and BARHL2.

The invention provides a method for the analysis of biological samples for features associated with the development of cell proliferative disorders, preferably those according to (most preferably lung carcinoma), the method characterized in that the nucleic acid, or a fragment thereof of SEQ ID NO: 1 to SEQ ID NO: 7 is contacted with a reagent or series of reagents capable of distinguishing between methylated and non methylated CpG dinucleotides within the genomic sequence.

The present invention provides a method for ascertaining epigenetic parameters of genomic DNA associated with the development of cell proliferative disorders, preferably those according to (most preferably lung carcinoma). The method has utility for the improved detection and diagnosis of said disease.

Preferably, the source of the test sample is selected from the group consisting of cells or cell lines, histological slides, biopsies, paraffin-embedded tissue, body fluids, ejaculate, urine, blood plasma, blood serum, whole blood, isolated blood cells, sputum and biological matter derived from bronchoscopy (including, but not limited to, lavage, bronchial alveolar lavage, bronchial brushing, bronchial abrasion, and combinations thereof. More preferably the sample type is selected from the group consisting of blood plasma, sputum and biological matter derived from bronchoscopy (including, but not limited to, bronchial lavage, bronchial alveolar lavage, bronchial brushing, and bronchial abrasion) and all possible combinations thereof.

Specifically, the present invention provides a method for detecting cell proliferative disorders, preferably those according to Table 2 (most preferably lung carcinoma) suitable for use in a diagnostic tool, comprising: obtaining a biological sample comprising genomic nucleic acid(s); contacting the nucleic acid(s), or a fragment thereof, with a reagent or a plurality of reagents sufficient for distinguishing between methylated and non methylated CpG dinucleotide sequences within a target sequence of the subject nucleic acid, wherein the target sequence comprises, or hybridises under stringent conditions to, a sequence comprising at least 16 contiguous nucleotides of SEQ ID NO: 1 to SEQ ID NO: 7, said contiguous nucleotides comprising at least one CpG dinucleotide sequence; and determining, based at least in part on said distinguishing, the methylation state of at least one CpG dinucleotide within said target sequence, or an average, or a value reflecting an average methylation state of a plurality of CpG dinucleotides within said target sequence of the subject nucleic acid, wherein the target sequence comprises, or hybridises under stringent conditions to a sequence comprising at least 16 contiguous nucleotides of SEQ ID NO: 1 to SEQ ID NO: 7, said contiguous nucleotides comprising at least one CpG dinucleotide sequence.

Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO: 8 to SEQ ID NO: 35 and contiguous regions thereof corresponding to the target sequence.

Additional embodiments provide a method for the detection of cell proliferative disorders, preferably those according to Table 2 (most preferably lung carcinoma) comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 8 to SEQ ID NO: 35 and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one, but more preferably a plurality of CpG dinucleotides of SEQ ID NO: 1 to SEQ ID NO: 7.

Preferably, determining comprises use of at least one method selected from the group consisting of: i) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 8 to SEQ ID NO: 35 and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 8 to SEQ ID NO: 35 and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 8 to SEQ ID NO: 35 and complements thereof, and extending at least one such hybridized nucleic acid molecule by at least one nucleotide base; and iv) sequencing of the amplificate.

Further embodiments provide a method for the analysis (i.e. detection or diagnosis) of cell proliferative disorders, preferably those according to Table 2 (most preferably lung carcinoma), comprising: obtaining a biological sample having subject genomic DNA; extracting the genomic DNA; contacting the genomic DNA, or a fragment thereof, comprising one or more sequences selected from the group consisting of SEQ ID NO: 1 to SEQ ID NO: 7; or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of SEQ ID NO: 1 to SEQ ID NO: 7; or an average, or a value reflecting an average methylation state of a plurality of CpG dinucleotide sequences thereof. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within SEQ ID NO: 1 to SEQ ID NO: 7.

Additional embodiments provide novel analytical assays, as well as specific favourable combinations of primers and blockers or primers and probes, resulting in especially well performing diagnostic or analytical tests.

### DETAILED DESCRIPTION OF THE INVENTION

### Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analysed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state or metylation level of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers)* covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridisation" is to be understood as a bond of an oligonucleotide to a complementary sequence along the lines of the Watson-Crick base pairings in the sample DNA, forming a duplex structure.

"Stringent hybridisation conditions," as defined herein, involve hybridising at 68°C in 5x SSC/5x Denhardt's solution/1.0% SDS, and washing in 0.2x SSC/0.1% SDS at room temperature, or involve the art-recognized equivalent thereof (e.g., conditions in which a hybridisation is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specific restriction enzymes" or "methylation-sensitive restriction enzymes" shall be taken to mean an enzyme that selectively digests a nucleic acid dependend on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5.

"Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

The term "at least one gene or genomic sequence selected from the group consisting of ONECUT1; FOXL-2 and TFAP2E; EN2-2, EN2-3, SHOX2-2 and BARHL2shall be taken to include any transcript variant thereof. Furthermore as a plurality of SNPs are known within said genes the term shall be taken to include all sequence variants thereof.

If within the present specification the genomic regions EN2-2, EN2-3 and SHOX2-2 are mentioned these terms are referring to the genomic sequences as presented in the sequence protocol (as listed in Table 1). These regions represent CpG islands associated with the genes EN2 or SHOX2.

The sample types which may be analysed with any of the methods according to the invention may be any from the group comprising cells or cell lines, histological slides, biopsies, paraffin-embedded tissue, body fluids, ejaculate, urine, blood plasma, blood serum, whole blood, isolated blood cells, sputum and biological matter derived from bronchoscopy (including, but not limited to, bronchial lavage, bronchial alveolar lavage, bronchial brushing, bronchial abrasion, and combinations thereof. More preferably the sample type is selected from the group consisting of blood plasma, sputum and biological matter derived from bronchoscopy (including, but not limited to, bronchial lavage, bronchial alveolar lavage, bronchial brushing, and bronchial abrasion) and all possible combinations thereof.

The sample types which may be analysed with any of the methods according to the invention preferably belong to the group of fluids which are derived from the bloodstream.

The sample types which may be analysed with any of the methods according to the invention also preferably belong to the group of biological samples derived from the lung. The term "biological samples derived from the lung" shall therefore comprise fluids and/or cells obtained from the bronchial system of the lung. Such biological samples derived from the lung may be taken from a subject (e.g. a patient) without adding an external fluid, in which case typical sample types are sputum, tracheal or bronchial fluid, exhaled fluid, brushings or biopsies. Such fluids from the bronchial system however may also be taken after adding or rinsing with external fluid, in which case the typical sample would be e.g. induced sputum, bronchial lavage or bronchoalveolar lavage. Such biological samples derived from the lung may be taken by use of instruments (suction catheters, bronchoscope, brushes, forceps, Water absorbing trap) or without using instruments. The method may also be employed to analyse DNA already obtained from any such material.

The bronchial system (also called "airways") is to be understood as the system of organs involved in the intake and exchange of air (especially oxygen and carbon dioxide) between an organism and the environment, e.g. trachea, bronchi, bronchioles, alveolar duct, alveoli).

The terms Bronchial lavage (BL) or Bronchoalveolar lavage (BAL) are to be understood as the types of fluids which are collected when the according medical procedures BL and BAL have been performed. BL and BAL are medical procedures in which a bronchoscope is passed through the mouth or nose into the lungs and fluid is squirted into a small part of the lung and then recollected for examination. BL/BAL is typically performed to diagnose lung disease. In particular, BAL is commonly used to diagnose infections in people with immune system problems, pneumonia in people on ventilators, some types of lung cancer, and scarring of the lung (interstitial lung disease). BAL is the most common manner to sample the components of the epithelial lining fluid (ELF) and to determine the protein composition of the pulmonary airways, and it is often used in immunological research as a means of sampling cells or pathogen levels in the lung. Examples of these include T-cell populations and influenza viral levels.

BL and BAL differ in the area (segment) of the bronchial system rinsed and the amount of fluid used:
- BL focusses on the bronchi using approximately 10ml of fluid.
- BAL reaches further towards bronchioli and alveolar ducts using a higher amount of fluid (about 100ml).

The term Bronchoscopy is understood to comprise a medical test to view the airways and diagnose lung disease. It may also be used during the treatment of some lung conditions.

Biological samples derived from the lung may also be achieved with a suction catheter for the trachea and the bronchial system, for example tubular, flexible suction catheter may be used for insertion into the trachea and the bronchial system, containing at least one continuous lumen for suction of fluids from the lungs.

The term lung carcinoma shall be taken to comprise lung adenocarcinoma; large cell lung cancer; squamous cell lung carcinoma and small cell lung carcinoma, as well as other forms of rare carcinoma types, which may be identified in a tumor which is located in the lung, whenever the specification refers to detection of lung carcinoma or diagnosis of lung carcinoma.

The term "methylation" is meant to be understood as cytosine methylation or CpG methylation. These terms are used to describe methylation at the C5 atom of the cytosine within a CpG context.

The present invention provides a method for detecting cell proliferative disorders, preferably those according to Table 2 (most preferably lung carcinoma) in a subject comprising determining the expression or methylation levels of at least one gene or genomic sequence selected from the group consisting of FOXL-2; ONECUT1; TFAP2E (including promoter or regulatory elements thereof) and EN2-2, EN2-3, SHOX2-2 and BARHL2 in a biological sample isolated from said subject wherein hyper-methylation and /or under-expression is indicative of the presence of said disorder. Said markers may be used for the diagnosis of cell proliferative disorders, preferably those according to Table 2 (most preferably lung carcinoma).

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g*., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analysed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyse individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g*., Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridisation has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 97/46705 and WO 95/15373).

The present invention provides for the use of the bisulfite technique, in combination with one or more methylation assays, for determination of the methylation status of CpG dinucleotide sequences within SEQ ID NO: 1 to SEQ ID NO: 7. Genomic CpG dinucleotides can be methylated or unmethylated (alternatively known as up- and down- methylated respectively). However the methods of the present invention are suitable for the analysis of biological samples of a heterogeneous nature e.g. a low concentration of tumor cells within a background of body fluid analyte, such as for example biological samples derived from the lung, such as sputum or bronchial lavage or bronchoalveolar lavage. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position, in other words the methylation level. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) at least one gene or genomic sequence selected from the group consisting of FOXL-2; ONECUT1; TFAP2E (including promoter or regulatory elements thereof) and EN2-2, EN2-3, SHOX2-2 and BARHL2.

According to the present invention, determination of the methylation status of CpG dinucleotide sequences within SEQ ID NO: 1 to SEQ ID NO: 7 have utility in the diagnosis and detection of cell proliferative disorders, preferably those according to Table 2 (most preferably lung carcinoma).

*Methylation Assay Procedures.* Various methylation assay procedures are known in the art, and can be used in conjunction with the present invention. These assays allow for determination of the methylation state of one or a plurality of CpG dinucleotides (*e.g*., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, *e.g*., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

*COBRA.* COBRA™ analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (*e.g*., as might be found in a typical COBRA^{™}-based kit) for COBRA^{™} analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (*e.g*., precipitation, ultrafiltration, affinity column); desulfonation buffer; and DNA recovery components.

Preferably, assays such as "MethyLight^{™}" (a fluorescence-based real-time PCR technique) (Eads et al., cell proliferative disorders, preferably those according to Cancer Res. 59:2302-2306, 1999), Ms-SNuPET^{™} (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., cell proliferative disorders, preferably those according to Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers. The HeavyMethyl™ assay may also be used in combination with methylation specific amplification primers.

Typical reagents (*e.g*., as might be found in a typical MethyLight□-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g*., as might be found in a typical MSP-based kit) for MSP analysis may include, but are not limited to: methylation-specific and unmethylation-specific PCR primers for specific gene(s) (or bisulfite treated DNA sequence or CpG island), optimized PCR buffers and deoxynucleotides, and specific probes.

*TSP Method.* The method was performed as described in the application EP08159227.1 (see p 29-28, under Examples). In brief, the DNA restriction Enzyme Tsp509I is used instead of the blocking oligonucleotides. This enzyme specifically cuts unmethylated DNA during amplicfication after bisulfite-treatment. As a result, unmethylated DNA is prevented from being amplified.

*MethyLight*^{™}*.* The MethyLight^{™} assay is a high-throughput quantitative methylation assay that utilizes fluorescence-based real-time PCR (TaqMan^{™}) technology that requires no further manipulations after the PCR step (Eads et al., Cancer Res. 59:2302-2306, 1999). Briefly, the MethyLight^{™} process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight^{™} assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight^{™} process can by used with any suitable probes e.g. "TaqMan®" , Lightcycler®, Scorpion^{™}, etc.... For example, double-stranded genomic DNA is treated with sodium bisulfite and subjected to one of two sets of PCR reactions using TaqMan® probes; *e.g*., with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g*., as might be found in a typical MethyLight□-based kit) for MethyLight^{™} analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM^{™} (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM^{™} process can by used with any suitable probes e.g. "TaqMan®" ,Lightcycler®, Scorpion®, etc. in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g*., as might be found in a typical QM^{™} -based kit) for QM^{™} analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

*Ms-SNuPE.* The Ms-SNuPE^{™} technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g*., microdissected pathology sections), and it avoids utilization of restriction enzymes for determining the methylation status at CpG sites.

Typical reagents (*e.g*., as might be found in a typical Ms-SNuPE^{™}-based kit) for Ms-SNuPE^{™} analysis may include, but are not limited to: PCR primers for specific gene (or bisulfite treated DNA sequence or CpG island); optimized PCR buffers and deoxynucleotides; gel extraction kit; positive control primers; Ms-SNuPE^{™} primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (*e.g*., precipitation, ultrafiltration, affinity column); desulfonation buffer; and DNA recovery components.

The genomic sequence(s) according to SEQ ID NO: 1 TO SEQ ID NO: 7 and non-naturally occurring treated variants thereof according to SEQ ID NO: 8 TO SEQ ID NO: 35 were determined to have novel utility for the detection of cell proliferative disorders, preferably those according to Table 2 (most preferably lung carcinoma). This utility has been exemplified in the specific assays described within the specification, especially in the examples.

The Scorpion® technique (generally described in patent application EP 9812768.1) has been adapted for the analysis of CpG methylation as described in detail within the published EP patent EP 1 654 388.

In one embodiment the method of the invention comprises the following steps: i) determining the expression of at least one gene or genomic sequence selected from the group consisting of ONECUT1; FOXL-2 and TFAP2E and ii) determining the presence or absence of a subject's risk or increased risk of suffering from a cell proliferative disorder, or detecting a cell proliferative disorder preferably those according to Table 2 (most preferably lung carcinoma). Preferred is the detection of a lung cancer selected from the group consisting of lung adenocarcinoma; large cell lung cancer; squamous cell lung carcinoma and small cell lung carcinoma.

The method of the invention may be enabled by means of any analysis of the expression of an RNA transcribed therefrom or polypeptide or protein translated from said RNA, preferably by means of mRNA expression analysis or polypeptide expression analysis. However, in the most preferred embodiment of the invention the detection of cell proliferative disorders, preferably those according to Table 2 (most preferably lung carcinoma), is enabled by means of analysis of the methylation status or methylation level of at least one gene or genomic sequence selected from the group consisting of FOXL-2; ONECUT1; TFAP2E (including promoter or regulatory elements thereof) and EN2-2, EN2-3, SHOX2-2 and BARHL2.

Accordingly the present invention also provides diagnostic assays and methods, both quantitative and qualitative for detecting the expression of at least one gene or genomic sequence selected from the group consisting of ONECUT1; FOXL-2 and TFAP2E in a subject and determining therefrom upon the presence or absence of a subject's risk or increased risk to suffer from a cell proliferative disorders, or to detect a cell proliferative disorder preferably those according to Table 2 (most preferably lung carcinoma) in said subject. Particularly preferred is that the cell proliferative disorder is lung cancer and particularly preferred that it is selected from the group consisting of lung adenocarcinoma; large cell lung cancer; squamous cell lung carcinoma and small cell lung carcinoma.

Aberrant expression of mRNA transcribed from at least one gene or genomic sequence selected from the group consisting of ONECUT1; FOXL-2 and TFAP2E is associated with the presence of cell proliferative disorders, preferably those according to Table 2 (most preferably lung carcinoma) in a subject. Particularly preferred is that the cell proliferative disorder is a lung cancer, preferably a lung cancer selected from the group consisting of lung adenocarcinoma, large cell lung cancer, squamous cell lung carcinoma and small cell lung carcinoma.

According to the present invention, hyper-methylation and /or under-expression is associated with the presence of cell proliferative disorders, in particular those according to Table 2 (most preferably lung carcinoma).

To detect the presence of mRNA encoding a gene or genomic sequence, a sample is obtained from a patient. The sample may be any suitable sample comprising cellular matter of the tumor. Suitable sample types include cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion, and all possible combinations thereof. More preferably the sample type is selected form the group consisting of blood plasma, sputum and biological matter derived from bronchoscopy (including but not limited to bronchial lavage, bronchial alveolar lavage, bronchial brushing, and bronchial abrasion), and all possible combinations thereof.

The sample may be treated to extract the RNA contained therein. The resulting nucleic acid from the sample is then analysed. Many techniques are known in the state of the art for determining absolute and relative levels of gene expression, commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotide dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987 which are incorporated by reference). Further preferred is the "Real-time" variant of RT- PCR, wherein the PCR product is detected by means of hybridisation probes (e.g. TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridisation to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, mass labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used.

In an RNase protection assay, the RNA target and an RNA probe of a defined length are hybridised in solution. Following hybridisation, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (*Ausubel et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridisation of the fluorescently labelled cDNA (comprising the sequences to be interrogated) to the known genes immobilized on the glass slide (or other solid phase). After hybridisation, arrays are scanned using a fluorescent microarray scanner. Analysing the relative fluorescent intensity of different genes provides a measure of the differences in gene expression.

DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of at least one gene or genomic sequence selected from the group consisting of ONECUT1; FOXL-2 and TFAP2E and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (e.g., Cy^{®}3- or Cy^{®}5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy^{®}3. The resulting labelled cDNA is purified to remove unincorporated nucleotides, free dye and residual RNA. Following purification, the labelled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridisation using a fluorescent microarray scanner. The fluorescent intensity of each spot indicates the level of expression of the analysed gene; bright spots correspond to strongly expressed genes, while dim spots indicate weak expression.

Once the images are obtained, the raw data must be analysed. First, the background fluorescence must be subtracted from the fluorescence of each spot. The data is then normalized to a control sequence, such as exogenously added nucleic acids (preferably RNA or DNA), or a housekeeping gene panel to account for any non-specific hybridisation, array imperfections or variability in the array set-up, cDNA labelling, hybridisation or washing. Data normalization allows the results of multiple arrays to be compared.

Another aspect of the invention relates to a kit for use in diagnosis of cell proliferative disorders, preferably those according to Table 2 (most preferably lung carcinoma and further preferred is a lung cancer selected from the group consisting of lung adenocarcinoma; large cell lung cancer; squamous cell lung carcinoma; small cell lung carcinoma.) in a subject according to the methods of the present invention, said kit comprising: a means for measuring the level of transcription of at least one gene or genomic sequence selected from the group consisting of ONECUT1; FOXL-2 and TFAP2E . In a preferred embodiment the means for measuring the level of transcription comprise oligonucleotides or polynucleotides able to hybridise under stringent or moderately stringent conditions to the transcription products of at least one gene or genomic sequence selected from the group consisting of FOXL-2; ONECUT1; TFAP2E (including promoter or regulatory elements thereof) and EN2-2, EN2-3, SHOX2-2 and BARHL2. In a most preferred embodiment the level of transcription is determined by techniques selected from the group of Northern Blot analysis, reverse transcriptase PCR, real-time PCR, RNAse protection, and microarray. In another embodiment of the invention the kit further comprises means for obtaining a biological sample of the patient. Preferred is a kit, which further comprises a container which is most preferably suitable for containing the means for measuring the level of transcription and the biological sample of the patient, and most preferably further comprises instructions for use and interpretation of the kit results.

In a preferred embodiment the kit comprises (a) a plurality of oligonucleotides or polynucleotides able to hybridise under stringent or moderately stringent conditions to the transcription products of at least one gene or genomic sequence selected from the group consisting of FOXL-2; ONECUT1; TFAP2E (including promoter or regulatory elements thereof) and EN2-2, EN2-3, SHOX2-2 and BARHL2; (b) a container , preferably suitable for containing the oligonucleotides or polynucleotides and a biological sample of the patient comprising the transcription products wherein the oligonucleotides or polynucleotides can hybridise under stringent or moderately stringent conditions to the transcription products, (c) means to detect the hybridisation of (b); and optionally, (d) instructions for use and interpretation of the kit results.

The kit may also contain other components such as hybridisation buffer (where the oligonucleotides are to be used as a probe) packaged in a separate container. Alternatively, where the oligonucleotides are to be used to amplify a target region, the kit may contain, packaged in separate containers, a polymerase and a reaction buffer optimised for primer extension mediated by the polymerase, such as PCR. Preferably said polymerase is a reverse transcriptase. It is further preferred that said kit further contains an Rnase reagent.

The present invention further provides for methods for the detection of the presence of the polypeptide encoded by said gene sequences in a sample obtained from a patient.

Aberrant levels of polypeptide expression of the polypeptides encoded at least one gene or genomic sequence selected from the group consisting of ONECUT1; FOXL-2 and TFAP2E are associated with the presence of cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma.

According to the present invention under-expression of said polypeptides is associated with the presence of cell proliferative disorders, preferably those according to Table 2 (most preferably lung carcinoma). It is particularly preferred that the cell proliferative disorder is lung cancer and that it is selected from the group consisting of lung adenocarcinoma; large cell lung cancer; squamous cell lung carcinoma and small cell lung carcinoma.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled polypeptide or derivative thereof.

Certain embodiments of the present invention comprise the use of antibodies specific to the polypeptide(s) encoded by at least one gene or genomic sequence selected from the group consisting of ONECUT 1; FOXL-2 and TFAP2E.

Such antibodies are useful for cell proliferative disorders, preferably of those diseases according to Table 2, and most preferably in the diagnosis of lung carcinoma. Particularly preferred is a lung cancer selected from the group consisting of lung adenocarcinoma; large cell lung cancer; squamous cell lung carcinoma; small cell lung carcinoma. In certain embodiments production of monoclonal or polyclonal antibodies can be induced by the use of an epitope encoded by a polypeptide of at least one gene or genomic sequence selected from the group consisting of ONECUT1; FOXL-2 and TFAP2E as an antigene. Such antibodies may in turn be used to detect expressed polypeptides as markers for cell proliferative disorders, preferably those according to Table 2 and most preferably the diagnosis of lung carcinoma. Particularly preferred is a lung cancer selected from the group consisting of lung adenocarcinoma; large cell lung cancer; squamous cell lung carcinoma; small cell lung carcinoma. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or cofactors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a blocking agent to bind remaining sticky places on the membrane, commonly used agents include generic protein (e.g. milk protein). An antibody specific to the protein of interest is then added, said antibody being detectably labelled for example by dyes or enzymatic means (e.g. alkaline phosphatase or horseradish peroxidase). The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference in its entirety). Methods for preparation of the polypeptides or epitopes thereof include, but are not limited to chemical synthesis, recombinant DNA techniques or isolation from biological samples.

In the final step of the method, the diagnosis of the patient is determined, whereby under-expression (of mRNA or polypeptides) is indicative of the presence of cell proliferative disorders, preferably those according to Table 2 (most preferably lung carcinoma). Particularly preferred it is a lung cancer, preferably selected from the group consisting of lung adenocarcinoma; large cell lung cancer; squamous cell lung carcinoma and small cell lung carcinoma. The term under-expression shall be taken to mean expression at a detected level less than a pre-determined cut off which may be selected from the group consisting of the mean, median or an optimised threshold value. The term over-expression shall be taken to mean expression at a detected level greater than a pre-determined cut off which may be selected from the group consisting of the mean, median or an optimised threshold value. Another aspect of the invention provides a kit for use in diagnosis of cell proliferative disorders, preferably those according to Table 2 (most preferably lung carcinoma) in a subject according to the methods of the present invention, comprising: a means for detecting polypeptides of at least one gene or genomic sequence selected from the group consisting of ONECUT1; FOXL-2 and TFAP2E. The means for detecting the polypeptides comprise preferably antibodies, antibody derivatives, or antibody fragments. The polypeptides are most preferably detected by means of Western Blotting utilizing a labelled antibody. In another embodiment of the invention the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit comprises: (a) a means for detecting polypeptides of at least one gene or genomic sequence selected from the group consisting of ONECUT1; FOXL-2 and TFAP2E; (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.

The kit may also contain other components such as buffers or solutions suitable for blocking, washing or coating , packaged in a separate container.

Particular embodiments of the present invention provide a novel application of the analysis of methylation status, methylation levels and/or patterns within at least one gene or genomic sequence selected from the group consisting of FOXL-2; ONECUT1; TFAP2E (including promoter or regulatory elements thereof) and EN2-2, EN2-3, SHOX2-2 and BARHL2. that enables a precise detection, characterisation, assessment of risk to suffer from cell proliferative disorders, preferably those according to Table 2 (most preferably lung carcinoma). It is particularly preferred that this lung cancer is selected from the group consisting of lung adenocarcinoma; large cell lung cancer; squamous cell lung carcinoma and small cell lung carcinoma. Early detection of cell proliferative disorders, in particular lung carcinoma, is directly linked with disease prognosis, and the disclosed method thereby enables the physician and patient to make better and more informed treatment decisions. Therefore it is preferred that the method of the invention which allows detection of disease in an early stage is performed as a screening tool, or as an additional diagnostic test, whenever a first diagnosis is unclear.

The preferred sample type used within the method of the invention is sputum or biological samples derived from the lung, preferably, bronchial fluid, bronchial lavage and bronchoalveolar lavage. This sample type has the advantage that it is a sample which is currently used in common practice and obtainable by established and routine diagnostic procedures of lung disease as part of the standard care (e.g. histology procedures and/or cytology procedures). The advantage of using available samples is that additional information from the same sample can be achieved. The second advantage is, that these samples can be obtained non-invasively (for example sputum) or with low risk to the subject or patient.

Another important advantage of using samples which are collected from the bronchial system is, that the marker that can be used for a specific diagnosis of lung cancer or risk assessment of lung cancer may be less specific in terms cancer type. It would not harm, if the same marker is also detecting other cancer types (if tested on other sample types, for example blood).

In the most preferred embodiment of the method, the presence or absence of risk or increased risk of a subject to suffer from a cell proliferative disorder, or detecting of a cell proliferative disorder, preferably those according to Table 2 (most preferably lung carcinoma, in particular a lung cancer selected from the group consisting of lung adenocarcinoma; large cell lung cancer; squamous cell lung carcinoma and small cell lung carcinoma.) is determined by analysis of the methylation status or level of one or more CpG dinucleotides of at least one gene or genomic sequence selected from the group consisting of FOXL-2; ONECUT1; TFAP2E (including promoter or regulatory elements thereof) and EN2-2, EN2-3, SHOX2-2 and BARHL2.

In one embodiment the invention of said method comprises the following steps: i) contacting genomic DNA (preferably isolated from body fluids) obtained from the subject with at least one reagent, or series of reagents that distinguishes between methylated and non-methylated CpG dinucleotides within at least one gene or genomic sequence selected from the group consisting of FOXL-2; ONECUT1; TFAP2E (including promoter or regulatory elements thereof) and EN2-2, EN2-3, SHOX2-2 and BARHL2and ii) detecting cell proliferative disorders, preferably those according to Table 2 (most preferably lung carcinoma). Particularly preferred is a lung cancer selected from the group consisting of lung adenocarcinoma; large cell lung cancer; squamous cell lung carcinoma and small cell lung carcinoma.

It is preferred that said one or more CpG dinucleotides of at least one gene or genomic sequence selected from the group consisting of FOXL-2; ONECUT1; TFAP2E (including promoter or regulatory elements thereof) and EN2-2, EN2-3, SHOX2-2 and BARHL2are comprised within a respective genomic target sequence thereof as provided in SEQ ID NO: 1 to SEQ ID NO: 7 and complements thereof. The present invention further provides a method for ascertaining genetic and/or epigenetic parameters of at least one gene or genomic sequence selected from the group consisting of FOXL-2; ONECUT1; TFAP2E (including promoter or regulatory elements thereof) and EN2-2, EN2-3, SHOX2-2 and BARHL2and/or the genomic sequence according to SEQ ID NO: 1 to SEQ ID NO: 7 within a subject by analysing cytosine methylation. Said method comprising contacting a nucleic acid comprising SEQ ID NO: 1 to SEQ ID NO: 7 in a biological sample obtained from said subject with at least one reagent or a series of reagents, wherein said reagent or series of reagents, distinguishes between methylated and non-methylated CpG dinucleotides within the target nucleic acid.

In a preferred embodiment, said method comprises the following steps: In the *first step,* a sample of the tissue to be analysed is obtained. The source may be any suitable source, such as cells or cell lines, histological slides, biopsies, paraffin-embedded tissue, body fluids, ejaculate, urine, blood plasma, blood serum, whole blood, isolated blood cells, sputum, biological samples derived from the lung, preferablybiological matter derived from bronchoscopy including but not limited to bronchial lavage, bronchial alveolar lavage, bronchial brushing, bronchial abrasion, and all possible combinations thereof. More preferably the sample type is selected form the group consisting of blood plasma, sputum, biological samples derived from the lung, preferablybiological matter derived from bronchoscopy (including, but not limited to, bronchial lavage, bronchial alveolar lavage, bronchial brushing, and bronchial abrasion) and all possible combinations thereof. It is a preferred embodiment of the method of the invention that the sample type is selected from the group consisting of sputum and biological samples derived from the lung (as described earlier), most preferably this biological matter is derived from bronchoscopy (including but not limited to bronchial lavage, bronchial alveolar lavage, bronchial brushing, and bronchial abrasion).

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g. ultrafiltration, silica surfaces or membranes, magnetic particles, polystyrol particles, polystyrol surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pre-treatment' or 'treatment' herein.

This explicit order of steps is only one embodiment of the method of the invention, because it is also possible and sometimes advantageous to omit the DNA isolation step prior to the bisulfite treatment. In that case the bisulfite treatment (see in detail below) is performed before the DNA is isolated and/or purified, for example if the sample DNA is not enclosed in a membrane. Hence the bisulfite treatment may be performed on a crude sample, i.e. the biological material itself. In some cases, the presence of a surfactant, such as for example SDS, may be needed.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. PCT/EP2004/011715, which is incorporated by reference in its entirety). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2,5,7,8,-tetramethylchromane-2-carboxylic acid or trihydroxybenzoe acid and derivates thereof, e.g. Gallic acid (see: PCT/EP2004/011715 which is incorporated by reference in its entirety). The bisulfite conversion is preferably carried out at a reaction temperature between 30°C and 70°C, whereby the temperature is increased to over 85°C for short periods of times during the reaction (see: PCT/EP2004/011715 which is incorporated by reference in its entirety). The bisulfite treated DNA is preferably purified priori to the quantification. This may be conducted by any means known in the art, such as but not limited to ultrafiltration, preferably carried out by means of Microcon^(TM) columns (manufactured by Millipore^(TM)). The purification is carried out according to a modified manufacturer's protocol (see: PCT/EP2004/011715 which is incorporated by reference in its entirety).

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridise under stringent or highly stringent conditions to an at least 16-base-pair long segment of the base sequences of one of SEQ ID NO: 8 to SEQ ID NO: 35 and sequences complementary thereto.

In an alternate embodiment of the method, the methylation status or level of pre-selected CpG positions within at least one gene or genomic sequence selected from the group consisting of FOXL-2; ONECUT1; TFAP2E (including promoter or regulatory elements thereof) and EN2-2, EN2-3, SHOX2-2 and BARHL2 and preferably within the nucleic acid sequences according to SEQ ID NO: 1 to SEQ ID NO: 7 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primer pairs contain at least one primer which hybridises to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridises to a treated nucleic acid sequence according to one of SEQ ID NO: 8 to SEQ ID NO: 35 and sequences complementary thereto, wherein the base sequence of said oligomers comprises at least one CpG dinucleotide. A further preferred embodiment of the method comprises the use of *blocker* oligonucleotides (the HeavyMethyl™ assay). The use of such blocker oligonucleotides has been described by Yu et al., BioTechniques 23:714-720, 1997. Blocking probe oligonucleotides are hybridised to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-termini thereof that render the blocker molecule nuclease-resistant. Particular applications may not require such 5' modifications of the blocker. For example, if the blocker- and primer-binding sites overlap, thereby precluding binding of the primer (*e.g.,* with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridises to a treated nucleic acid sequence according to one of SEQ ID NO: 8 to SEQ ID NO: 35 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, *e.g*., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analysed by means of based-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG, TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence selected from the group consisting SEQ ID NO: 1 to SEQ ID NO: 7, and the equivalent positions within SEQ ID NO: 8 to SEQ ID NO: 35. Said oligonucleotides may also be present in the form of peptide nucleic acids. The non-hybridised amplificates are then removed. The hybridised amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res: 6:986-994, 1996; *also see* United States

Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridise to a CpG-rich sequence located between the forward and reverse amplification primers. The TaqMan™ probe further comprises a fluorescent "reporter moiety" and a "quencher moiety" covalently bound to linker moieties (*e.g*., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the *fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

In the most preferred embodiment of the method the genomic nucleic acids are isolated and treated according to the first three steps of the method outlined above, namely:
a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or methylation specific *blocking oligonucleotides,* and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridises to a treated nucleic acid sequence according to one of SEQ ID NO: 8 to SEQ ID NO: 35 and sequences complementary thereto, wherein the base sequence of said oligomers comprises at least one CpG dinucleotide, but preferably two or three.

Step e) of the method, namely the detection of the specific amplificates indicative of the methylation status of one or more CpG positions according to SEQ ID NO: 1 to SEQ ID NO: 7 is carried out by means of *real-time* detection methods as described above.

Additional embodiments of the invention provide a method for the analysis of the methylation status of the at least one gene or genomic sequence selected from the group consisting of FOXL-2; ONECUT1; TFAP2E (including promoter or regulatory elements thereof) and EN2-2, EN2-3, SHOX2-2 and BARHL2 (preferably SEQ ID NO: 1 to SEQ ID NO: 7 and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In the *first step* of such additional embodiments, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, *e.g*., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for use in the present method, preferred are cells or cell lines, histological slides, biopsies, paraffin-embedded tissue, body fluids, ejaculate, urine, blood plasma, blood serum, whole blood, isolated blood cells, and biological samples derived from the lung, such as sputum and biological matter derived from bronchoscopy (including but not limited to bronchial lavage, bronchial alveolar lavage, bronchial brushing, bronchial abrasion, and combinations thereof. More preferably the sample type is selected form the group consisting of blood plasma, sputum and biological matter derived from bronchoscopy (including but not limited to bronchial lavage, bronchial alveolar lavage, bronchial brushing, bronchial abrasion) and all possible combinations thereof.

Once the nucleic acids have been extracted, the genomic double-stranded DNA is used in the analysis.

In a preferred embodiment, the DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of MseI, BfaI, Csp6I, TrulI, TvulI, Tru9I, Tvu9I, MaeI and XspI. Particularly preferred is the use of two or three such enzymes. Particularly preferred is the use of a combination of MseI, BfaI and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the *third step,* the DNA (or fragments thereof) is then digested with one or more methylation sensitive restriction enzymes. The digestion is carried out such that hydrolysis of the DNA at the restriction site is informative of the methylation status of a specific CpG dinucleotide of at least one gene or genomic sequence selected from the group consisting of FOXL-2; ONECUT1; TFAP2E (including promoter or regulatory elements thereof) and EN2-2, EN2-3, SHOX2-2 and BARHL2.

Preferably, the methylation-specific restriction enzyme is selected from the group consisting *of Bsi El, Hga I HinPl, Hpy99I, Ava I, Bce AI, Bsa HI, BisI, BstUI, BihI236I, AccII, BstFNI, McrBC, GlaI, MvnI, HpaII (HapII), HhaI, AciI, SmaI, HinP1I, HpyCH4IV, EagI* and mixtures of two or more of the above enzymes. Preferred is a mixture containing the restriction enzymes BstUI, HpaII, HpyCH4IV and HinPII.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence selected from the group consisting SEQ ID NO: 1 to SEQ ID NO: 7, and complements thereof. Preferably said contiguous sequence is at least 16, 20 or 25 nucleotides in length. In an alternative embodiment said primers may be complementary to any adaptors linked to the fragments.

In the *fifth step* the amplificates are detected. The detection may be by any means standard in the art, for example, but not limited to, gel electrophoresis analysis, hybridisation analysis, incorporation of detectable tags within the PCR products, DNA array analysis, MALDI or ESI analysis. Preferably said detection is carried out by hybridisation to at least one nucleic acid or peptide nucleic acid comprising in each case a contiguous sequence at least 16 nucleotides in length that is complementary to, or hybridizes under moderately stringent or stringent conditions to a sequence selected from the group consisting of SEQ ID NO: 1 to SEQ ID NO: 7, and complements thereof. Preferably said contiguous sequence is at least 16, 20 or 25 nucleotides in length.

Subsequent to the determination of the methylation state or methylation level of the genomic nucleic acids obtained from a subject's sample, the risk or increased risk of a subject to suffer froma cell proliferative disorder, preferably those according to Table 2 (most preferably lung carcinoma), or the presence of such a cell proliferative disorder is deduced based upon the methylation state or level of at least one CpG dinucleotide sequence of SEQ ID NO: 1 to SEQ ID NO: 7, or an average, or a value reflecting an average methylation state of a plurality of CpG dinucleotide sequences of SEQ ID NO: 1 to SEQ ID NO: 7 wherein methylation is associated with the presence of cell proliferative disorders, preferably those according to Table 2 (most preferably lung carcinoma). Wherein said methylation is determined by quantitative means the cut-off point for determining said presence of methylation is preferably zero (i.e. wherein a sample displays any degree of methylation it is determined as having a methylated status at the analyzed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected form the group consisting of 0%-5%, 5%-10%, 10%-15%, 15%-20%, 20%-30% and 30%-50%. Particularly preferred are the cut-offs 10%, 15%, 25%, and 30%.

Upon determination of the methylation and/or expression of at least one gene or genomic sequence selected from the group consisting of FOXL-2; ONECUT1; TFAP2E (including promoter or regulatory elements thereof) and EN2-2, EN2-3, SHOX2-2 and BARHL2 the presence or absence of a cell proliferative disorder or an increased risk of a subject to suffer from a cell proliferative disorder, preferably those according to Table 2 (most preferably lung carcinoma) is determined, wherein hyper-methylation and /or under-expression indicates the presence of cell proliferative disorders and/or the presence of an increased risk of the subject to suffer from such a disorder, preferably those according to Table 2 (most preferably lung carcinoma) and hypo-methylation and /or over-expression indicates the absence of cell proliferative disorders within the subject, and/or the absence of an increased risk of the subject to suffer from such a disorder, preferably those according to Table 2 (most preferably lung carcinoma). It is particularly preferred that said proliferative disorder is a lung cancer selected from the group consisting of lung adenocarcinoma; large cell lung cancer; squamous cell lung carcinoma and small cell lung carcinoma.

An *increased risk* is to be understood as a risk that is at least two fold higher than the average risk of the population with the same gender in the same age group (wherein subjects belong to the same age group if they are not more than 5 years older or younger than the subject analysed.

### Further improvements

The disclosed invention provides treated nucleic acids, derived from genomic SEQ ID NO: 1 to SEQ ID NO: 7, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. Said treatment may however also comprise an appropriate enzymatic treatment (instead of the bisulfiite treatment), resulting in conversion of the unmethylated cytosines into base pairs with a different base pairing behavious. In a preferred embodiment of the invention, the invention provides a non-naturally occurring modified nucleic acid comprising a sequence of at least 16 contiguous nucleotide bases in length of a sequence selected from the group consisting of SEQ ID NO: 8 TO SEQ ID NO: 35. In further preferred embodiments of the invention said nucleic acid is at least 50, 100, 150, 200, 250 or 500 base pairs in length of a segment of the nucleic acid sequence disclosed in SEQ ID NO: 8 to SEQ ID NO: 35. Particularly preferred is a nucleic acid molecule that is identical or complementary to all or a portion of the sequences SEQ ID NO: 8 to SEQ ID NO: 35 but not to SEQ ID NO: 1 to SEQ ID NO: 7 or other naturally occurring DNA.

It is preferred that said sequence comprises at least one CpG, TpA or CpA dinucleotide and sequences complementary thereto. The sequences of SEQ ID NO: 8 TO SEQ ID NO: 35 provide non-naturally occurring modified versions of the nucleic acid according to SEQ ID NO: 1 TO SEQ ID NO: 7, wherein the modification of each genomic sequence results in the synthesis of a nucleic acid having a sequence that is unique and distinct from said genomic sequence as follows. For each sense strand genomic DNA, *e.g*., SEQ ID NO: 1 to SEQ ID NO: 7, four converted versions are disclosed. A first version wherein "C" is converted to "T," but "CpG" remains "CpG" (*i.e.,* corresponds to case where, for the genomic sequence, all "C" residues of CpG dinucleotide sequences are methylated and are thus not converted); a second version discloses the complement of the disclosed genomic DNA sequence (i.e. *antisense* strand), wherein "C" is converted to "T," but "CpG" remains "CpG" (*i.e*., corresponds to case where, for all "C" residues of CpG dinucleotide sequences are methylated and are thus not converted). The 'upmethylated' converted sequences of SEQ ID NO: 1 to SEQ ID NO: 7 correspond to SEQ ID NO: 8 to SEQ ID NO: 21. A third chemically converted version of each genomic sequences is provided, wherein "C" is converted to "T" for all "C" residues, including those of "CpG" dinucleotide sequences (*i.e*., corresponds to case where, for the genomic sequences, all "C" residues of CpG dinucleotide sequences are unmethylated); a final chemically converted version of each sequence, discloses the complement of the disclosed genomic DNA sequence (i.e. antisense strand), wherein "C" is converted to "T" for all "C" residues, including those of "CpG" dinucleotide sequences (*i.e*., corresponds to case where, for the complement (antisense strand) of each genomic sequence, all "C" residues of CpG dinucleotide sequences are unmethylated). The 'downmethylated' converted sequences of SEQ ID NO: 1 to SEQ ID NO: 7 correspond to SEQ ID NO: 19 to SEQ ID NO: 30.

Significantly, heretofore, the nucleic acid sequences and molecules according to SEQ ID NO: 8 to SEQ ID NO: 35were not implicated in or connected with the detection or diagnosis of cell proliferative disorders, preferably those according to Table 2 (most preferably lung carcinoma). It is particularly preferred that the cell proliferative disorder is a lung cancer selected from the group consisting of lung adenocarcinoma; large cell lung cancer; squamous cell lung carcinoma and small cell lung carcinoma.

In an alternative preferred embodiment, the invention further provides oligonucleotides or oligomers suitable for use in the methods of the invention for detecting the cytosine methylation state within genomic or treated (chemically modified) DNA, according to SEQ ID NO: 1 to SEQ ID NO: 35 Said oligonucleotide or oligomer nucleic acids provide novel diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NO: 8 to SEQ ID NO: 35 and/or sequences complementary thereto, or to a genomic sequence according to SEQ ID NO: 1 to SEQ ID NO: 7; and/or sequences complementary thereto.

Thus, the present invention includes nucleic acid molecules (*e.g*., oligonucleotides and peptide nucleic acid (PNA) molecules (PNA-oligomers)) that hybridize under moderately stringent and/or stringent hybridization conditions to all or a portion of the sequences SEQ ID NO: 1 to SEQ ID NO: 35 or to the complements thereof. Particularly preferred is a nucleic acid molecule that hybridizes under moderately stringent and/or stringent hybridization conditions to all or a portion of the sequences SEQ ID NO: 8 to SEQ ID NO: 35 but not to SEQ ID NO: 1 to SEQ ID NO: 7 or other human genomic DNA.

The identical or hybridizing portion of the hybridizing nucleic acids is typically at least 9, 16, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO: 8 to SEQ ID NO: 35 , or to the complements thereof.

Hybridizing nucleic acids of the type described herein can be used, for example, as a primer (*e.g*., a PCR primer), or a diagnostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 1 to SEQ ID NO: 7 (such as allelic variants and SNPs), rather than identical, it is useful to first establish the lowest temperature at which only homologous hybridization occurs with a particular concentration of salt (*e.g*., SSC or SSPE). Then, assuming that 1% mismatching results in a 1 °C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of inventive oligonucleotides of length X (in nucleotides), as indicated by polynucleotide positions with reference to, *e.g*., SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
n to (n + (X-1));
where n=1, 2, 3,...(Y-(X-1));
where Y equals the length (nucleotides or base pairs) of SEQ ID NO: 1 (3905);
where X equals the common length (in nucleotides) of each oligonucleotide in the set (*e.g*., X=20 for a set of consecutively overlapping 20-mers); and
where the number (Z) of consecutively overlapping oligomers of length X for a given SEQ ID NO 1 of length Y is equal to Y- (X-1). For example Z= 3905-19= 3886 for either sense or antisense sets of SEQ ID NO: 1, where X=20.

Preferably, the set is limited to those oligomers that comprise at least one CpG, TpG or CpA dinucleotide, and thus hybridise in any case to a region of the converted target DNA, that comprises at least one (methylated or unmethylated) CpG in its unconverted version.

Examples of inventive 20-mer oligonucleotides include the following set of 3905 oligomers (and the antisense set complementary thereto), indicated by polynucleotide positions with reference to SEQ ID NO: 1:
1-20, 2-21, 3-22, 4-23, 5-24, .............. and 3886- 3905

Preferably, the set is limited to those oligomers that comprise at least one CpG, TpG or CpA dinucleotide and thus hybridise in any case to a region of the converted target DNA, that comprises at least one (methylated or unmethylated) CpG in its unconverted version.

Likewise, examples of inventive 25-mer oligonucleotides include the following set of 3881 oligomers (and the antisense set complementary thereto), indicated by polynucleotide positions with reference to SEQ ID NO: 1:
1-25, 2-26, 3-27, 4-28, 5-29, ...... and 3881- 3905.

Preferably, the set is limited to those oligomers that comprise at least one CpG, TpG or CpA dinucleotide and thus hybridise in any case to a region of the converted target DNA, that comprises at least one (methylated or unmethylated) CpG in its unconverted version.

The present invention encompasses, for *each* of SEQ ID NO: 1 to SEQ ID NO: 35 (sense and antisense), multiple consecutively overlapping sets of oligonucleotides or modified oligonucleotides of length X, where, *e.g.,* X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO: 1 to SEQ ID NO: 7. Preferred sets of such oligonucleotides or modified oligonucleotides of length X are those consecutively overlapping sets of oligomers corresponding to SEQ ID NO: 1 to SEQ ID NO: 35 (and to the complements thereof). Preferably, said oligomers comprise at least one CpG, TpG or CpA dinucleotide and thus hybridise in any case to a region of the converted target DNA, that comprises at least one (methylated or unmethylated) CpG in its unconverted version.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, *e.g*., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of each of the CpG dinucleotides of a genomic sequence or parts thereof selected from the group consisting of SEQ ID NO: 1 to SEQ ID NO: 7 and sequences complementary thereto, or to the corresponding CpG, TpG or CpA dinucleotide within a sequence of the treated nucleic acids according to SEQ ID NO: 8 to SEQ ID NO: 35 and sequences complementary thereto. However, it is anticipated that for economic or other factors it may be preferable to analyse a limited selection of the CpG dinucleotides within said sequences, and the content of the set of oligonucleotides is altered accordingly.

Therefore, in particular embodiments, the present invention provides a set of at least two (2) (oligonucleotides and/or PNA-oligomers) useful for detecting the cytosine methylation state in treated genomic DNA (SEQ ID NO: 8 to SEQ ID NO: 35), or in genomic DNA (SEQ ID NO: 1 to SEQ ID NO: 7 and sequences complementary thereto). These probes enable diagnosis and detection of cell proliferative disorders, preferably those according to Table 2 (most preferably lung carcinoma). It is particularly preferred that it is a lung cancer selected from the group consisting of lung adenocarcinoma; large cell lung cancer; squamous cell lung carcinoma and small cell lung carcinoma. The set of oligomers may also be used for detecting single nucleotide polymorphisms (SNPs) in treated genomic DNA (SEQ ID NO: 8 to SEQ ID NO: 35), or in genomic DNA (SEQ ID NO: 1 to SEQ ID NO: 7 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NO: 1 to SEQ ID NO: 35 and sequences complementary thereto, or segments thereof.

It is anticipated that the oligonucleotides may constitute all or part of an "array" or "DNA chip" (*i.e.,* an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003), which is hereby incorporated by reference. In such methods, enough labels are generated so that each nucleic acid in the complex mixture (*i.e*., each analyte) can be uniquely bound by a unique label and thus detected (each label is directly counted, resulting in a digital read-out of each molecular species in the mixture).

It is particularly preferred that the oligomers according to the invention are utilised for detecting, or for diagnosing cell proliferative disorders, preferably those according to Table 2 (most preferably lung carcinoma) or for detecting the presence or absence of an increased risk of a subject to suffer from a cell proliferative disorder, preferably those according to Table 2 (most preferably lung carcinoma). It is particularly preferred that the disorder is a lung cancer and that it is selected from the group consisting of lung adenocarcinoma; large cell lung cancer; squamous cell lung carcinoma and small cell lung carcinoma.

### Kits

Moreover, an additional aspect of the present invention is a kit comprising: a means for determining the expression or methylation status or levels of at least one gene or genomic sequence selected from the group consisting of FOXL-2; ONECUT1; TFAP2E (including promoter or regulatory elements thereof) and EN2-2, EN2-3, SHOX2-2 and BARHL2. The means for determining the expression or methylation status or levels of at least one gene or genomic sequence selected from the group consisting of FOXL-2; ONECUT1; TFAP2E (including promoter or regulatory elements thereof) and EN2-2, EN2-3, SHOX2-2 and BARHL2preferably comprise a bisulfite-containing reagent; one or a plurality of oligonucleotides wherein the sequences thereof are identical, are complementary, or hybridise under stringent or highly stringent conditions to a 9 or more preferably 18 base long segment of a sequence selected from SEQ ID NO: 8 to SEQ ID NO: 35; and optionally instructions for carrying out and evaluating the described method of methylation analysis. In one embodiment the base sequence of said oligonucleotides comprises at least one CpG, CpA or TpG dinucleotide.

In a further embodiment, said kit may further comprise standard reagents for performing a CpG position-specific methylation analysis, wherein said analysis comprises one or more of the following techniques: MS-SNuPE, MSP, MethyLight^{™}, HeavyMethyl, COBRA, and nucleic acid sequencing. However, a kit along the lines of the present invention can also contain only part of the aforementioned components.

In a preferred embodiment the kit may comprise additional bisulfite conversion reagents selected from the group consisting: DNA denaturation buffer; sulfonation buffer; DNA recovery reagents or kits (*e.g*., precipitation, ultrafiltration, affinity column); desulfonation buffer; and DNA recovery components.

In a further alternative embodiment, the kit may contain, packaged in separate containers, a polymerase and a reaction buffer optimised for primer extension mediated by the polymerase, such as PCR. In another embodiment of the invention the kit further comprising means for obtaining a biological sample of the patient. Preferred is a kit, which further comprises a container suitable for containing the means for determining methylation of at least one gene or genomic sequence selected from the group consisting of FOXL-2; ONECUT1; TFAP2E (including promoter or regulatory elements thereof) and EN2-2, EN2-3, SHOX2-2 and BARHL2in the biological sample of the patient, and most preferably further comprises instructions for use and interpretation of the kit results. In a preferred embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 8 to SEQ ID NO: 35; and optionally (d) instructions for use and interpretation of the kit results. In an alternative preferred embodiment the kit comprises: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridises to a pre-treated nucleic acid sequence according to one of SEQ ID NO: 8 to SEQ ID NO: 35 and sequences complementary thereto; and optionally (d) instructions for use and interpretation of the kit results.

In an alternative embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 8 to SEQ ID NO: 35; (d) at least one oligonucleotides and/or PNA-oligomer having a length of at least 9 or 16 nucleotides which is identical to or hybridises to a pre-treated nucleic acid sequence according to one of SEQ ID NO: 8 to SEQ ID NO: 35 and sequences complementary thereto; and optionally (e) instructions for use and interpretation of the kit results.

The kit may also contain other components such as buffers or solutions suitable for blocking, washing or coating, packaged in a separate container.

Another aspect of the invention relates to a kit for use in determining the presence of and/or diagnosing cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma.

Said kit prefereably comprises: a means for measuring the level of transcription of at least one gene or genomic sequence selected from the group consisting of ONECUT1; FOXL-2 and TFAP2E and a means for determining methylation status or level of at least one gene or genomic sequence selected from the group consisting of FOXL-2; ONECUT1; TFAP2E (including promoter or regulatory elements thereof) and EN2-2, EN2-3, SHOX2-2 and BARHL2.

Typical reagents (*e.g*., as might be found in a typical COBRA^{™}-based kit) for COBRA^{™} analysis may include, but are not limited to: PCR primers for at least one gene or genomic sequence selected from the group consisting of FOXL-2; ONECUT1; TFAP2E (including promoter or regulatory elements thereof) and EN2-2, EN2-3, SHOX2-2 and BARHL2and/or their bisulfite converted sequences; restriction enzyme and appropriate buffer; gene-hybridization oligo; control hybridization oligo; kinase labeling kit for oligo probe; and labeled nucleotides. Typical reagents (*e.g*., as might be found in a typical MethyLight ^{™} - based kit) for MethyLight^{™} analysis may include, but are not limited to: PCR primers for the bisulfite converted sequence of at least one gene or genomic sequence selected from the group consisting of ONECUT1; FOXL-2 and TFAP2E (including promoter or regulatory elements thereof) and EN2-2, EN2-3, SHOX2-2 and BARHL2; bisulfite specific probes (*e.g*. TaqMan ^{™} or Lightcycler ^{™}); optimized PCR buffers and deoxynucleotides; and Taq polymerase.

Typical reagents (*e.g*., as might be found in a typical Ms-SNuPE^{™}-based kit) for Ms-SNuPE^{™} analysis may include, but are not limited to: PCR primers for specific gene (or bisulfite treated DNA sequence or CpG island); optimized PCR buffers and deoxynucleotides; gel extraction kit; positive control primers; Ms-SNuPET^{™} primers for the bisulfite converted sequence of at least one gene or genomic sequence selected from the group consisting of ONECUT1; FOXL-2 and TFAP2E (including promoter or regulatory elements thereof) and EN2-2, EN2-3, SHOX2-2 and BARHL2; reaction buffer (for the Ms-SNuPE reaction); and labelled nucleotides.

Typical reagents (*e.g*., as might be found in a typical MSP-based kit) for MSP analysis may include, but are not limited to: methylation-specific and unmethylation-specific PCR primers for the bisulfite converted sequence of at least one gene or genomic sequence selected from the group consisting of ONECUT1; FOXL-2 and TFAP2E (including promoter or regulatory elements thereof) and EN2-2, EN2-3, SHOX2-2 and BARHL2, optimized PCR buffers and deoxynucleotides, and specific probes.

Moreover, an additional aspect of the present invention is an alternative kit comprising a means for determining methylation (status or level) of at least one gene or genomic sequence selected from the group consisting of ONECUT1; FOXL-2 and TFAP2E (including promoter or regulatory elements thereof) and EN2-2, EN2-3, SHOX2-2 and BARHL2, wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence selected from SEQ ID NO: 1 to SEQ ID NO: 7 and optionally instructions for carrying out and evaluating the described method of methylation analysis. In one embodiment the base sequence of said oligonucleotides are identical, are complementary, or hybridise under stringent or highly stringent conditions to an at least 18 base long segment of a sequence selected from SEQ ID NO: 1 to SEQ ID NO: 7.

In a further embodiment said kit may comprise one or a plurality of oligonucleotide probes for the analysis of the digest fragments, preferably said oligonucleotides are identical, are complementary, or hybridise under stringent or highly stringent conditions to an at least 16 base long segment of a sequence selected from SEQ ID NO: 1 to SEQ ID NO: 7.

In a preferred embodiment the kit may comprise additional reagents selected from the group consisting: buffer (e.g. restriction enzyme, PCR, storage or washing buffers); DNA recovery reagents or kits (*e.g*., precipitation, ultrafiltration, affinity column) and DNA recovery components.

In a further alternative embodiment, the kit may contain, packaged in separate containers, a polymerase and a reaction buffer optimised for primer extension mediated by the polymerase, such as PCR. In another embodiment of the invention the kit further comprising means for obtaining a biological sample of the patient. In a preferred embodiment the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridise under stringent or highly stringent conditions to an at least 9 base long segment of a sequence selected from SEQ ID NO: 1 to SEQ ID NO: 7 and optionally (d) instructions for use and interpretation of the kit results.

In an alternative preferred embodiment the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 7 and optionally (d) instructions for use and interpretation of the kit results.

In an alternative embodiment the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 7 (d) at least one set of oligonucleotides one or a plurality of nucleic acids or peptide nucleic acids which are identical , are complementary, or hybridise under stringent or highly stringent conditions to an at least 9 base long segment of a sequence selected from SEQ ID NO: 1 to SEQ ID NO: 7 and optionally (e) instructions for use and interpretation of the kit results.

The kit may also contain other components such as buffers or solutions suitable for blocking, washing or coating, packaged in a separate container.

The invention further relates to a kit for use in providing a diagnosis of the presence or absence of cell proliferative disorders, preferably those according to Table 2 (most preferably lung carcinoma), in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for at least one gene or genomic sequence selected from the group consisting of ONECUT1; FOXL-2 and TFAP2E (including promoter or regulatory elements thereof) and EN2-2, EN2-3, SHOX2-2 and BARHL2and comprises a sequence of at least 15 base pairs in length but no more than 200 bp of a sequence according to one of SEQ ID NO: 1 to SEQ ID NO: 7. Preferably said sequence is at least 15 base pairs in length but no more than 80 bp of a sequence according to one of SEQ ID NO: 1 to SEQ ID NO: 7. It is further preferred that said sequence is at least 20 base pairs in length but no more than 30 bp of a sequence according to one of SEQ ID NO: 1 to SEQ ID NO: 7.

Said test kit preferably further comprises a restriction enzyme component comprising one or a plurality of methylation-sensitive restriction enzymes.

In a further embodiment said test kit is further characterized in that it comprises at least one methylation-specific restriction enzyme, and wherein the oligonucleotides comprise a restriction site of said at least one methylation specific restriction enzymes.

The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

The described invention further provides a composition of matter useful for detecting, or for diagnosing cell proliferative disorders, preferably those according to Table 2 (most preferably lung carcinoma) . Particularly preferred is a lung cancer selected from the group consisting of lung adenocarcinoma; large cell lung cancer; squamous cell lung carcinoma; small cell lung carcinoma.

Said composition preferably comprises at least one nucleic acid 18 base pairs in length of a segment of the nucleic acid sequence disclosed in SEQ ID NO: 8 to SEQ ID NO: 35, and one or more substances taken from the group comprising :
1-5 mM Magnesium Chloride, 100-500 µM dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 8 to SEQ ID NO: 35 and sequences complementary thereto. It is preferred that said composition of matter comprises a buffer solution appropriate for the stabilization of said nucleic acid in an aqueous solution and enabling polymerase based reactions within said solution.. Suitable buffers are known in the art and commercially available.

In further preferred embodiments of the invention said at least one nucleic acid is at least 50, 100, 150, 200, 250 or 500 base pairs in length of a segment of the nucleic acid sequence disclosed in SEQ ID NO: 8 to SEQ ID NO: 35.

**Table 1**

| **Gene** | **Genomic SEQ ID NO:** | **Pretreated methylated sequence (sense) SEQ ID NO:** | **Pretreated methylated strand (antisense) SEQ ID NO:** | **Pretreated unmethylated sequence (sense) SEQ ID NO:** | **Pretreated unmethylated sequence (antisense) SEQ ID NO:** |
|---|---|---|---|---|---|
| **SHOX2-2** | 1 | 8 | 9 | 22 | 23 |
| **EN2-2** | | | | | |
| Second CpG island associated with Homeobox protein engrailed-2 (Hu-En-2); EN2; HME2 | 2 | 10 | 11 | 24 | 25 |
| **EN2-3** | | | | | |
| Third CpG island associated with Homeobox protein engrailed-2 (Hu-En-2); EN2; HME2 | 3 | 12 | 13 | 26 | 27 |
| **ONECUT1** | 4 | 14 | 15 | 28 | 29 |
| **FOXL2** | 5 | 16 | 17 | 30 | 31 |
| **TFAP2E** | 6 | 18 | 19 | 32 | 33 |
| **BARHL2** | 7 | 20 | 21 | 34 | 35 |

**Table 2**

| **Gene** | **Preferred disorder** |
|---|---|
| SHOX2-2 | Cancer, preferably lung |
| EN2-2 | Cancer, preferably lung |
| EN2-3 | Cancer, preferably lung |
| ONECUT 1 | Cancer, preferably lung |
| FOXL-2 | Cancer, preferably lung |
| TFAP2E | Cancer, preferably lung |
| BARHL2 | Cancer, preferably lung |

**Table 3A MSP Assays**

| **Gene/ Genomic region** | **MSP-Amplicon/ SEQ ID NO:** | **Forward Primer** / **SEQ ID NO:** | **Reverse Primer** / **SEQ ID NO:** | **Probe/ SEQ ID NO:** |
|---|---|---|---|---|
| ONECUT1 | gttttgaaatttattagaat aacgacgttttaaaaata aaggcgtagtaagtattt tttttttcgttgtcgcgggt tgaattacggacgttcg cgggtcgtttagtttcga cggttcgtagggggcg cgcgtcgtagtcgtagt atagttcggttatttttag aaag/36 | gttttgaaatttattagaata acgacgtt /41 | ctttctaaaaataacc gaactatactacgac /42 | tacggacgttcgcgggtcgtt /43 |
| TFAP2E | tttagaagcggttttcgta tcgttgcggtgggcgttt tcgggtttcgatttcgtta gcgtcgcggggtag aggtatttggagttcgta gggtttagatttgggttg gaaaagtttcgttgattgt aggtaagcgttcgg /37 | tttagaagcggttttcgtat c /52 | ccgaacgcttaccta caatc /53 | ttgcggtgggcgttttcgggtt /54 |

**Table 3B HeavyMethyl Assays**

| **Gene/ Genom ic region** | **HM-Amplicon/ SEQ ID NO:** | **Forward Primer/ SEQ ID NO:** | **Reverse Primer/ SEQ ID NO:** | **Forward Blocker** / **SEQ ID NO:** | **Reverse Blocker** / **SEQ ID NO:** | **Probe/ SEQ ID NO:** |
|---|---|---|---|---|---|---|
| FOXL-2 | ccaagacctgggcttgcagc gccgccaacaggcccggggaca cgaggcgctccaggccggggtct tcccg gctgctggcc cctctcgctccccacccgctgg cggcgcctcggtcgcccgcaatt gacccaacccgcttcctgcgtttgccc ctcaggtt tcc /39 | ccaaaaccta aacttacaac /44 | gagaggggtt agtagt /45 | tacaacacca ccaacaaacc caaaaacaca a /46 | ttgggaagat tttggtttgg agt /47 | ccgccgaaa acacgaaac ggcgggag aggggttagt agt /48; cccgggaag attttggtttg gagcccgg gccaaaacc taa acttacaac /49; ctccaaacc aaaatcttcc c /50; ccgaaaaca c gaaacgctc /51 |
| TFAP2 E | aaacccaaacctaaattaaa aaaacttcgc taactacaaa caaacgtccgaaaaaacgacca | aaacccaaacct aaattaaa /55 | ggaagtgtgt ggtaaag /56 | gtaaagtgtt ggggttttgt ttggttgttt /57 | / | aaaaacttcg ctaactacaa acaaac |
| | aacgaaaccccgacgct ttaccacaca cttcc /40 | | | | | /58 |

**Table 3C TSP assays**

| **Gene/Genomi c region** | **TSP PCR Amplicon** | **Primer 1/ SEQ ID NO:** | **Primer 2/ SEQ ID NO:** | **Probe/ SEQ ID NO:** |
|---|---|---|---|---|
| BARHL2 | attgtttgttagtttttaagttaat cgtagtaataatcgttggatta ttttaaatgtggttaaaatcga cgttggataaaattaatttgtta tatgt /38 | gttttgaaatttattagaa taacgacgtt /59 | acatataacaaatatatt tt atccaac /60 | ttggattattttaaat gtggttaaaa/61 |

**Table 4**

| **Gene/Marker** | **AUC** | **Sensitivity** | **Specificity** |
|---|---|---|---|
| **FOXL2** | 0.911 | 0.575 | 0.957 |
| **BARHL2** | 0.766 | 0.400 | 0.957 |

### EXAMPLES

The following analysis was performed to examine the methylation status of FOXL2 and BARHL2 gene markers. DNA was first extracted from bronchial lavage samples and bisulfite treated. The treated DNA was analyzed using HeavyMethyl-based real-time PCR on the ABI PRISM 7900HT platform.

### Preanalytics

### DNA extraction

Genomic DNA from unfixed bronchial lavage specimens was isolated using a QIAamp DNA Micro Kit (Qiagen, Hilden, Germany). The viscosity of the bronchial lavage samples was reduced, before DNA extraction, by adding 1,4-Dithiothreitol (DTT, Carl Roth, Germany) to a final concentration of 0,225% and incubating the samples at room temperature for at least 30 minutes or until the desired fluidity was obtained. After centrifugation at 3200xg for 12 minutes, the pellet was processed using a QIAamp DNA Micro Kit according to the manufacturer's protocol.

### Bisulfite treatment

Bisulfite treatment of extracted sample DNA was performed using an EpiTect Kit (Qiagen, Hilden, Germany) according to the manufacturer's instructions with the following modifications. A fixed volume of 15 µl DNA from sample extractions was mixed with 5µl water, 85µl bisulfite mix and 35 µl protection buffer. Two elution steps were performed using 25µl elution buffer each time.

### Analytics

### Principle

The quantification af the methylation of a specific locus is achieved via two PCRs. The first PCR is comprised of two gene specific primers and and a gene specific probe which detectes DNA irrespective of its methylation state (quatification of total DNA). The second PCR is comprised of the same primers but contains a probe specific for methylated DNA and two blockers to supress the amplification of unmethylated DNA.

For FOXL2 (SEQ ID NO: 123)
SEQ ID NO: 44 Forward primer ccaaaacctaaacttacaac
SEQ ID NO: 45 Reverse primer gagaggggttagtagt
SEQ ID NO: 46 Forward blocker tacaacaccaccaacaaacccaaaaacacaa
SEQ ID NO: 47 Reverse blocker ttgggaagattttggtttggagt

In one assay (for BARHL2) the DNA restriction Enzyme Tsp509I is used instead of the blocking oligonucleotides. This enzyme specifically cuts unmethylated DNA after bisulfite-treatment leading to methylation specific amplification.

For BARHL2 (SEQ ID NO: 125)
SEQ ID NO: 59 Primer gttttgaaatttattagaataacgacgtt
SEQ ID NO: 60 Primer acatataacaaatatattttatccaac

### Biomarkers/Assays

The following assays were performed with Scorpion probes:, FOXL2,
SEQ ID NO: 48: Probe ccgccgaaaacacgaaacggcgggagaggggttagtagt
SEQ ID NO: 49 Probe
cccgggaagattttggtttggagcccgggccaaaacctaaacttacaac

### The following assays were performed with TaqMan probes: BARHL2

SEQ ID NO: 61 Probe ttggattattttaaatgtggttaaaa

### Heavy Methyl based real-time PCR

Real-time PCR experiments were performed using the Applied Biosystems ABI PRISM 7900HT instrument. Each real-time assay for one biomarker consisted of two independend reactions: a reference reaction for quantification of total input DNA and a HM-reaction for quantification of methylated target template. The reference assay was composed of two methylation-unspecific oligonucleotides and a methylation unspecific probe, whereas the HM-assay consisted of the same two methylation-unspecific primers, but in addition two methylation-specific blockers (one for each primer) and a methylation-specific probe. For the biomarker BARHL2, the DNA restriction Enzyme Tsp509I is used instead of the blocking oligonucleotides. This enzyme specifically cuts unmethylated DNA during amplicfication after bisulfite-treatment. As a result, unmethylated DNA is prevented from being amplified.

Two different probe systems were used for RT-PCR analysis, depending on the biomarker/assay. For FOXL2, Scorpion® probes consisting of a methylation-unspecific primer part and a methylation-specific probe part were used. The Scorpion® probes contained BHQ1 as quencher and 6-FAM as fluorescent reporter. For the markers BARHL2 TaqMan probes with BHQ1 and 6-FAM were used as detection system. Each assay was tested with 86 BL samples (40 cancer, 46 benign lung disease). Each PCR plate contained several PCR controls. These included 50ng of bisulfit-treated Sperm DNA (0%BisStd), which is usually unmethylated, 0.5 ng methylated Chemicon DNA in 50 ng Sperm DNA (1% BisStd) and non template controls (NTCs). These controls were used to monitor the general RT-PCR performance and to define concentration limits for sample exclusion (see Data and Statistical analyses).

The 20 µl PCR reactions contained 0.25µl of bisulfite treated sample DNA (without any prior determination of concentration), 10 µ of QuantiTect Multiplex PCR NoROX mixture (Qiagen, Hilden), 0.3 µM unspecific forward and reverse primer and either 0,3µM TaqMan probe oder 0.15 µM Scorpion® probe. When a Scorpion® probe was used in the experiment, the concentration of the respective non-probe primer was reduced to 0,15µM. TaqMan probe concentration was 0.30 µM. For HM-reactions, blockers where added to a final concentration of 1 µM each. For Tsp509I-based assay, 1U of restriction enzyme was used for the methylation-specific amplification.

Thermocycling conditions were as follows: an initial denaturation at 95°C for 15 minutes followed by 50 cycles of 95°C for 15 seconds and a annealing/dentauration step at 56°C for 30 seconds. Single fluorescent detection was performed during the annealing/elongation step.

### Clinical samples

Number of clinical samples: 86
Cancer samples: 40
Benign samples: 46

## Claims

1. A method for detecting cell proliferative disorders, preferably lung carcinoma, by detecting the presence or absence of CpG methylation within the FOXL2 gene and/or its promoter or regulatory elements in a subject, wherein CpG methylation is indicative of the presence of said disorder.

2. The method according to claim 1, further comprising contacting genomic DNA isolated from a biological sample obtained from said subject with at least one reagent, or series of reagents that distinguishes between methylated and non-methylated CpG dinucleotides within at least one target region of the genomic DNA, wherein said target region comprises, or hybridizes under stringent conditions to a sequence of at least 16 contiguous nucleotides of SEQ ID NO: 80, wherein said contiguous nucleotides comprise at least one CpG dinucleotide sequence.

3. The method according to claim 2, further comprising:
a) contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NOs: 87, 88, 99, and 100, and complements thereof, wherein the treated genomic DNA or the fragment thereof is either amplified to produce at least one amplificate, or is not amplified; and
b) determining, based on a presence or absence of, or on a property of said amplificate, the methylation state or level of at least one CpG dinucleotide of SEQ ID NO: 80, or an average, or a value reflecting an average methylation state or level of a plurality of CpG dinucleotides of SEQ ID NO: 80, whereby at least one of detecting and diagnosing cell proliferative disorders, preferably lung carcinoma, is afforded.

4. The method according to claim 2, wherein treating the genomic DNA, or the fragment thereof, comprises using a reagent selected from the group comprising bisulfite, hydrogen sulfite, disulfite, and combinations thereof.

5. The method according to any of claims 1 to 4, wherein said biological sample obtained from the subject is selected from the group comprising cell lines, histological slides, biopsies, paraffin-embedded tissue, body fluids, urine, blood plasma, blood serum, whole blood, isolated blood cells, cells isolated from the blood, and combinations thereof.

6. The method according to claim 3, further comprising at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NOs: 87, 88, 99, and 100, and complements thereof.

7. The method according to claim 6, wherein said nucleic acid molecule or peptide nucleic acid molecule suppresses amplification of the nucleic acid to which it is hybridized.

8. The method of claim 3, wherein determining comprises sequencing of the amplificate.

9. The method of claim 3, wherein contacting or amplifying comprises use of methylation-specific primers.

10. The method according to claim 1, further comprising:
a) extracting or otherwise isolating genomic DNA from a biological sample obtained from the subject,
b) digesting the genomic DNA of a), or a fragment thereof, with one or more methylation sensitive restriction enzymes,
c) contacting the DNA restriction enzyme digest of b), with an amplification enzyme and at least two primers suitable for the amplification of a sequence comprising at least one CpG dinucleotide of SEQ ID NO.: 80, and
d) determining based on a presence or absence of an amplificate the methylation state or level of at least one CpG dinucleotide of SEQ ID NO: 80, whereby at least detecting cell proliferative disorders, preferably lung carcinoma is afforded.

11. The method according to claim 10, wherein the presence or absence of an amplificate is determined by means of hybridization to at least one nucleic acid or peptide nucleic acid which is identical , complementary, or hybridizes under stringent or highly stringent conditions to an at least 16 base long segment of SEQ ID NO: 80.

12. A nucleic acid comprising at least 16 contiguous nucleotides of a DNA sequence selected from the group consisting of SEQ ID NOs: 87, 88, 99, and 100, and sequences complementary thereto.

13. An oligonucleotide which is identical, is complementary, or hybridizes under stringent or highly stringent conditions to an at least 9 base long segment of a DNA sequence selected from the group consisting of SEQ ID NOs: 87, 88, 99, and 100, and sequences complementary thereto.

14. A kit suitable for performing the method according to any of the proceeding claims comprising (a) a bisulfite reagent, (b) at least one oligonucleotide which is identical, is complementary, or hybridizes under stringent or highly stringent conditions to at least 9 base long segment of a sequence selected from SEQ ID NOs: 87, 88, 99, and 100.

15. Use of a method according to any of claims 1 to 11, a nucleic acid and an oligonucleotide according to any of claims 12 to 13, and/or a kit according to claim 14 in the diagnosis, prognoses, monitoring and/or classification of cellular proliferative disorders.


Sequence listing

<110> Epigenomics AG

<120> METHODS AND NUCLEIC ACIDS FOR ANALYSES OF CELL PROLIFERATIVE
      DISORDERS

<130> E31136PCT

<160> 61

<210> 1
<211> 9739
<212> DNA
<213> Homo Sapiens

<400> 1

acggaagcat agaaatgtat tcaataaata tgcaacagaa ggacaatcaa tctaatatca      60
aagtgactag agagttaatg ccagggctcc atgggtatgc tgacattcca gaatagagag     120
gactgaaggt cagagactct gggtaacctg cacattaaga acgtcctcaa gttatcgggg     180
ggaaagatag agtcctttct gttctacaca aaggggcaga tctaaaatca cagtccctca     240
aaatctctaa aaatttttgc aagatttgaa gagagctttg ttccaaactc cagtgcacac     300
aatataaaca cacattatca atacttacaa taagagattg gaattaaagt gcacaccacc     360
tcccaaagtt tttcaaaact gcttctaatc acaaaaaggt aaattaaaaa tcctctctat     420
gttacagtag tttaaaaggt aaataatgga agtagaaagg agatgtacat ttgttgaaac     480
ctgttatgta ccaggcacct tacataactt attttatttt gatcctcaca ataattggca     540
aggtgtgtgc tgcaggacac taagaggtca tagattttag gaggctaaga aatgacctaa     600
aactttacat ctaatagttg gtatagcaaa gatttgaagt caagaatatc tgcctctaaa     660
aattagccgt atactatgac aacacatccc aaataaagat gtataaaaag tacttggctc     720
ttcatagata ttatataaaa tatgggacaa tattagtgat atacctaaat ttagacatta     780
gaaagaagtc cttcttaatt tactctgtaa ttgtaactca gttgttcatc ttacaggtcc     840
ctatagtaat aattaaacat tcctttcttt gttttatatg tgttttcaaa ctgctattaa     900
agatccttgg tcagcagcct ttatatcttt tggcgttttg ctgctaatgc tcggtaaaca     960
tgggcagcca aggaaaatgc agatggtgct atggaacaca aaccacgaca acatatggta    1020
tacatttagt cctaacaaag tgcgcttctt aacctctggg tgtcgcttca ccattccttt    1080
ataagactac tcatttccag ggaaggttta gtttccaaaa ccagggcaac aggcccacat    1140
gtgttatatg gccattcaga tgcacagatt atgtgtccta atacctttaa gcaagtgcat    1200
ttagcacatg cttcctagca ctatcatgtg tatttatgaa ctgttttgcc cagatggtaa    1260
gctcctcgag gacaagtgcc atgcctgaaa tctcacctta ttaactgtgc atggcagagt    1320
aacaatgtta tctatcttgc atatagagac acacaaccta taaattgctc ttctaggtaa    1380
acctcctaac tacactgcta cattgtgaca attgtcacat cttctagatg aggctcacac    1440
aggctaagca attagccaga atcacaaact aacaggcaat gttttcttct tgcccacaag    1500
agaaagcttt tcttgtcttg caacacttta ttgttttttt cttttaaggt ttttattaca    1560
aaggtcaatg tctattggcc ttttaataaa agtaattatt atttagtact tagtatacat    1620
atgcaaggca ttgttctaag ggtttacaaa atattaactc atttaatcct cctaaccatt    1680
ccatgagata gacactattt ttatccacat tttacagata aggaaactga gggacagaga    1740
gattaacttg tccaaaatca gaggactggt aaacagtagc agtgaaattt gaatccagca    1800
gtgaagcata gctccacagt gtgtgctctt aaacatacac tattctgctt gaataagagt    1860
ctacaaagat ccttactcag gacatatgag gagttgttca gcaagaaaag aagcatgttt    1920
actcctccct atttgatgta tttgattata ttctaaaacc tttcctcatg ccaatagaac    1980
tccgcgtatt atactaaact ttcctgcacc gaaggcatat gtaatggtta ccttgactga    2040
catgtaaaat acccaaaaca aaataccccg agactcgagg attttttgtt tcatgaatta    2100
gacaccaaaa aaaaaattgt ctaggcatca gaagtgttat acactcacat ctgtaagtct    2160
ttcttgggat tttcagattt cttatacaag attttttttt gtctgaaata tttcagtgct    2220
cactcaatgc ctgagcttga aaattatcta atccttttta attttttcat tttcagctac    2280
tttgccttca ggcatagggc agggcaagca cttgctttca atatgatgtt taacactttt    2340
tatgttttta ttttcgtcat cttgaaaaca tgctagtaaa catccaaaca tactgtaaag    2400
atttaaaatt tggggtgtgt atacgtatat caacaggagt tatgaattat gtatattgca    2460
ggcctatata aattagattt tgaagaacaa gcatcacagt aatcaagcgg gtcataataa    2520
gacattccat gtgaaatgta aaaactacct tgaataaatt atctgtaagt taacattctc    2580
attagaatga catatttatt atttctgggt ttgtgatatt gctttaatgt attttggcta    2640
actgttttaa tggcatatta agaaaccatt tcaggccttt tttccccgtt ggaatttgca    2700

gacttttatt tccgtttgaa ctaaaataat ataatattaa aaacaatccc ccacttccca 2760
atgtacaata ccatttcttt ctgcccagaa atatttaatt aaagcaggat ataacctcag 2820
taattatttt tacacagaat gactcttaaa ttaatagtca gttgtatgac taaaaattgg 2880
gagactgaca atcaaaacaa tcttaaatgc tttgtttatg tgatgaaaat gagtgatcct 2940
ttaattccct cacacacatt aaactgattt cacagatttt ctgtactggt gtttaacata 3000
gtcaagtgct tgaggttatg taaaataaac aatcttgaga tcttattgca aatgtttgca 3060
atttatgatg taaactgatt tgtgaagaaa aaacaggatc ctatgtcgct gaagccaaag 3120
aggcattttt cagaaatcaa aatagttccg aaatttgagc attgcattat actaaagtat 3180
taccatctga ggacccaaaa aagttataaa tctgggaaaa aactcaaaat agatgtacat 3240
cagttcagct ccaagcaaag gcaccgatct tactatctta ctgtgtctct tgtatcatag 3300
ggtccttaac actaatgatg tcttaaacaa tctcttttaa ctcagttttt cccagtcatt 3360
gattttgcaa ctcgggaagg ttttgcatgc ccaaaagatt tggggagcag aggatggggg 3420
tgtcctttat taattatatt aggattattg agtttcgacc caaactacaa gcatgttggg 3480
ccttatttaa atttaaagtg aattcctcag accccttctt gggatggttc ggggaactac 3540
gagtttggaa ctcttgttca cacaccgagg cgcctgcccc agagtttgga cactggcatt 3600
ccgggagagc aggccttgcg ggagtctgga cccgaagggc gagactccac agggccaagg 3660
aaagcggcct ctgtcctccg ttagtcttgg gggagcagac gcaagaggag gcaagggcgc 3720
cgcgagctcc ccggatgcac tggtcccaca ggccgtgccc gagtggagca ctgcgaatgg 3780
ggccaagaaa ttttggcctt tctcgccgga cctggctgcc tccgcgggcc tctccgccta 3840
ccgcgctccc gccgcggccc gactcccgcg ggtctccgcg ccgaacccac ctggctccta 3900
tcgcacggga cattcccgac ccacccacgc cgcgtcactg agcctctgta ccgatacccg 3960
gcgcctccgc cagcagggcc tggacgcacc gcctcctttg acctcgggct tcccccgcgc 4020
tccgctgctt ggggcagact ggccccgaga gggagccacc atctcccctg ctccagggtc 4080
tccagggtcc gaacccgtgt tgggatctgg gttaggatta gggtttggag cttggagcct 4140
gcctgttagg acccccggcc ccggcgccga ctggagctcg ctggaggcca caggaccacg 4200
gcggatggcc tggctgcttc agggcgtacc atgcccgcag gcagatgttt attattaaaa 4260
actaccgacg ttcatcaacc aggagacccg caaggcctgc gcactgaata cggcccaaat 4320
cctgttcagt ggtctttgaa agttaaaaga aagaaaccta tcgcccacgt ctatgctgag 4380
gaacagcttt gaatacgagc taagacctgg gagagggcca agtgggggtg gtggggaaca 4440
ctgctggagg atgtgggcct ttggcagggg ttttacgcac cctccagcac gagggtgggg 4500
ggtcccaggg gacgctcagg actctttcag tctttgcgga aggtcccgtc atcacaagag 4560
cccgcgcggg aaggaaagtt cctgccttgg acatggtcag ggccgagtct tcaaagtctc 4620
caactatccc cacacaggca gcacctttgg gctttactag tgggatttct tccagaaggg 4680
ggccacgaca tggggagaga gagctcctca ttattttcaa ggccaggctt ttcctaaacc 4740
cgattctggc ttccaccttc taaatttaac caatgaagaa gctgcttcag ccaaccctaa 4800
acaggagtgt cagatgggga atcctccctc cacagtgccc tggcctgccc gcctttgcgg 4860
ctcttttccc gctccgaagt cagcacctgc cccgctccag agagggtcaa cgaactggga 4920
ctgattgtcg attatgccat accaaaccca ggttgatttc gctccgcagg atccctcctt 4980
ccttcctccc aaaagtgtcc ccagataaag actggaatta tagcaaaacg aataacgaga 5040
gtccatcttg gggaaggaag ttactctatc ctatgttatt ttacttctta tctgttcttt 5100
cattaatttg gtataaccct gtttctatgc tgggtggata aaatggaggg ggccggcgga 5160
aatgcgctca gcgctaacga cagccacaga gccacccttc ttactgcccc ttgactgcgg 5220
cacgtaagag cagaggcaag cgcttttcca agttggtata ttcgagagag tgatacgcat 5280
taatcaaaag ggaaagacta tcccggatat tttaatagta acaggaacaa agatgactcg 5340
aaccccatca aatgaaacga tattttcatt caattgatcg caaagtgtct tcaattaata 5400
acttggcact tatttaaaag gtttaacaga attggaggcg acacattttg tactgacaac 5460
acgtcatgag ataacatctt ttgtttaaaa taacttaata cactagaaaa aaatatgtca 5520
aatataaata attttgtttt catggagaac aaatagttac aaagctcaac cgcataccaa 5580
agttcagtta gaagagcttt tcctctttct tgtaaattag aaggtaaaac aaaaacaaaa 5640
acaaaacccc acaaacttag gcttgctagc gaattgggat acagactggc ttagtccaag 5700
gtatcccagt ttaaatagct acatgaattg aaacaaacaa ccaaaagaaa aaaaaaaaca 5760
aaaaagaaac aaacaaacaa aaaaaaaagg ttacttgaat agatacacct ttgtgagata 5820
aaatgcaaat gttgaaagtc agtccacaca ttatagtcaa aataaacttt gttcgtgtgt 5880
atcaacatat cttacacaaa cctagtgcct gtatctagct gggtgttatt taaggtgaat 5940
ttgacgggat agagaggggg aaataaacca ctgtcttcaa ataggacttc aggaaaccaa 6000
caagaaggaa cacaagaaaa ggggaatggt gggaactaat actgattgag cgcctactgt 6060
gtctggcaca gcgaggcgct gtaccttcat tatttcattt ggtggtcttg ttttcttttt 6120
cctttcccca catattctgc ccacctctgt cttcttagga caatgagtgt aattttttcc 6180
ctttctgact tttttctttt gttccccagg atcaaagaca gggcaaatat atatatatat 6240
atatatatat atatatatat atatatatgg caaatatatg atatatatat atggatatat 6300
atatatcaat ttccagatac ttttggtatt gtttttcata gtgaagatga aaatggagtg 6360
agtgcatggg agataagggg gtgggaggag acacaccatc aatggaatac aagtaacatg 6420
caaaccggaa tttactgtca gcagtccaga cagttttgcc ataataattt ctcagagaaa 6480

tcattgtctg tggcattttt ttgtgttaat attatttttc tctctcctcg ttttaatatt 6540
ttctcttttc ttgctcttcc atacatggtg ggtttaaact caaagcatct gattcaacgt 6600
aaaaggaggc ccgcctctct catcaaattt ctcgtgttaa cgtgaacttc gcagaaaaag 6660
gtcagtttca aaacctgtga acttcccagc tgcgcacttt tttcatctta gttttttaaa 6720
taagtatacc tcttctttct gctttgcctt cttagcgcat ttttaaaaac acacatttaa 6780
gttggtaact ccccccagtc tctcgagaat cgtctgggtt gtttttccat atttttaaat 6840
gtataaaata ctttaatttt aaaatttgtt tttgctcctt ggttctccaa tttcagcttc 6900
tgccaaatag cagttaagaa ataagttccc ttcctctttt tctctcccgt ttgtctttcg 6960
atttttttgt ttgctcattt tttcattgtt aacaagattt ttttttctat gcaagagtcc 7020
atcgttgcag ctttgcggtg agccaaactc cgcggttcca gcactcccct gtccagtctc 7080
tctccagact cccccaaacc cgctcctaca aaacccaatt ctaggccctc gagtaggaaa 7140
acgggcagga gccacggagc ctgcgtgcct cgtgagatcc ctggtcctgc gtggagtctg 7200
gctttccgag tccaagatgc gataggggac gagggatggt cagtgaggcg ggaagagggc 7260
cggctcccga ggtctcaaag gggtaacgga gaagcagcgg ggcgcggagg gcgtgcaggc 7320
tgagtgccgc gggacaggcg cgacattggt gctggcgttg gcgtcacaga cccagggctg 7380
cggcgtgctt tttggctttc agtctgagat cggcgatgct ggagttcttg ctggtggtct 7440
tggcggctgc tgcggccgcc actaccgagg cggcggaagc cgaatccgcg gccagcgtgg 7500
cgagcggcag tccgaagggc ggtgctggga acatcatgta gggcgcgtgc gcggccaggt 7560
gcggatgcag gtggtggtgc gcgtgcgcca cagcgctgtc cagctgcagc tgcgcctgaa 7620
cctgaaagga caagggcgtc acgttgcaat gactatccta gggtgacaac agaatagaaa 7680
cagagcatta acggcgtcca gcttggccag agagagagtt gggttgtgtt tgggcacggg 7740
gagagggcat ttggctgtgc atcttgttcc gacagtgacc ttctcagcct gttgctgaat 7800
ctggggttcc acatggatac tccagtgtcc cctgggtctc cgaaactcgc ttagggtagg 7860
cttatttccc cggaccccgg gaaccccctt ctgcaggaat gtggccgtct cccctgggaa 7920
ctgggctgaa atggtatatt ggaaagactg cgggtgagga cccttaccct cttctcttga 7980
tgctgccatg catctcttac cccactacca aaacaacgga gtatcaacaa gcaaactacg 8040
caacttttta atgtcattca gggaagttac tccatcttca ccacaatagg cacaattgtg 8100
agtcggtact tctaaccttt attgccataa caattaccca gggatctcgt taaaattcag 8160
attctgattc gcaaggtcta ggttggggcc tgagattctg aatttctaac aatctcccag 8220
acggtgttgt tgctgctggc ccaaagatta ccttttgagt agtaaaggtc tccttttaaa 8280
gtcacccctc cctctttctt cagtcattct ctcttgtttc caaagccaag catcaggatg 8340
ccagagtcct gtggcaccaa gagagctaag atctccctaa caccgactct caattcatgg 8400
tcacacctat ctgtctcccc ggtccaagtc actggagact gagagctaat tcatgacatc 8460
acaggcatgc caagcagggg tcctggctga ggatagggaa ggtcagacac tggttgggaa 8520
aggatttcgc cctcaggctt ggaaagtggg gagttcagag cactgggatt tcccaggatt 8580
aaatcattgc tgcaggctgt ccttttaaaa tgtactctta aaggtttctt tatggctaag 8640
ggattgcaaa ggcagggcag ccctgggaag accaagactt ctctctttac cagagatgaa 8700
gctttgtttg cagaaacaga tttaaaaaca aaagaacgaa aaaacaaaag tatgagctgg 8760
gagtacttgt gtttattttt cttctgtcag agttatttaa tgtgactaag aggtagaaaa 8820
tacctagaaa agtatctaga gggttgcctt agaaatacct taggactaac ctctgtattg 8880
gatttgacaa aatcttaatc aaaaggcttt tccttcctct ctctctaagg gcaatcttta 8940
gtgtattttt aaaggaccag ttactgcccc caggcccctt cagggattcc catgaggaca 9000
gaagggacta ggcattcagg cctttaatac cgtataggtt tttaaagggt agtgggtcca 9060
ctatatcatc tcaaaatgat tctccaacta tgcagacatt gacatctggg ctcagagaca 9120
ggtgatgttc aacagttcaa gcaaacattc aggtattttc atatttaaaa acagcttgaa 9180
actaggctgt acttgcctgc tgaaatggca tccttaaagc acctacgttg acataaggtg 9240
cgactctaca agcttcaaac tggctggcgg cccctatgag aacacctgta aaaagtacaa 9300
gagaaaaata atgtgaggtt aacgtttgta gcatttttca gggttccaaa gggtacaaga 9360
cagaacgaat tcctttgaaa atggactatt atctttctaa actgcatcca aaatttaaat 9420
aaatgtaact tactcccaaa ctttaggact ccattaacag cctttgaaga ttattaagtc 9480
aagaactatt gtcatttttg aggttccttt ttttctgtca ttgtaatagt aatattcact 9540
acttctctct ttgctcagac tatcaaatgt tccttgtata agaagcatac ctttatggag 9600
ttgattttct tgttttctac atttagctct tcgattttga aaccaaacct ataggttgga 9660
gggggaaaaa aaataaaacc tagatgttat gactaaaaat ttttttaaat tacaaaagta 9720
caaagagaaa agagtcgtg 9739

<210> 2
<211> 4313
<212> DNA
<213> Homo Sapiens

<400> 2

gggtttgatt ttttgagact cggggaggac ccctggcaga tgtgtgccta gccagaacat 60
ttggtaagga ctcctccaat gaagaaaaag tggaggaatc cagccccagc gagaagaggc 120
ttccccaccc tgccctagac acaccggaca gagggcacac tctgaccaga gccacgtcca 180
gtggccagga ggccagccca gcacctcctc ccccaccatt cccgtcctgg gtgggggggt 240
aattttcctg ggagcagctg tgggaactgt cgtttctcat cccagcccag ccagcactct 300
gaagcctgca ggggaaggac agcacgtggg atggacactg gggaaggagc ttcgcaaggc 360
cagggtgcaa ccttcaggcc ccaggtggcc tggcaggcca cgctgcctcg gagatgcttg 420
ccagactccc caagttcact cagggcctgg cagcaacctg ctggctgcct ctgcgggggt 480
ctgggttgct gagcacggtg cagctgccca gggccaatca gccctagggt gtccgtgcca 540
ggctgcggcc tccccgcctc ccccgcactg agggtactca tggcgtgcaa atgctcccgc 600
acccccagag ctgccctatc ggatgtttcc aggaattcac acatttccat aaaaatgcat 660
tttaaatgat ggacaggcga gcctggggta acaacgggtg tttggtgggt agacaagagc 720
aaatgggaag gagcccgagg gaggaggggg aagagaagag gaaacagaac ttccagttgg 780
acattctgac aacagctgga aggaaagtct agaaaagatg aagagagagg aggggagaaa 840
ccaactgggg ctcccaccct tgccgttgga ttcctaattc tcgtttcaaa tgggccctgc 900
tctccggcaa aattagttta aaggatttta aaacaaagaa aacgagatga ccggtctggg 960
agctcctcaa tcagagtaga gaagttagag gggggcgggc gacttggttt tgaagtctta 1020
gctgaacagt cacccctcct ctccttggca aaaaggattc ctttagaacc tccgaggctc 1080
ctggatttct cccttcgcaa atggagccgc atactgcatt cccccgctct ttcggatcgc 1140
taagcatgtt tcatgagggt cgctgtcccc gggtggaatg cggccgtatg cacgcgcctc 1200
cctgcacacg cacacacacg cacacttaca ataagtgtct gcaggaggag tgtcctgcgc 1260
gccagctctg cgtttaagac aggaagctgc cgggttaccg agtcaaatgg gagtgacact 1320
attcctctcc atcagcaagg aaagcggacc acaaaagtcc ctttgtatct cggcagctca 1380
tttaatatta tttatgcatt ttgtgcaagg aattgtggga tttcgcccca cggtaaacaa 1440
tatggaaatc ttaaaaatag cgatcttcct gtgcgtgtcc acctacgcgc cccggggtga 1500
cctggcgggg ctgtcgccgg gtgactcaca cccctgaacc gcgaagcgac agggaaagcg 1560
cgggcgagcg caggagacgc ggtcgggggt ctctccgggt tcctgggctc ccgcacccgg 1620
agcgggggac gcggccgctt taaggggagg aggggcggcg ggctgctcct gtcacccagc 1680
ggcggccgga gcgtcacgtg ggcgcgcggc gccgcggcca ttggcccgag gcacgtgtcc 1740
aggagaccgg cctgcgacgt cactcgaggg ggctctgtta aaaataagaa caaaaatcca 1800
gagtgaaagt gtctcaggtt gcgccgagtg gcctggaaat ttccgagccc gcgcggaggc 1860
cgaggcggcg agggcggcgg acggccgggg agcgcgggcg gcccagcccg gcccggccgg 1920
gccctggcct cgcgtctctc acccatgcga ctcgggccgc ggagctctgc ggggctcggc 1980
gggggcgcgg ccgcacgccg gtggggcgcc ccggcccgca gcggggcggc ggccgcgagg 2040
agggggcctc catgtgcgtg cgggcggtgg cgggcgcgct gaccgcgggc gcccggcacc 2100
ctcgagggcc ggctagggcg tgcgggcggg gacggccggg cggcggcggc ggccggagcc 2160
ggcccgggcg ggcgtgagcg ccggggaacg cgctgcctgc atgcgcgcag ctctcgcccc 2220
gggcggccca ggcggcggcg ccggagcccg aggcggccgg acgcggagag gagcggggag 2280
cccgggaggc ggcccgcgtc cccgccggac cactgcgact gtctagaccc cggctgcgcg 2340
gcgaagtcga ggacttggct ctgttgaatc tctcatcgtc tgggcgagcg gggcggctcg 2400
tggtgtttct aacccagttc gtggattcaa aggtggctcc gcgccgagcg cggccggcga 2460
cttgtaggac ctcagccctg gccgcggccg ccgcgcacgc cctcggaaga ctcggcgggg 2520
tgggggcgcg ggggtctccg tgtgcgccgc gggagggccg aaggctgatt tggaagggcg 2580
tccccggaga accagtgtgg gatttactgt gaacagcatg gaggagaatg accccaagcc 2640
tggcgaagca gcggcggcgg tggagggaca gcggcagccg gaatccagcc ccggcggcgg 2700
ctcgggcggc ggcggcggta gcagcccggg cgaagcggac accgggcgcc ggcgggctct 2760
gatgctgccc gcggtcctgc aggcgcccgg caaccaccag cacccgcacc gcatcaccaa 2820
cttcttcatc gacaacatcc tgcggcccga gttcggccgg cgaaaggacg cggggacctg 2880
ctgtgcgggc gcgggaggag gaaggggcgg cggagccggc ggcgaaggcg gcgcgagcgg 2940
tgcggaggga ggcggcggcg cgggcggctc ggagcagctc ttgggctcgg gctcccgaga 3000
gccccggcag aacccgccat gtgcgcccgg cgcgggcggg ccgctcccag ccgccggcag 3060
cgactctccg ggtgacgggg aaggcggctc caagacgctc tcgctgcacg gtggcgccaa 3120
gaaaggcggc gaccccggcg gccccctgga cgggtcgctc aaggcccgcg gcttgggcgg 3180
cggcgacctg tcggtgagct cggactcgga cagctcgcaa gccggcgcca acctgggcgc 3240
gcagcccatg ctctggccgg cgtgggtcta ctgtacgcgc tactcggacc ggccttcttc 3300
aggtgagccc gcggggacca cgcgtcccgg ctcgccgcgg ggaggcccgc ggagctgggg 3360
ggcggtgctg gcgcgggaac ttaccgggag gaaaacatct cgaacctccc ccgcgcacac 3420
gcacaaagac tcacgcgaca ttgtgtgaag ctgacgccgg cccgggcagc ggccaggagt 3480
ccagcggcag gactgattcg ctagggggta cagacttctt aggaccgcag aaggaccttt 3540
ctttctttct ctgtctctct ctctccttcc tctctccctg tctcccctct ctgtcttcca 3600
ccccgccttg gcgcatctct tcccagcccc tagcccatgt ctcccccact gtagtttttt 3660
ttggtgggaa cgtggtggct ggaagatggg cccggaagtg cacactctca tccccttccc 3720
tacgatcttc caactcaggc caggccgggg acgcatgccc cagcccaccc cagacttgtc 3780

ctaccatccg gttatcccgg ctgtgctcgg ggaagaaaag gcgaggccct ttgccgctct 3840
gccttctgcc cctcgggcct gcgctgaccg gtgggactca ggaggatgca cacagggaag 3900
gaggaaaata aaggcgccct ttccccttgg ctccactttg tttgccagcg ccagcccgca 3960
gtggtggggc tcagccccct tcctgcacac agcgaggaca agggaggcag ccgtccctcc 4020
cggcacctgc catccccaaa tagaaaggac cttctctcag ggtttcctgg gggctgctga 4080
tgggaaagag gcagcattcg caggggccct gcagagatgc tggatatatt ttttcataga 4140
tctgcgattt taaaaaacta agtccatgtc cttgtagaaa tcatcaactg cattccatgc 4200
gggtctgcgg ctgggaaccg ccattagaag tggactgttt gaccccgagc tggcagcgga 4260
tccccgctgc ccccaaaccc tcaactattt tgcgggggtc atttgcccag atc 4313

<210> 3
<211> 16197
<212> DNA
<213> Homo Sapiens

<400> 3

ccctgcaggt ggagggggaa agggcttggg ggctggtgga ggacgcagga gtatggggga 60
gctgtggaaa agacgtggag gcagagccag caggccttgc taagggacag gaggtggctc 120
tagagagaaa atgagggatc agggatgtct cctggggggt ggccagctgg gtgactggga 180
gaaactggga aggcgcagat cagggcagca ggggtagcgg gatcgggggt gctctgaagt 240
gaacacgtga aattgaaggt gctcgtcagc cttccagtgg aggcatccag gaggcagctg 300
gaactggaag gaaatggtga cagtcgtcag caccgtagag gtggcggtgg tggtggttac 360
agctggaccc ctctgggctg gtgggaagtg tggaggtgaa ggagcaggga gcagggggag 420
gtggagaagg aaacctcagg cttacatttt caccctattc ttggctgcca tattttcagg 480
aagtccctct tgaggctggg acagagggtg gggacagttc agtctccctg agagagttta 540
ttctcggagg cctgcggtgg gagccagggc gcggcggaga gggtttccca cctcttcccc 600
agcaagcggg agggaggcgc cgggctgagg ctgcgctgag ctcgagctcg ccgcccgggt 660
ggacccgccc tgttcaagcg cggagggcgg gaggcctggt tgggttgtag cgtggtgcgg 720
agcaggacgc cgcctcgtgc catggtcact ggagacgcac gcccatcttc cctcccgggc 780
ccgtcgatcc tgccatcttc cgctcccggc cgctcgtggg tgttcgtcat ttagaccttc 840
cccgtgcttc atgggacgca agccctcccc cagagttcgg ttttgcaaaa gaggtctgga 900
gccctcgcta cagatcccct ccctgggcca cggaggatga gaagggtcac cgagccgagc 960
cgtaatcctg cgtaacccct gacctctctc cttccctgcc cccacggcac cccatcgtcc 1020
cctccccgtc tccgaggtcc tccagaaaac agcgcagaat tgtgcagatg tttaggagat 1080
gtgaagatgc tggagatgct taggaggcga cggttacgcg aggagaattg cttccaggtg 1140
cgcttctgga acgcacggag atcccccggc ggggaagggg ccggggctcg cgtcacttag 1200
tgtctgctca ccgatttccc tctgaagagg gggcccaggg cctctgctga gggagcgggg 1260
aggcggtgcg ggcccctccc gggcacacat gggtgtccgc tccttcctcc ccctcccctc 1320
ccctcgcacg gagagcggag agcggagagc ggagagcgac agggaggcag ccgaagactt 1380
gaattttgaa aggggagctg gcggcgaatg gtgaatgaga cagctactta ggaagcgagt 1440
ccagccagcc cgggaggcgg tggagaccca cgcccggaag taaccggatt aggtctcaga 1500
atgtgatcgc cccccgggtc ccgggggaga cgccaaggac gcgcaagcgg agggcgcgga 1560
gacaactggg agtcagagtc tccatcactt gggctgggaa cgcctctggg cgttccgacg 1620
gggtggcggt gggggtcggg ggaggccctt gagaaccgtg cggcccgggg agagcccacc 1680
cattgctgag ccccgacaca ggctttgaag ttgctgcagt ggctcagccc ctcccccgtc 1740
cggccctgcg cagcgcggtg ccgcagagtc caggccgtgc ctccgtttcg tcgctcagag 1800
cttattgcgg cggctcattg gaccacgtcg gtggggcgat cgcagcctct gatctgtgag 1860
ctgcaaagag ctccgaggtt cattcacaaa tctgcgcccc cagccgtccc tccacgcacc 1920
cgagccacgt cccgggaccc ggagagcccg gggcgttggg cgctgcggag gaggcccggc 1980
ttctgtcgct cccttcacct cccagcccgc gagggacttg ggggaagggg gagcaagctt 2040
tcgctccgga agaaacgttc ggaaccaagg agtctgactc ctggatccgg gtgcctgttg 2100
gatccaggct cccactcccg ggccctccgg tcgaaccaag ggtcccgaca gggcccgagg 2160
cacccgcatt cctaggagac aggagctcgg ccagggcgca tcaccgggtc ctgctccgag 2220
ccagaggatg tagtgtagac acccaccccc acaccgcccc tccacagagc tcctctcccc 2280
ttggggcgtc gctgggcaca gggcaggtcg ctaggaatcc ccagtaaaac cacgctcgct 2340
cagaggttcg cggcttcctc agagggcgct ggggaaagag aggggacctg atttcttcga 2400
ccctcggagg aaaagtgctc ggggcctcct agggacacag ccctggaagc tcacctcatc 2460
ttagcttagg ccgcggcgag gtggggagga gagttaggag agggggagag gggctctgcg 2520
ccctgcagag gcctttaatt ctggaggaaa aagactggga cacacccaag cgagccaagg 2580
cccgagcccc acgcacctcc acccacccgg ggcggcgcac agccagctcc tgccgggcgt 2640
gagcactcga tcaagggagc aagtggaatg aaaatccagc tgggggggtc cccatcgaca 2700
caactgtccc gcagtcgagc ttctggattc ctgggagatg tggagagcct ggggccggct 2760

cccgctccgc agagcagact ggactgccct aggtgcctgg aatgcgcctg caccctgtcc 2820
ctcggacccg cgggagaccc gtgttccgca agtccccctt ctcaccctag tctgccccta 2880
cctacatccc gccggcgagt gtgccaccgc gaggcgcctc cttccccggg aagggagctc 2940
ccttccgcgc agacccgcat tgcccttctt ttcgctcggt tttgttttcc aggggtagtt 3000
tctgcagaaa ggagattctc ctccgggccg aagggctacc agcctgcagt cagttcagcc 3060
ccggaccctg ggagatgctc accactctgc ggatcttgat ccgaaatcct ttctggccgc 3120
ccactgcgga gagtgtcctc gtagagaggt tttcaatcga aggagcctgg gctccacatt 3180
ttgtcttcaa gcctgagctt tcagggctgc tttgcacgag gtgcagatga acttgtgtct 3240
gcaaacaaga cagaaaaccc taagtgccgc ccgaccttcc tcttttgggg aacccgcact 3300
ttgtcctggg agcgtgccca gcgccttagt accaaacttt ctggccgggg ctggcagagc 3360
tcagagtccc gcttccccct aggcgcggcc ccctaacatc tgtaacccaa atgttgcgcc 3420
gcggccaaaa ttagccctgg cagtgcgaac agagaactaa aagcaggcag tgaatgagaa 3480
cagctcgcat ttctccttcc tggtagacgg ggaggtgtaa atcccgagga attctagggc 3540
attcgctcca aacgtgggaa atcttcgcgc gtatcccgtt tcttcctccc agcctgtgtt 3600
aatgctccaa atggtgtcga gttgctcaac tttgccgtca tcacagaggc tgttgtgtct 3660
taggggatta actgatgtga gacacacaaa accctgcaac tccataacat aaactatagc 3720
acagcctttc tggagagggc tggaatattt gagtgagttt ccgagaggaa aagaggagtt 3780
ttttagagga gaaacagagc acttctataa tgtgccctaa ctgagaaatc ttgttctact 3840
gagcttttct ttaagtggaa ccagaagtgc tgggatgaga gggaaaggat gggagtgcgt 3900
ccaaaggtgg acagcaggtc cccatccctg gtgggagtga gactggacgg catcccccgg 3960
aaaggtggtt tgggccttgg acaaggctag aggcaggagt ccatgatgca gagatgacac 4020
agtgcccctc cgcgtgtgag tccacgaagg tcactactga ggctttgtgc ttgtaaaagg 4080
tcgccacgct tcacacaagc ttctatactc aacacaggga ttgattgggt acagggatcc 4140
ccccatacca tacatgtaag catgtatgtc aattaaagat gctcgtgcta aagaaacggc 4200
caactttgtt gaactcagag gaactgatca atcatttaac taagtagagg aaatgtttga 4260
atttaattcg caatttagtt gcctttttat ataaaactat atatctttat ttatatttga 4320
tgaatgaaaa aagaaatcag ttcatgactt taacttaaac atatgttttt aaaaatatat 4380
ttcccccagc ttagccagta tataaactaa ttgagttctc ctggttaagc atgattcagt 4440
tgtgatattt aagagcggga gtggttgttt agatattttc ttcctcacgt gaaacctaga 4500
ctaatgagtt atcatctaac aagctgtagg tagtttggct gggctggatc cagatggttt 4560
gagttaaatc tagattgtac ttgcctaaaa ttctgtaaac ctctagctac gcaaatccta 4620
tttccaaaaa tgctgggaag tcactgtata agagtttaag ctacactagc ctctctgtga 4680
ttacttgtag cctttgggga aagaatagaa gaaaagaaaa tgtcagcctc tgtgaggtgg 4740
ggctggtgtt tcaggggttc cttttgaaca tcttgttttc tttccaaagg tcaaaaggaa 4800
ggcagtggat acatactaga atttcttcca tctgtgaatg gtcgcaaggc tggagaaggt 4860
ggctagtgta cttcaaggct cattatcctt ttctgtgttt tccttcctgt tttggtaggc 4920
ttagccagct caagccttgg gtgccattct tcaaattcct ttgccaaact aattttactt 4980
atttgactgg attatttgag aggtgccact ttcttctggg ttgctgtgat tttgaggggg 5040
cattttcata agagtccagg cattaggtgg tgaaacagtc tgtgctccca aacctgcccc 5100
tcccagggct cccgggagac tccagagtgc aggtctgtct ggggagtctc aggggtgggc 5160
cttgagtgga agtgggccca ccttccacag gagtccaaat tttataggaa taagaacagc 5220
agtaaaacag gacaagagag caggcaggga gctgccaagg aaaggcgatt cttgggaagg 5280
caggtcaccc agaataaggc tcccctggcg ctggagagcc ggaaggggga gcgggcacag 5340
aggacctggt ttaggtgtgg gggttactag cacaagtaga gccatccttc agattctcct 5400
tcagaagcag ctgctttcca gagaaaccag gtgagggaca gtttctgcat ctttacacgc 5460
cagctctgga gatctgccca cctgccccca gccgcccccc tccccgggcg agcctgggct 5520
aagtatcaag tcaggacaga agggcggtcc ccagtcggct cctggcccac tctgcttccc 5580
caacacctag ggagcttggg ctcagcacag gcgcttctcc agcgatcggg gcagaaccag 5640
gacgtgcaat gcgactgccc ccccctcccc gctccccggc agagcttctg tccgcgccaa 5700
cccacccacc aggctctgcc cgcgccacgc gcgcccctgg caggcctggg gcggggaaag 5760
gcgaagcgct gggcacgcga gggcctgtgc accccagttc ctacgacgct cctggccctt 5820
ttcaggcccg ccgctgccgc atttaacccc ctccttccgg gggttattct gaagaagtct 5880
cagaccctag acccagcctc ccagaccccg tccccgagct cgcccggtgg gcctgtaggc 5940
cggtcctcct tcgcccagag gagagcgcag acacgcaatg tccgcccgtt ggccccgccc 6000
gccccacccc tgctccccgc gccccttcgt ttcggccttt gtctacaggc cgggtcggaa 6060
cgtcagcccc aggagccgac ggtggctctc tgctcgcccc ggggaaggtg ttgctctcct 6120
atcggcccca attccccgcc cggcgcctgg ggcttggctg cggggctcgg ctcccagccg 6180
agggcgcagg gctggccagg cctgctttgg ctgaggtgga gatctcgctc tcagggattg 6240
ttgggcgtct ctgccccgcg agtaacgaga ccgccgcgag cgaacggctt tcattgagtc 6300
catttttcca agtgtcacca cgtcaagcta gagaagcgag gcgagtggag gggacgcaga 6360
ggggccggaa aagtcaccct tctctggcct cgttttcaga taaaaacggg agcctggctc 6420
ggcatccggg cgcccggtgt ctccggggcg cttcactgag gttttgtttg caaaactagc 6480
gtccgtgtcc tgaagcgcac ggcgtctgga agctgctttc ctgctcgccc tctccgaggc 6540

ccctctttgt gcagcgagcc tgagaaatat ggaggaccgt cctccgcaag cgggtggtcg 6600
cgggcgctct ccgattcctg ggtgaagcta gagggaaaac ggggttcccg ggtcagtgcc 6660
ccactttcca tcccgggaga aactaatgtc cggagagggc tcgtctgatc cgcacagaaa 6720
ggccgacctt gagggcggcg gtcgttcggg ggagaaagcg gaggctctgg gctcgcggga 6780
gcgcggcagc cggggctggc atcgcagagg agagacacgc cactgccccg catccccaga 6840
aagcgcgagg cgccgtccca gctggggcag gcggccgagg ccggtcctca tgcgcgcttc 6900
tcgaagcttc ctgaaacacc ttgcggagtt ccgtgtgtat agaactcagc accgcccagc 6960
ccctgggcag ctcaactttc tgcagcccca tcccagcttc cccggccctt tgaacggtga 7020
cccctcttac cgctttccca gagttgtttc gtgtttgggt ccactctggg gggcccggta 7080
ccctctagtt atctctctct tcatttattt ctctttctcc tattcctccc tccacctctc 7140
accttcctcc tttccaggag agcgacccac gaattcctct tcctccagcc aaccccaggg 7200
cccagcccgc agaccctgcg agggcaggtc tctgcccacc ggcccgccag gcgccctgga 7260
ggtgacgctc tgcttcccag agtctctgtt gcagccacga attggggtct gggtcgcagg 7320
aaagcacagg gctgaagccc agcgtcttgg ggccatttat actgaggcag tcagaggcaa 7380
agagtcccaa gaattcagaa aatacttctc aggaagccgc ctaattggct ttcatgggac 7440
aggtggagcc attaacctgg gatggttttg caggaaccaa agagctcagg gcccctcctc 7500
cccccaatat catgttcagg agacccagag tcgttggacc ctccctttcc tgactggtga 7560
tcatcagagt ttccagagtt gcagaaaatt ctccccccaa aaaaccaagt aagcgtcaat 7620
aagacttccc acaaactctc accagcccta ttttcccggg gggcaggtag actgtggggt 7680
ttgatttttt gagactcggg gaggacccct ggcagatgtg tgcctagcca gaacatttgg 7740
taaggactcc tccaatgaag aaaaagtgga ggaatccagc cccagcgaga agaggcttcc 7800
ccaccctgcc ctagacacac cggacagagg gcacactctg accagagcca cgtccagtgg 7860
ccaggaggcc agcccagcac ctcctccccc accattcccg tcctgggtgg gggggtaatt 7920
ttcctgggag cagctgtggg aactgtcgtt tctcatccca gcccagccag cactctgaag 7980
cctgcagggg aaggacagca cgtgggatgg acactgggga aggagcttcg caaggccagg 8040
gtgcaacctt caggccccag gtggcctggc aggccacgct gcctcggaga tgcttgccag 8100
actccccaag ttcactcagg gcctggcagc aacctgctgg ctgcctctgc gggggtctgg 8160
gttgctgagc acggtgcagc tgcccagggc caatcagccc tagggtgtcc gtgccaggct 8220
gcggcctccc cgcctccccc gcactgaggg tactcatggc gtgcaaatgc tcccgcaccc 8280
ccagagctgc cctatcggat gtttccagga attcacacat ttccataaaa atgcatttta 8340
aatgatggac aggcgagcct ggggtaacaa cgggtgtttg gtgggtagac aagagcaaat 8400
gggaaggagc ccgagggagg agggggaaga gaagaggaaa cagaacttcc agttggacat 8460
tctgacaaca gctggaagga aagtctagaa aagatgaaga gagaggaggg gagaaaccaa 8520
ctggggctcc cacccttgcc gttggattcc taattctcgt ttcaaatggg ccctgctctc 8580
cggcaaaatt agtttaaagg attttaaaac aaagaaaacg agatgaccgg tctgggagct 8640
cctcaatcag agtagagaag ttagaggggg gcgggcgact tggttttgaa gtcttagctg 8700
aacagtcacc cctcctctcc ttggcaaaaa ggattccttt agaacctccg aggctcctgg 8760
atttctccct tcgcaaatgg agccgcatac tgcattcccc cgctctttcg gatcgctaag 8820
catgtttcat gagggtcgct gtccccgggt ggaatgcggc cgtatgcacg cgcctccctg 8880
cacacgcaca cacacgcaca cttacaataa gtgtctgcag gaggagtgtc ctgcgcgcca 8940
gctctgcgtt taagacagga agctgccggg ttaccgagtc aaatgggagt gacactattc 9000
ctctccatca gcaaggaaag cggaccacaa aagtcccttt gtatctcggc agctcattta 9060
atattattta tgcattttgt gcaaggaatt gtgggatttc gccccacggt aaacaatatg 9120
gaaatcttaa aaatagcgat cttcctgtgc gtgtccacct acgcgccccg gggtgacctg 9180
gcggggctgt cgccgggtga ctcacacccc tgaaccgcga agcgacaggg aaagcgcggg 9240
cgagcgcagg agacgcggtc gggggtctct ccgggttcct gggctcccgc acccggagcg 9300
ggggacgcgg ccgctttaag ggaggagggg gcggcgggct gctcctgtca cccagcggcg 9360
gccggagcgt cacgtgggcg cgcggcgccg cggccattgg cccgaggcac gtgtccagga 9420
gaccggcctg cgacgtcact cgagggggct ctgttaaaaa taagaacaaa aatccagagt 9480
gaaagtgtct caggttgcgc cgagtggcct ggaaatttcc gagcccgcgc ggaggccgag 9540
gcggcgaggg cggcggacgg ccggggagcg cgggcggccc agcccggccc ggccgggccc 9600
tggcctcgcg tctctcaccc atgcgactcg ggccgcggag ctctgcgggg ctcggcgggg 9660
gcgcggccgc acgccggtgg ggcgccccgg cccgcagcgg ggcggcggcc gcgaggaggg 9720
ggcctccatg tgcgtgcggg cggtggcggg cgcgctgacc gcgggcgccc ggcaccctcg 9780
agggccggct agggcgtgcg ggcggggacg gccgggcggc ggcggcggcc ggagccggcc 9840
cgggcgggcg tgagcgccgg ggaacgcgct gcctgcatgc gcgcagctct cgccccgggc 9900
ggcccaggcg gcggcgccgg agcccgaggc ggccggacgc ggagaggagc ggggagcccg 9960
ggaggcggcc cgcgtccccg ccggaccact gcgactgtct agaccccggc tgcgcggcga 10020
agtcgaggac ttggctctgt tgaatctctc atcgtctggg cgagcggggc ggctcgtggt 10080
gtttctaacc cagttcgtgg attcaaaggt ggctccgcgc cgagcgcggc cggcgacttg 10140
taggacctca gccctggccg cggccgccgc gcacgccctc ggaagactcg gcggggtggg 10200
ggcgcggggg tctccgtgtg cgccgcggga gggccgaagg ctgatttgga aggcgtcccc 10260
cggagaacca gtgtgggatt tactgtgaac agcatggagg agaatgaccc caagcctggc 10320

gaagcagcgg cggcggtgga gggacagcgg cagccggaat ccagccccgg cggcggctcg 10380
ggcggcggcg gcggtagcag cccgggcgaa gcggacaccg ggcgccggcg ggctctgatg 10440
ctgcccgcgg tcctgcaggc gcccggcaac caccagcacc cgcaccgcat caccaacttc 10500
ttcatcgaca acatcctgcg gcccgagttc ggccggcgaa aggacgcggg gacctgctgt 10560
gcgggcgcgg gaggaggaag ggcggcggga gccggcggcg aaggcggcgc gagcggtgcg 10620
gagggaggcg gcggcgcggg cggctcggag cagctcttgg gctcgggctc ccgagagccc 10680
cggcagaacc cgccatgtgc gcccggcgcg ggcgggccgc tcccagccgc cggcagcgac 10740
tctccgggtg acggggaagg cggctccaag acgctctcgc tgcacggtgg cgccaagaaa 10800
ggcggcgacc ccggcggccc cctggacggg tcgctcaagg cccgcggctt gggcggcggc 10860
gacctgtcgg tgagctcgga ctcggacagc tcgcaagccg gcgccaacct gggcgcgcag 10920
cccatgctct ggccggcgtg ggtctactgt acgcgctact cggaccggcc ttcttcaggt 10980
gagcccgcgg ggaccacgcg tcccggctcg ccgcggggag gcccgcggag ctggggggcg 11040
gtgctggcgc gggaacttac cgggaggaaa acatctcgaa cctcccccgc gcacacgcac 11100
aaagactcac gcgacattgt gtgaagctga cgccggcccg ggcagcggcc aggagtccag 11160
cggcaggact gattcgctag ggggtacaga cttcttagga ccgcagaagg gaccttcttt 11220
ctttctctgt ctctctctct ccttcctctc tccctgtctc ccctctctgt cttccacccc 11280
gccttggcgc atctcttccc agcccctagc ccatgtctcc cccactgtag ttttttttgg 11340
tgggaacgtg gtggctggaa gatgggcccg gaagtgcaca ctctcatccc cttccctacg 11400
atcttccaac tcaggccagg ccggggacgc atgccccagc ccaccccaga cttgtcctac 11460
catccggtta tcccggctgt gctcggggaa gaaaaggcga ggccctttgc cgctctgcct 11520
tctgcccctc gggcctgcgc tgaccggtgg gactcaggag gatgcacaca gggaaggagg 11580
aaaataaagg cgccctttcc ccttggctcc actttgtttg ccagcgccag cccgcagtgg 11640
tggggctcag cccccttcct gcacacagcg aggacaaggg aggcagccgt ccctcccggc 11700
acctgccatc cccaaataga aaggaccttc tctcagggtt tcctgggggc tgctgatggg 11760
aaagaggcag cattcgcagg ggccctgcag agatgctgga tatatttttt catagatctg 11820
cgattttaaa aaactaagtc catgtccttg tagaaatcat caactgcatt ccatgcgggt 11880
ctgcggctgg gaaccgccat tagaagtgga ctgtttgacc ccgagctggc agcggatccc 11940
cgctgccccc aaaccctcaa ctattttgcg ggggtcattt gcccagatca cagcaggagt 12000
gagccaaccc ttgggccgcc atcccgcaga actatgcgtg catatttctg atgaaattca 12060
gatttctcag ctagatctga aatttgctcc attgttctcg tcttcctcct ttgttaatat 12120
ttaattaaca tacaggctca caatgccggg cgaggagact cggccgggct ttgtgcggcg 12180
cgggagttcg ctgagccagc ccccaacggc ccgggagctg ggcagcaccg cccggcccgg 12240
cctggcccgg cccagctcag cccagcccaa gtcgcctatc ttcatgggct ttaaaatatc 12300
cctgcaagat aatgtttctg ttctttggtt tctccgaaag aaaggggaga gagagttttc 12360
ttggggaggt ttgattctgt ttctgagact cctaagtatt tgtcttttga aagaaaatca 12420
agaaaaaaac ctaaaaatta ttattttagg gaaatttatt gccataaaat ggtgtccttt 12480
tgcgggctgc ttcatgagtg cattaacaag agccccagga ccagaagagt ttgggggtag 12540
agttctgggg aagggagtgg ttggaaaccc agacagagat gggctctggg agcaggaggc 12600
tggggcctct cctggagcct tgtgccccat cccccaccac cgttccggag ggccaacccc 12660
atctccaaat ctgcacctac ccctaccaaa gccaggccca ctggcctgga gccctgggcg 12720
tgagcaagac aggcactgag tgtgtacgtg tgcatggggt gggtgcccaa gtatagggtg 12780
tgtgctctca tgggtggtga gcctgcctat gggttgcttt aaaagctgcc cttggtgctc 12840
ctgaggtggt gcccacagat cctctttctc caggcctgtc tcctggagag agcacaagac 12900
tcacttggcc atgagggagt gcttggcatt caccctgggc ctccagttcg tcccccacct 12960
cttgctgggc acagccccag caccctagtt gactctcctg acctgggcag ggtgcagtcc 13020
cagggcctcc aaggagatcc acattcctct tctcctcagt gtgcccggca gctctccggc 13080
cctgaagggt ggggggcccc cagccttctc cagccacagg gacctgtgat gaagctgggg 13140
ccagatgctc cctaaagccg attcatacac cgcacaaatt gaaacccaga ggcgaggtca 13200
ccactccctg ccagtggcct tgcccccttc ttcccccaca gggaacgcca gggggttgag 13260
cctcttatca ccaaaaagaa actgatgaca cttccctcct tctgctctcc tccctctgcc 13320
ctttccccat ggatagcagg tcctagaagc cttacagcga ccctgtccaa aacctggggc 13380
aggtccacag ggagaaggcc aggtcaggtt cataagtctg aatcccagtt gggaggcaca 13440
gtggggaggg tcagaagtgg acctggacaa ggtcagctgg gctaccctgc tgcccacagt 13500
gaagcagtcc catgtctggg gaaagggtgg tgcagtcaac acccttgtag agccaggtcc 13560
cctctcctgg acaggaaacc tgggagattt ccagtgggtg aaggactcac ccactgtgag 13620
tagctcagtg cccctcccca ccaaggaggg aagtacatgc actgactctt ccttaaagga 13680
atgaacttgg gtccacagag cccttggctg ggagtcatag aggagcttgg gtggaggcag 13740
acactctggg cccctcctgt cctcagggcc cacctgcccc tgattcccac agcccttggc 13800
accctgtggg gtatccccat gagggtcccc atcatagccc tcagggcgcc tcctgcttct 13860
gtgaccgccc tgcagccttt cccagtcttc tctcctcccc tctctctcag cacctaccgc 13920
catccctgtt cctgaacaga gagcctcaga aaggactagg aaaaaccagg ccagaaagtg 13980
tggggagttt tgcttacatc caggagtttt acttttatct agagacccct catttgtggt 14040
cagcctgccc actaggcctg ccccatagtc ccacttacat cacacgcagt cctccctcac 14100

caagtggtgg aggtccgcgt tgagcccatg cccagtccga agtctagccc cacatctggc 14160
ggttcagcct cgagtggcct tgggcgagtc acttccctct tggggcccca gaatccttac 14220
ccggtgccta ttgggtggca cacctctggg taacctgact tctctctgtc caccgtatct 14280
acccaggtcc caggtctcga aaaccaaaga agaagaaccc gaacaaagag gacaagcggc 14340
cgcgcacggc ctttaccgcc gagcagctgc agaggctcaa ggccgagttc cagaccaaca 14400
ggtacctgac ggagcagcgg cgccagagcc tggcgcagga gctgagcctc aacgagtcac 14460
agatcaagat ttggttccag aacaagcgcg ccaagatcaa gaaggccacg ggcaacaaga 14520
acacgctggc cgtgcacctc atggcacagg gcttgtacaa ccactccacc acagccaagg 14580
agggcaagtc ggacagcgag tagggcgggg ggcatggagg ccaggtctca gtccgcgcta 14640
aacaatgcaa taatttaaaa tcataaaggg ccagtgtata aagattatac cagcattaat 14700
agtgaaaata ttgtgtatta gctaaggttc tgaaatattc tatgtatata tcatttacag 14760
gtggtataaa atccaaaata tctgactata aaatattttt ttgagttttt tgtgtttatg 14820
agattatgct aatttatggg gtttttttct tttttgcgaa gggggctgct tagggtttca 14880
ccttttttta atcccctaag ctccattata tgacattgga cactttttta ttattccaaa 14940
agaagaaaaa attaaaacaa cttgctgaag tccaaagatt ttttattgct gcatttcaca 15000
caactgtgaa ccgaataaat agctcctatt tggtctatga cttctgccac tttgtttgtg 15060
ttggcttggt gaggacagca ggaggggccc acacctcaag cctggaccag ccacctcaag 15120
gccttgggga gcttagggga cctggtggga gagaggggac ttccagggtc cttgggccag 15180
ttctgggatt tggccctggg aagcagccca gcgtacccca ggcctgctct gggaagtcgg 15240
ctccatgctc accagcagcc gcccaggccc gcagcctcac ccggctccct ctcctcaccc 15300
tcctgcacct aactccctcc tccttctcct ttttcctcct cttcctcctt cctccttcct 15360
cctgctcctc ctttcttctt ctttttcttc tcctcctcct ccttccttcc tcctcctcct 15420
tctctttcct cctcctcctc accaagggcc caaccgtgtg catacatcgt ctgcgtctgt 15480
ggtctgtgtc gctgtcccca gtcccaccgc agtcctgccg caggcctaac cctcctgccc 15540
tgggcactgc ctccatgcag aagcgcttcg aggttctggg gctaaaggcc tggggtgtgt 15600
ggcctaaagc ccaagagcgg tggggcgacc ctccttttgg cttggcccca ggaatttcct 15660
gtgactccac cagccatcat gggtgccagc caggtcccca gaaatgaggc catggctcac 15720
tgtttctggg cgggcagaag gctctgtaga gggagatggc atcatctatc ttcctttcct 15780
ttttcttttc ttccctattt ttttcttttt ttcctttatt tttttctttt cttggagtgg 15840
ctgcttctgc tatagagaac attcttccaa gataaatatg tgtgtttaca catatgtctg 15900
catgcatgtg aacacacaca cacacacaca cacaccaggc gtgtttgagt ccacagttct 15960
gaaacatgtg gctaccttgt ctttcaaaag aactcagaat cctccaggat ctagaagaag 16020
gaagaaagtg tgtaaataat catttcttat catcactttt tgtcttttct tgttttttaa 16080
aatatacatt ttatttttga aggtgtggta cagtgtaaat taaatatatt caatatattt 16140
cccaccaagt acctatatat gtatataaac aaacacatta tctatatata acgccac 16197

<210> 4
<211> 2609
<212> DNA
<213> Homo Sapiens

<400> 4

cgtagattag agatgactat agattttccc cagcgcggat caaagggact gaattgaacg 60
ttttagctta atgatccaac ccctgtcaca cccacaggga cgcgaattcc gattttataa 120
gcaggtgtgc acgtgcactc agatactcac acaaagccgc gtggagggac gaaaagatca 180
accacccgat cgacgaggat aggtttgatc ttttcgatta cctcagtgtg ccagtgtata 240
ttcccggctg ggcctagcgc cctaagaaac ttcggaactt tagctgttaa tttttgtttt 300
cttactatga cttcccaaag acattctatc tgcctaccgg ggcgaagaga aatgggacca 360
ggcgtcaggg cggtgggacc ctgtccaggg tcctgactcc gcgccagggc tccagaccag 420
tcggcctccg aaggcccttt cacccacccc acacaagagg aaacaaagac ttctcagctc 480
aaggcctcag ggctgcctcc tgactccggt cagtttgcag gaagaggaaa caacaaaaca 540
aaggaaccgc caatccgccg ggtattatat ccctcagctc caacctccga cttcggaccg 600
ccagggtcac cttctctacg ctgaccccgc ttttcttaaa tgaaaacacg ccaacaaaag 660
catacttcgg atacaaaatc caagtacgca tcttttttgg ggaggttaga gctgaggtgt 720
acttcggaag atgagaattt tgttttcatg aattgggtaa cacccaggca ttgttaggta 780
ccccgacaga ccctctagat atccttctct tcctccttca cactttcttc ctatcaaaat 840
agttattgtt ttgaaattca ctagaacaac gacgttctaa aaacaaaggc gcagcaagca 900
tccctttctt cgctgccgcg ggctgaacca cggacgctcg cgggtcgccc agccccgacg 960
gcccgcaggg ggcgcgcgcc gcagccgcag cacagcccgg ctacccccag aaagggagcc 1020
gaatggaggg aagcagggag cgcggagggc tcgaggcttg cagataagga gaggcgcatc 1080
ctgggatttg ggtcctctgc tgctacaaca caaccgtgct attgttggca ctgtccgacc 1140
caagtgtcgg tggtaagcgg cgatgtcggg gctgggtctc tagcaaccgc tgtgccctgg 1200

gtcaggctgg tcgccccagc taccgggatc cctctccgga tgcttccagg gcgataggtg 1260
ctgcacccat tgacgggata gccgcatacc tccgaacagg tagtggagtt cgtctctggc 1320
aggcatccta gctgcgctga caccaaggtc gccacacaat agccatcagc cccccttaag 1380
ccccagaagt aggtttcccc tgccccagcc atagcggttc cagtcgtcgc cacgagccca 1440
cttcttatcc ccaagcgcac ctccctctcc tcacccgggt ttatgcccgt tacacagaga 1500
gaactacaca gggggaacta tggtcctaca ccctcgaggg gacagacacc ggccgtgaga 1560
caggcaccac gcagagcctt ctggtgactg tccgcaggag cgagaccttt tttggttttg 1620
cagtcggcta ggtgtgtgtg tgtgagggtc cccagttgac taccgggatg cactgccact 1680
tttcggcctg gcgggctctg ggactccttg gtctccgtag gaggccatcc taggccttcg 1740
gaggaggcgc tcccagctgg cggcgcccct cgccccgggc tcagaggcgg acacggtcgg 1800
tcgcgccctg ctggcccttt gttcgcgccg cagcgggctg ggagcagctg cgcgacacca 1860
gacccacagc gccaagacgc gaagcgcgag gaaaccgctg cgcttgactc cttctccccc 1920
aactcttgga cccaggaacg cttccagctc ctgcgtccca cacgcccagt cctggcttct 1980
ctcccggccc agaagtcttc aaggattgaa gggctctgcc tagggccccg cactcctgcc 2040
tgactcccat ggcccagaaa agcaggggga catttgaaat gtcaccccgg gataaatatt 2100
aacaaaaaag caaatggact tgtgcagggg ccagttatca atcaaccagg ccgcaaggcc 2160
actcagggca aacacagccc aggttgggct gggcgagact ttctcatggc cgccccccga 2220
ccgaacccct ccccccttgg ctcaggtcct tcttaggtcg ttctggggca aacaccggac 2280
gggaaggggg cgccgccaac tcccccgcgg ggtttggagg tttcctcgcc tccaagtccc 2340
gcagggcagg gtcggagtgc ccaacaccca cccccgcccg aacctcggcc ctgcgcgccg 2400
ccttcccctg ggtccgcggt gctgcgcttg ctgttgggtg tgtgtcgctg tctctttccg 2460
agccggcagc tcctgctgtg tggccgaagc cctctggaat ctttaattgg aaactaatct 2520
tggtcttgat agacgcccac gtcagaggcg cgccacccac ccacaccccc cgcctcatcc 2580
cggaggagac gcggcgagaa ccctgtcgc 2609

<210> 5
<211> 11667
<212> DNA
<213> Homo Sapiens

<400> 5

cgggacccgt gggcgccaat caatgctatg gtggcggaga gtaaagggga cgaacacagc 60
ctggggccat ccccggagtc ccccagcgtc cgcctgtggc cgtgcctggt tggccgcgga 120
tcccggcggg cgccgcaaag cggcgggatt gccagcgcag agctccggct ctctgccttg 180
tccctgggtc cgagcaccgg agcctctggt gtctgcgggg agaagtctcg gattgagaaa 240
tacgggaggg tctcgtcagt ggctgcaggt gcggcagcca ctctggggac ccagtgagaa 300
tggggtcgcc tggctctgcg cgaacccctc acgtgggtgc agctcctgag ccgccgaggg 360
aggcggtggt aatgtcgccc agtgccagca gagggcagcc tcgaggccgt gagcccgaac 420
ggcgacctcg ccaaaccgcg ggctcccttc cagctcccgg attctgcggg gtagaggcgg 480
ccttggagcc cagagaccag cgacctcagt ctgcaggagc ccggcgcaga ggcccaaggg 540
cacccccggg atgtggtcaa gctacagccc ttaggcagcc tcactctgcg acggcaaggg 600
cttagagggt ggagggggac cagatgcctt aggaggggtt agaaagccaa cgcacacagg 660
gaatttgttt tcaagcaaca gattccaagc atgtggaaac cttttaaatg atgctggtga 720
gagctatgat agttttcgca ttcttggcga gggaagtcgg aggctagtag cgggatggct 780
cctgggcgcg cgggagacag agggaccagc gatccttgct gggggcagag ggactgtgga 840
ccaaggatcc agacacactt aattggggat tccagtcccg actgtggcca ctcaactggc 900
gttgaacttg agcaactcac tataccgctc tagcctccga ctaatcactt ctgtaaaatg 960
ggcattgtgg gctccgagtc tcgtaagggc gctagggatt cgagacagca ggaatttcct 1020
cactgctcca gcaaccctgg gttcctgggt ttgtaggtgg gctgaaagga tcctttcatc 1080
gcacgtttgc ctggcgtggc tgctttagag accgaggccc gccaggcttt gcaacccagt 1140
tgcgcgtggt cagccatggc cttggaaccg ccgggatcgc ctcagcggca ctccggccag 1200
ctcccgactt cccgccctgg gcactagggt cacccagctc cagaagatag tgcttatcag 1260
cacaggaatt gagaccccac acccggcgca gtgggtctcc caggaagcct ggcgaagagc 1320
caaggcccgc cggacctgag gtcgcctggg gcgctaaaca gacccatcta tgagcacatt 1380
cggccaacac acccaagttc aacagtgggc gggatctttg gctggcctcg atctctctca 1440
cagtgtgtgc aaacgggtac ccattagatt ctttttgctt gtttcctcat ctgtaaactt 1500
tgaaaggaga gtatttcccg gggtgattct gaaaatcatg ccagaactga aaaggcctgt 1560
gtaaatgcga ggtgtaatga tgacttatta gaagcagcca gccctgtacg tgcctgtagg 1620
atagatgcta gaacgttcta caaatgtgtg cacatacaca tcacgtatgt gtgaaggtgc 1680
acacctgtcc ctagacactt gacaaatgca cacacgtatg cgtgtacctc tggctgagag 1740
caaaggggct aatacagggc tcatacagga catcatcctg cagtcttgtg cgtgcacagt 1800
acacacgcga gcacgactgt ctgtgcctgg agacaccgta tggcaccgga ggagctgctc 1860

agccgtcggt gccacctggg aagtagggtt tcgggtgctg ctctggagcg cgggaaagtc 1920
cggatccggg tctgctggtc agcgcttcgg cgccgccggg ctcctttatc tctcatccgc 1980
cggccggtga gcggctccag tttcaagacc cccgatcctg ccggtgaggg gagggagcga 2040
gaaggagtgc gctccgggcc caagaggctg cagggcccca ctccccaggt ctgtcgtcct 2100
tcctttatct cctcaggtca cacccccact taaaataaag aagggtgcct gactcattca 2160
ggtcagggaa gtcggccccc gggagcctcc tgccctcaca agccatggtg aagggaggcg 2220
aagccagggt ttgccacggc ctgggagaaa atgcggctgc agggtctctt tgggctgcag 2280
cgctggcccg gggccccaag actgttaagg tgtgtgtggg aggcgcgcag tgtggctacc 2340
gaagagccct ccatcaccgc accggcaggc cgccgggctc gtcctcctct catgcttcca 2400
agggttctga gctgcgcagc actcgcatca cccagaagtg tctgtggaga aaacgacctc 2460
aggtgtaagc ccaaggctgc tgcctgcaaa ggcaatgccg tggagactgg gttccacagc 2520
gacctgggtt tttaagtcac cgaaagctac agggcagggt cacaaacact ctcccgcctt 2580
ctctgcagaa ccgtgcggct gacaggagag cgttgcgcag aaaattcgag gccgggcgct 2640
ggaggagtct ccgcggcccg gagaaggcac agaggcgccc ctgagaggcg cagctggaac 2700
aggcgatgca cgggttcgga tctggccggc caaccgagcc cagcggtcgc gaaaaccaga 2760
gtcgccacag aggttgagca cgattccatt tctggggatc gggtccggtg gggagccact 2820
gtccctaacg cctccagaga ctttaagtag gacaatataa tgctggtagg agcaaggtgc 2880
aaggaattta gcggcagaag cctttcgggg gggtggggaa aggcagatcc gtgcctcgtc 2940
tgagcctggg ggtggggaca gcccctggcc ttgtagcccc tgttccgggg atcagctggg 3000
cccacctaac ccccctacat ggggttgaaa ggggccaatg ggacggcccc gccgcccctc 3060
cctcgggatt ccacaggccc ctgacccggc ctttatctct gcacggtcag cagtcgcggg 3120
ggcttcagga aggaggacaa aggcccggtg tcaccgcggg cgggggctcg gtttcctggc 3180
tctctgctca cactcacagc ctttagcggt tgttggggga agatttttaa aaatatgtgt 3240
cgaatttcct ttttttctct tctagaaaca aacaaacaaa aaaggcaaaa ggcgaattcc 3300
ccttcactcc tcgtccatag agattaaagt ttcctgggat cctgcccttt tttttctttt 3360
gactgcctag aaatacttgt tctcccttgt acatagagga aaatgcgggg aaaggttttt 3420
taaaactcgg tttcatacta ttattattac taaggacaac cgggcaggct gaggtcccaa 3480
cgtggatgat ccgagttggc ctcgcgccgg ggctctgcag ccactgccct gtgcgctcag 3540
cacctctggg ggcgatcagg gcccctgcgc ttccgcccgc cgcccggcag tcgagagcac 3600
cctgtgccca gactggccga ctcattctcc cccgaatttt gtttagagct ggcaaggggg 3660
acttagctcg cgccccaaga cctgggcttg cagcgccgcc aacaggcccg gggacacgag 3720
gcgctccagg ccggggtctt cccggctgct ggcccctctc gctccccacc cgctggcggc 3780
gcctcggtcg cccgcaattg acccaacccg cttcctgcgt ttgcccctca ggtttcccgt 3840
ttctccacaa aggcctaggg gagcctcgcc cacaggctga ccctgcaacc tctggcccgg 3900
tggctacctt ctgcttttct gaaaaagaaa aggaaaaaaa aaaaaaaaaa gaaaaaatca 3960
accagtcgag ggaggcgcgt gaggactgga ggcgccagcc ggacagccta tgagtaggtc 4020
cctgggtcgg tgccgcttcg cgggtcagca cggcctttct cagagaaaac ctcctaaacg 4080
tgtgaagacc gctttggggg aagcgagagg gaggttggag gagccccggg cggggtctca 4140
gcgcccacca gctgtgcctt cagggcttgg gtgttcgctg caacggcaac cgcgtgagcc 4200
tcactcccac ggccaagggg ctagggcagg gtggatgcaa tcgcgtgcgc ctggccccgg 4260
aaggtgctcg cagggggtgc ctgtggccag ctgggttagg aagtcaaatc ctagaaagtt 4320
attactaaag gtttttttttc cccttccttt tcttctttcc tttttgcttt ccttccttcc 4380
tttttttttt ttaatagggg aagggaaaaa acattattga acatctacta taaactgggc 4440
acttaaaaat tagaaaattt aattatttta ataattctga tacacgcacg tatttctgtt 4500
ttatagagga gaacactgaa atttgaatgg gtattttgcc caaggttaca tagctgttaa 4560
agaggacacc aggctccaac ccagttcttc ctgactaaac aggccaggtc tttgcctcat 4620
gctgcacagg ccacctccgc gaagtgacaa gatcccagaa catgcttttc acagctcagc 4680
accaagcctg tgcggcaaac agcctgtgca gcagcagttt ctgtggtaca gaaacaaaca 4740
aaaaaggcaa aaggcacagc tcagactgtg cttccttctt ctcctgggag gctctccctt 4800
actactcctg ggaggctctc ccttactacg cagctccctt gagcatcagt ggcagctagg 4860
ctcagctgct gtcaagtctc ctttaaaaat ttcggaggcc atttgcctca cagatacctg 4920
gaagacctct ggttggtctc ttctcttctt aggacctgag attcctcaaa tgtaatgtaa 4980
ttttgggttc ctagacccaa gacactcttt ttatgacagc tgggagtcct ctctccagaa 5040
agtggaaagt tgctaagact gctcggtttt aagaatcctt aattcctggt tgtcccgaag 5100
gcatggggct cttagtccca tcctttcccc atcttttcta tgggtcccac agtctactcc 5160
aagaagtagg ggctgccaca cagtggtggt aggcctgacg ggcagggccc cagggtcacc 5220
accaggttag atctgcagaa tggggacact gcagggatgc tgagcctgat caagatgttt 5280
cctggcctta gacccccacc cagaagccag gagtctggtc cggtgatcct gtccatgaag 5340
cctggcccag accttcataa agggaagagc tcagttgaga gcaaactatc ttgggccaga 5400
ctcagattcc tggcccatat tctgcttgtc tggctgcatg aagcctgggt ctggccccaa 5460
tttccacccg ccgccagggg gctccttgca gccccatctc cagccttggc ttccaggaaa 5520
ccctggcaaa ggctggatgc acctaacctc ggatgaaagg tccagggcct cttgctcatc 5580
taagcctcag atctggagca gtgaactatt tactgatgca agctggaaac aggacgtcaa 5640

taccctctgg agtagggtac aggaccatct ccagaactcc gtaggtccct ttcccacagt 5700
cccttggtgc atgtacttca atggcgaaga tttctccagg tccctcttga gaacaggaca 5760
cctccccctg ctttctccaa accaacccac ttactctgac tgggcaattt tagtgaggtg 5820
gaggtgactc taagaatttc tgtgtaaatg tgaggagagg gctcatactg tcagcagtgt 5880
gtgagatgtt tacacccacc acacacataa tcttcatgca agaacgactc attacccagg 5940
acattcagaa attggctggg tatttgtatt aaactctatt tacattgtat ttacacactg 6000
gccaatatat atacacacac tcacatggcc cagccatcta ggccagactg tatatgctaa 6060
ggcataattg ctatataact caaatacttt tgccctcagg aattgcttaa atcagcttct 6120
ataatagaaa ctcaaaagtg cgaagaggga gagaagagaa aggaaatgag agatgaagaa 6180
agcctgccat tgaactaaac tttcggccct ggggaaaatg accaaactta agagctttgg 6240
agaaatcccg agggctggag ttagacagat ttttcctgtg ggcatccatg gggaagtggg 6300
gtatgatttg gctcccttct ctcacctcct tcaatccctg tgtagaccca ggaaaccctc 6360
actgattgct tcattttgtc ttccttactt ggtgaggctg ctgctctctt cccagccttg 6420
ggtgcctttt gcctttcagt tctcggggag gagacaggct ggtccttcat tgctggcccc 6480
ggccaacttc tccgtctcac tggaggtggg ctgctacctg cccaccaggg cttggtcctg 6540
gaagcttccc tcccttgctt tctctgcttg agacggcagt tgggggagtg ctgacagcca 6600
gcttcagaac agggagcctc tgcctgatga agcaaaggct ctgtccatat tactttattt 6660
ttcagcaagc caggaataca cacccacaaa tgcacacatc accaatgagg tatgaatgcc 6720
acacacactg tggtcccagt aagcacttcc taagtgttag ccatatgatt attacaatgc 6780
caaaaatata tgcccactct gcaggcgaat gacagacaca cacacctccc accctgtcac 6840
caatacacaa ctgacactgt acacacacat ttgcagcaga tacagactac acaattactt 6900
gcccatgggc taaattttca cgctagacaa agcacaaaca gacatgcaca cagcaggctg 6960
ctttctgttc tgcaactccc cttagccaga tagtggagca gccgggcacc aaaggcaccc 7020
agacaccagg cctgaccagc ctctagggct tcctgctcca gctgagcctc cctctccatc 7080
ctgctcctgc cggttcctgg taagctccag tcccagtctt gaggtgatgc cccctgagaa 7140
tccccgtggc ttggtctcct gccttgtagc ctctcgcagg atgcctgtgg ggggcggggg 7200
gcattctgag cagaagccac ttgggcccaa ctatacataa caggatacca ccctgaggtt 7260
cctaccgcct gaccactgga gggctgaagt gagaggcctg caggctcatt cccgtggcgc 7320
aaaaccgctc ctggcccacc agccggcctg gacctgccgc cacattgctg gccaggcccc 7380
gagatagacg gggggggggc agtgagggct gggtgcgggg tctagtctca gctgagcagc 7440
agcccgcaag aaccggcgga tctatcctat cagaagtcgg aagtcatcta tcccatcaga 7500
agagtgaaag ccgaccaccc cctaatccgc ctgggagaac cagactgcaa ccaggtcaaa 7560
cgcctgtgtg cctgcgcgga agcatggatc ttaattcgtt atttggggat ctcagtaaag 7620
gaagagagag aaagaaaaaa gactaaattt ctgcagccgc gaagtatgtt caatccttac 7680
tgaaacctcc aaatctccta cccatatctc aaggtaaatg aattctgact attaaaaata 7740
ataaagcagt cacttctcac atttgtacag aggttagcag tttgcagcgc atttccacaa 7800
ccagaatttt atctattaat gtttggaaca aactcttggc tgttatagtt gactcaaaat 7860
cgaaaagttt gagaacaata ataaaaacac agccagccca tacacctagt acaactgtgt 7920
atgaggtaga atacaaactt gaataggtac aaaaataaac caagtcctac ctacagtcgc 7980
gccttgtgaa cgaaccacgg cagaaaaagt ctttctgctg acgttaaaca gccgcaacgg 8040
gccagtgatg acacagaaaa caaacaatcc caggaaaaca aagcagcagc gacagccaca 8100
aacgcctgcc accccggttg ctctcaatct ttgtttaact cattctgaaa aagataaaat 8160
tttgctgaaa atcttcctct tttaactagc tgctgaacta aggagcgaca gaaataaaga 8220
agtccgtgtt aaaagaagga acacaaaatt tattcgggaa tcgacaaggc aaaaaacaaa 8280
aacaaaacaa aaaaacacaa caaaagtgct ctagttctct ctagaaaatt tggtccccca 8340
aacaacaagc tacagtacaa ccaggtctat ggttttcggg ccgggccggc tccgggccac 8400
aagaccgcct aggtcggccg ctgccgggtt tcacatttct ccttcccaag gtgggagaag 8460
caggaggttt gaaaaacaaa aagcagggag gacgagccgt cccagcagcg tgggccaggc 8520
aggcagtgat ctccctgcct ccaggacttg catcgaaaga tccggggaca tttgtttttg 8580
atttttttct tcagataggg agagggtgaa acttccccaa ttggtagacg aaaccatctt 8640
ttattggata tattctccta actctgaaca cagctttttg aaggcccaac aaaaactcta 8700
acgcaaatct tccagaagag aagatgagga atactaaagc ttttgatttt catatttgga 8760
tttagcaaac tccaaggcca caatacttcc ccggttgccc ggtgaatttt ttttcctttt 8820
ttttttttta aatactctct taaaaagttc caccgatctt tcaaacaaaa cattagccag 8880
ccgccggggc ccggaggtgc ccgaagcgac gggactgata gcggaggaaa cgcagcctcc 8940
ctccgcgccc gcccgcggtg taaaccgagt acaggccgtg gagccaggct gccccggctc 9000
ccgctgggtc ccaacccccg ccccgcctag tgggccccgc ggcaagcggc ttctgaacag 9060
cttcaagagg gttcgcggag caaacacacg tattggtccg tccctttctc gggcagcgcg 9120
gtcccgccat cagtcctgtc cggcgcgtct agccatggac tgcacggcag tcgggcgggg 9180
aacgcggaga gcgagcgcac cgacctgtga gagaaggcca agaggtctgc gctgccgacg 9240
cccggtcgca cctccgcccc gggccctttc cgcggtgaat ttgggcagga gacgctgggg 9300
ctccggaaag agacgagccc agtagaaagc gcgcagagag gcagcttcag gccaggggag 9360
tgcaaggtca cagaggtcag ggaggtgagc acaggaggac ataaactgag gggacaaaga 9420

ggagcgacag gagcttagga aagcgaaaaa gcacagaggg accctgggcg ctggctccag 9480
aggcgggccc agagggtgtg aggtcaggct ggcggcggcg tcgtcggctg cgaccggggc 9540
cggcgtcgcg cgtccctgca tcctcgcatc cgtctgcacc ggcatgcggt gggctctcag 9600
agatcgaggc gcgaatgcag cgcgccggtc ttgctgtcgt ggtcccagta agagcaatgc 9660
atcatggcga gctcgggctg ccgggcacaa gcgaactgca ggcccggcgc actggtgggc 9720
gcgggcgccg ggggcgcggc ggtggctggg ctggcagggc tgagctggcc cggcggcggc 9780
gcggcggccc cgtggtgcgg tggggcaggc ggcggtgcgg cggccgcgtg cagatggtgt 9840
gcgtgcggat gcgggtgggg gtgcggcgga ggcgggggtg cggccggcgg gcctcccagg 9900
ccattgtacg agttcactac gccggggggc agcgccatgc tctgcacgcg tgtgtacggc 9960
ccgtacgagg cggccgggcc cgccagcccc ttgaccacag cggccgcgcc agggctaccg 10020
gggcccgcgg ctgcagccgc agctgctgca gccgctgcgg ctgccgccat ctggcaggag 10080
gcatagggca tgggtgaggg aggctgcggt agcggccacg agttgttgag gaagccagac 10140
tgcaggtact tggggggcgc caggtagccg tagccgtcgg ccccggcgcc cgccacgccg 10200
cacccgcctg cggcgcctcc ggccccgaag agccccttgc cgggctggaa gtgcgcgggc 10260
ggcggccgga agggcctctt catgcggcgg cggcgccggt agttgccctt ctcgaacatg 10320
tcttcgcagg ccgggtccag cgtccagtag ttgcccttgc gctcgccgcc gccctcgcgc 10380
ggcaccttga tgaagcactc gttgaggctg aggttgtggc ggatgctatt ttgccagccc 10440
ttcttattct tctcgtagaa cgggaacttc gcgatgatgt actggtagat gccggacagc 10500
gtgagcctct tctccgcgct ctcgcggatc gccatggcga tgagcgccac gtacgagtac 10560
gggggcttct gcgccgggtc cggcttctcc ggggctgtcc cgccgccacc cccaccgccc 10620
ttgcctgggc tcggcggcgg cccttctggc tccttgactg tgcgaccggt ctctggggcc 10680
agcagggccc ccgccgcgtc ctcgggctcg gggtagctgg ccatcatgac aaagccggcg 10740
cgccgcggcc gggccgcctc tgctctccgc tccaggcgct ggcgcggcaa agagttgggg 10800
cgcacgagtc cgcttacggc caagtctcaa acttctggag actgcggatg ccgcccgcgc 10860
ttgcttgctg gaggcctgtc gctgctctcc cctctccttc cccttcccct agggagcggc 10920
cggcgggagt ggagctcagc ctctggccat ggggagtccg cccaacagag aggggctccg 10980
gcctcgccgc ccctccccgc tcaggccagt ccccgccttg gtgggttttc ttttctgcgc 11040
tcttcccctc cccccgcccc ccggtttccc gaagcacgac ccgcgtctct ggcggagctg 11100
cctcctggag tccctagtgc gccaggagcc tcgctctgtt ctgattcgta tgggctccac 11160
cgagttccgc ttgcgtcagg cgccttcgcc cctatagcgg ggcggccagc cgcgcacggg 11220
cgagttcatc tccaagtcac tttttgtaaa cgccccgcac agcctggacc ggcctgcccc 11280
cgcccagcga gcctcagggg cccagccgac agccaggctc acgcgccctt gaaatctgcc 11340
ggtactcgct ctgcgggctg ggctgggaga tgacgaggac cccggtgggg tctgcccgca 11400
cccggccaaa gcccaggaag ctcggccccc agcgaggaaa ggcgctccaa gcctcctcgc 11460
ggctttcagg tgaaagaaaa cgactccttt gctctgccgt ttgctgccgt cttgaggctg 11520
aacttctagc tcggggctgg ggaggggcga gacggcgagg gggctggacg gggtagggtg 11580
gggagagctg ctctgaggct ttgggaaagt cagcccagaa acgggtgtga ctgtacgaag 11640
aagcctcggc ctggcctgtc cctcgcg 11667

<210> 6
<211> 1394
<212> DNA
<213> Homo Sapiens

<400> 6

cggaaccccg gtccgaagcc gagacaggag actggatgcg aggccctccc agagctggtt 60
tctctcaaac aacttccaaa actcctagat cctaggggta cgccgaaatc ccccaaagca 120
gtccaaagaa cacaacgaga gtcctaacat cccaggtggc ggcgcgctgg ctccctggag 180
cggggcggga cgcggccgcg cggactcacg tgcacaaccg cgcgggacgg ggccacgcgg 240
actcacgtgc acaaccgcgg gaccccagcg ccagcgggac cccagcgcca gcgggacccc 300
agcgccagcg ggaccccagc gccagcggga ccccagcgcc agcgggaccc cagcgccagc 360
gggaccccag cgccagcggg tctgtggccc agtggagcga gtggagcgct ggcgacctga 420
gcggagactg cgccctggac gccccagcct agacgtcaag ttacagcccg cgcagcagca 480
gcaaagggga aggggcagga gccgggcaca gttggatccg gaggtcgtga cccaggggaa 540
agcgtgggcg gtcgacccag ggcagctgcg gcggcgaggc aggtgggctc cttgctccct 600
ggagccgccc ctccccacac ctgccctcgg cgcccccagc agttttcacc ttggccctcc 660
gcggtcactg cgggattcgg cgttgccgcc agcccagtgg ggagtgaatt agcgccctcc 720
ttcgtcctcg gcccttccga cggcacgagg aactcctgtc ctgccccaca gaccttcggc 780
ctccgccgag tgcggtactg gagcctgccc cgccaggccc ctggaatcag agaaagtcgc 840
tctttggcca cctgaagcgt cggatcccta cagtgcctcc cagcctgggc gggagcggcg 900
gctgcgtcgc tgaaggttgg ggtccttggt gcgaaaggga ggcagctgca gcctcagccc 960
caccccagaa gcggccttcg catcgctgcg gtgggcgttc tcgggcttcg acttcgccag 1020

cgccgcgggg cagaggcacc tggagctcgc agggcccaga cctgggttgg aaaagcttcg 1080
ctgactgcag gcaagcgtcc gggaggggcg gccaggcgaa gccccggcgc tttaccacac 1140
acttccgggt cccatgccag ttgcatccgc ggtattgggc aggaaatggc agggctgagg 1200
ccgaccctag gagtataagg gagccctcca tttcctgccc acatttgtca cctccagttt 1260
tgcaacctat cccagacaca cagaaagcaa gcaggactgg tggggagacg gagcttaaca 1320
ggaatatttt ccagcagtga gcaggggctg tatgggacgc gggaggagct cagaggaggc 1380
gcggagagtg cccg 1394

<210> 7
<211> 6357
<212> DNA
<213> Homo Sapiens

<400> 7

gtgtggagat tgggaaggtg acaaggtgaa ggcaattgaa ggaagagccg agggggacat 60
ggggaaggat tttgtttcac ccctcctaag ttgaaccatt gtcctttgaa ggccggctcc 120
tggagaaatt aaagggcccc tgtgtgacac agccatgtca tacataaaca gaactctgaa 180
gcctatcaac tcctgaggct aagtaagagg gaatgtaggg gccaaggcag aagagaaacc 240
aaaacctcag agcgctgagc aaagatgcca atcagagaaa gagaaattca tttgcgatgt 300
taattaacaa gcggctaatt aaaacggcac tttgagtgct aatcaatcgc cttattaagt 360
tacagccatc actggaacaa attgaaacct ccccgccccg ttttctgcct ttggtgcagg 420
cggggccgcg ttcccagata ccgtgagagg ccttggggcg cggaggttgg gggcagcctc 480
ggtcagcttt ctcagtctct cccaggtcta cagaatacgc cactggacaa gtgcctaagc 540
agcgacttct ggtccagaca caccgcccgg ggagtaagta gttgcgtcga agaacaactc 600
attcagcagc agttaacacc gacgtttcct cctagaaaga gctcccgcaa agcgggggga 660
tgtgacctgt gggcccccag caggggtagg aggcagttca gcccgagagg gggcgctcta 720
gggcctggat cctgcatccc tatttcctgg aacacaccca acgcctcatt ctgaaaaccc 780
tgcttaggcc ctggccctgg tgccgctcag caaccaggaa agagctggac ctgccttcag 840
gcagcaagaa caggactgcc agcctcctgt ggctctgtct cccgaggctc catgagaagg 900
ggatgggggt gcaagaaggg aagagtgagg tggtgtgctg ggcgtcgggg acgaggacgc 960
acgccagcca agacgtgcct cccacccagc ccacgcgcgc ttccccaccc ccctggccct 1020
ccaaaatcgg taagagaatt aagatttcga atccctattt tgaggagcct tccgcatttc 1080
ctaattgtta aattcctgct tttcaccaaa ttcccggggg agaaacattt ggcaataaga 1140
agggactgtg aatttaaatg ctaattgagt gggtcctttt tccgcagctc cacctgcctg 1200
gcagcctctg ttgaaaccaa acacactcgg agcgcccagt gcaacattct tggggtgccg 1260
agtagaagcg cagtaaagag agaccctagc ggactcctgt ctggtttgct ttttaccgac 1320
tcttacagaa aaaaagagaa tgccattgga agaagctctt ttgcgtggtg ggcgatgtgt 1380
gggtggggga cttgtggcat ggcccacggt gttgtttctg tgcctgcgat gacacacgta 1440
tgtcttgagc tgtgggctcg ccttcctgga ggtgcgcccg accgcatctg ctggtgggtc 1500
tgagcgtgct tggggtgtcc caggagaact gagagaacgg ctcccacgtg caaagttcca 1560
aagcattaat attttcatca tattatcatt attcaatata ataatatttg ttcggttagc 1620
ggcactaatt aggccacatt aaaaccgtag tgtgtcccta atggtgcgta atgtgctcac 1680
actcacattt ttctctctga ggatgggcgg ctgcaggctg gtaggggagg agagacaggc 1740
aagcggcggg ctggattagg gcgtgacgcc ccccaccacg cacacaaaca tacacagccc 1800
actggatgtc tgccgggtgg gagccgcaat ctccgcgcgg tcgatggggc cctccgctgc 1860
gcactcggcc ctgcgccgag caccctgcag cctcctcccg cgacacggcg ctttgaactc 1920
ggcggattga ttttgcttcc cttccccctt ttgtgtgtgt ttgcgttcaa ttggttaggt 1980
ttttaagatt tgggagggct ggtgtgaaag aattaaaata ctcttaactg gagcccctcc 2040
gccgagaact ggaggtcccg cctcctagtt cggcgctttc aggaccctct tcccagaggg 2100
aatttctttc agaaattcca gggtgggctt gtaaaagacg cttccgcaga gcaggtcccg 2160
tcagggtctt tttcctgttc ctggtgccag cggtcggccc gggcgccccg cagacctcgg 2220
cgaggtagat gttaagctcg gagagtgccc ctcccgcagg cgccgtggcg agatcactct 2280
gaatatgtaa catatttgta acgtgcgccg aggtgtgatg tgtgtgctga aataggggga 2340
tgggggaatt cgaagccgga ttgggaaggc gggggggagg cgcacagaac tcacaatgta 2400
cttcgcaatc taacaatctg aacattcatt tattaaaagc tgctgcgtga catttacact 2460
gagccaccag tctctgcctc taatccgggc gaaaacgatt gtactgccga gttatggctg 2520
cagcgtatgg ggacgctgct gtccgcggcc ggacagagcc catcagctac aacgcggaag 2580
gcctctgcac ccccttgggg gcgggaggaa agtactgcca gtcctgcctg ggggccgagg 2640
gtaacaagca ccgagcctct cgctccacgc agggccagct gcccagctca gcgaagctct 2700
tgtgatctgg tgcgtgtctc tcgctcttcc ctccccatca aagaagtaaa ctttctacct 2760
actcccccta atccgatcgt ttagagctgc tgttttcctt ttgtcagatt cctcctcccc 2820
gatcagtctg agtacacgat cagaactgct cagagagcag gaagcacatt gatttcagct 2880

tgttctgtcc acagacaggc cctgacaagg ttgttagaac agccggagag gtctatacaa 2940
tcacttaatt accaaaactg tcagtcaggc gggacgcgga tccgcgtccc gggctgcgct 3000
aggcattcca gcactgggcc gcgcgcgtga ttgatcggtg ctgatagcac cgcaaaataa 3060
ttacggcgaa ttttctgatg tgtgatttta tcccaagttc atgcttcaga gaggtaatcg 3120
gagaatgaga agggtcagtg ccatttcgga ttacctggaa tctgcgagaa agggtaaaat 3180
gggggaagga gctccgagga aaacgggaga gatgggggtg cagagagaga gggaagaaga 3240
aagcgagtta tggattgctg gagggactgc aagcaattcg tcaaactgtg caagtgattt 3300
ccttcagagc cagcatatgg cagattgatt ttgtccaacg tcggttttag ccacatttaa 3360
aatgatccag cggttattac tgcgattggc ttaggaactg acaggcagtt ttaggcgcaa 3420
ggagtataga tcctgtttac cggagatgtg ttcgtaactg ctgtcaaata cagttaagta 3480
aatatcatta gcgaagagct ctgttaagag aaatgccaat ccaataaata tgcttttcct 3540
ccccgccctc cgcatggctg cctgcgcttc ctccagaggt tctccttcct gctcctttgc 3600
tgcttgggtc agacgtccca ggcatggtgc tgactcccgc caccttggag ccccgagctg 3660
agcctcgggc agaagatgac aggccagccg tggggcaagg aggccgcgga aacgcggaac 3720
ggcttcgggg agacggaagc gcccaatgag attcaccctg cagcccgggt ccagcccacc 3780
ttcctcggag attgccgcgg ccctcgaacc cggcctaggg tcttcatgtc ccggcggcca 3840
gaggacgttg cggggaccac tggggagctg ccctcagtca gctctctgcc ccacgccgga 3900
ggtcctggcg cggcttcttt cccgaactag actggcgact ctgggccagg ccccaaggac 3960
cgccccggcc tctccggctt tgcggggaga atctgaggaa ccgagtccaa gatagccgac 4020
ctaggctgtt ttcacccaga ccctgcgtcc ccgacccgct ggagtgaatc tgacactgcc 4080
aggttctctc tcatggcatg gagtgaatga agagggccat agatcccctt acccagcaca 4140
gtccctcggc aggccctgga aatccacagg gagcagaagc acagtatttt ctgaaccgct 4200
ccctctccct gggcctgtgg ccatttgaag gcagagctct gtgcctccaa gacagtaggt 4260
tttcggtcaa gtttggagcc tggggcccca acacatttac acagggttgg catcaccgtt 4320
tccttggact aaaggcaggc tcctatatcc tttttaaagg aacagaagga aggaaaagga 4380
aaccaacacg ggttatgttc agatagtagg cctatggcaa ttcttcacag ccatagagtc 4440
ctaatccgag tatcttccca gagaggaaaa acccaaaaaa cttttaaaag ggggaaagct 4500
gggtagatca tagcacccat tcttcatgcc taggcagaaa aactaaccca gagggagcaa 4560
aggggtaaga aatatgaaga gatcccctct gggagctgag gagcacccta gtttataatt 4620
tggtcaaagg agaaagtcac tggcctcctc ctttgataga ggcgtgtcat ctatcttccc 4680
agggaacatg atggttcaca aatgaagagg ctagccctcc tgcagctttt ttctacagag 4740
tgtaaaacac acaccgcctt catcagtgtt tgggatgtaa agaaccctgt ctatttaaaa 4800
gagatactgc atttttaaag tcaaacagta ccaatgtatg tggcgaatca agtaggtaaa 4860
caacttacat atggttgctg cacttgaagg aaccatccat tctcatgcac agcaaattga 4920
agaaacaatg gcactaatga gccttgcaaa atgcaactgt gaataatgaa agacaacact 4980
gcattttgca acagaaagaa taaaggtgaa ataatcagct agcaaagagg aaaagaaagc 5040
gagcaatgat taaatgatca aaagctggca gagtgaattc aatgtcactg ccagacgcag 5100
ccatctaccc acaagtgaaa gttaggtttc aagcacagtg taattatagc tggggttgtc 5160
agtttgacat taatgcagcc agcagaaatt tcctaattgg cctcagagga gaaagtgaac 5220
cagaaaatat attaacattt taaaaaagca tattttgcct aatcctttca ctttcgaaca 5280
atatttgaag accaaaatgc cccaggcata agaatttaaa tgagcaattt tgtttttgaa 5340
ggaaacggcc aatgagacag aaaatagact aaagggaaat cattagtgga tgagagatac 5400
tgacaggctt gccttgctga ctggctggcc tgtcacttgc agtctgtgtt ctttagttcc 5460
acgctatgag ctaagttgat aacatgaaaa gacccataaa cgtgcagcca gaagtcacag 5520
cctattatct ggaaattcaa atgcaagggg agggggtggc agagaaggca tcggcgaggt 5580
tgggagggag aggtgtgcat cgagggagga ggaggaggag gaaggggagg agggaaagga 5640
ggaggaggag gagaaaagaa gccctcattc tttggcataa aatcggccat atcagagaac 5700
aataataagc tattatctgt cataaatgtg ctatggactg ccaaaaaatg tagtcccgaa 5760
tcgacaacat tgttcgaact gaagatagca acaaaatgct taaagttgcg gatgtaattt 5820
cacatgcgtc cgggttgatg tgatatgacc gtatcaggga aacaaagcta agtgcagtca 5880
ggatctgtta gtacagtggc ttttgatgga acagctgagg cacacatcgc ccgtggcatg 5940
gactccgggg ccgaacgctc acgaccaaga cttttgccct tttgaaatga aatagaaata 6000
ggggagctgc aggaaaaccg aatcgcgctt agggtcagga gcaagacagg agctttcagc 6060
gaagatctga acattcagaa ctggaacggg taattagcag atagccagga aaaaataaat 6120
aaataaataa aaaagcctgg atggacctct gtaaacaatc attaagaaaa ataaaaatga 6180
accttcttat tagcctgcct tggaggtagt cagaaacaaa caaccaaagc aagagaggat 6240
gaagatttaa ataaaataat tatgtgcatc attaaaataa tcatatatgt ttgtacagac 6300
acgtatacac caaggaacgt aatgggggct cctcgcacag tcccaggaga tgcagga 6357

<210> 8
<211> 9739
<212> DNA
<213> Artificial Sequence

<220>
<223> chemically treated genomic DNA (Homo sapiens)

<400> 8

acggaagtat agaaatgtat ttaataaata tgtaatagaa ggataattaa tttaatatta 60
aagtgattag agagttaatg ttagggtttt atgggtatgt tgatatttta gaatagagag 120
gattgaaggt tagagatttt gggtaatttg tatattaaga acgtttttaa gttatcgggg 180
ggaaagatag agtttttttt gttttatata aaggggtaga tttaaaatta tagtttttta 240
aaatttttaa aaatttttgt aagatttgaa gagagttttg ttttaaattt tagtgtatat 300
aatataaata tatattatta atatttataa taagagattg gaattaaagt gtatattatt 360
ttttaaagtt ttttaaaatt gtttttaatt ataaaaaggt aaattaaaaa ttttttttat 420
gttatagtag tttaaaaggt aaataatgga agtagaaagg agatgtatat ttgttgaaat 480
ttgttatgta ttaggtattt tatataattt atttttattt gatttttata ataattggta 540
aggtgtgtgt tgtaggatat taagaggtta tagattttag gaggttaaga aatgatttaa 600
aattttatat ttaatagttg gtatagtaaa gatttgaagt taagaatatt tgtttttaaa 660
aattagtcgt atattatgat aatatatttt aaataaagat gtataaaaag tatttggttt 720
tttatagata ttatataaaa tatgggataa tattagtgat atatttaaat ttagatatta 780
gaaagaagtt ttttttaatt tattttgtaa ttgtaattta gttgtttatt ttataggttt 840
ttatagtaat aattaaatat tttttttttt gttttatatg tgtttttaaa ttgttattaa 900
agatttttgg ttagtagttt ttatattttt tggcgttttg ttgttaatgt tcggtaaata 960
tgggtagtta aggaaaatgt agatggtgtt atggaatata aattacgata atatatggta 1020
tatatttagt tttaataaag tgcgtttttt aattttgggg tgtcgtttta ttattttttt 1080
ataagattat ttatttttag ggaaggttta gtttttaaaa ttagggtaat aggtttatat 1140
gtgttatatg gttatttaga tgtatagatt atgtgtttta atatttttaa gtaagtgtat 1200
ttagtatatg ttttttagta ttattatgtg tatttatgaa ttgttttgtt tagatggtaa 1260
gtttttcgag gataagtgtt atgtttgaaa ttttatttta ttaattgtgt atggtagagt 1320
aataatgtta tttattttgt atatagagat atataattta taaattgttt ttttaggtaa 1380
attttttaat tatattgtta tattgtgata attgttatat tttttagatg aggtttatat 1440
aggttaagta attagttaga attataaatt aataggtaat gttttttttt tgtttataag 1500
agaaagtttt ttttgttttg taatatttta ttgttttttt tttttaaggt ttttattata 1560
aaggttaatg tttattggtt ttttaataaa agtaattatt atttagtatt tagtatatat 1620
atgtaaggta ttgttttaag ggttttataa atattaattt atttaatttt tttaattatt 1680
ttatgagata gatattattt ttatttatat tttatagata aggaaattga gggatagaga 1740
gattaatttg tttaaaatta gaggattggt aaatagtagt agtgaaattt gaatttagta 1800
gtgaagtata gttttatagt gtgtgttttt aaatatatat tattttgttt gaataagagt 1860
ttataaagat ttttatttag gatatatgag gagttgttta gtaagaaaag aagtatgttt 1920
attttttttt atttgatgta tttgattata ttttaaaatt tttttttatg ttaatagaat 1980
ttcgcgtatt atattaaatt tttttgtatc gaaggtatat gtaatggtta ttttgattga 2040
tatgtaaaat atttaaaata aaatatttcg agattcgagg attttttgtt ttatgaatta 2100
gatattaaaa aaaaaattgt ttaggtatta gaagtgttat atatttatat ttgtaagttt 2160
tttttgggat ttttagattt tttatataag attttttttt gtttgaaata ttttagtgtt 2220
tatttaatgt ttgagtttga aaattattta atttttttta atttttttat ttttagttat 2280
tttgttttta ggtatagggt agggtaagta tttgttttta atatgatgtt taatattttt 2340
tatgttttta ttttcgttat tttgaaaata tgttagtaaa tatttaaata tattgtaaag 2400
atttaaaatt tggggtgtgt atacgtatat taataggagt tatgaattat gtatattgta 2460
ggtttatata aattagattt tgaagaataa gtattatagt aattaagcgg gttataataa 2520
gatattttat gtgaaatgta aaaattattt tgaataaatt atttgtaagt taatattttt 2580
attagaatga tatatttatt atttttgggt ttgtgatatt gttttaatgt atttggttta 2640
attgttttaa tggtatatta agaaattatt ttaggttttt ttttttcgtt ggaatttgta 2700
gatttttatt ttcgtttgaa ttaaaataat ataatattaa aaataatttt ttatttttta 2760
atgtataata ttattttttt ttgtttagaa atatttaatt aaagtaggat ataattttag 2820
taattatttt tatatagaat gatttttaaa ttaatagtta gttgtatgat taaaaattgg 2880
gagattgata attaaaataa ttttaaatgt tttgtttatg tgatgaaaat gagtgatttt 2940
ttaatttttt tatatatatt aaattgattt tatagatttt ttgtattggt gtttaatata 3000
gttaagtgtt tgaggttatg taaaataaat aattttgaga ttttattgta aatgtttgta 3060
atttatgatg taaattgatt tgtgaagaaa aaataggatt ttatgtcgtt gaagttaaag 3120
aggtattttt tagaaattaa aatagtttcg aaatttgagt attgtattat attaaagtat 3180
tattatttga ggatttaaaa aagttataaa tttggggaaa aatttaaaat agatgtatat 3240
tagtttagtt ttaagtaaag gtatcgattt tattatttta ttgtgttttt tgtattatag 3300
ggtttttaat attaatgatg ttttaaataa ttttttttaa tttagttttt tttagttatt 3360
gattttgtaa ttcgggaagg ttttgtatgt ttaaaagatt tggggagtag aggatggggg 3420

tgttttttat taattatatt aggattattg agtttcgatt taaattataa gtatgttggg 3480
ttttatttaa atttaaagtg aattttttag attttttttt gggatggttc ggggaattac 3540
gagtttggaa tttttgttta tatatcgagg cgtttgtttt agagtttgga tattggtatt 3600
tcgggagagt aggttttgcg ggagtttgga ttcgaagggc gagattttat agggttaagg 3660
aaagcggttt ttgtttttcg ttagttttgg gggagtagac gtaagaggag gtaagggcgt 3720
cgcgagtttt tcggatgtat tggttttata ggtcgtgttc gagtggagta ttgcgaatgg 3780
ggttaagaaa ttttggtttt tttcgtcgga tttggttgtt ttcgcgggtt ttttcgttta 3840
tcgcgttttc gtcgcggttc gattttcgcg ggttttcgcg tcgaatttat ttggttttta 3900
tcgtacggga tattttcgat ttatttacgt cgcgttattg agtttttgta tcgatattcg 3960
gcgttttcgt tagtagggtt tggacgtatc gttttttttg atttcgggtt tttttcgcgt 4020
ttcgttgttt ggggtagatt ggtttcgaga gggagttatt attttttttg ttttagggtt 4080
tttagggttc gaattcgtgt tgggatttgg gttaggatta gggtttggag tttggagttt 4140
gtttgttagg attttcggtt tcggcgtcga ttggagttcg ttggaggtta taggattacg 4200
gcggatggtt tggttgtttt agggcgtatt atgttcgtag gtagatgttt attattaaaa 4260
attatcgacg tttattaatt aggagattcg taaggtttgc gtattgaata cggtttaaat 4320
tttgtttagt ggtttttgaa agttaaaaga aagaaattta tcgtttacgt ttatgttgag 4380
gaatagtttt gaatacgagt taagatttgg gagagggtta agtgggggtg gtggggaata 4440
ttgttggagg atgtggggtt ttggtagggg ttttacgtat tttttagtac gagggtgggg 4500
ggttttaggg gacgtttagg atttttttag tttttgcgga aggtttcgtt attataagag 4560
ttcgcgcggg aaggaaagtt tttgttttgg atatggttag ggtcgagttt ttaaagtttt 4620
taattatttt tatataggta gtatttttgg gttttattag tgggattttt tttagaaggg 4680
ggttacgata tggggagaga gagtttttta ttatttttaa ggttaggttt tttttaaatt 4740
cgattttggt ttttattttt taaatttaat taatgaagaa gttgttttag ttaattttaa 4800
ataggagtgt tagatgggga attttttttt tatagtgttt tggtttgttc gtttttgcgg 4860
tttttttttc gtttcgaagt tagtatttgt ttcgttttag agagggttaa cgaattggga 4920
ttgattgtcg attatgttat attaaattta ggttgatttc gtttcgtagg attttttttt 4980
tttttttttt aaaagtgttt ttagataaag attggaatta tagtaaaacg aataacgaga 5040
gtttattttg gggaaggaag ttattttatt ttatgttatt ttatttttta tttgtttttt 5100
tattaatttg gtataatttt gtttttatgt tgggtggata aaatggaggg ggtcggcgga 5160
aatgcgttta gcgttaacga tagttataga gttatttttt ttattgtttt ttgattgcgg 5220
tacgtaagag tagaggtaag cgttttttta agttggtata ttcgagagag tgatacgtat 5280
taattaaaag ggaaagatta tttcggatat tttaatagta ataggaataa agatgattcg 5340
aattttatta aatgaaacga tatttttatt taattgatcg taaagtgttt ttaattaata 5400
atttggtatt tatttaaaag gtttaataga attggaggcg atatattttg tattgataat 5460
acgttatgag ataatatttt ttgtttaaaa taatttaata tattagaaaa aaatatgtta 5520
aatataaata attttgtttt tatggagaat aaatagttat aaagtttaat cgtatattaa 5580
agtttagtta gaagagtttt tttttttttt tgtaaattag aaggtaaaat aaaaataaaa 5640
ataaaatttt ataaatttag gtttgttagc gaattgggat atagattggt ttagtttaag 5700
gtattttagt ttaaatagtt atatgaattg aaataaataa ttaaaagaaa aaaaaaaata 5760
aaaaagaaat aaataaataa aaaaaaaagg ttatttgaat agatatattt ttgtgagata 5820
aaatgtaaat gttgaaagtt agtttatata ttatagttaa aataaatttt gttcgtgtgt 5880
attaatatat tttatataaa tttagtgttt gtatttagtt gggtgttatt taaggtgaat 5940
ttgacgggat agagaggggg aaataaatta ttgtttttaa ataggatttt aggaaattaa 6000
taagaaggaa tataagaaaa ggggaatggt gggaattaat attgattgag cgtttattgt 6060
gtttggtata gcgaggcgtt gtatttttat tattttattt ggtggttttg tttttttttt 6120
tttttttta tatatttgt ttattttgt tttttagga taatgagtgt aattttttt 6180
ttttttgatt tttttttttt gttttttagg attaaagata gggtaaatat atatatatat 6240
atatatatat atatatatat atatatatgg taaatatatg atatatatat atggatatat 6300
atatattaat ttttagatat ttttggtatt gttttttata gtgaagatga aaatggagtg 6360
agtgtatggg agataagggg gtgggaggag atatattatt aatggaatat aagtaatatg 6420
taaatcggaa tttattgtta gtagtttaga tagttttgtt ataataattt tttagagaaa 6480
ttattgtttg tggtattttt ttgtgttaat attatttttt ttttttttcg ttttaatatt 6540
tttttttttt ttgttttttt atatatggtg ggtttaaatt taaagtattt gatttaacgt 6600
aaaaggaggt tcgttttttt tattaaattt ttcgtgttaa cgtgaatttc gtagaaaaag 6660
gttagtttta aaatttgtga attttttagt tgcgtatttt ttttatttta gttttttaaa 6720
taagtatatt tttttttttt gttttgtttt tttagcgtat ttttaaaaat atatatttaa 6780
gttggtaatt ttttttagtt tttcgagaat cgtttgggtt gtttttttat atttttaaat 6840
gtataaaata ttttaatttt aaaatttgtt tttgtttttt ggttttttaa ttttagtttt 6900
tgttaaatag tagttaagaa ataagttttt tttttttttt tttttttcgt ttgtttttcg 6960
atttttttgt ttgtttattt ttttattgtt aataagattt ttttttttat gtaagagttt 7020
atcgttgtag ttttgcggtg agttaaattt cgcggtttta gtattttttt gtttagtttt 7080
tttttagatt tttttaaatt cgtttttata aaatttaatt ttaggttttc gagtaggaaa 7140
acgggtagga gttacggagt ttgcgtgttt cgtgagattt ttggttttgc gtggagtttg 7200

gtttttcgag tttaagatgc gataggggac gagggatggt tagtgaggcg ggaagagggt 7260
cggttttcga ggttttaaag gggtaacgga gaagtagcgg ggcgcggagg gcgtgtaggt 7320
tgagtgtcgc gggataggcg cgatattggt gttggcgttg gcgttataga tttagggttg 7380
cggcgtgttt tttggttttt agtttgagat cggcgatgtt ggagtttttg ttggtggttt 7440
tggcggttgt tgcggtcgtt attatcgagg cggcggaagt cgaattcgcg gttagcgtgg 7500
cgagcggtag ttcgaagggc ggtgttggga atattatgta gggcgcgtgc gcggttaggt 7560
gcggatgtag gtggtggtgc gcgtgcgtta tagcgttgtt tagttgtagt tgcgtttgaa 7620
tttgaaagga taagggcgtt acgttgtaat gattatttta gggtgataat agaatagaaa 7680
tagagtatta acggcgttta gtttggttag agagagagtt gggttgtgtt tgggtacggg 7740
gagagggtat ttggttgtgt attttgtttc gatagtgatt tttttagttt gttgttgaat 7800
ttggggtttt atatggatat tttagtgttt tttgggtttt cgaaattcgt ttagggtagg 7860
tttatttttt cggatttcgg gaattttttt ttgtaggaat gtggtcgttt tttttgggaa 7920
ttgggttgaa atggtatatt ggaaagattg cgggtgagga tttttatttt ttttttttga 7980
tgttgttatg tattttttat tttattatta aaataacgga gtattaataa gtaaattacg 8040
taatttttta atgttattta gggaagttat tttattttta ttataatagg tataattgtg 8100
agtcggtatt tttaattttt attgttataa taattattta gggatttcgt taaaatttag 8160
attttgattc gtaaggttta ggttggggtt tgagattttg aatttttaat aattttttag 8220
acggtgttgt tgttgttggt ttaaagatta ttttttgagt agtaaaggtt tttttttaaa 8280
gttatttttt tttttttttt tagttatttt tttttgtttt taaagttaag tattaggatg 8340
ttagagtttt gtggtattaa gagagttaag atttttttaa tatcgatttt taatttatgg 8400
ttatatttat ttgttttttc ggtttaagtt attggagatt gagagttaat ttatgatatt 8460
ataggtatgt taagtagggg ttttggttga ggatagggaa ggttagatat tggttgggaa 8520
aggatttcgt ttttaggttt ggaaagtggg gagtttagag tattgggatt ttttaggatt 8580
aaattattgt tgtaggttgt tttttaaaa tgtattttta aaggtttttt tatggttaag 8640
ggattgtaaa ggtagggtag ttttgggaag attaagattt ttttttttat tagagatgaa 8700
gttttgtttg tagaaataga tttaaaaata aaagaacgaa aaaataaaag tatgagttgg 8760
gagtatttgt gtttattttt tttttgttag agttatttaa tgtgattaag aggtagaaaa 8820
tatttagaaa agtatttaga gggttgtttt agaaatattt taggattaat ttttgtattg 8880
gatttgataa aattttaatt aaaaggtttt tttttttttt ttttttaagg gtaattttta 8940
gtgtattttt aaaggattag ttattgtttt taggtttttt tagggatttt tatgaggata 9000
gaagggatta ggtatttagg tttttaatat cgtataggtt tttaaagggt agtgggttta 9060
ttatattatt ttaaaatgat tttttaatta tgtagatatt gatatttggg tttagagata 9120
ggtgatgttt aatagtttaa gtaaatattt aggtattttt atatttaaaa atagtttgaa 9180
attaggttgt atttgtttgt tgaaatggta tttttaaagt atttacgttg atataaggtg 9240
cgattttata agttttaaat tggttggcgg tttttatgag aatatttgta aaaagtataa 9300
gagaaaaata atgtgaggtt aacgtttgta gtatttttta gggttttaaa gggtataaga 9360
tagaacgaat ttttttgaaa atggattatt atttttttaa attgtattta aaatttaaat 9420
aaatgtaatt tatttttaaa ttttaggatt ttattaatag tttttgaaga ttattaagtt 9480
aagaattatt gttatttttg aggttttttt ttttttgtta ttgtaatagt aatatttatt 9540
attttttttt ttgtttagat tattaaatgt tttttgtata agaagtatat ttttatggag 9600
ttgatttttt tgttttttat atttagtttt tcgattttga aattaaattt ataggttgga 9660
gggggaaaaa aaataaaatt tagatgttat gattaaaaat ttttttaaat tataaaagta 9720
taaagagaaa agagtcgtg 9739

<210> 9
<211> 9739
<212> DNA
<213> Artificial Sequence

<220>
<223> chemically treated genomic DNA (Homo sapiens)

<400> 9

tacgattttt ttttttttgt atttttgtaa tttaaaaaaa tttttagtta taatatttag 60
gttttatttt tttttttttt ttaatttata ggtttggttt taaaatcgaa gagttaaatg 120
tagaaaataa gaaaattaat tttataaagg tatgtttttt atataaggaa tatttgatag 180
tttgagtaaa gagagaagta gtgaatatta ttattataat gatagaaaaa aaggaatttt 240
aaaaatgata atagtttttg atttaataat ttttaaaggt tgttaatgga gttttaaagt 300
ttgggagtaa gttatattta tttaaatttt ggatgtagtt tagaaagata atagtttatt 360
tttaaaggaa ttcgttttgt tttgtatttt ttggaatttt gaaaaatgtt ataaacgtta 420
attttatatt attttttttt tgtatttttt ataggtgttt ttataggggt cgttagttag 480
tttgaagttt gtagagtcgt attttatgtt aacgtaggtg ttttaaggat gttattttag 540

taggtaagta tagtttagtt ttaagttgtt tttaaatatg aaaatatttg aatgtttgtt 600
tgaattgttg aatattattt gtttttgagt ttagatgtta atgtttgtat agttggagaa 660
ttattttgag atgatatagt ggatttatta ttttttaaaa atttatacgg tattaaaggt 720
ttgaatgttt agtttttttt gtttttatgg gaatttttga aggggtttgg gggtagtaat 780
tggtttttta aaaatatatt aaagattgtt tttagagaga gaggaaggaa aagttttttg 840
attaagattt tgttaaattt aatatagagg ttagttttaa ggtattttta aggtaatttt 900
ttagatattt ttttaggtat tttttatttt ttagttatat taaataattt tgatagaaga 960
aaaataaata taagtatttt tagtttatat ttttgttttt tcgttttttt gtttttaaat 1020
ttgtttttgt aaataaagtt ttatttttgg taaagagaga agttttggtt tttttagggt 1080
tgttttgttt ttgtaatttt ttagttataa agaaattttt aagagtatat tttaaaagga 1140
tagtttgtag taatgattta attttgggaa attttagtgt tttgaatttt ttatttttta 1200
agtttgaggg cgaaattttt ttttaattag tgtttgattt tttttatttt tagttaggat 1260
ttttgtttgg tatgtttgtg atgttatgaa ttagttttta gtttttagtg atttggatcg 1320
gggagataga taggtgtgat tatgaattga gagtcggtgt tagggagatt ttagtttttt 1380
tggtgttata ggattttggt attttgatgt ttggttttgg aaataagaga gaatgattga 1440
agaaagaggg aggggtgatt ttaaaaggag atttttatta tttaaaaggt aatttttggg 1500
ttagtagtaa taatatcgtt tgggagattg ttagaaattt agaattttag gttttaattt 1560
agattttgcg aattagaatt tgaattttaa cgagattttt gggtaattgt tatggtaata 1620
aaggttagaa gtatcgattt ataattgtgt ttattgtggt gaagatggag taattttttt 1680
gaatgatatt aaaaagttgc gtagtttgtt tgttgatatt tcgttgtttt ggtagtgggg 1740
taagagatgt atggtagtat taagagaaga gggtaagggt ttttattcgt agttttttta 1800
atatattatt ttagtttagt ttttagggga gacggttata tttttgtaga agggggtttt 1860
cggggttcgg ggaaataagt ttattttaag cgagtttcgg agatttaggg gatattggag 1920
tatttatgtg gaattttaga tttagtaata ggttgagaag gttattgtcg gaataagatg 1980
tatagttaaa tgtttttttt tcgtgtttaa atataattta attttttttt tggttaagtt 2040
ggacgtcgtt aatgttttgt ttttattttg ttgttatttt aggatagtta ttgtaacgtg 2100
acgtttttgt ttttttaggt ttaggcgtag ttgtagttgg atagcgttgt ggcgtacgcg 2160
tattattatt tgtattcgta tttggtcgcg tacgcgtttt atatgatgtt tttagtatcg 2220
tttttcggat tgtcgttcgt tacgttggtc gcggattcgg ttttcgtcgt ttcggtagtg 2280
gcggtcgtag tagtcgttaa gattattagt aagaatttta gtatcgtcga ttttagattg 2340
aaagttaaaa agtacgtcgt agttttgggt ttgtgacgtt aacgttagta ttaatgtcgc 2400
gtttgtttcg cggtatttag tttgtacgtt tttcgcgttt cgttgttttt tcgttatttt 2460
tttgagattt cgggagtcgg tttttttttc gttttattga ttatttttcg ttttttatcg 2520
tattttggat tcggaaagtt agattttacg taggattagg gattttacga ggtacgtagg 2580
tttcgtggtt tttgttcgtt tttttattcg agggtttaga attgggtttt gtaggagcgg 2640
gtttggggga gtttggagag agattggata ggggagtgtt ggaatcgcgg agtttggttt 2700
atcgtaaagt tgtaacgatg gatttttgta tagaaaaaaa aattttgtta ataatgaaaa 2760
aatgagtaaa taaaaaaatc gaaagataaa cgggagagaa aaagaggaag ggaatttatt 2820
ttttaattgt tatttggtag aagttgaaat tggagaatta aggagtaaaa ataaatttta 2880
aaattaaagt attttatata tttaaaaata tggaaaaata atttagacga ttttcgagag 2940
attgggggga gttattaatt taaatgtgtg tttttaaaaa tgcgttaaga aggtaaagta 3000
gaaagaagag gtatatttat ttaaaaaatt aagatgaaaa aagtgcgtag ttgggaagtt 3060
tataggtttt gaaattgatt tttttttgcg aagtttacgt taatacgaga aatttgatga 3120
gagaggcggg ttttttttta cgttgaatta gatgttttga gtttaaattt attatgtatg 3180
gaagagtaag aaaagagaaa atattaaaac gaggagagag aaaaataata ttaatataaa 3240
aaaatgttat agataatgat ttttttgaga aattattatg gtaaaattgt ttggattgtt 3300
gatagtaaat ttcggtttgt atgttatttg tattttattg atggtgtgtt tttttttatt 3360
tttttatttt ttatgtattt attttatttt tatttttatt atgaaaaata atattaaaag 3420
tatttggaaa ttgatatata tatatttata tatatatatt atatatttgt tatatatata 3480
tatatatata tatatatata tatatatata tatttgtttt gtttttgatt ttggggaata 3540
aaagaaaaaa gttagaaagg gaaaaaatta tatttattgt tttaagaaga tagaggtggg 3600
tagaatatgt ggggaaagga aaaagaaaat aagattatta aatgaaataa tgaaggtata 3660
gcgtttcgtt gtgttagata tagtaggcgt ttaattagta ttagttttta ttattttttt 3720
ttttttgtgt ttttttttgt tggttttttg aagttttatt tgaagatagt ggtttatttt 3780
ttttttttta tttcgttaaa tttattttaa ataatattta gttagatata ggtattaggt 3840
ttgtgtaaga tatgttgata tatacgaata aagtttattt tgattataat gtgtggattg 3900
atttttaata tttgtatttt attttataaa ggtgtattta tttaagtaat tttttttttt 3960
tgtttgtttg tttttttttt gttttttttt tttttttggt tgtttgtttt aatttatgta 4020
gttatttaaa ttgggatatt ttggattaag ttagtttgta ttttaattcg ttagtaagtt 4080
taagtttgtg gggttttgtt tttgtttttg ttttattttt taatttataa gaaagaggaa 4140
aagttttttt aattgaattt tggtatgcgg ttgagttttg taattatttg ttttttatga 4200
aaataaaatt atttatattt gatatatttt tttttagtgt attaagttat tttaaataaa 4260
agatgttatt ttatgacgtg ttgttagtat aaaatgtgtc gtttttaatt ttgttaaatt 4320

ttttaaataa gtgttaagtt attaattgaa gatattttgc gattaattga atgaaaatat 4380
cgttttattt gatggggttc gagttatttt tgtttttgtt attattaaaa tattcgggat 4440
agtttttttt ttttgattaa tgcgtattat tttttcgaat atattaattt ggaaaagcgt 4500
ttgtttttgt ttttacgtgt cgtagttaag gggtagtaag aagggtggtt ttgtggttgt 4560
cgttagcgtt gagcgtattt tcgtcggttt tttttatttt atttatttag tatagaaata 4620
gggttatatt aaattaatga aagaatagat aagaagtaaa ataatatagg atagagtaat 4680
tttttttttt aagatggatt ttcgttattc gttttgttat aattttagtt tttatttggg 4740
gatatttttg ggaggaagga aggagggatt ttgcggagcg aaattaattt gggtttggta 4800
tggtataatc gataattagt tttagttcgt tgattttttt tggagcgggg taggtgttga 4860
tttcggagcg ggaaaagagt cgtaaaggcg ggtaggttag ggtattgtgg agggaggatt 4920
ttttatttga tatttttgtt tagggttggt tgaagtagtt tttttattgg ttaaatttag 4980
aaggtggaag ttagaatcgg gtttaggaaa agtttggttt tgaaaataat gaggagtttt 5040
ttttttttat gtcgtggttt ttttttggaa gaaattttat tagtaaagtt taaaggtgtt 5100
gtttgtgtgg ggatagttgg agattttgaa gattcggttt tgattatgtt taaggtagga 5160
attttttttt tcgcgcgggt ttttgtgatg acgggatttt tcgtaaagat tgaaagagtt 5220
ttgagcgttt tttgggattt tttattttcg tgttggaggg tgcgtaaaat ttttgttaaa 5280
gttttatatt ttttagtagt gttttttatt atttttattt ggtttttttt taggttttag 5340
ttcgtattta aagttgtttt ttagtataga cgtgggcgat aggttttttt tttttaattt 5400
ttaaagatta ttgaatagga tttgggtcgt atttagtgcg taggttttgc gggttttttg 5460
gttgatgaac gtcggtagtt tttaataata aatatttgtt tgcgggtatg gtacgttttg 5520
aagtagttag gttattcgtc gtggttttgt ggtttttagc gagttttagt cggcgtcggg 5580
gtcgggggtt ttaataggta ggttttaagt tttaaatttt aattttaatt tagattttaa 5640
tacgggttcg gattttggag attttggagt aggggagatg gtggtttttt ttcggggtta 5700
gtttgtttta agtagcggag cgcgggggaa gttcgaggtt aaaggaggcg gtgcgtttag 5760
gttttgttgg cggaggcgtc gggtatcggt atagaggttt agtgacgcgg cgtgggtggg 5820
tcgggaatgt ttcgtgcgat aggagttagg tgggttcggc gcggagattc gcgggagtcg 5880
ggtcgcggcg ggagcgcggt aggcggagag gttcgcggag gtagttaggt tcggcgagaa 5940
aggttaaaat tttttggttt tattcgtagt gttttattcg ggtacggttt gtgggattag 6000
tgtattcggg gagttcgcgg cgtttttgtt tttttttgcg tttgtttttt taagattaac 6060
ggaggataga ggtcgttttt tttggttttg tggagtttcg tttttcgggt ttagattttc 6120
gtaaggtttg ttttttcgga atgttagtgt ttaaattttg gggtaggcgt ttcggtgtgt 6180
gaataagagt tttaaattcg tagtttttcg aattatttta agaaggggtt tgaggaattt 6240
attttaaatt taaataaggt ttaatatgtt tgtagtttgg gtcgaaattt aataatttta 6300
atataattaa taaaggatat ttttattttt tgttttttaa attttttggg tatgtaaaat 6360
tttttcgagt tgtaaaatta atgattggga aaaattgagt taaaagagat tgtttaagat 6420
attattagtg ttaaggattt tatgatataa gagatatagt aagatagtaa gatcggtgtt 6480
tttgtttgga gttgaattga tgtatattta ttttgagttt ttttttagat ttataatttt 6540
tttgggtttt tagatggtaa tattttagta taatgtaatg tttaaatttc ggaattattt 6600
tgatttttga aaaatgtttt tttggtttta gcgatatagg attttgtttt ttttttataa 6660
attagtttat attataaatt gtaaatattt gtaataagat tttaagattg tttattttat 6720
ataattttaa gtatttgatt atgttaaata ttagtataga aaatttgtga aattagttta 6780
atgtgtgtga gggaattaaa ggattattta tttttattat ataaataaag tatttaagat 6840
tgttttgatt gttagttttt taatttttag ttatataatt gattattaat ttaagagtta 6900
ttttgtgtaa aaataattat tgaggttata ttttgtttta attaaatatt tttgggtaga 6960
agaaaatggt attgtatatt gggaagtggg ggattgtttt taatattata ttattttagt 7020
ttaaacggaa ataaaagttt gtaaatttta acggggaaaa aaggtttgaa atggtttttt 7080
aatatgttat taaaatagtt agttaaaata tattaaagta atattataaa tttagaaata 7140
ataaatatgt tattttaatg agaatgttaa tttatagata atttatttaa ggtagttttt 7200
atattttata tggaatgttt tattatgatt cgtttgatta ttgtgatgtt tgttttttaa 7260
aatttaattt atataggttt gtaatatata taatttataa tttttgttga tatacgtata 7320
tatattttaa attttaaatt tttatagtat gtttggatgt ttattagtat gtttttaaga 7380
tgacgaaaat aaaaatataa aaagtgttaa atattatatt gaaagtaagt gtttgttttg 7440
ttttatgttt gaaggtaaag tagttgaaaa tgaaaaaatt aaaaaggatt agataatttt 7500
taagtttagg tattgagtga gtattgaaat attttagata aaaaaaaatt ttgtataaga 7560
aatttgaaaa ttttaagaaa gatttataga tgtgagtgta taatattttt gatgtttaga 7620
taattttttt tttggtgttt aatttatgaa ataaaaaatt ttcgagtttc ggggtatttt 7680
gttttgggta ttttatatgt tagttaaggt aattattata tatgttttcg gtgtaggaaa 7740
gtttagtata atacgcggag ttttattggt atgaggaaag gttttagaat ataattaaat 7800
atattaaata gggaggagta aatatgtttt tttttttgtt gaataatttt ttatatgttt 7860
tgagtaagga tttttgtaga tttttattta agtagaatag tgtatgttta agagtatata 7920
ttgtggagtt atgttttatt gttggattta aattttattg ttattgttta ttagtttttt 7980
gattttggat aagttaattt ttttgttttt tagttttttt atttgtaaaa tgtggataaa 8040
aatagtgttt attttatgga atggttagga ggattaaatg agttaatatt tgtaaaattt 8100

ttagaataat gttttgtata tgtatattaa gtattaaata ataattattt ttattaaaag 8160
gttaatagat attgattttt gtaataaaaa ttttaaaaga aaaaaataat aaagtgttgt 8220
aagataagaa aagttttttt ttgtgggtaa gaagaaaata ttgtttgtta gtttgtgatt 8280
ttggttaatt gtttagtttg tgtgagtttt atttagaaga tgtgataatt gttataatgt 8340
agtagtgtag ttaggaggtt tatttagaag agtaatttat aggttgtgtg tttttatatg 8400
taagatagat aatattgtta ttttgttatg tatagttaat aaggtgagat tttaggtatg 8460
gtatttgttt tcgaggagtt tattatttgg gtaaaatagt ttataaatat atatgatagt 8520
gttaggaagt atgtgttaaa tgtatttgtt taaaggtatt aggatatata atttgtgtat 8580
ttgaatggtt atataatata tgtgggtttg ttgttttggt tttggaaatt aaattttttt 8640
tggaaatgag tagttttata aaggaatggt gaagcgatat ttagaggtta agaagcgtat 8700
tttgttagga ttaaatgtat attatatgtt gtcgtggttt gtgttttata gtattatttg 8760
tatttttttt ggttgtttat gtttatcgag tattagtagt aaaacgttaa aagatataaa 8820
ggttgttgat taaggatttt taatagtagt ttgaaaatat atataaaata aagaaaggaa 8880
tgtttaatta ttattatagg gatttgtaag atgaataatt gagttataat tatagagtaa 8940
attaagaagg attttttttt aatgtttaaa tttaggtata ttattaatat tgttttatat 9000
tttatataat atttatgaag agttaagtat tttttatata tttttatttg ggatgtgttg 9060
ttatagtata cggttaattt ttagaggtag atatttttga ttttaaattt ttgttatatt 9120
aattattaga tgtaaagttt taggttattt tttagttttt taaaatttat gattttttag 9180
tgttttgtag tatatatttt gttaattatt gtgaggatta aataaaaata agttatgtaa 9240
ggtgtttggt atataatagg ttttaataaa tgtatatttt ttttttattt ttattattta 9300
ttttttaaat tattgtaata tagagaggat ttttaattta tttttttgtg attagaagta 9360
gttttgaaaa attttgggag gtggtgtgta ttttaatttt aattttttat tgtaagtatt 9420
gataatgtgt gtttatattg tgtgtattgg agtttggaat aaagtttttt ttaaattttg 9480
taaaaatttt tagagatttt gagggattgt gattttagat ttgttttttt gtgtagaata 9540
gaaaggattt tatttttttt ttcgataatt tgaggacgtt tttaatgtgt aggttattta 9600
gagtttttga tttttagttt tttttatttt ggaatgttag tatatttatg gagttttggt 9660
attaattttt tagttatttt gatattagat tgattgtttt tttgttgtat atttattgaa 9720
tatattttta tgttttcgt 9739

<210> 10
<211> 4313
<212> DNA
<213> Artificial Sequence

<220>
<223> chemically treated genomic DNA (Homo sapiens)

<400> 10

gggtttgatt ttttgagatt cggggaggat ttttggtaga tgtgtgttta gttagaatat 60
ttggtaagga tttttttaat gaagaaaaag tggaggaatt tagttttagc gagaagaggt 120
ttttttattt tgttttagat atatcggata gagggtatat tttgattaga gttacgttta 180
gtggttagga ggttagttta gtattttttt ttttattatt ttcgttttgg gtgggggggt 240
aatttttttg ggagtagttg tgggaattgt cgttttttat tttagtttag ttagtatttt 300
gaagtttgta ggggaaggat agtacgtggg atggatattg gggaaggagt ttcgtaaggt 360
tagggtgtaa tttttaggtt ttaggtggtt tggtaggtta cgttgtttcg gagatgtttg 420
ttagattttt taagtttatt tagggtttgg tagtaatttg ttggttgttt ttgcgggggt 480
ttgggttgtt gagtacggtg tagttgttta gggttaatta gttttagggt gttcgtgtta 540
ggttgcggtt ttttcgtttt tttcgtattg agggtattta tggcgtgtaa atgttttcgt 600
atttttagag ttgttttatc ggatgttttt aggaatttat atatttttat aaaaatgtat 660
tttaaatgat ggataggcga gtttggggta ataacgggtg tttggtgggt agataagagt 720
aaatgggaag gagttcgagg gaggaggggg aagagaagag gaaatagaat ttttagttgg 780
atattttgat aatagttgga aggaaagttt agaaaagatg aagagagagg aggggagaaa 840
ttaattgggg tttttatttt tgtcgttgga tttttaattt tcgttttaaa tgggttttgt 900
ttttcggtaa aattagttta aaggatttta aaataaagaa aacgagatga tcggtttggg 960
agttttttaa ttagagtaga gaagttagag gggggcgggc gatttggttt tgaagtttta 1020
gttgaatagt tatttttttt ttttttggta aaaaggattt ttttagaatt ttcgaggttt 1080
ttggattttt tttttcgtaa atggagtcgt atattgtatt ttttcgtttt ttcggatcgt 1140
taagtatgtt ttatgagggt cgttgttttc gggtggaatg cggtcgtatg tacgcgtttt 1200
tttgtatacg tatatatacg tatatttata ataagtgttt gtaggaggag tgttttgcgc 1260
gttagttttg cgtttaagat aggaagttgt cgggttatcg agttaaatgg gagtgatatt 1320
attttttttt attagtaagg aaagcggatt ataaaagttt ttttgtattt cggtagttta 1380
tttaatatta tttatgtatt ttgtgtaagg aattgtggga tttcgtttta cggtaaataa 1440

tatggaaatt ttaaaaatag cgattttttt gtgcgtgttt atttacgcgt ttcggggtga 1500
tttggcgggg ttgtcgtcgg gtgatttata tttttgaatc gcgaagcgat agggaaagcg 1560
cgggcgagcg taggagacgc ggtcgggggt tttttcgggt ttttgggttt tcgtattcgg 1620
agcgggggac gcggtcgttt taaggggagg aggggcggcg ggttgttttt gttatttagc 1680
ggcggtcgga gcgttacgtg ggcgcgcggc gtcgcggtta ttggttcgag gtacgtgttt 1740
aggagatcgg tttgcgacgt tattcgaggg ggttttgtta aaaataagaa taaaaattta 1800
gagtgaaagt gttttaggtt gcgtcgagtg gtttggaaat tttcgagttc gcgcggaggt 1860
cgaggcggcg agggcggcgg acggtcgggg agcgcgggcg gtttagttcg gttcggtcgg 1920
gttttggttt cgcgtttttt atttatgcga ttcgggtcgc ggagttttgc ggggttcggc 1980
gggggcgcgg tcgtacgtcg gtggggcgtt tcggttcgta gcggggcggc ggtcgcgagg 2040
agggggtttt tatgtgcgtg cgggcggtgg cgggcgcgtt gatcgcgggc gttcggtatt 2100
ttcgagggtc ggttagggcg tgcgggcggg gacggtcggg cggcggcggc ggtcggagtc 2160
ggttcgggcg ggcgtgagcg tcggggaacg cgttgtttgt atgcgcgtag ttttcgtttc 2220
gggcggttta ggcggcggcg tcggagttcg aggcggtcgg acgcggagag gagcggggag 2280
ttcgggaggc ggttcgcgtt ttcgtcggat tattgcgatt gtttagattt cggttgcgcg 2340
gcgaagtcga ggatttggtt ttgttgaatt ttttatcgtt tgggcgagcg gggcggttcg 2400
tggtgttttt aatttagttc gtggatttaa aggtggtttc gcgtcgagcg cggtcggcga 2460
tttgtaggat tttagttttg gtcgcggtcg tcgcgtacgt tttcggaaga ttcggcgggg 2520
tgggggcgcg ggggttttcg tgtgcgtcgc gggagggtcg aaggttgatt tggaagggcg 2580
ttttcggaga attagtgtgg gatttattgt gaatagtatg gaggagaatg attttaagtt 2640
tggcgaagta gcggcggcgg tggagggata gcggtagtcg gaatttagtt tcggcggcgg 2700
ttcgggcggc ggcggcggta gtagttcggg cgaagcggat atcgggcgtc ggcgggtttt 2760
gatgttgttc gcggttttgt aggcgttcgg taattattag tattcgtatc gtattattaa 2820
tttttttatc gataatattt tgcggttcga gttcggtcgg cgaaaggacg cggggatttg 2880
ttgtgcgggc gcgggaggag gaaggggcgg cggagtcggc ggcgaaggcg gcgcgagcgg 2940
tgcggaggga ggcggcggcg cgggcggttc ggagtagttt ttgggttcgg gttttcgaga 3000
gtttcggtag aattcgttat gtgcgttcgg cgcgggcggg tcgtttttag tcgtcggtag 3060
cgatttttcg ggtgacgggg aaggcggttt taagacgttt tcgttgtacg gtggcgttaa 3120
gaaaggcggc gatttcggcg gttttttgga cgggtcgttt aaggttcgcg gtttgggcgg 3180
cggcgatttg tcggtgagtt cggattcgga tagttcgtaa gtcggcgtta atttgggcgc 3240
gtagtttatg ttttggtcgg cgtgggttta ttgtacgcgt tattcggatc ggtttttttt 3300
aggtgagttc gcggggatta cgcgtttcgg ttcgtcgcgg ggaggttcgc ggagttgggg 3360
ggcggtgttg gcgcgggaat ttatcgggag gaaaatattt cgaatttttt tcgcgtatac 3420
gtataaagat ttacgcgata ttgtgtgaag ttgacgtcgg ttcgggtagc ggttaggagt 3480
ttagcggtag gattgattcg ttagggggta tagatttttt aggatcgtag aagggatttt 3540
tttttttttt ttgttttttt tttttttttt tttttttttg tttttttttt ttgtttttta 3600
tttcgttttg gcgtattttt ttttagtttt tagtttatgt tttttttatt gtagtttttt 3660
ttggtgggaa cgtggtggtt ggaagatggg ttcggaagtg tatattttta tttttttttt 3720
tacgattttt taatttaggt taggtcgggg acgtatgttt tagtttattt tagatttgtt 3780
ttattattcg gttatttcgg ttgtgttcgg ggaagaaaag gcgaggtttt ttgtcgtttt 3840
gttttttgtt tttcgggttt gcgttgatcg gtgggattta ggaggatgta tatagggaag 3900
gaggaaaata aaggcgtttt ttttttttgg ttttattttg tttgttagcg ttagttcgta 3960
gtggtggggt ttagtttttt ttttgtatat agcgaggata agggaggtag tcgttttttt 4020
cggtatttgt tatttttaaa tagaaaggat ttttttttag ggttttttgg gggttgttga 4080
tgggaaagag gtagtattcg taggggtttt gtagagatgt tggatatatt tttttataga 4140
tttgcgattt taaaaaatta agtttatgtt tttgtagaaa ttattaattg tattttatgc 4200
gggtttgcgg ttgggaatcg ttattagaag tggattgttt gatttcgagt tggtagcgga 4260
ttttcgttgt ttttaaattt ttaattattt tgcgggggtt atttgtttag att 4313

<210> 11
<211> 4313
<212> DNA
<213> Artificial Sequence

<220>
<223> chemically treated genomic DNA (Homo sapiens)

<400> 11

gatttgggta aatgattttc gtaaaatagt tgagggtttg ggggtagcgg ggattcgttg 60
ttagttcggg gttaaatagt ttatttttaa tggcggtttt tagtcgtaga ttcgtatgga 120
atgtagttga tgatttttat aaggatatgg atttagtttt ttaaaatcgt agatttatga 180
aaaaatatat ttagtatttt tgtagggttt ttgcgaatgt tgtttttttt ttattagtag 240

tttttaggaa attttgagag aaggtttttt ttatttgggg atggtaggtg tcgggaggga 300
cggttgtttt ttttgttttc gttgtgtgta ggaagggggt tgagttttat tattgcgggt 360
tggcgttggt aaataaagtg gagttaaggg gaaagggcgt tttatttttt tttttttttt 420
gtgtgtattt ttttgagttt tatcggttag cgtaggttcg aggggtagaa ggtagagcgg 480
taaagggttt cgtttttttt ttttcgagta tagtcgggat aatcggatgg taggataagt 540
ttggggtggg ttggggtatg cgttttcggt ttggtttgag ttggaagatc gtagggaagg 600
ggatgagagt gtgtattttc gggtttattt tttagttatt acgtttttat taaaaaaaat 660
tatagtgggg gagatatggg ttaggggttg ggaagagatg cgttaaggcg gggtggaaga 720
tagagagggg agatagggag agaggaagga gagagagaga tagagaaaga aagaaggttt 780
tttttgcggt tttaagaagt ttgtattttt tagcgaatta gttttgtcgt tggatttttg 840
gtcgttgttc gggtcggcgt tagttttata taatgtcgcg tgagtttttg tgcgtgtgcg 900
cgggggaggt tcgagatgtt tttttttcgg taagttttcg cgttagtatc gttttttagt 960
ttcgcgggtt ttttcgcggc gagtcgggac gcgtggtttt cgcgggttta tttgaagaag 1020
gtcggttcga gtagcgcgta tagtagattt acgtcggtta gagtatgggt tgcgcgttta 1080
ggttggcgtc ggtttgcgag ttgttcgagt tcgagtttat cgataggtcg tcgtcgttta 1140
agtcgcgggt tttgagcgat tcgtttaggg ggtcgtcggg gtcgtcgttt tttttggcgt 1200
tatcgtgtag cgagagcgtt ttggagtcgt ttttttcgtt attcggagag tcgttgtcgg 1260
cggttgggag cggttcgttc gcgtcgggcg tatatggcgg gttttgtcgg ggttttcggg 1320
agttcgagtt taagagttgt ttcgagtcgt tcgcgtcgtc gttttttttc gtatcgttcg 1380
cgtcgttttc gtcgtcggtt tcgtcgtttt tttttttttt cgcgttcgta tagtaggttt 1440
tcgcgttttt tcgtcggtcg aattcgggtc gtaggatgtt gtcgatgaag aagttggtga 1500
tgcggtgcgg gtgttggtgg ttgtcgggcg tttgtaggat cgcgggtagt attagagttc 1560
gtcggcgttc ggtgttcgtt tcgttcgggt tgttatcgtc gtcgtcgttc gagtcgtcgt 1620
cggggttgga tttcggttgt cgttgttttt ttatcgtcgt cgttgtttcg ttaggtttgg 1680
ggttattttt ttttatgttg tttatagtaa attttatatt ggtttttcgg ggacgttttt 1740
ttaaattagt tttcggtttt ttcgcggcgt atacggagat tttcgcgttt ttatttcgtc 1800
gagtttttcg agggcgtgcg cggcggtcgc ggttagggtt gaggttttat aagtcgtcgg 1860
tcgcgttcgg cgcggagtta tttttgaatt tacgaattgg gttagaaata ttacgagtcg 1920
tttcgttcgt ttagacgatg agagatttaa tagagttaag ttttcgattt cgtcgcgtag 1980
tcggggttta gatagtcgta gtggttcggc ggggacgcgg gtcgtttttc gggtttttcg 2040
ttttttttcg cgttcggtcg tttcgggttt cggcgtcgtc gtttgggtcg ttcggggcga 2100
gagttgcgcg tatgtaggta gcgcgttttt cggcgtttac gttcgttcgg gtcggtttcg 2160
gtcgtcgtcg tcgttcggtc gttttcgttc gtacgtttta gtcggttttc gagggtgtcg 2220
ggcgttcgcg gttagcgcgt tcgttatcgt tcgtacgtat atggaggttt ttttttcgcg 2280
gtcgtcgttt cgttgcgggt cggggcgttt tatcggcgtg cggtcgcgtt ttcgtcgagt 2340
ttcgtagagt ttcgcggttc gagtcgtatg ggtgagagac gcgaggttag ggttcggtcg 2400
ggtcgggttg ggtcgttcgc gtttttcggt cgttcgtcgt tttcgtcgtt tcggttttcg 2460
cgcgggttcg gaaattttta ggttattcgg cgtaatttga gatattttta ttttggattt 2520
ttgtttttat ttttaataga gttttttcga gtgacgtcgt aggtcggttt tttggatacg 2580
tgtttcgggt taatggtcgc ggcgtcgcgc gtttacgtga cgtttcggtc gtcgttgggt 2640
gataggagta gttcgtcgtt tttttttttt ttaaagcggt cgcgtttttc gtttcgggtg 2700
cgggagttta ggaattcgga gagattttcg atcgcgtttt ttgcgttcgt tcgcgttttt 2760
tttgtcgttt cgcggtttag gggtgtgagt tattcggcga tagtttcgtt aggttatttc 2820
ggggcgcgta ggtggatacg tataggaaga tcgttatttt taagattttt atattgttta 2880
tcgtggggcg aaattttata attttttgta taaaatgtat aaataatatt aaatgagttg 2940
tcgagatata aagggatttt tgtggttcgt tttttttgtt gatggagagg aatagtgtta 3000
tttttatttg attcggtaat tcggtagttt tttgttttaa acgtagagtt ggcgcgtagg 3060
atattttttt tgtagatatt tattgtaagt gtgcgtgtgt gtgcgtgtgt agggaggcgc 3120
gtgtatacgg tcgtatttta ttcggggata gcgattttta tgaaatatgt ttagcgattc 3180
gaaagagcgg gggaatgtag tatgcggttt tatttgcgaa gggagaaatt taggagtttc 3240
ggaggtttta aaggaatttt ttttgttaag gagaggaggg gtgattgttt agttaagatt 3300
ttaaaattaa gtcgttcgtt tttttttaat ttttttattt tgattgagga gtttttagat 3360
cggttatttc gttttttttg ttttaaaatt ttttaaatta attttgtcgg agagtagggt 3420
ttatttgaaa cgagaattag gaatttaacg gtaagggtgg gagttttagt tggttttttt 3480
tttttttttt ttttattttt tttagatttt ttttttagtt gttgttagaa tgtttaattg 3540
gaagttttgt tttttttttt tttttttttt ttttttcggg ttttttttta tttgtttttg 3600
tttatttatt aaatattcgt tgttatttta ggttcgtttg tttattattt aaaatgtatt 3660
tttatggaaa tgtgtgaatt tttggaaata ttcgataggg tagttttggg ggtgcgggag 3720
tatttgtacg ttatgagtat ttttagtgcg ggggaggcgg ggaggtcgta gtttggtacg 3780
gatattttag ggttgattgg ttttgggtag ttgtatcgtg tttagtaatt tagattttcg 3840
tagaggtagt tagtaggttg ttgttaggtt ttgagtgaat ttggggagtt tggtaagtat 3900
tttcgaggta gcgtggtttg ttaggttatt tggggtttga aggttgtatt ttggttttgc 3960
gaagtttttt ttttagtgtt tattttacgt gttgtttttt ttttgtaggt tttagagtgt 4020

tggttgggtt gggatgagaa acgatagttt ttatagttgt ttttaggaaa attatttttt 4080
tatttaggac gggaatggtg ggggaggagg tgttgggttg gttttttggt tattggacgt 4140
ggttttggtt agagtgtgtt ttttgttcgg tgtgtttagg gtagggtggg gaagtttttt 4200
ttcgttgggg ttggattttt ttattttttt tttattggag gagtttttat taaatgtttt 4260
ggttaggtat atatttgtta ggggtttttt tcgagtttta aaaaattaaa ttt 4313

<210> 12
<211> 16197
<212> DNA
<213> Artificial Sequence

<220>
<223> chemically treated genomic DNA (Homo sapiens)

<400> 12

ttttgtaggt ggagggggaa agggtttggg ggttggtgga ggacgtagga gtatggggga 60
gttgtggaaa agacgtggag gtagagttag taggttttgt taagggatag gaggtggttt 120
tagagagaaa atgagggatt agggatgttt tttggggggt ggttagttgg gtgattggga 180
gaaattggga aggcgtagat tagggtagta ggggtagcgg gatcgggggt gttttgaagt 240
gaatacgtga aattgaaggt gttcgttagt tttttagtgg aggtatttag gaggtagttg 300
gaattggaag gaaatggtga tagtcgttag tatcgtagag gtggcggtgg tggtggttat 360
agttggattt ttttgggttg gtgggaagtg tggaggtgaa ggagtaggga gtagggggag 420
gtggagaagg aaattttagg tttatatttt tattttattt ttggttgtta tatttttagg 480
aagtttttt tgaggttggg atagagggtg gggatagttt agttttttg agagagttta 540
ttttcggagg tttgcggtgg gagttagggc gcggcggaga gggtttttta tttttttttt 600
agtaagcggg agggaggcgt cgggttgagg ttgcgttgag ttcgagttcg tcgttcgggt 660
ggattcgttt tgtttaagcg cggaggggcg gaggtttggt tgggttgtag cgtggtgcgg 720
agtaggacgt cgtttcgtgt tatggttatt ggagacgtac gtttattttt tttttcgggt 780
tcgtcgattt tgttattttt cgttttcggt cgttcgtggg tgttcgttat ttagattttt 840
ttcgtgtttt atgggacgta agtttttttt tagagttcgg ttttgtaaaa gaggtttgga 900
gttttcgtta tagatttttt ttttgggtta cggaggatga gaagggttat cgagtcgagt 960
cgtaattttg cgtaattttt gatttttttt tttttttgtt tttacggtat tttatcgttt 1020
ttttttcgtt ttcgaggttt tttagaaaat agcgtagaat tgtgtagatg tttaggagat 1080
gtgaagatgt tggagatgtt taggaggcga cggttacgcg aggagaattg tttttaggtg 1140
cgtttttgga acgtacggag atttttcggc ggggaagggg tcggggttcg cgttatttag 1200
tgtttgttta tcgatttttt tttgaagagg gggtttaggg tttttgttga gggagcgggg 1260
aggcggtgcg ggtttttttc gggtatatat gggtgttcgt tttttttttt tttttttttt 1320
ttttcgtacg gagagcggag agcggagagc ggagagcgat agggaggtag tcgaagattt 1380
gaattttgaa aggggagttg gcggcgaatg gtgaatgaga tagttattta ggaagcgagt 1440
ttagttagtt cgggaggcgg tggagattta cgttcggaag taatcggatt aggttttaga 1500
atgtgatcgt ttttcgggtt tcgggggaga cgttaaggac gcgtaagcgg agggcgcgga 1560
gataattggg agttagagtt tttattattt gggttgggaa cgtttttggg cgtttcgacg 1620
gggtggcggt gggggtcggg ggaggttttt gagaatcgtg cggttcgggg agagtttatt 1680
tattgttgag tttcgatata ggttttgaag ttgttgtagt ggtttagttt ttttttcgtt 1740
cggttttgcg tagcgcggtg tcgtagagtt taggtcgtgt tttcgtttcg tcgtttagag 1800
tttattgcgg cggtttattg gattacgtcg gtggggcgat cgtagttttt gatttgtgag 1860
ttgtaaagag tttcgaggtt tatttataaa tttgcgtttt tagtcgtttt tttacgtatt 1920
cgagttacgt ttcgggattc ggagagttcg gggcgttggg cgttgcggag gaggttcggt 1980
ttttgtcgtt ttttttattt tttagttcgc gagggatttg ggggaagggg gagtaagttt 2040
tcgtttcgga agaaacgttc ggaattaagg agtttgattt ttggattcgg gtgtttgttg 2100
gatttaggtt tttattttcg ggtttttcgg tcgaattaag ggtttcgata gggttcgagg 2160
tattcgtatt tttaggagat aggagttcgg ttagggcgta ttatcgggtt ttgtttcgag 2220
ttagaggatg tagtgtagat atttattttt atatcgtttt tttatagagt tttttttttt 2280
ttggggcgtc gttgggtata gggtaggtcg ttaggaattt ttagtaaaat tacgttcgtt 2340
tagaggttcg cggttttttt agagggcgtt ggggaaagag aggggatttg attttttcga 2400
ttttcggagg aaaagtgttc ggggtttttt agggatatag ttttggaagt ttattttatt 2460
ttagtttagg tcgcggcgag gtggggagga gagttaggag agggggagag gggttttgcg 2520
ttttgtagag gtttttaatt ttggaggaaa aagattggga tatatttaag cgagttaagg 2580
ttcgagtttt acgtattttt atttattcgg ggcggcgtat agttagtttt tgtcgggcgt 2640
gagtattcga ttaagggagt aagtggaatg aaaatttagt tgggggggtt tttatcgata 2700
taattgtttc gtagtcgagt ttttggattt ttgggagatg tggagagttt ggggtcggtt 2760
ttcgtttcgt agagtagatt ggattgtttt aggtgtttgg aatgcgtttg tattttgttt 2820

ttcggattcg cgggagattc gtgtttcgta agtttttttt tttattttag tttgttttta 2880
tttatatttc gtcggcgagt gtgttatcgc gaggcgtttt tttttcgggg aagggagttt 2940
tttttcgcgt agattcgtat tgtttttttt ttcgttcggt tttgtttttt aggggtagtt 3000
tttgtagaaa ggagattttt tttcgggtcg aaggttattt agtttgtagt tagtttagtt 3060
tcggattttg ggagatgttt attattttgc ggattttgat tcgaaatttt ttttggtcgt 3120
ttattgcgga gagtgtttcc gtagagaggt ttttaatcga aggagtttgg gttttatatt 3180
ttgtttttaa gtttgagttt ttagggttgt tttgtacgag gtgtagatga atttgtgttt 3240
gtaaataaga tagaaaattt taagtgtcgt tcgatttttt tttttggggg aattcgtatt 3300
ttgttttggg agcgtgttta gcgttttagt attaaatttt ttggtcgggg ttggtagagt 3360
ttagagtttc gttttttttt aggcgcggtt ttttaatatt tgtaatttaa atgttgcgtc 3420
gcggttaaaa ttagttttgg tagtgcgaat agagaattaa aagtaggtag tgaatgagaa 3480
tagttcgtat tttttttttt tggtagacgg ggaggtgtaa atttcgagga attttagggt 3540
attcgtttta aacgtgggaa attttcgcgc gtatttcgtt tttttttttt agtttgtgtt 3600
aatgttttaa atggtgtcga gttgtttaat tttgtcgtta ttatagaggt tgttgtgttt 3660
taggggatta attgatgtga gatatataaa attttgtaat tttataatat aaattatagt 3720
atagtttttt tggagagggt tggaatattt gagtgagttt tcgagaggaa aagaggagtt 3780
ttttagagga gaaatagagt atttttataa tgtgttttaa ttgagaaatt ttgttttatt 3840
gagttttttt ttaagtggaa ttagaagtgt tgggatgaga gggaaaggat gggagtgcgt 3900
ttaaaggtgg atagtaggtt tttatttttg gtgggagtga gattggacgg tatttttcgg 3960
aaaggtggtt tgggttttgg ataaggttag aggtaggagt ttatgatgta gagatgatat 4020
agtgtttttt cgcgtgtgag tttacgaagg ttattattga ggttttgtgt ttgtaaaagg 4080
tcgttacgtt ttatataagt ttttatattt aatataggga ttgattgggt atagggattt 4140
ttttatatta tatatgtaag tatgtatgtt aattaaagat gttcgtgtta aagaaacggt 4200
taattttgtt gaatttagag gaattgatta attatttaat taagtagagg aaatgtttga 4260
atttaattcg taatttagtt gtttttttat ataaaattat atatttttat ttatatttga 4320
tgaatgaaaa aagaaattag tttatgattt taatttaaat atatgttttt aaaaatatat 4380
tttttttagt ttagttagta tataaattaa ttgagttttt ttggttaagt atgatttagt 4440
tgtgatattt aagagcggga gtggttgttt agatattttt ttttttacgt gaaatttaga 4500
ttaatgagtt attatttaat aagttgtagg tagtttggtt gggttggatt tagatggttt 4560
gagttaaatt tagattgtat ttgtttaaaa ttttgtaaat ttttagttac gtaaatttta 4620
tttttaaaaa tgttgggaag ttattgtata agagtttaag ttatattagt tttttttgtga 4680
ttatttgtag tttttgggga aagaatagaa gaaaagaaaa tgttagtttt tgtgaggtgg 4740
ggttggtgtt ttaggggttt tttttgaata ttttgttttt tttttaaagg ttaaaaggaa 4800
ggtagtggat atatattaga atttttttta tttgtgaatg gtcgtaaggt tggagaaggt 4860
ggttagtgta ttttaaggtt tattattttt ttttgtgttt ttttttttgt tttggtaggt 4920
ttagttagtt taagttttgg gtgttatttt ttaaattttt ttgttaaatt aattttattt 4980
atttgattgg attatttgag aggtgttatt tttttttggg ttgttgtgat tttgaggggg 5040
tatttttata agagtttagg tattaggtgg tgaaatagtt tgtgttttta aatttgtttt 5100
ttttagggtt ttcgggagat tttagagtgt aggtttgttt ggggagtttt aggggtgggt 5160
tttgagtgga agtgggttta ttttttatag gagtttaaat tttataggaa taagaatagt 5220
agtaaaatag gataagagag taggtaggga gttgttaagg aaaggcgatt tttgggaagg 5280
taggttattt agaataaggt ttttttggcg ttggagagtc ggaaggggga gcgggtatag 5340
aggatttggt ttaggtgtgg gggttattag tataagtaga gttatttttt agattttttt 5400
ttagaagtag ttgtttttta gagaaattag gtgagggata gtttttgtat ttttatacgt 5460
tagttttgga gatttgttta tttgttttta gtcgtttttt ttttcgggcg agtttgggtt 5520
aagtattaag ttaggataga agggcggttt ttagtcggtt tttggtttat tttgtttttt 5580
taatatttag ggagtttggg tttagtatag gcgttttttt agcgatcggg gtagaattag 5640
gacgtgtaat gcgattgttt tttttttttc gtttttcggt agagtttttg ttcgcgttaa 5700
tttatttatt aggttttgtt cgcgttacgc gcgtttttgg taggtttggg gcggggaaag 5760
gcgaagcgtt gggtacgcga gggtttgtgt attttagttt ttacgacgtt tttggttttt 5820
tttaggttcg tcgttgtcgt atttaatttt tttttttcgg gggttatttt gaagaagttt 5880
tagattttag atttagtttt ttagatttcg ttttcgagtt cgttcggtgg gtttgtaggt 5940
cggttttttt tcgtttagag gagagcgtag atacgtaatg ttcgttcgtt ggtttcgttc 6000
gttttatttt tgtttttcgc gttttttcgt ttcggttttt gtttataggt cgggtcggaa 6060
cgttagtttt aggagtcgac ggtggttttt tgttcgtttc ggggaaggtg ttgttttttt 6120
atcggtttta atttttcgtt cggcgtttgg ggtttggttg cggggttcgg tttttagtcg 6180
agggcgtagg gttggttagg tttgttttgg ttgaggtgga gatttcgttt ttagggattg 6240
ttgggcgttt ttgtttcgcg agtaacgaga tcgtcgcgag cgaacggttt ttattgagtt 6300
tattttttta agtgttatta cgttaagtta gagaagcgag gcgagtggag gggacgtaga 6360
ggggtcggaa aagttatttt tttttggttt cgtttttaga taaaaacggg agtttggttc 6420
ggtattcggg cgttcggtgt tttcggggcg ttttattgag gttttgtttg taaaattagc 6480
gttcgtgttt tgaagcgtac ggcgtttgga agttgttttt ttgttcgttt ttttcgaggt 6540
ttttttttgt gtagcgagtt tgagaaatat ggaggatcgt ttttcgtaag cgggtggtcg 6600

cgggcgtttt tcgatttttg ggtgaagtta gagggaaaac ggggttttcg ggttagtgtt 6660
ttatttttta tttcgggaga aattaatgtt cggagagggt tcgtttgatt cgtatagaaa 6720
ggtcgatttt gagggcggcg gtcgttcggg ggagaaagcg gaggttttgg gttcgcggga 6780
gcgcggtagt cggggttggt atcgtagagg agagatacgt tattgtttcg tatttttaga 6840
aagcgcgagg cgtcgtttta gttggggtag gcggtcgagg tcggttttta tgcgcgtttt 6900
tcgaagtttt ttgaaatatt ttgcggagtt tcgtgtgtat agaatttagt atcgtttagt 6960
ttttgggtag tttaattttt tgtagtttta ttttagtttt ttcggttttt tgaacggtga 7020
ttttttttat cgttttttta gagttgtttc gtgtttgggt ttattttggg gggttcggta 7080
tttttagtt atttttttt ttatttattt ttttttttt tattttttt tttatttttt 7140
attttttttt tttttaggag agcgatttac gaattttttt ttttttagtt aattttaggg 7200
tttagttcgt agattttgcg agggtaggtt tttgtttatc ggttcgttag gcgttttgga 7260
ggtgacgttt tgttttttag agtttttgtt gtagttacga attggggttt gggtcgtagg 7320
aaagtatagg gttgaagttt agcgttttgg ggttatttat attgaggtag ttagaggtaa 7380
agagttttaa gaatttagaa aatatttttt aggaagtcgt ttaattggtt tttatgggat 7440
aggtggagtt attaatttgg gatggttttg taggaattaa agagtttagg gttttttttt 7500
tttttaatat tatgtttagg agatttagag tcgttggatt tttttttttt tgattggtga 7560
ttattagagt ttttagagtt gtagaaaatt ttttttttaa aaaattaagt aagcgttaat 7620
aagatttttt ataaattttt attagtttta ttttttcggg gggtaggtag attgtggggt 7680
ttgatttttt gagattcggg gaggattttt ggtagatgtg tgtttagtta gaatatttgg 7740
taaggatttt tttaatgaag aaaaagtgga ggaatttagt tttagcgaga agaggttttt 7800
ttattttgtt ttagatatat cggatagagg gtatattttg attagagtta cgtttagtgg 7860
ttaggaggtt agtttagtat tttttttttt attattttcg ttttgggtgg gggggtaatt 7920
tttttgggag tagttgtggg aattgtcgtt ttttatttta gtttagttag tattttgaag 7980
tttgtagggg aaggatagta cgtgggatgg atattgggga aggagtttcg taaggttagg 8040
gtgtaatttt taggttttag gtggtttggt aggttacgtt gtttcggaga tgtttgttag 8100
attttttaag tttatttagg gtttggtagt aatttgttgg ttgtttttgc gggggtttgg 8160
gttgttgagt acggtgtagt tgtttagggt taattagttt tagggtgttc gtgttaggtt 8220
gcggtttttt cgtttttttc gtattgaggg tatttatggc gtgtaaatgt tttcgtattt 8280
ttagagttgt tttatcggat gtttttagga atttatatat ttttataaaa atgtatttta 8340
aatgatggat aggcgagttt ggggtaataa cgggtgtttg gtgggtagat aagagtaaat 8400
gggaaggagt tcgagggagg agggggaaga gaagaggaaa tagaattttt agttggatat 8460
tttgataata gttggaagga aagtttagaa aagatgaaga gagaggaggg gagaaattaa 8520
ttggggtttt tatttttgtc gttggatttt taattttcgt tttaaatggg ttttgttttt 8580
cggtaaaatt agtttaaagg attttaaaat aaagaaaacg agatgatcgg tttgggagtt 8640
ttttaattag agtagagaag ttagaggggg gcgggcgatt tggttttgaa gttttagttg 8700
aatagttatt tttttttttt ttggtaaaaa ggattttttt agaattttcg aggtttttgg 8760
attttttttt tcgtaaatgg agtcgtatat tgtatttttt cgttttttcg gatcgttaag 8820
tatgttttat gagggtcgtt gttttcgggt ggaatgcggt cgtatgtacg cgtttttttg 8880
tatacgtata tatacgtata tttataataa gtgtttgtag gaggagtgtt ttgcgcgtta 8940
gttttgcgtt taagatagga agttgtcggg ttatcgagtt aaatgggagt gatattattt 9000
ttttttatta gtaaggaaag cggattataa aagttttttt gtatttcggt agtttattta 9060
atattattta tgtattttgt gtaaggaatt gtgggatttc gttttacggt aaataatatg 9120
gaaattttaa aaatagcgat ttttttgtgc gtgtttattt acgcgtttcg gggtgatttg 9180
gcggggttgt cgtcgggtga tttatatttt tgaatcgcga agcgataggg aaagcgcggg 9240
cgagcgtagg agacgcggtc gggggttttt tcgggttttt gggttttcgt attcggagcg 9300
ggggacgcgg tcgttttaag gggaggaggg gcggcgggtt gtttttgtta tttagcggcg 9360
gtcggagcgt tacgtgggcg cgcggcgtcg cggttattgg ttcgaggtac gtgtttagga 9420
gatcggtttg cgacgttatt cgagggggtt ttgttaaaaa taagaataaa aatttagagt 9480
gaaagtgttt taggttgcgt cgagtggttt ggaaattttc gagttcgcgc ggaggtcgag 9540
gcggcgaggg cggcggacgg tcggggagcg cgggcggttt agttcggttc ggtcgggttt 9600
tggtttcgcg ttttttattt atgcgattcg ggtcgcggag ttttgcgggg ttcggcgggg 9660
gcgcggtcgt acgtcggtgg ggcgtttcgg ttcgtagcgg ggcggcggtc gcgaggaggg 9720
ggtttttatg tgcgtgcggg cggtggcggg cgcgttgatc gcgggcgttc ggtattttcg 9780
agggtcggtt agggcgtgcg ggcggggacg gtcgggcggc ggcggcggtc ggagtcggtt 9840
cgggcgggcg tgagcgtcgg ggaacgcgtt gtttgtatgc gcgtagtttt cgtttcgggc 9900
ggtttaggcg gcggcgtcgg agttcgaggc ggtcggacgc ggagaggagc ggggagttcg 9960
ggaggcggtt cgcgttttcg tcggattatt gcgattgttt agatttcggt tgcgcggcga 10020
agtcgaggat ttggttttgt tgaatttttt atcgtttggg cgagcggggc ggttcgtggt 10080
gtttttaatt tagttcgtgg atttaaaggt ggtttcgcgt cgagcgcggt cggcgatttg 10140
taggatttta gttttggtcg cggtcgtcgc gtacgttttc ggaagattcg gcggggtggg 10200
ggcgcggggg ttttcgtgtg cgtcgcggga gggtcgaagg ttgatttgga agggcgtttt 10260
cggagaatta gtgtgggatt tattgtgaat agtatggagg agaatgattt taagtttggc 10320
gaagtagcgg cggcggtgga gggatagcgg tagtcggaat ttagtttcgg cggcggttcg 10380

ggcggcggcg gcggtagtag ttcgggcgaa gcggatatcg ggcgtcggcg ggttttgatg 10440
ttgttcgcgg ttttgtaggc gttcggtaat tattagtatt cgtatcgtat tattaatttt 10500
tttatcgata atattttgcg gttcgagttc ggtcggcgaa aggacgcggg gatttgttgt 10560
gcgggcgcgg gaggaggaag gggcggcgga gtcggcggcg aaggcggcgc gagcggtgcg 10620
gagggaggcg gcggcgcggg cggttcggag tagtttttgg gttcgggttt tcgagagttt 10680
cggtagaatt cgttatgtgc gttcggcgcg ggcgggtcgt ttttagtcgt cggtagcgat 10740
ttttcgggtg acggggaagg cggttttaag acgttttcgt tgtacggtgg cgttaagaaa 10800
ggcggcgatt tcggcggttt tttggacggg tcgtttaagg ttcgcggttt gggcggcggc 10860
gatttgtcgg tgagttcgga ttcggatagt tcgtaagtcg gcgttaattt gggcgcgtag 10920
tttatgtttt ggtcggcgtg ggtttattgt acgcgttatt cggatcggtt ttttttaggt 10980
gagttcgcgg ggattacgcg tttcggttcg tcgcggggag gttcgcggag ttggggggcg 11040
gtgttggcgc gggaatttat cgggaggaaa atatttcgaa tttttttcgc gtatacgtat 11100
aaagatttac gcgatattgt gtgaagttga cgtcggttcg ggtagcggtt aggagtttag 11160
cggtaggatt gattcgttag ggggtataga ttttttagga tcgtagaagg gatttttttt 11220
ttttttttgt tttttttttt tttttttttt tttttgtttt ttttttttgt tttttatttc 11280
gttttggcgt atttttttt agttttagt ttatgtttt tttattgtag tttttttgg 11340
tgggaacgtg gtggttggaa gatgggttcg gaagtgtata tttttatttt tttttttacg 11400
attttttaat ttaggttagg tcggggacgt atgttttagt ttattttaga tttgttttat 11460
tattcggtta tttcggttgt gttcggggaa gaaaaggcga ggttttttgt cgttttgttt 11520
tttgtttttc gggtttgcgt tgatcggtgg gatttaggag gatgtatata gggaaggagg 11580
aaaataaagg cgtttttttt ttttggtttt attttgtttg ttagcgttag ttcgtagtgg 11640
tggggtttag tttttttttt gtatatagcg aggataaggg aggtagtcgt ttttttcggt 11700
atttgttatt tttaaataga aaggattttt ttttagggtt ttttgggggt tgttgatggg 11760
aaagaggtag tattcgtagg ggttttgtag agatgttgga tatatttttt tatagatttg 11820
cgattttaaa aaattaagtt tatgtttttg tagaaattat taattgtatt ttatgcgggt 11880
ttgcggttgg gaatcgttat tagaagtgga ttgtttgatt tcgagttggt agcggatttt 11940
cgttgttttt aaatttttaa ttattttgcg ggggttattt gtttagatta tagtaggagt 12000
gagttaattt ttgggtcgtt atttcgtaga attatgcgtg tatatttttg atgaaattta 12060
gattttttag ttagatttga aatttgtttt attgttttcg tttttttttt ttgttaatat 12120
ttaattaata tataggttta taatgtcggg cgaggagatt cggtcgggtt ttgtgcggcg 12180
cgggagttcg ttgagttagt ttttaacggt tcgggagttg ggtagtatcg ttcggttcgg 12240
tttggttcgg tttagtttag tttagtttaa gtcgtttatt tttatgggtt ttaaaatatt 12300
tttgtaagat aatgtttttg ttttttggtt ttttcgaaag aaaggggaga gagagttttt 12360
ttggggaggt ttgattttgt ttttgagatt tttaagtatt tgttttttga aagaaaatta 12420
agaaaaaaat ttaaaaatta ttattttagg gaaatttatt gttataaaat ggtgtttttt 12480
tgcgggttgt tttatgagtg tattaataag agttttagga ttagaagagt ttgggggtag 12540
agttttgggg aagggagtgg ttggaaattt agatagagat gggttttggg agtaggaggt 12600
tggggttttt tttggagttt tgtgttttat tttttattat cgtttcggag ggttaatttt 12660
atttttaaat ttgtatttat ttttattaaa gttaggttta ttggtttgga gttttgggcg 12720
tgagtaagat aggtattgag tgtgtacgtg tgtatggggt gggtgtttaa gtatagggtg 12780
tgtgttttta tgggtggtga gtttgtttat gggttgtttt aaaagttgtt tttggtgttt 12840
ttgaggtggt gtttatagat tttttttttt taggtttgtt ttttggagag agtataagat 12900
ttatttggtt atgagggagt gtttggtatt tattttgggt ttttagttcg ttttttattt 12960
tttgttgggt atagttttag tattttagtt gatttttttg atttgggtag ggtgtagttt 13020
tagggttttt aaggagattt atattttttt tttttttagt gtgttcggta gtttttcggt 13080
tttgaagggt gggggttttt tagttttttt tagttatagg gatttgtgat gaagttgggg 13140
ttagatgttt tttaaagtcg atttatatat cgtataaatt gaaatttaga ggcgaggtta 13200
ttatttttg ttagtggttt tgttttttt ttttttata gggaacgtta ggggttgag 13260
ttttttatta ttaaaaagaa attgatgata tttttttttt tttgtttttt tttttttgtt 13320
ttttttttat ggatagtagg ttttagaagt tttatagcga ttttgtttaa aatttggggt 13380
aggtttatag ggagaaggtt aggttaggtt tataagtttg aattttagtt gggaggtata 13440
gtggggaggg ttagaagtgg atttggataa ggttagttgg gttattttgt tgtttatagt 13500
gaagtagttt tatgtttggg gaaagggtgg tgtagttaat atttttgtag agttaggttt 13560
ttttttttgg ataggaaatt tgggagattt ttagtgggtg aaggatttat ttattgtgag 13620
tagtttagtg ttttttttta ttaaggaggg aagtatatgt attgattttt ttttaaagga 13680
atgaatttgg gtttatagag tttttggttg ggagttatag aggagttggg gtggaggtag 13740
atattttggg ttttttttgt ttttagggtt tatttgtttt tgatttttat agtttttggt 13800
attttgtggg gtatttttat gagggttttt attatagttt ttagggcgtt ttttgttttt 13860
gtgatcgttt tgtagttttt tttagttttt tttttttttt ttttttttag tatttatcgt 13920
tatttttgtt tttgaataga gagttttaga aaggattagg aaaaattagg ttagaaagtg 13980
tggggagttt tgtttatatt taggagtttt atttttattt agagattttt tatttgtggt 14040
tagtttgttt attaggtttg ttttatagtt ttatttatat tatacgtagt ttttttttat 14100
taagtggtgg aggttcgcgt tgagtttatg tttagttcga agtttagttt tatatttggc 14160

ggtttagttt cgagtggttt tgggcgagtt attttttttt tggggtttta gaatttttat 14220
tcggtgttta ttgggtggta tatttttggg taatttgatt tttttttgtt tatcgtattt 14280
atttaggttt taggtttcga aaattaaaga agaagaattc gaataaagag gataagcggt 14340
cgcgtacggt ttttatcgtc gagtagttgt agaggtttaa ggtcgagttt tagattaata 14400
ggtatttgac ggagtagcgg cgttagagtt tggcgtagga gttgagtttt aacgagttat 14460
agattaagat ttggttttag aataagcgcg ttaagattaa gaaggttacg ggtaataaga 14520
atacgttggt cgtgtatttt atggtatagg gtttgtataa ttattttatt atagttaagg 14580
agggtaagtc ggatagcgag tagggcgggg ggtatggagg ttaggtttta gttcgcgtta 14640
aataatgtaa taatttaaaa ttataaaggg ttagtgtata aagattatat tagtattaat 14700
agtgaaaata ttgtgtatta gttaaggttt tgaaatattt tatgtatata ttatttatag 14760
gtggtataaa atttaaaata tttgattata aaatattttt ttgagttttt tgtgtttatg 14820
agattatgtt aattttatgg gttttttttt tttttgcgaa gggggttgtt tagggtttta 14880
ttttttttta attttttaag ttttattata tgatattgga tattttttta ttattttaaa 14940
agaagaaaaa attaaaataa tttgttgaag tttaaagatt ttttattgtt gtattttata 15000
taattgtgaa tcgaataaat agtttttatt tggtttatga tttttgttat tttgtttgtg 15060
ttggtttggt gaggatagta ggaggggttt atattttaag tttggattag ttattttaag 15120
gttttgggga gtttagggga tttggtggga gagaggggat ttttagggtt tttgggttag 15180
ttttgggatt tggttttggg aagtagttta gcgtatttta ggtttgtttt gggaagtcgg 15240
ttttatgttt attagtagtc gtttaggttc gtagttttat tcggtttttt ttttttattt 15300
ttttgtattt aatttttttt tttttttttt tttttttttt tttttttttt tttttttttt 15360
ttttgttttt tttttttttt tttttttttt tttttttttt tttttttttt tttttttttt 15420
tttttttttt tttttttttt attaagggtt taatcgtgtg tatatatcgt ttgcgtttgt 15480
ggtttgtgtc gttgttttta gttttatcgt agttttgtcg taggtttaat ttttttgttt 15540
tgggtattgt ttttatgtag aagcgtttcg aggttttggg gttaaaggtt tggggtgtgt 15600
ggtttaaagt ttaagagcgg tggggcgatt ttttttttgg tttggtttta ggaatttttt 15660
gtgattttat tagttattat gggtgttagt tagggtttta gaaatgaggt tatggtttat 15720
tgtttttggg cgggtagaag gttttgtaga gggagatggt attatttatt tttttttttt 15780
tttttttttt ttttttattt tttttttttt tttttttatt tttttttttt tttggagtgg 15840
ttgtttttgt tatagagaat atttttttaa gataaatatg tgtgtttata tatatgtttg 15900
tatgtatgtg aatatatata tatatatata tatattaggc gtgtttgagt ttatagtttt 15960
gaaatatgtg gttattttgt tttttaaaag aatttagaat tttttaggat ttagaagaag 16020
gaagaaagtg tgtaaataat tattttttat tattattttt tgtttttttt tgttttttaa 16080
aatatatatt ttatttttga aggtgtggta tagtgtaaat taaatatatt taatatattt 16140
tttattaagt atttatatat gtatataaat aaatatatta tttatatata acgttat 16197

<210> 13
<211> 16197
<212> DNA
<213> Artificial Sequence

<220>
<223> chemically treated genomic DNA (Homo sapiens)

<400> 13

gtggcgttat atatagataa tgtgtttgtt tatatatata tataggtatt tggtgggaaa 60
tatattgaat atatttaatt tatattgtat tatattttta aaaataaaat gtatatttta 120
aaaaataaga aaagataaaa agtgatgata agaaatgatt atttatatat tttttttttt 180
tttttagatt ttggaggatt ttgagttttt ttgaaagata aggtagttat atgttttaga 240
attgtggatt taaatacgtt tggtgtgtgt gtgtgtgtgt gtgtgtttat atgtatgtag 300
atatatgtgt aaatatatat atttattttg gaagaatgtt ttttatagta gaagtagtta 360
ttttaagaaa agaaaaaaat aaaggaaaaa aagaaaaaaa tagggaagaa aagaaaaagg 420
aaaggaagat agatgatgtt attttttttt atagagtttt ttgttcgttt agaaatagtg 480
agttatggtt ttattttggg gattttggtt ggtatttatg atggttggtg gagttatagg 540
aaatttttgg ggttaagtta aaaggagggt cgttttatcg tttttgggtt ttaggttata 600
tattttaggt ttttagtttt agaatttcga agcgtttttg tatggaggta gtgtttaggg 660
taggagggtt aggtttgcgg taggattgcg gtgggattgg ggatagcgat atagattata 720
gacgtagacg atgtatgtat acggttgggt ttttggtgag gaggaggagg aaagagaagg 780
aggaggagga aggaaggagg aggaggagaa gaaaaagaag aagaaaggag gagtaggagg 840
aaggaggaag gaggaagagg aggaaaaagg agaaggagga gggagttagg tgtaggaggg 900
tgaggagagg gagtcgggtg aggttgcggg tttgggcggt tgttggtgag tatggagtcg 960
attttttaga gtaggtttgg ggtacgttgg gttgtttttt agggttaaat tttagaattg 1020
gtttaaggat tttggaagtt tttttttttt tattaggttt tttaagtttt ttaaggtttt 1080

gaggtggttg gtttaggttt gaggtgtggg ttttttttgt tgttttttatt aagttaatat 1140
aaataaagtg gtagaagtta tagattaaat aggagttatt tattcggttt atagttgtgt 1200
gaaatgtagt aataaaaaat tttggatttt tagtaagttg ttttaatttt tttttttttt 1260
ggaataataa aaaagtgttt aatgttatat aatggagttt aggggattaa aaaaaggtga 1320
aattttaagt agtttttttc gtaaaaaaga aaaaaattta taaaattagt ataattttat 1380
aaatataaaa aatttaaaaa aatattttat agttagatat tttggatttt atattatttg 1440
taaatgatat atatatagaa tattttagaa ttttagttaa tatataatat ttttattatt 1500
aatgttggta taatttttat atattggttt tttatgattt taaattattg tattgtttag 1560
cgcggattga gatttggttt ttatgttttt cgttttattc gttgttcgat ttgttttttt 1620
tggttgtggt ggagtggttg tataagtttt gtgttatgag gtgtacggtt agcgtgtttt 1680
tgttgttcgt ggtttttttg attttggcgc gtttgttttg gaattaaatt ttgatttgtg 1740
attcgttgag gtttagtttt tgcgttaggt tttggcgtcg ttgtttcgtt aggtatttgt 1800
tggtttggaa ttcggttttg agtttttgta gttgttcggc ggtaaaggtc gtgcgcggtc 1860
gtttgttttt tttgttcggg tttttttttt ttggttttcg agatttggga tttgggtaga 1920
tacggtggat agagagaagt taggttattt agaggtgtgt tatttaatag gtatcgggta 1980
aggattttgg ggttttaaga gggaagtgat tcgtttaagg ttattcgagg ttgaatcgtt 2040
agatgtgggg ttagatttcg gattgggtat gggtttaacg cggattttta ttatttggtg 2100
agggaggatt gcgtgtgatg taagtgggat tatggggtag gtttagtggg taggttgatt 2160
ataaatgagg ggtttttaga taaaagtaaa atttttggat gtaagtaaaa ttttttatat 2220
tttttggttt ggtttttttt agtttttttt gaggtttttt gtttaggaat agggatggcg 2280
gtaggtgttg agagagaggg gaggagagaa gattgggaaa ggttgtaggg cggttataga 2340
agtaggaggc gttttgaggg ttatgatggg gatttttatg ggatattttt ataggtgttt 2400
aagggttgtg ggaattaggg gtaggtgggt tttgaggata ggagggggttt agagtgtttg 2460
tttttattta agttttttta tgatttttag ttaagggttt tgtggattta agtttatttt 2520
tttaaggaag agttagtgta tgtatttttt tttttggtgg ggaggggtat tgagttattt 2580
atagtgggtg agttttttat ttattggaaa ttttttaggt tttttgttta ggagagggga 2640
tttggtttta taagggtgtt gattgtatta tttttttttt agatatggga ttgttttatt 2700
gtgggtagta gggtagttta gttgattttg tttaggttta tttttgattt tttttattgt 2760
gttttttaat tgggatttag atttatgaat ttgatttggt tttttttttg tggatttgtt 2820
ttaggttttg gatagggtcg ttgtaaggtt tttaggattt gttatttatg gggaaagggt 2880
agagggagga gagtagaagg agggaagtgt tattagtttt tttttggtga taagaggttt 2940
aattttttgg cgttttttgt gggggaagaa gggggtaagg ttattggtag ggagtggtga 3000
tttcgttttt gggttttaat ttgtgcggtg tatgaatcgg ttttagggag tatttggttt 3060
tagttttatt ataggttttt gtggttggag aaggttgggg gttttttatt ttttagggtc 3120
ggagagttgt cgggtatatt gaggagaaga ggaatgtgga tttttttgga ggttttggga 3180
ttgtattttg tttaggttag gagagttaat tagggtgttg gggttgtgtt tagtaagagg 3240
tgggggacga attggaggtt tagggtgaat gttaagtatt tttttatggt taagtgagtt 3300
ttgtgttttt tttaggagat aggtttggag aaagaggatt tgtgggtatt attttaggag 3360
tattaagggt agtttttaaa gtaatttata ggtaggttta ttatttatga gagtatatat 3420
tttatatttg ggtatttatt ttatgtatac gtatatattt agtgtttgtt ttgtttacgt 3480
ttagggtttt aggttagtgg gtttggtttt ggtaggggta ggtgtagatt tggagatggg 3540
gttggttttt cggaacggtg gtgggggatg gggtataagg ttttaggaga ggttttagtt 3600
ttttgttttt agagtttatt tttgtttggg tttttaatta tttttttttt tagaatttta 3660
tttttaaatt tttttggttt tggggttttt gttaatgtat ttatgaagta gttcgtaaaa 3720
ggatattatt ttatggtaat aaattttttt aaaataataa tttttaggtt ttttttttga 3780
ttttttttta aaagataaat atttaggagt tttagaaata gaattaaatt tttttaagaa 3840
aatttttttt tttttttttt tcggagaaat taaagaatag aaatattatt ttgtagggat 3900
attttaaagt ttatgaagat aggcgatttg ggttgggttg agttgggtcg ggttaggtcg 3960
ggtcgggcgg tgttgtttag ttttcgggtc gttgggggtt ggtttagcga attttcgcgt 4020
cgtataaagt tcggtcgagt tttttcgttc ggtattgtga gtttgtatgt taattaaata 4080
ttaataaagg aggaagacga gaataatgga gtaaatttta gatttagttg agaaatttga 4140
attttattag aaatatgtac gtatagtttt gcgggatggc ggtttaaggg ttggtttatt 4200
tttgttgtga tttgggtaaa tgattttcgt aaaatagttg agggtttggg ggtagcgggg 4260
attcgttgtt agttcggggt taaatagttt atttttaatg gcggttttta gtcgtagatt 4320
cgtatggaat gtagttgatg atttttataa ggatatggat ttagtttttt aaaatcgtag 4380
atttatgaaa aaatatattt agtatttttg tagggttttt gcgaatgttg tttttttttt 4440
attagtagtt tttaggaaat tttgagagaa ggtttttttt atttggggat ggtaggtgtc 4500
gggagggacg gttgtttttt ttgttttcgt tgtgtgtagg aagggggttg agttttatta 4560
ttgcgggttg gcgttggtaa ataaagtgga gttaagggga aagggcgttt ttattttttt 4620
tttttttttgt gtgtattttt ttgagtttta tcggttagcg taggttcgag ggtagaaggg 4680
tagagcggta aagggtttcg tttttttttt ttcgagtata gtcgggataa tcggatggta 4740
ggataagttt ggggtgggtt ggggtatgcg ttttcggttt ggtttgagtt ggaagatcgt 4800
agggaagggg atgagagtgt gtatttttcgg gtttattttt tagttattac gtttttatta 4860

aaaaaaatta tagtggggga gatatgggtt aggggttggg aagagatgcg ttaaggcggg 4920
gtggaagata gagaggggag atagggagag aggaaggaga gagagagata gagaaagaaa 4980
gaaggttttt tttgcggttt taagaagttt gtatttttta gcgaattagt tttgtcgttg 5040
gatttttggt cgttgttcgg gtcggcgtta gttttatata atgtcgcgtg agtttttgtg 5100
cgtgtgcgcg ggggaggttc gagatgtttt tttttcggta agttttcgcg ttagtatcgt 5160
tttttagttt cgcgggtttt ttcgcggcga gtcgggacgc gtggttttcg cgggtttatt 5220
tgaagaaggt cggttcgagt agcgcgtata gtagatttac gtcggttaga gtatgggttg 5280
cgcgtttagg ttggcgtcgg tttgcgagtt gttcgagttc gagtttatcg ataggtcgtc 5340
gtcgtttaag tcgcgggttt tgagcgattc gtttaggggg tcgtcggggt cgtcgttttt 5400
tttggcgtta tcgtgtagcg agagcgtttt ggagtcgttt ttttcgttat tcggagagtc 5460
gttgtcggcg gttgggagcg gttcgttcgc gtcgggcgta tatggcgggt tttgtcgggg 5520
ttttcgggag ttcgagttta agagttgttt cgagtcgttc gcgtcgtcgt tttttttcgt 5580
atcgttcgcg tcgttttcgt cgtcggtttc gtcgtttttt ttttttttcg cgttcgtata 5640
gtaggttttc gcgttttttc gtcggtcgaa ttcgggtcgt aggatgttgt cgatgaagaa 5700
gttggtgatg cggtgcgggt gttggtggtt gtcgggcgtt tgtaggatcg cgggtagtat 5760
tagagttcgt cggcgttcgg tgttcgtttc gttcgggttg ttatcgtcgt cgtcgttcga 5820
gtcgtcgtcg gggttggatt tcggttgtcg ttgttttttt atcgtcgtcg ttgtttcgtt 5880
aggtttgggg ttattttttt ttatgttgtt tatagtaaat tttatattgg tttttcgggg 5940
acgttttttt aaattagttt tcggtttttt cgcggcgtat acggagattt tcgcgttttt 6000
atttcgtcga gtttttcgag ggcgtgcgcg gcggtcgcgg ttagggttga ggttttataa 6060
gtcgtcggtc gcgttcggcg cggagttatt tttgaattta cgaattgggt tagaaatatt 6120
acgagtcgtt tcgttcgttt agacgatgag agatttaata gagttaagtt ttcgatttcg 6180
tcgcgtagtc ggggtttaga tagtcgtagt ggttcggcgg ggacgcgggt cgtttttcgg 6240
gtttttcgtt ttttttcgcg ttcggtcgtt tcgggtttcg gcgtcgtcgt ttgggtcgtt 6300
cggggcgaga gttgcgcgta tgtaggtagc gcgtttttcg gcgtttacgt tcgttcgggt 6360
cggtttcggt cgtcgtcgtc gttcggtcgt tttcgttcgt acgttttagt cggttttcga 6420
gggtgtcggg cgttcgcggt tagcgcgttc gttatcgttc gtacgtatat ggaggttttt 6480
ttttcgcggt cgtcgtttcg ttgcgggtcg ggcgttttta tcggcgtgcg gtcgcgtttt 6540
cgtcgagttt cgtagagttt cgcggttcga gtcgtatggg tgagagacgc gaggttaggg 6600
ttcggtcggg tcgggttggg tcgttcgcgt ttttcggtcg ttcgtcgttt tcgtcgtttc 6660
ggttttcgcg cgggttcgga aatttttagg ttattcggcg taatttgaga tatttttatt 6720
ttggattttt gtttttattt ttaatagagt tttttcgagt gacgtcgtag gtcggttttt 6780
tggatacgtg tttcgggtta atggtcgcgg cgtcgcgcgt ttacgtgacg tttcggtcgt 6840
cgttgggtga taggagtagt tcgtcgtttt tttttttttt aaagcggtcg cgtttttcgt 6900
ttcgggtgcg ggagtttagg aattcggaga gattttcgat cgcgtttttt gcgttcgttc 6960
gcgttttttt tgtcgtttcg cggtttaggg gtgtgagtta ttcggcgata gtttcgttag 7020
gttatttcgg ggcgcgtagg tggatacgta taggaagatc gttattttta agatttttat 7080
attgtttatc gtggggcgaa attttataat tttttgtata aaatgtataa ataatattaa 7140
atgagttgtc gagatataaa gggatttttg tggttcgttt tttttgttga tggagaggaa 7200
tagtgttatt tttatttgat tcggtaattc ggtagttttt tgttttaaac gtagagttgg 7260
cgcgtaggat attttttttg tagatattta ttgtaagtgt gcgtgtgtgt gcgtgtgtag 7320
ggaggcgcgt gtatacggtc gtattttatt cggggatagc gatttttatg aaatatgttt 7380
agcgattcga aagagcgggg gaatgtagta tgcggtttta tttgcgaagg gagaaattta 7440
ggagtttcgg aggttttaaa ggaatttttt ttgttaagga gaggaggggt gattgtttag 7500
ttaagatttt aaaattaagt cgttcgtttt tttttaattt ttttattttg attgaggagt 7560
ttttagatcg gttatttcgt tttttttgtt ttaaaatttt ttaaattaat tttgtcggag 7620
agtagggttt atttgaaacg agaattagga atttaacggt aagggtggga gttttagttg 7680
gttttttttt tttttttttt ttattttttt tagatttttt ttttagttgt tgttagaatg 7740
tttaattgga agttttgttt tttttttttt tttttttttt ttttcgggtt tttttttatt 7800
tgtttttgtt tatttattaa atattcgttg ttattttagg ttcgtttgtt tattatttaa 7860
aatgtatttt tatggaaatg tgtgaatttt tggaaatatt cgatagggta gttttggggg 7920
tgcgggagta tttgtacgtt atgagtattt ttagtgcggg ggaggcgggg aggtcgtagt 7980
ttggtacgga tattttaggg ttgattggtt ttgggtagtt gtatcgtgtt tagtaattta 8040
gattttcgta gaggtagtta gtaggttgtt gttaggtttt gagtgaattt ggggagtttg 8100
gtaagtattt tcgaggtagc gtggtttgtt aggttatttg gggtttgaag gttgtatttt 8160
ggttttgcga agtttttttt ttagtgttta ttttacgtgt tgtttttttt ttgtaggttt 8220
tagagtgttg gttgggttgg gatgagaaac gatagttttt atagttgttt ttaggaaaat 8280
tattttttta tttaggacgg gaatggtggg ggaggaggtg ttgggttggt tttttggtta 8340
ttggacgtgg ttttggttag agtgtgtttt ttgttcggtg tgtttagggt agggtgggga 8400
agtttttttt cgttggggtt ggattttttt attttttttt tattggagga gttttttatta 8460
aatgttttgg ttaggtatat atttgttagg ggtttttttc gagttttaaa aaattaaatt 8520
ttatagttta tttgtttttc gggaaaatag ggttggtgag agtttgtggg aagttttatt 8580
gacgtttatt tggttttttg gggggagaat tttttgtaat tttggaaatt ttgatgatta 8640

ttagttagga aagggagggt ttaacgattt tgggtttttt gaatatgata ttggggggag 8700
gaggggtttt gagttttttg gtttttgtaa aattatttta ggttaatggt tttatttgtt 8760
ttatgaaagt taattaggcg gttttttgag aagtattttt tgaatttttg ggattttttg 8820
tttttgattg ttttagtata aatggtttta agacgttggg ttttagtttt gtgttttttt 8880
gcgatttaga ttttaattcg tggttgtaat agagattttg ggaagtagag cgttattttt 8940
agggcgtttg gcgggtcggt gggtagagat ttgttttcgt agggtttgcg ggttgggttt 9000
tggggttggt tggaggaaga ggaattcgtg ggtcgttttt ttggaaagga ggaaggtgag 9060
aggtggaggg aggaatagga gaaagagaaa taaatgaaga gagagataat tagagggtat 9120
cgggtttttt agagtggatt taaatacgaa ataattttgg gaaagcggta agaggggtta 9180
tcgtttaaag ggtcggggaa gttgggatgg ggttgtagaa agttgagttg tttaggggtt 9240
gggcggtgtt gagttttata tatacggaat ttcgtaaggt gttttaggaa gtttcgagaa 9300
gcgcgtatga ggatcggttt cggtcgtttg ttttagttgg gacggcgttt cgcgtttttt 9360
ggggatgcgg ggtagtggcg tgtttttttt ttgcgatgtt agtttcggtt gtcgcgtttt 9420
cgcgagttta gagttttcgt tttttttttc gaacgatcgt cgtttttaag gtcggttttt 9480
ttgtgcggat tagacgagtt tttttcggat attagttttt ttcgggatgg aaagtggggt 9540
attgattcgg gaatttcgtt ttttttttag ttttatttag gaatcggaga gcgttcgcga 9600
ttattcgttt gcggaggacg gttttttata ttttttaggt tcgttgtata aagaggggtt 9660
tcggagaggg cgagtaggaa agtagttttt agacgtcgtg cgttttagga tacggacgtt 9720
agttttgtaa ataaaatttt agtgaagcgt ttcggagata tcgggcgttc ggatgtcgag 9780
ttaggttttc gtttttattt gaaaacgagg ttagagaagg gtgatttttt cggttttttt 9840
gcgttttttt tattcgtttc gtttttttag tttgacgtgg tgatatttgg aaaaatggat 9900
ttaatgaaag tcgttcgttc gcggcggttt cgttattcgc ggggtagaga cgtttaataa 9960
tttttgagag cgagattttt attttagtta aagtaggttt ggttagtttt gcgttttcgg 10020
ttgggagtcg agtttcgtag ttaagtttta ggcgtcgggc ggggaattgg ggtcgatagg 10080
agagtaatat ttttttcggg gcgagtagag agttatcgtc ggtttttggg gttgacgttt 10140
cgattcggtt tgtagataaa ggtcgaaacg aaggggcgcg gggagtaggg gtggggcggg 10200
cggggttaac gggcggatat tgcgtgtttg cgtttttttt tgggcgaagg aggatcggtt 10260
tataggttta tcgggcgagt tcggggacgg ggtttgggag gttgggttta gggtttgaga 10320
tttttttaga ataattttcg gaaggagggg gttaaatgcg gtagcggcgg gtttgaaaag 10380
ggttaggagc gtcgtaggaa ttggggtgta taggttttcg cgtgtttagc gtttcgtttt 10440
ttttcgtttt aggtttgtta ggggcgcgcg tggcgcgggt agagtttggt gggtgggttg 10500
gcgcggatag aagttttgtc ggggagcggg gagggggggg tagtcgtatt gtacgttttg 10560
gttttgtttc gatcgttgga gaagcgtttg tgttgagttt aagtttttta ggtgttgggg 10620
aagtagagtg ggttaggagt cgattgggga tcgttttttt gttttgattt gatatttagt 10680
ttaggttcgt tcggggaggg gggcggttgg gggtaggtgg gtagattttt agagttggcg 10740
tgtaaagatg tagaaattgt tttttatttg gtttttttgg aaagtagttg tttttgaagg 10800
agaatttgaa ggatggtttt atttgtgtta gtaattttta tatttaaatt aggttttttg 10860
tgttcgtttt ttttttcggt tttttagcgt taggggagtt ttattttggg tgatttgttt 10920
ttttaagaat cgtttttttt tggtagtttt ttgtttgttt ttttgttttg ttttattgtt 10980
gtttttattt ttataaaatt tggatttttg tggaaggtgg gtttattttt atttaaggtt 11040
tatttttgag attttttaga tagatttgta ttttggagtt tttcgggagt tttgggaggg 11100
gtaggtttgg gagtatagat tgttttatta tttaatgttt ggatttttat gaaaatgttt 11160
ttttaaaatt atagtaattt agaagaaagt ggtatttttt aaataattta gttaaataag 11220
taaaattagt ttggtaaagg aatttgaaga atggtattta aggtttgagt tggttaagtt 11280
tattaaaata ggaaggaaaa tatagaaaag gataatgagt tttgaagtat attagttatt 11340
ttttttagtt ttgcgattat ttatagatgg aagaaatttt agtatgtatt tattgttttt 11400
tttttgattt ttggaaagaa aataagatgt ttaaaaggaa tttttgaaat attagtttta 11460
ttttatagag gttgatattt tttttttttt tatttttttt ttaaaggtta taagtaatta 11520
tagagaggtt agtgtagttt aaatttttat atagtgattt tttagtattt ttggaaatag 11580
gatttgcgta gttagaggtt tatagaattt taggtaagta taatttagat ttaatttaaa 11640
ttatttggat ttagtttagt taaattattt atagtttgtt agatgataat ttattagttt 11700
aggttttacg tgaggaagaa aatatttaaa taattatttt cgtttttaaa tattataatt 11760
gaattatgtt taattaggag aatttaatta gtttatatat tggttaagtt gggggaaata 11820
tatttttaaa aatatatgtt taagttaaag ttatgaattg attttttttt ttatttatta 11880
aatataaata aagatatata gttttatata aaaaggtaat taaattgcga attaaattta 11940
aatatttttt ttatttagtt aaatgattga ttagtttttt tgagtttaat aaagttggtc 12000
gtttttttag tacgagtatt tttaattgat atatatgttt atatgtatgg tatgggggga 12060
tttttgtatt taattaattt ttgtgttgag tatagaagtt tgtgtgaagc gtggcgattt 12120
tttataagta taaagtttta gtagtgattt tcgtggattt atacgcggag gggtattgtg 12180
ttatttttgt attatggatt tttgttttta gtttgtttta aggtttaaat tattttttcg 12240
ggggatgtcg tttagtttta tttttattag ggatggggat ttgttgttta tttttggacg 12300
tatttttatt tttttttttt tattttagta tttttggttt tatttaaaga aaagtttagt 12360
agaataagat tttttagtta gggtatatta tagaagtgtt ttgttttttt tttaaaaaat 12420

tttttttttt ttttcggaaa tttatttaaa tattttagtt ttttttagaa aggttgtgtt 12480
atagtttatg ttatggagtt gtagggtttt gtgtgtttta tattagttaa ttttttaaga 12540
tataatagtt tttgtgatga cggtaaagtt gagtaattcg atattatttg gagtattaat 12600
ataggttggg aggaagaaac gggatacgcg cgaagatttt ttacgtttgg agcgaatgtt 12660
ttagaatttt tcggatttta tatttttcg tttattagga aggagaaatg cgagttgttt 12720
ttatttattg tttgttttta gttttttgtt cgtattgtta gggttaattt tggtcgcggc 12780
gtaatatttg ggttatagat gttagggggt cgcgtttagg gggaagcggg attttgagtt 12840
ttgttagttt cggttagaaa gtttggtatt aaggcgttgg gtacgttttt aggataaagt 12900
gcgggttttt taaaagagga aggtcgggcg gtatttaggg ttttttgttt tgtttgtaga 12960
tataagttta tttgtatttc gtgtaaagta gttttgaaag tttaggtttg aagataaaat 13020
gtggagttta ggttttttcg attgaaaatt tttttacgag gatatttttc gtagtgggcg 13080
gttagaaagg atttcggatt aagattcgta gagtggtgag tattttttag ggttcggggt 13140
tgaattgatt gtaggttggt agtttttcgg ttcggaggag aatttttttt ttgtagaaat 13200
tatttttgga aaataaaatc gagcgaaaag aagggtaatg cgggtttgcg cggaagggag 13260
tttttttttc ggggaaggag gcgtttcgcg gtggtatatt cgtcggcggg atgtaggtag 13320
gggtagatta gggtgagaag ggggatttgc ggaatacggg tttttcgcgg gttcgaggga 13380
tagggtgtag gcgtatttta ggtatttagg gtagtttagt ttgttttgcg gagcgggagt 13440
cggttttagg ttttttatat tttttaggaa tttagaagtt cgattgcggg atagttgtgt 13500
cgatggggat ttttttagtt ggatttttat tttatttgtt tttttgatcg agtgtttacg 13560
ttcggtagga gttggttgtg cgtcgtttcg ggtgggtgga ggtgcgtggg gttcgggttt 13620
tggttcgttt gggtgtgttt tagttttttt tttttagaat taaaggtttt tgtagggcgt 13680
agagtttttt tttttttttt ttaatttttt tttttatttc gtcgcggttt aagttaagat 13740
gaggtgagtt tttagggttg tgtttttagg aggtttcgag tatttttttt tcgagggtcg 13800
aagaaattag gttttttttt tttttttagc gttttttgag gaagtcgcga atttttgagc 13860
gagcgtggtt ttattgggga tttttagcga tttgttttgt gtttagcgac gttttaaggg 13920
gagaggagtt ttgtggaggg gcggtgtggg ggtgggtgtt tatattatat tttttggttc 13980
ggagtaggat tcggtgatgc gttttggtcg agtttttgtt ttttaggaat gcgggtgttt 14040
cgggttttgt cgggattttt ggttcgatcg gagggttcgg gagtgggagt ttggatttaa 14100
taggtattcg gatttaggag ttagattttt tggtttcgaa cgtttttttc ggagcgaaag 14160
tttgtttttt ttttttttaa gtttttcgcg ggttgggagg tgaagggagc gatagaagtc 14220
gggttttttt cgtagcgttt aacgtttcgg gtttttcggg tttcgggacg tggttcgggt 14280
gcgtggaggg acggttgggg gcgtagattt gtgaatgaat ttcggagttt tttgtagttt 14340
atagattaga ggttgcgatc gttttatcga cgtggtttaa tgagtcgtcg taataagttt 14400
tgagcgacga aacggaggta cggtttggat tttgcggtat cgcgttgcgt agggtcggac 14460
gggggagggg ttgagttatt gtagtaattt taaagtttgt gtcggggttt agtaatgggt 14520
gggttttttt cgggtcgtac ggtttttaag ggtttttttc gatttttatc gttatttcgt 14580
cggaacgttt agaggcgttt ttagtttaag tgatggagat tttgattttt agttgttttc 14640
gcgtttttcg tttgcgcgtt tttggcgttt ttttcgggat tcggggggcg attatatttt 14700
gagatttaat tcggttattt tcgggcgtgg gtttttatcg tttttcgggt tggttggatt 14760
cgttttttaa gtagttgttt tatttattat tcgtcgttag tttttttttt aaaatttaag 14820
ttttcggttg tttttttgtc gtttttcgtt tttcgttttt cgtttttcgt gcgaggggag 14880
gggaggggga ggaaggagcg gatatttatg tgtgttcggg aggggttcgt atcgtttttt 14940
cgttttttta gtagaggttt tgggtttttt ttttagaggg aaatcggtga gtagatatta 15000
agtgacgcga gtttcggttt ttttttcgtc gggggatttt cgtgcgtttt agaagcgtat 15060
ttggaagtaa tttttttcgc gtaatcgtcg ttttttaagt atttttagta tttttatatt 15120
ttttaaatat ttgtataatt ttgcgttgtt ttttggagga tttcggagac ggggagggga 15180
cgatggggtg tcgtgggggt agggaaggag agaggttagg ggttacgtag gattacggtt 15240
cggttcggtg atttttttta tttttcgtgg tttagggagg ggatttgtag cgagggtttt 15300
agattttttt tgtaaaatcg aattttgggg gagggtttgc gttttatgaa gtacggggaa 15360
ggtttaaatg acgaatattt acgagcggtc gggagcggaa gatggtagga tcgacgggtt 15420
cgggagggaa gatgggcgtg cgtttttagt gattatggta cgaggcggcg ttttgtttcg 15480
tattacgtta taatttaatt aggttttcgt tttttcgcgt ttgaataggg cgggtttatt 15540
cgggcggcga gttcgagttt agcgtagttt tagttcggcg tttttttttc gtttgttggg 15600
gaagaggtgg gaaatttttt tcgtcgcgtt ttggttttta tcgtaggttt tcgagaataa 15660
atttttttag ggagattgaa ttgtttttat tttttgtttt agttttaaga gggatttttt 15720
gaaaatatgg tagttaagaa tagggtgaaa atgtaagttt gaggtttttt tttttatttt 15780
tttttgtttt ttgtttttt attttatat tttttattag tttagagggg tttagttgta 15840
attattatta tcgttatttt tacggtgttg acgattgtta ttattttttt ttagttttag 15900
ttgttttttg gatgttttta ttggaaggtt gacgagtatt tttaatttta cgtgtttatt 15960
ttagagtatt ttcgatttcg ttatttttgt tgttttgatt tgcgtttttt tagttttttt 16020
tagttattta gttggttatt ttttaggaga tatttttgat tttttatttt ttttttagag 16080
ttattttttg ttttttagta aggtttgttg gttttgtttt tacgtttttt ttatagtttt 16140
tttatatttt tgcgtttttt attagttttt aagttttttt tttttttatt tgtaggg 16197

<210> 14
<211> 2609
<212> DNA
<213> Artificial Sequence

<220>
<223> chemically treated genomic DNA (Homo sapiens)

<400> 14

cgtagattag agatgattat agattttttt tagcgcggat taaagggatt gaattgaacg 60
ttttagttta atgatttaat ttttgttata tttataggga cgcgaatttc gattttataa 120
gtaggtgtgt acgtgtattt agatatttat ataaagtcgc gtggagggac gaaaagatta 180
attattcgat cgacgaggat aggtttgatt ttttcgatta ttttagtgtg ttagtgtata 240
ttttcggttg ggtttagcgt tttaagaaat ttcggaattt tagttgttaa tttttgtttt 300
tttattatga ttttttaaag atattttatt tgtttatcgg ggcgaagaga aatgggatta 360
ggcgttaggg cggtgggatt ttgtttaggg ttttgatttc gcgttagggt tttagattag 420
tcggttttcg aaggtttttt tatttatttt atataagagg aaataaagat tttttagttt 480
aaggttttag ggttgttttt tgatttcggt tagtttgtag gaagaggaaa taataaaata 540
aaggaatcgt taattcgtcg ggtattatat tttttagttt taattttcga tttcggatcg 600
ttagggttat tttttttacg ttgatttcgt tttttttaaa tgaaaatacg ttaataaaag 660
tatatttcgg atataaaatt taagtacgta ttttttttgg ggaggttaga gttgaggtgt 720
atttcggaag atgagaattt tgtttttatg aattgggtaa tatttaggta ttgttaggta 780
tttcgataga ttttttagat attttttttt ttttttttta tatttttttt ttattaaaat 840
agttattgtt ttgaaattta ttagaataac gacgttttaa aaataaaggc gtagtaagta 900
tttttttttt cgttgtcgcg ggttgaatta cggacgttcg cgggtcgttt agtttcgacg 960
gttcgtaggg ggcgcgcgtc gtagtcgtag tatagttcgg ttatttttag aaagggagtc 1020
gaatggaggg aagtagggag cgcggagggt tcgaggtttg tagataagga gaggcgtatt 1080
ttgggatttg ggttttttgt tgttataata taatcgtgtt attgttggta ttgttcgatt 1140
taagtgtcgg tggtaagcgg cgatgtcggg gttgggtttt tagtaatcgt tgtgttttgg 1200
gttaggttgg tcgttttagt tatcgggatt ttttttcgga tgtttttagg gcgataggtg 1260
ttgtatttat tgacgggata gtcgtatatt ttcgaatagg tagtggagtt cgtttttggt 1320
aggtatttta gttgcgttga tattaaggtc gttatataat agttattagt tttttttaag 1380
ttttagaagt aggttttttt tgttttagtt atagcggttt tagtcgtcgt tacgagttta 1440
ttttttattt ttaagcgtat tttttttttt ttattcgggt ttatgttcgt tatatagaga 1500
gaattatata gggggaatta tggttttata ttttcgaggg gatagatatc ggtcgtgaga 1560
taggtattac gtagagtttt ttggtgattg ttcgtaggag cgagattttt tttggttttg 1620
tagtcggtta ggtgtgtgtg tgtgagggtt tttagttgat tatcgggatg tattgttatt 1680
tttcggtttg gcgggttttg ggattttttg gttttcgtag gaggttattt taggttttcg 1740
gaggaggcgt ttttagttgg cggcgttttt cgtttcgggt ttagaggcgg atacggtcgg 1800
tcgcgttttg ttggtttttt gttcgcgtcg tagcgggttg ggagtagttg cgcgatatta 1860
gatttatagc gttaagacgc gaagcgcgag gaaatcgttg cgtttgattt tttttttttt 1920
aatttttgga tttaggaacg tttttagttt ttgcgtttta tacgtttagt tttggttttt 1980
ttttcggttt agaagttttt aaggattgaa gggttttgtt tagggtttcg tatttttgtt 2040
tgatttttat ggtttagaaa agtaggggga tatttgaaat gttatttcgg gataaatatt 2100
aataaaaaag taaatggatt tgtgtagggg ttagttatta attaattagg tcgtaaggtt 2160
atttagggta aatatagttt aggttgggtt gggcgagatt tttttatggt cgtttttcga 2220
tcgaattttt ttttttttgg tttaggtttt ttttaggtcg ttttggggta aatatcggac 2280
gggaaggggg cgtcgttaat tttttcgcgg ggtttggagg ttttttcgtt tttaagtttc 2340
gtagggtagg gtcggagtgt ttaatattta ttttcgttcg aatttcgggt ttgcgcgtcg 2400
tttttttttg ggttcgcggt gttgcgtttg ttgttgggtg tgtgtcgttg ttttttttcg 2460
agtcggtagt ttttgttgtg tggtcgaagt tttttggaat ttttaattgg aaattaattt 2520
tggttttgat agacgtttac gttagaggcg cgttatttat ttatattttt cgttttattt 2580
cggaggagac gcggcgagaa ttttgtcgt 2609

<210> 15
<211> 2609
<212> DNA
<213> Artificial Sequence

<220>
<223> chemically treated genomic DNA (Homo sapiens)

<400> 15

gcgatagggt tttcgtcgcg tttttttcgg gatgaggcgg ggggtgtggg tgggtggcgc 60
gtttttgacg tgggcgttta ttaagattaa gattagtttt taattaaaga ttttagaggg 120
tttcggttat atagtaggag ttgtcggttc ggaaagagat agcgatatat atttaatagt 180
aagcgtagta tcgcggattt aggggaaggc ggcgcgtagg ttcgaggttc gggcgggggt 240
gggtgttggg tatttcgatt ttgttttgcg ggatttggag gcgaggaaat ttttaaattt 300
cgcgggggag ttggcggcgt ttttttttcg ttcggtgttt gttttagaac gatttaagaa 360
ggatttgagt taagggggga ggggttcggt cggggggcgg ttatgagaaa gtttcgttta 420
gtttaatttg ggttgtgttt gttttgagtg gttttgcggt ttggttgatt gataattggt 480
ttttgtataa gtttatttgt ttttttgtta atatttattt cggggtgata ttttaaatgt 540
tttttttgttt ttttgggtta tgggagttag gtaggagtgc ggggtttag gtagagtttt 600
ttaatttttg aagatttttg ggtcgggaga gaagttagga ttgggcgtgt gggacgtagg 660
agttggaagc gtttttgggt ttaagagttg ggggagaagg agttaagcgt agcggttttt 720
tcgcgtttcg cgttttggcg ttgtgggttt ggtgtcgcgt agttgttttt agttcgttgc 780
ggcgcgaata aagggttagt agggcgcgat cgatcgtgtt cgtttttgag ttcggggcga 840
ggggcgtcgt tagttgggag cgtttttttc gaaggtttag gatggttttt tacggagatt 900
aaggagtttt agagttcgtt aggtcgaaaa gtggtagtgt atttcggtag ttaattgggg 960
atttttatat atatatattt agtcgattgt aaaattaaaa aaggtttcgt ttttgcggat 1020
agttattaga aggttttgcg tggtgtttgt tttacggtcg gtgtttgttt tttcgagggt 1080
gtaggattat agtttttttt gtgtagtttt ttttgtgtaa cgggtataaa ttcgggtgag 1140
gagagggagg tgcgtttggg gataagaagt gggttcgtgg cgacgattgg aatcgttatg 1200
gttggggtag gggaaattta tttttggggt ttaagggggg ttgatggtta ttgtgtggcg 1260
attttggtgt tagcgtagtt aggatgtttg ttagagacga attttattat ttgttcggag 1320
gtatgcggtt atttcgttaa tgggtgtagt atttatcgtt ttggaagtat tcggagaggg 1380
atttcggtag ttggggcgat tagtttgatt tagggtatag cggttgttag agatttagtt 1440
tcgatatcgt cgtttattat cgatatttgg gtcggatagt gttaataata gtacggttgt 1500
gttgtagtag tagaggattt aaattttagg atgcgttttt ttttatttgt aagtttcgag 1560
tttttcgcgt tttttgtttt tttttattcg gttttttttt tgggggtagt cgggttgtgt 1620
tgcggttgcg gcgcgcgttt tttgcgggtc gtcggggttg ggcgattcgc gagcgttcgt 1680
ggtttagttc gcggtagcga agaaagggat gtttgttgcg tttttgtttt tagaacgtcg 1740
ttgttttagt gaattttaaa ataataatta ttttgatagg aagaaagtgt gaaggaggaa 1800
gagaaggata tttagagggt ttgtcggggt atttaataat gtttgggtgt tatttaattt 1860
atgaaaataa aatttttatt tttcgaagta tattttagtt ttaatttttt taaaaaagat 1920
gcgtatttgg attttgtatt cgaagtatgt ttttgttggc gtgtttttat ttaagaaaag 1980
cggggttagc gtagagaagg tgattttggc ggttcgaagt cggaggttgg agttgaggga 2040
tataatattc ggcggattgg cggttttttt gttttgttgt tttttttttt tgtaaattga 2100
tcggagttag gaggtagttt tgaggttttg agttgagaag tttttgtttt tttttgtgtg 2160
gggtgggtga aagggttttc ggaggtcgat tggtttggag ttttggcgcg gagttaggat 2220
tttggatagg gttttatcgt tttgacgttt ggttttattt tttttcgttt cggtaggtag 2280
atagaatgtt tttgggaagt tatagtaaga aaataaaaat taatagttaa agtttcgaag 2340
ttttttaggg cgttaggttt agtcgggaat atatattggt atattgaggt aatcgaaaag 2400
attaaattta ttttcgtcga tcgggtggtt gattttttcg tttttttacg cggttttgtg 2460
tgagtatttg agtgtacgtg tatatttgtt tataaaatcg gaattcgcgt ttttgtgggt 2520
gtgatagggg ttggattatt aagttaaaac gtttaattta gtttttttga ttcgcgttgg 2580
ggaaaattta tagttatttt taatttacg 2609

<210> 16
<211> 11667
<212> DNA
<213> Artificial Sequence

<220>
<223> chemically treated genomic DNA (Homo sapiens)

<400> 16

cgggattcgt gggcgttaat taatgttatg gtggcggaga gtaaagggga cgaatatagt 60
ttggggttat tttcggagtt ttttagcgtt cgtttgtggt cgtgtttggt tggtcgcgga 120
tttcggcggg cgtcgtaaag cggcgggatt gttagcgtag agtttcggtt ttttgttttg 180
tttttgggtt cgagtatcgg agtttttggt gtttgcgggg agaagtttcg gattgagaaa 240
tacgggaggg tttcgttagt ggttgtaggt gcggtagtta ttttggggat ttagtgagaa 300

tggggtcgtt tggttttgcg cgaatttttt acgtgggtgt agtttttgag tcgtcgaggg 360
aggcggtggt aatgtcgttt agtgttagta gagggtagtt tcgaggtcgt gagttcgaac 420
ggcgatttcg ttaaatcgcg ggtttttttt tagttttcgg attttgcggg gtagaggcgg 480
ttttggagtt tagagattag cgattttagt ttgtaggagt tcggcgtaga ggtttaaggg 540
tattttcggg atgtggttaa gttatagttt ttaggtagtt ttattttgcg acggtaaggg 600
tttagagggt ggagggggat tagatgtttt aggaggggtt agaaagttaa cgtatatagg 660
gaatttgttt ttaagtaata gattttaagt atgtggaaat tttttaaatg atgttggtga 720
gagttatgat agttttcgta tttttggcga gggaagtcgg aggttagtag cgggatggtt 780
tttgggcgcg cgggagatag agggattagc gatttttgtt gggggtagag ggattgtgga 840
ttaaggattt agatatattt aattggggat tttagtttcg attgtggtta tttaattggc 900
gttgaatttg agtaatttat tatatcgttt tagttttcga ttaattattt ttgtaaaatg 960
ggtattgtgg gtttcgagtt tcgtaagggc gttagggatt cgagatagta ggaatttttt 1020
tattgtttta gtaattttgg gtttttgggt ttgtaggtgg gttgaaagga tttttttatc 1080
gtacgtttgt ttggcgtggt tgttttagag atcgaggttc gttaggtttt gtaatttagt 1140
tgcgcgtggt tagttatggt tttggaatcg tcgggatcgt tttagcggta tttcggttag 1200
ttttcgattt ttcgttttgg gtattagggt tatttagttt tagaagatag tgtttattag 1260
tataggaatt gagattttat attcggcgta gtgggttttt taggaagttt ggcgaagagt 1320
taaggttcgt cggatttgag gtcgtttggg gcgttaaata gatttattta tgagtatatt 1380
cggttaatat atttaagttt aatagtgggc gggatttttg gttggtttcg atttttttta 1440
tagtgtgtgt aaacgggtat ttattagatt ttttttgttt gtttttttat ttgtaaattt 1500
tgaaaggaga gtatttttcg gggtgatttt gaaaattatg ttagaattga aaaggtttgt 1560
gtaaatgcga ggtgtaatga tgatttatta gaagtagtta gttttgtacg tgtttgtagg 1620
atagatgtta gaacgtttta taaatgtgtg tatatatata ttacgtatgt gtgaaggtgt 1680
atatttgttt ttagatattt gataaatgta tatacgtatg cgtgtatttt tggttgagag 1740
taaaggggtt aatatagggt ttatatagga tattattttg tagttttgtg cgtgtatagt 1800
atatacgcga gtacgattgt ttgtgtttgg agatatcgta tggtatcgga ggagttgttt 1860
agtcgtcggt gttatttggg aagtagggtt tcgggtgttg ttttggagcg cgggaaagtt 1920
cggattcggg tttgttggtt agcgtttcgg cgtcgtcggg tttttttatt ttttattcgt 1980
cggtcggtga gcggttttag ttttaagatt ttcgattttg tcggtgaggg gagggagcga 2040
gaaggagtgc gtttcgggtt taagaggttg tagggtttta ttttttaggt ttgtcgtttt 2100
tttttatttt ttttaggtta tatttttatt taaaataaag aagggtgttt gatttattta 2160
ggttagggaa gtcggttttc gggagttttt tgtttttata agttatggtg aagggaggcg 2220
aagttagggt ttgttacggt ttgggagaaa atgcggttgt agggtttttt tgggttgtag 2280
cgttggttcg ggggtttaag attgttaagg tgtgtgtggg aggcgcgtag tgtggttatc 2340
gaagagtttt ttattatcgt atcggtaggt cgtcgggttc gttttttttt tatgttttta 2400
agggttttga gttgcgtagt attcgtatta tttagaagtg tttgtggaga aaacgatttt 2460
aggtgtaagt ttaaggttgt tgtttgtaaa ggtaatgtcg tggagattgg gttttatagc 2520
gatttgggtt tttaagttat cgaaagttat agggtagggt tataaatatt ttttcgtttt 2580
ttttgtagaa tcgtgcggtt gataggagag cgttgcgtag aaaattcgag gtcgggcgtt 2640
ggaggagttt tcgcggttcg gagaaggtat agaggcgttt ttgagaggcg tagttggaat 2700
aggcgatgta cgggttcgga tttggtcggt taatcgagtt tagcggtcgc gaaaattaga 2760
gtcgttatag aggttgagta cgatttttatt tttggggatc gggttcggtg ggagttattt 2820
gtttttaacg tttttagaga ttttaagtag gataatataa tgttggtagg agtaaggtgt 2880
aaggaattta gcggtagaag tttttcgggg gggtggggaa aggtagattc gtgtttcgtt 2940
tgagtttggg ggtggggata gtttttggtt ttgtagtttt tgtttcgggg attagttggg 3000
tttatttaat ttttttatat ggggttgaaa ggggttaatg ggacggtttc gtcgtttttt 3060
tttcgggatt ttatagggtt ttgattcggt ttttattttt gtacggttag tagtcgcggg 3120
ggttttagga aggaggataa aggttcggtg ttatcgcggg cgggggttcg gttttttggt 3180
tttttgttta tatttatagt ttttagcggt tgttggggga agatttttaa aaatatgtgt 3240
cgaatttttt tttttttttt tttagaaata aataaataaa aaaggtaaaa ggcgaatttt 3300
tttttatttt tcgtttatag agattaaagt tttttgggat tttgtttttt tttttttttt 3360
gattgtttag aaatatttgt ttttttttgt atatagagga aaatgcgggg aaaggttttt 3420
taaaattcgg ttttatatta ttattattat taaggataat cgggtaggtt gaggttttaa 3480
cgtggatgat tcgagttggt ttcgcgtcgg ggttttgtag ttattgtttt gtgcgtttag 3540
tatttttggg ggcgattagg gtttttgcgt tttcgttcgt cgttcggtag tcgagagtat 3600
tttgtgttta gattggtcga tttatttttt ttcgaatttt gtttagagtt ggtaaggggg 3660
atttagttcg cgttttaaga tttgggtttg tagcgtcgtt aataggttcg gggatacgag 3720
gcgttttagg tcggggtttt ttcggttgtt ggtttttttc gttttttatt cgttggcggc 3780
gtttcggtcg ttcgtaattg atttaattcg ttttttgcgt ttgtttttta ggtttttcgt 3840
ttttttataa aggtttaggg gagtttcgtt tataggttga ttttgtaatt tttggttcgg 3900
tggttatttt ttgttttttt gaaaaagaaa aggaaaaaaa aaaaaaaaaa gaaaaaatta 3960
attagtcgag ggaggcgcgt gaggattgga ggcgttagtc ggatagttta tgagtaggtt 4020
tttgggtcgg tgtcgtttcg cgggttagta cggttttttt tagagaaaat tttttaaacg 4080

tgtgaagatc gttttggggg aagcgagagg gaggttggag gagtttcggg cggggtttta 4140
gcgtttatta gttgtgtttt tagggtttgg gtgttcgttg taacggtaat cgcgtgagtt 4200
ttatttttac ggttaagggg ttagggtagg gtggatgtaa tcgcgtgcgt ttggtttcgg 4260
aaggtgttcg tagggggtgt ttgtggttag ttgggttagg aagttaaatt ttagaaagtt 4320
attattaaag gttttttttt tttttttttt tttttttttt tttttgtttt tttttttttt 4380
tttttttttt ttaatagggg aagggaaaaa atattattga atatttatta taaattgggt 4440
atttaaaaat tagaaaattt aattatttta ataattttga tatacgtacg tatttttgtt 4500
ttatagagga gaatattgaa atttgaatgg gtatttgttt taaggttata tagttgttaa 4560
agaggatatt aggttttaat ttagtttttt ttgattaaat aggttaggtt tttgttttat 4620
gttgtatagg ttattttcgc gaagtgataa gatttagaaa tatgtttttt atagtttagt 4680
attaagtttg tgcggtaaat agtttgtgta gtagtagttt ttgtggtata gaaataaata 4740
aaaaaggtaa aaggtatagt ttagattgtg tttttttttt tttttgggag gttttttttt 4800
attatttttg ggaggttttt ttttattacg tagttttttt gagtattagt ggtagttagg 4860
tttagttgtt gttaagtttt ttttaaaaat ttcggaggtt atttgtttta tagatatttg 4920
gaagattttt ggttggtttt tttttttttt aggatttgag attttttaaa tgtaatgtaa 4980
ttttgggttt ttagatttaa gatatttttt ttatgatagt tgggagtttt ttttttagaa 5040
agtggaaagt tgttaagatt gttcggtttt aagaattttt aatttttggt tgtttcgaag 5100
gtatggggtt tttagtttta tttttttttt atttttttta tgggttttat agtttatttt 5160
aagaagtagg ggttgttata tagtggtggt aggtttgacg ggtagggttt tagggttatt 5220
attaggttag atttgtagaa tggggatatt gtagggatgt tgagtttgat taagatgttt 5280
tttggtttta gatttttatt tagaagttag gagtttggtt cggtgatttt gtttatgaag 5340
tttggtttag atttttataa agggaagagt ttagttgaga gtaaattatt ttgggttaga 5400
tttagatttt tggtttatat tttgtttgtt tggttgtatg aagtttgggt ttggttttaa 5460
tttttattcg tcgttagggg gttttttgta gttttatttt tagttttggt ttttaggaaa 5520
ttttggtaaa ggttggatgt atttaatttc ggatgaaagg tttagggttt tttgtttatt 5580
taagttttag atttggagta gtgaattatt tattgatgta agttggaaat aggacgttaa 5640
tattttttgg agtagggtat aggattattt ttagaatttc gtaggttttt tttttatagt 5700
tttttggtgt atgtatttta atggcgaaga tttttttagg ttttttttga gaataggata 5760
tttttttttg ttttttttaa attaatttat ttattttgat tgggtaattt tagtgaggtg 5820
gaggtgattt taagaatttt tgtgtaaatg tgaggagagg gtttatattg ttagtagtgt 5880
gtgagatgtt tatatttatt atatatataa tttttatgta agaacgattt attatttagg 5940
atatttagaa attggttggg tatttgtatt aaattttatt tatattgtat ttatatattg 6000
gttaatatat atatatatat ttatatggtt tagttattta ggttagattg tatatgttaa 6060
ggtataattg ttatataatt taaatatttt tgtttttagg aattgtttaa attagttttt 6120
ataatagaaa tttaaaagtg cgaagaggga gagaagagaa aggaaatgag agatgaagaa 6180
agtttgttat tgaattaaat tttcggtttt ggggaaaatg attaaattta agagttttgg 6240
agaaatttcg agggttggag ttagatagat tttttttgtg ggtatttatg gggaagtggg 6300
gtatgatttg gttttttttt tttatttttt ttaatttttg tgtagattta ggaaattttt 6360
attgattgtt ttattttgtt ttttttattt ggtgaggttg ttgttttttt tttagttttg 6420
ggtgtttttt gttttttagt tttcggggag gagataggtt ggttttttat tgttggtttc 6480
ggttaatttt ttcgttttat tggaggtggg ttgttatttg tttattaggg tttggttttg 6540
gaagtttttt ttttttgttt tttttgtttg agacggtagt tgggggagtg ttgatagtta 6600
gttttagaat agggagtttt tgtttgatga agtaaaggtt ttgtttatat tattttattt 6660
tttagtaagt taggaatata tatttataaa tgtatatatt attaatgagg tatgaatgtt 6720
atatatattg tggttttagt aagtattttt taagtgttag ttatatgatt attataatgt 6780
taaaaatata tgtttatttt gtaggcgaat gatagatata tatatttttt attttgttat 6840
taatatataa ttgatattgt atatatatat ttgtagtaga tatagattat ataattattt 6900
gtttatgggt taaattttta cgttagataa agtataaata gatatgtata tagtaggttg 6960
ttttttgttt tgtaattttt tttagttaga tagtggagta gtcgggtatt aaaggtattt 7020
agatattagg tttgattagt ttttagggtt ttttgtttta gttgagtttt tttttttatt 7080
ttgtttttgt cggtttttgg taagttttag ttttagtttt gaggtgatgt tttttgagaa 7140
ttttcgtggt ttggtttttt gttttgtagt ttttcgtagg atgtttgtgg ggggcggggg 7200
gtattttgag tagaagttat ttgggtttaa ttatatataa taggatatta ttttgaggtt 7260
tttatcgttt gattattgga gggttgaagt gagaggtttg taggtttatt ttcgtggcgt 7320
aaaatcgttt ttggtttatt agtcggtttg gatttgtcgt tatattgttg gttaggtttc 7380
gagatagacg gggggggggt agtgagggtt gggtgcgggg tttagtttta gttgagtagt 7440
agttcgtaag aatcggcgga tttattttat tagaagtcgg aagttattta ttttattaga 7500
agagtgaaag tcgattattt tttaattcgt ttgggagaat tagattgtaa ttaggttaaa 7560
cgtttgtgtg tttgcgcgga agtatggatt ttaattcgtt atttggggat tttagtaaag 7620
gaagagagag aaagaaaaaa gattaaattt ttgtagtcgc gaagtatgtt taatttttat 7680
tgaaattttt aaatttttta tttatatttt aaggtaaatg aattttgatt attaaaaata 7740
ataaagtagt tattttttat atttgtatag aggttagtag tttgtagcgt atttttataa 7800
ttagaatttt atttattaat gtttggaata aatttttggt tgttatagtt gatttaaaat 7860

cgaaaagttt gagaataata ataaaaatat agttagttta tatatttagt ataattgtgt 7920
atgaggtaga atataaattt gaataggtat aaaaataaat taagttttat ttatagtcgc 7980
gttttgtgaa cgaattacgg tagaaaaagt ttttttgttg acgttaaata gtcgtaacgg 8040
gttagtgatg atatagaaaa taaataattt taggaaaata aagtagtagc gatagttata 8100
aacgtttgtt atttcggttg tttttaattt ttgtttaatt tattttgaaa aagataaaat 8160
tttgttgaaa attttttttt tttaattagt tgttgaatta aggagcgata gaaataaaga 8220
agttcgtgtt aaaagaagga atataaaatt tattcgggaa tcgataaggt aaaaaataaa 8280
aataaaataa aaaaatataa taaaagtgtt ttagtttttt ttagaaaatt tggtttttta 8340
aataataagt tatagtataa ttaggtttat ggttttcggg tcgggtcggt ttcgggttat 8400
aagatcgttt aggtcggtcg ttgtcgggtt ttatattttt tttttttaag gtgggagaag 8460
taggaggttt gaaaaataaa aagtagggag gacgagtcgt tttagtagcg tgggttaggt 8520
aggtagtgat ttttttgttt ttaggatttg tatcgaaaga ttcggggata tttgtttttg 8580
attttttttt ttagataggg agagggtgaa atttttttaa ttggtagacg aaattatttt 8640
ttattggata tattttttta attttgaata tagttttttg aaggtttaat aaaaatttta 8700
acgtaaattt tttagaagag aagatgagga atattaaagt ttttgatttt tatatttgga 8760
tttagtaaat tttaaggtta taatattttt tcggttgttc ggtgaatttt tttttttttt 8820
ttttttttta aatatttttt taaaaagttt tatcgatttt ttaaataaaa tattagttag 8880
tcgtcggggt tcggaggtgt tcgaagcgac gggattgata gcggaggaaa cgtagttttt 8940
tttcgcgttc gttcgcggtg taaatcgagt ataggtcgtg gagttaggtt gtttcggttt 9000
tcgttgggtt ttaattttcg tttcgtttag tgggtttcgc ggtaagcggt ttttgaatag 9060
ttttaagagg gttcgcggag taaatatacg tattggttcg tttttttttc gggtagcgcg 9120
gtttcgttat tagttttgtt cggcgcgttt agttatggat tgtacggtag tcgggcgggg 9180
aacgcggaga gcgagcgtat cgatttgtga gagaaggtta agaggtttgc gttgtcgacg 9240
ttcggtcgta ttttcgtttc gggttttttt cgcggtgaat ttgggtagga gacgttgggg 9300
tttcggaaag agacgagttt agtagaaagc gcgtagagag gtagttttag gttaggggag 9360
tgtaaggtta tagaggttag ggaggtgagt ataggaggat ataaattgag gggataaaga 9420
ggagcgatag gagtttagga aagcgaaaaa gtatagaggg attttgggcg ttggttttag 9480
aggcgggttt agagggtgtg aggttaggtt ggcggcggcg tcgtcggttg cgatcggggt 9540
cggcgtcgcg cgtttttgta ttttcgtatt cgtttgtatc ggtatgcggt gggtttttag 9600
agatcgaggc gcgaatgtag cgcgtcggtt ttgttgtcgt ggttttagta agagtaatgt 9660
attatggcga gttcgggttg tcgggtataa gcgaattgta ggttcggcgt attggtgggc 9720
gcgggcgtcg ggggcgcggc ggtggttggg ttggtagggt tgagttggtt cggcggcggc 9780
gcggcggttt cgtggtgcgg tggggtaggc ggcggtgcgg cggtcgcgtg tagatggtgt 9840
gcgtgcggat gcgggtgggg gtgcggcgga ggcgggggtg cggtcggcgg gttttttagg 9900
ttattgtacg agtttattac gtcggggggt agcgttatgt tttgtacgcg tgtgtacggt 9960
tcgtacgagg cggtcgggtt cgttagtttt ttgattatag cggtcgcgtt agggttatcg 10020
gggttcgcgg ttgtagtcgt agttgttgta gtcgttgcgg ttgtcgttat ttggtaggag 10080
gtatagggta tgggtgaggg aggttgcggt agcggttacg agttgttgag gaagttagat 10140
tgtaggtatt tggggggcgt taggtagtcg tagtcgtcgg tttcggcgtt cgttacgtcg 10200
tattcgtttg cggcgttttc ggtttcgaag agttttttgt cgggttggaa gtgcgcgggc 10260
ggcggtcgga agggtttttt tatgcggcgg cggcgtcggt agttgttttt ttcgaatatg 10320
ttttcgtagg tcgggtttag cgtttagtag ttgtttttgc gttcgtcgtc gttttcgcgc 10380
ggtattttga tgaagtattc gttgaggttg aggttgtggc ggatgttatt ttgttagttt 10440
tttttatttt tttcgtagaa cgggaatttc gcgatgatgt attggtagat gtcggatagc 10500
gtgagttttt ttttcgcgtt ttcgcggatc gttatggcga tgagcgttac gtacgagtac 10560
gggggttttt gcgtcgggtt cggttttttc ggggttgttt cgtcgttatt tttatcgttt 10620
ttgtttgggt tcggcggcgg tttttttggt tttttgattg tgcgatcggt ttttggggtt 10680
agtagggttt tcgtcgcgtt ttcgggttcg gggtagttgg ttattatgat aaagtcggcg 10740
cgtcgcggtc gggtcgtttt tgtttttcgt tttaggcgtt ggcgcggtaa agagttgggg 10800
cgtacgagtt cgtttacggt taagttttaa atttttggag attgcggatg tcgttcgcgt 10860
ttgtttgttg gaggtttgtc gttgtttttt tttttttttt tttttttttt agggagcggt 10920
cggcgggagt ggagtttagt ttttggttat ggggagttcg tttaatagag aggggtttcg 10980
gtttcgtcgt tttttttcgt ttaggttagt tttcgttttg gtgggttttt ttttttgcgt 11040
tttttttttt ttttcgtttt tcggtttttc gaagtacgat tcgcgttttt ggcggagttg 11100
ttttttggag tttttagtgc gttaggagtt tcgttttgtt ttgattcgta tgggttttat 11160
cgagtttcgt ttgcgttagg cgttttcgtt tttatagcgg ggcggttagt cgcgtacggg 11220
cgagtttatt tttaagttat tttttgtaaa cgtttcgtat agtttggatc ggtttgtttt 11280
cgtttagcga gttttagggg tttagtcgat agttaggttt acgcgttttt gaaatttgtc 11340
ggtattcgtt ttgcgggttg ggttgggaga tgacgaggat ttcggtgggg tttgttcgta 11400
ttcggttaaa gtttaggaag ttcgggtttt agcgaggaaa ggcgttttaa gttttttcgc 11460
ggtttttagg tgaaagaaaa cgattttttt gttttgtcgt ttgttgtcgt tttgaggttg 11520
aatttttagt tcggggttgg ggaggggcga gacggcgagg gggttggacg gggtagggtg 11580
gggagagttg ttttgaggtt ttgggaaagt tagtttagaa acgggtgtga ttgtacgaag 11640

aagtttcggt ttggtttgtt tttcgcg 11667

<210> 17
<211> 11667
<212> DNA
<213> Artificial Sequence

<220>
<223> chemically treated genomic DNA (Homo sapiens)

<400> 17
   cgcgagggat aggttaggtc gaggtttttt cgtatagtta tattcgtttt tgggttgatt 60
   tttttaaagt tttagagtag ttttttttat tttatttcgt ttagtttttt cgtcgtttcg 120
   ttttttttta gtttcgagtt agaagtttag ttttaagacg gtagtaaacg gtagagtaaa 180
   ggagtcgttt ttttttattt gaaagtcgcg aggaggtttg gagcgttttt tttcgttggg 240
   gttcgagttt tttgggtttt ggtcgggtgc gggtagattt tatcggggtt ttcgttattt 300
   tttagtttag ttcgtagagc gagtatcggt agattttaag ggcgcgtgag tttggttgtc 360
   ggttgggttt ttgaggttcg ttgggcgggg gtaggtcggt ttaggttgtg cggggcgttt 420
   ataaaaagtg atttggagat gaattcgttc gtgcgcggtt ggtcgtttcg ttataggggc 480
   gaaggcgttt gacgtaagcg gaattcggtg gagtttatac gaattagaat agagcgaggt 540
   ttttggcgta ttagggattt taggaggtag tttcgttaga gacgcgggtc gtgtttcggg 600
   aaatcggggg gcggggggag gggaagagcg tagaaaagaa aatttattaa ggcggggatt 660
   ggtttgagcg ggagggggcg gcgaggtcgg agtttttttt tgttgggcgg attttttatg 720
   gttagaggtt gagttttatt ttcgtcggtc gttttttagg ggaaggggaa ggagagggga 780
   gagtagcgat aggtttttag taagtaagcg cgggcggtat tcgtagtttt tagaagtttg 840
   agatttggtc gtaagcggat tcgtgcgttt taattttttg tcgcgttagc gtttggagcg 900
   gagagtagag gcggttcggt cgcggcgcgt cggttttgtt atgatggtta gttatttcga 960
   gttcgaggac gcggcggggg ttttgttggt tttagagatc ggtcgtatag ttaaggagtt 1020
   agaagggtcg tcgtcgagtt taggtaaggg cggtgggggt ggcggcggga tagtttcgga 1080
   gaagtcggat tcggcgtaga agttttcgta ttcgtacgtg gcgtttatcg ttatggcgat 1140
   tcgcgagagc gcggagaaga ggtttacgtt gttcggtatt tattagtata ttatcgcgaa 1200
   gttttcgttt tacgagaaga ataagaaggg ttggtaaaat agtattcgtt ataattttag 1260
   ttttaacgag tgttttatta aggtgtcgcg cgagggcggc ggcgagcgta agggtaatta 1320
   ttggacgttg gattcggttt gcgaagatat gttcgagaag ggtaattatc ggcgtcgtcg 1380
   tcgtatgaag aggttttttc ggtcgtcgtt cgcgtatttt tagttcggta aggggttttt 1440
   cggggtcgga ggcgtcgtag gcgggtgcgg cgtggcgggc gtcggggtcg acggttacgg 1500
   ttatttggcg ttttttaagt atttgtagtt tggttttttt aataattcgt ggtcgttatc 1560
   gtagtttttt ttatttatgt tttatgtttt ttgttagatg gcggtagtcg tagcggttgt 1620
   agtagttgcg gttgtagtcg cgggtttcgg tagttttggc gcggtcgttg tggttaaggg 1680
   gttggcgggt tcggtcgttt cgtacgggtc gtatatacgc gtgtagagta tggcgttgtt 1740
   tttcggcgta gtgaattcgt ataatggttt gggaggttcg tcggtcgtat tttcgttttc 1800
   gtcgtatttt tattcgtatt cgtacgtata ttatttgtac gcggtcgtcg tatcgtcgtt 1860
   tgttttatcg tattacgggg tcgtcgcgtc gtcgtcgggt tagtttagtt ttgttagttt 1920
   agttatcgtc gcgttttcgg cgttcgcgtt tattagtgcg tcgggtttgt agttcgtttg 1980
   tgttcggtag ttcgagttcg ttatgatgta ttgtttttat tgggattacg atagtaagat 2040
   cggcgcgttg tattcgcgtt tcgatttttg agagtttatc gtatgtcggt gtagacggat 2100
   gcgaggatgt agggacgcgc gacgtcggtt tcggtcgtag tcgacgacgt cgtcgttagt 2160
   ttgattttat attttttggg ttcgtttttg gagttagcgt ttagggtttt tttgtgtttt 2220
   ttcgtttttt taagtttttg tcgttttttt ttgttttttt agtttatgtt tttttgtgtt 2280
   tatttttttg atttttgtga ttttgtattt ttttggtttg aagttgtttt tttgcgcgtt 2340
   ttttattggg ttcgtttttt ttcggagttt tagcgttttt tgtttaaatt tatcgcggaa 2400
   agggttcggg gcggaggtgc gatcgggcgt cggtagcgta gattttttgg ttttttttta 2460
   taggtcggtg cgttcgtttt tcgcgttttt cgttcgattg tcgtgtagtt tatggttaga 2520
   cgcgtcggat aggattgatg gcgggatcgc gttgttcgag aaagggacgg attaatacgt 2580
   gtgtttgttt cgcgaatttt tttgaagttg tttagaagtc gtttgtcgcg gggtttatta 2640
   ggcggggcgg gggttgggat ttagcgggag tcggggtagt ttggttttac ggtttgtatt 2700
   cggtttatat cgcgggcggg cgcggaggga ggttgcgttt ttttcgttat tagtttcgtc 2760
   gtttcgggta ttttcgggtt tcggcggttg gttaatgttt tgtttgaaag atcggtggaa 2820
   ttttttaaga gagtatttaa aaaaaaaaaa aggaaaaaaa atttatcggg taatcgggga 2880
   agtattgtgg ttttggagtt tgttaaattt aaatatgaaa attaaaagtt ttagtatttt 2940
   ttattttttt ttttggaaga tttgcgttag agtttttgtt gggtttttaa aaagttgtgt 3000
   ttagagttag gagaatatat ttaataaaag atggtttcgt ttattaattg gggaagtttt 3060
   attttttttt tatttgaaga aaaaaattaa aaataaatgt tttcggattt ttcgatgtaa 3120
   gttttggagg tagggagatt attgtttgtt tggtttacgt tgttgggacg gttcgttttt 3180
   tttgtttttt gttttttaaa ttttttgttt tttttatttt gggaaggaga aatgtgaaat 3240
   tcggtagcgg tcgatttagg cggttttgtg gttcggagtc ggttcggttc gaaaattata 3300
   gatttggttg tattgtagtt tgttgtttgg gggattaaat tttttagaga gaattagagt 3360
   atttttgttg tgtttttttg ttttgttttt gttttttgtt ttgtcgattt tcgaataaat 3420
   tttgtgtttt tttttttaat acggattttt ttatttttgt cgttttttag tttagtagtt 3480
   agttaaaaga ggaagatttt tagtaaaatt ttattttttt tagaatgagt taaataaaga 3540
   ttgagagtaa tcggggtggt aggcgtttgt ggttgtcgtt gttgttttgt ttttttggga 3600
   ttgtttgttt tttgtgttat tattggttcg ttgcggttgt ttaacgttag tagaaagatt 3660
   ttttttgtcg tggttcgttt ataaggcgcg attgtaggta ggatttggtt tatttttgta 3720
   tttatttaag tttgtatttt attttatata tagttgtatt aggtgtatgg gttggttgtg 3780
   tttttattat tgtttttaaa tttttcgatt ttgagttaat tataatagtt aagagtttgt 3840
   tttaaatatt aatagataaa attttggttg tggaaatgcg ttgtaaattg ttaatttttg 3900
   tataaatgtg agaagtgatt gttttattat ttttaatagt tagaatttat ttattttgag 3960
   atatgggtag gagatttgga ggttttagta aggattgaat atatttcgcg gttgtagaaa 4020
   tttagttttt tttttttttt tttttttttt tattgagatt tttaaataac gaattaagat 4080
   ttatgttttc gcgtaggtat ataggcgttt gatttggttg tagtttggtt tttttaggcg 4140
   gattaggggg tggtcggttt ttattttttt gatgggatag atgattttcg atttttgata 4200
   ggatagattc gtcggttttt gcgggttgtt gtttagttga gattagattt cgtatttagt 4260
   ttttattgtt tttttttcgt ttatttcggg gtttggttag taatgtggcg gtaggtttag 4320
   gtcggttggt gggttaggag cggttttgcg ttacgggaat gagtttgtag gttttttatt 4380
   ttagtttttt agtggttagg cggtaggaat tttagggtgg tattttgtta tgtatagttg 4440
   ggtttaagtg gtttttgttt agaatgtttt tcgtttttta taggtatttt gcgagaggtt 4500
   ataaggtagg agattaagtt acggggattt ttagggggta ttattttaag attgggattg 4560
   gagtttatta ggaatcggta ggagtaggat ggagagggag gtttagttgg agtaggaagt 4620
   tttagaggtt ggttaggttt ggtgtttggg tgtttttggt gttcggttgt tttattattt 4680
   ggttaagggg agttgtagaa tagaaagtag tttgttgtgt gtatgtttgt ttgtgttttg 4740
   tttagcgtga aaatttagtt tatgggtaag taattgtgta gtttgtattt gttgtaaatg 4800
   tgtgtgtata gtgttagttg tgtattggtg atagggtggg aggtgtgtgt gtttgttatt 4860
   cgtttgtaga gtgggtatat atttttggta ttgtaataat tatatggtta atatttagga 4920
   agtgtttatt gggattatag tgtgtgtggt atttatattt tattggtgat gtgtgtattt 4980
   gtgggtgtgt atttttggtt tgttgaaaaa taaagtaata tggatagagt ttttgtttta 5040
   ttaggtagag gttttttgtt ttgaagttgg ttgttagtat ttttttaatt gtcgttttaa 5100
   gtagagaaag taagggaggg aagtttttag gattaagttt tggtgggtag gtagtagttt 5160
   atttttagtg agacggagaa gttggtcggg gttagtaatg aaggattagt ttgttttttt 5220
   ttcgagaatt gaaaggtaaa aggtatttaa ggttgggaag agagtagtag ttttattaag 5280
   taaggaagat aaaatgaagt aattagtgag ggttttttgg gtttatatag ggattgaagg 5340
   aggtgagaga agggagttaa attatatttt atttttttat ggatgtttat aggaaaaatt 5400
   tgtttaattt tagttttcgg gattttttta aagtttttaa gtttggttat tttttttagg 5460
   gtcgaaagtt tagtttaatg gtaggttttt tttatttttt attttttttt tttttttttt 5520
   tttttcgtat ttttgagttt ttattataga agttgattta agtaattttt gagggtaaaa 5580
   gtatttgagt tatatagtaa ttatgtttta gtatatatag tttggtttag atggttgggt 5640
   tatgtgagtg tgtgtatata tattggttag tgtgtaaata taatgtaaat agagtttaat 5700
   ataaatattt agttaatttt tgaatgtttt gggtaatgag tcgtttttgt atgaagatta 5760
   tgtgtgtggt gggtgtaaat attttatata ttgttgatag tatgagtttt ttttttatat 5820
   ttatatagaa atttttagag ttatttttat tttattaaaa ttgtttagtt agagtaagtg 5880
   ggttggtttg gagaaagtag ggggaggtgt tttgttttta agagggattt ggagaaattt 5940
   tcgttattga agtatatgta ttaagggatt gtgggaaagg gatttacgga gttttggaga 6000
   tggttttgta ttttatttta gagggtattg acgttttgtt tttagtttgt attagtaaat 6060
   agtttattgt tttagatttg aggtttagat gagtaagagg ttttggattt tttattcgag 6120
   gttaggtgta tttagttttt gttagggttt tttggaagtt aaggttggag atggggttgt 6180
   aaggagtttt ttggcggcgg gtggaaattg gggttagatt taggttttat gtagttagat 6240
   aagtagaata tgggttagga atttgagttt ggtttaagat agtttgtttt taattgagtt 6300
   ttttttttta tgaaggtttg ggttaggttt tatggatagg attatcggat tagatttttg 6360
   gtttttgggt gggggtttaa ggttaggaaa tattttgatt aggtttagta tttttgtagt 6420
   gtttttattt tgtagattta atttggtggt gattttgggg ttttgttcgt taggtttatt 6480
   attattgtgt ggtagttttt attttttgga gtagattgtg ggatttatag aaaagatggg 6540
   gaaaggatgg gattaagagt tttatgtttt cgggataatt aggaattaag gatttttaaa 6600
   atcgagtagt tttagtaatt ttttattttt tggagagagg atttttagtt gttataaaaa 6660
   gagtgttttg ggtttaggaa ttttaaatta tattatattt gaggaatttt aggttttaag 6720
   aagagaagag attaattaga ggttttttag gtatttgtga ggtaaatggt tttcgaaatt 6780
   tttaaaggag atttgatagt agttgagttt agttgttatt gatgtttaag ggagttgcgt 6840
   agtaagggag agttttttag gagtagtaag ggagagtttt ttaggagaag aaggaagtat 6900
   agtttgagtt gtgttttttg ttttttttgt ttgtttttgt attatagaaa ttgttgttgt 6960
   ataggttgtt tgtcgtatag gtttggtgtt gagttgtgaa aagtatgttt tgggattttg 7020
   ttatttcgcg gaggtggttt gtgtagtatg aggtaaagat ttggtttgtt tagttaggaa 7080
   gaattgggtt ggagtttggt gtttttttta atagttatgt aattttgggt aaaatattta 7140
   tttaaatttt agtgtttttt tttataaaat agaaatacgt gcgtgtatta gaattattaa 7200
   aataattaaa ttttttaatt tttaagtgtt tagtttatag tagatgttta ataatgtttt 7260
   tttttttttt ttattaaaaa aaaaaaagga aggaaggaag taaaaaagga aagaagaaag 7320
   gaagggggaa aaaaaatttt tagtaataat tttttaggat ttgatttttt aatttagttg 7380
   gttataggta ttttttgcga gtatttttcg gggttaggcg tacgcgattg tatttatttt 7440
   gttttagttt tttggtcgtg ggagtgaggt ttacgcggtt gtcgttgtag cgaatattta 7500
   agttttgaag gtatagttgg tgggcgttga gatttcgttc ggggtttttt taattttttt 7560
   ttcgtttttt ttaaagcggt ttttatacgt ttaggaggtt ttttttgaga aaggtcgtgt 7620
   tgattcgcga agcggtatcg atttagggat ttatttatag gttgttcggt tggcgttttt 7680
   agtttttacg cgtttttttc gattggttga tttttttttt tttttttttt tttttttttt 7740
   tttttttaga aaagtagaag gtagttatcg ggttagaggt tgtagggtta gtttgtgggc 7800
   gaggtttttt taggtttttg tggagaaacg ggaaatttga ggggtaaacg taggaagcgg 7860
   gttgggttaa ttgcgggcga tcgaggcgtc gttagcgggt ggggagcgag aggggttagt 7920
   agtcgggaag atttcggttt ggagcgtttc gtgttttcgg gtttgttggc ggcgttgtaa 7980
   gtttaggttt tggggcgcga gttaagtttt ttttgttagt tttaaataaa attcggggga 8040
   gaatgagtcg gttagtttgg gtatagggtg ttttcgattg tcgggcggcg ggcggaagcg 8100
   taggggtttt gatcgttttt agaggtgttg agcgtatagg gtagtggttg tagagtttcg 8160
   gcgcgaggtt aattcggatt atttacgttg ggattttagt ttgttcggtt gtttttagta 8220
   ataataatag tatgaaatcg agttttaaaa aatttttttt cgtatttttt tttatgtata 8280
   agggagaata agtattttta ggtagttaaa gaaaaaaaaa gggtaggatt ttaggaaatt 8340
   ttaattttta tggacgagga gtgaagggga attcgttttt tgtttttttt gtttgtttgt 8400
   ttttagaaga gaaaaaaagg aaattcgata tatattttta aaaatttttt tttaataatc 8460
   gttaaaggtt gtgagtgtga gtagagagtt aggaaatcga gttttcgttc gcggtgatat 8520
   cgggtttttg tttttttttt tgaagttttc gcgattgttg atcgtgtaga gataaaggtc 8580
   gggttagggt tttgtggaat ttcgagggag gggcggcggg gtcgttttat tggttttttt 8640
   taattttatg taggggggtt aggtgggttt agttgatttt cggaataggg gttataaggt 8700
   taggggttgt ttttattttt aggtttagac gaggtacgga tttgtttttt tttatttttt 8760
   cgaaaggttt ttgtcgttaa attttttgta ttttgttttt attagtatta tattgtttta 8820
   tttaaagttt ttggaggcgt tagggatagt ggttttttat cggattcgat ttttagaaat 8880
   ggaatcgtgt ttaatttttg tggcgatttt ggttttcgcg atcgttgggt tcggttggtc 8940
   ggttagattc gaattcgtgt atcgtttgtt ttagttgcgt tttttagggg cgtttttgtg 9000
   tttttttcgg gtcgcggaga tttttttagc gttcggtttc gaattttttg cgtaacgttt 9060
   ttttgttagt cgtacggttt tgtagagaag gcgggagagt gtttgtgatt ttgttttgta 9120
   gttttcggtg atttaaaaat ttaggtcgtt gtggaattta gtttttacgg tattgttttt 9180
   gtaggtagta gttttgggtt tatatttgag gtcgtttttt ttatagatat ttttgggtga 9240
   tgcgagtgtt gcgtagttta gaatttttgg aagtatgaga ggaggacgag ttcggcggtt 9300
   tgtcggtgcg gtgatggagg gtttttcggt agttatattg cgcgtttttt atatatattt 9360
   taatagtttt ggggtttcgg gttagcgttg tagtttaaag agattttgta gtcgtatttt 9420
   tttttaggtc gtggtaaatt ttggtttcgt ttttttttat tatggtttgt gagggtagga 9480
   ggttttcggg ggtcgatttt tttgatttga atgagttagg tatttttttt tattttaagt 9540
   gggggtgtga tttgaggaga taaaggaagg acgatagatt tggggagtgg ggttttgtag 9600
   ttttttgggt tcggagcgta ttttttttcg tttttttttt ttatcggtag gatcgggggt 9660
   tttgaaattg gagtcgttta tcggtcggcg gatgagagat aaaggagttc ggcggcgtcg 9720
   aagcgttgat tagtagattc ggattcggat tttttcgcgt tttagagtag tattcgaaat 9780
   tttatttttt aggtggtatc gacggttgag tagttttttc ggtgttatac ggtgttttta 9840
   ggtatagata gtcgtgttcg cgtgtgtatt gtgtacgtat aagattgtag gatgatgttt 9900
   tgtatgagtt ttgtattagt ttttttgttt ttagttagag gtatacgtat acgtgtgtgt 9960
   atttgttaag tgtttaggga taggtgtgta tttttatata tacgtgatgt gtatgtgtat 10020
   atatttgtag aacgttttag tatttatttt ataggtacgt atagggttgg ttgtttttaa 10080
   taagttatta ttatatttcg tatttatata ggttttttta gttttggtat gatttttaga 10140
   attatttcgg gaaatatttt ttttttaaag tttatagatg aggaaataag taaaaagaat 10200
   ttaatgggta ttcgtttgta tatattgtga gagagatcga ggttagttaa agatttcgtt 10260
   tattgttgaa tttgggtgtg ttggtcgaat gtgtttatag atgggtttgt ttagcgtttt 10320
   aggcgatttt aggttcggcg ggttttggtt tttcgttagg ttttttggga gatttattgc 10380
   gtcgggtgtg gggttttaat ttttgtgttg ataagtatta ttttttggag ttgggtgatt 10440
   ttagtgttta gggcgggaag tcgggagttg gtcggagtgt cgttgaggcg atttcggcgg 10500
   ttttaaggtt atggttgatt acgcgtaatt gggttgtaaa gtttggcggg tttcggtttt 10560
   taaagtagtt acgttaggta aacgtgcgat gaaaggattt ttttagttta tttataaatt 10620
   taggaattta gggttgttgg agtagtgagg aaatttttgt tgtttcgaat ttttagcgtt 10680
   tttacgagat tcggagttta taatgtttat tttatagaag tgattagtcg gaggttagag 10740
   cggtatagtg agttgtttaa gtttaacgtt agttgagtgg ttatagtcgg gattggaatt 10800
   tttaattaag tgtgtttgga tttttggttt atagtttttt tgtttttagt aaggatcgtt 10860
   ggtttttttg tttttcgcgc gtttaggagt tatttcgtta ttagttttcg atttttttcg 10920
   ttaagaatgc gaaaattatt atagttttta ttagtattat ttaaaaggtt tttatatgtt 10980
   tggaatttgt tgtttgaaaa taaatttttt gtgtgcgttg gttttttaat tttttttaag 11040
   gtatttggtt tttttttatt ttttaagttt ttgtcgtcgt agagtgaggt tgtttaaggg 11100
   ttgtagtttg attatatttc gggggtgttt ttgggttttt gcgtcgggtt tttgtagatt 11160
   gaggtcgttg gtttttgggt tttaaggtcg tttttatttc gtagaattcg ggagttggaa 11220
   gggagttcgc ggtttggcga ggtcgtcgtt cgggtttacg gtttcgaggt tgttttttgt 11280
   tggtattggg cgatattatt atcgtttttt tcggcggttt aggagttgta tttacgtgag 11340
   gggttcgcgt agagttaggc gattttattt ttattgggtt tttagagtgg ttgtcgtatt 11400
   tgtagttatt gacgagattt tttcgtattt tttaattcga gatttttttt cgtagatatt 11460
   agaggtttcg gtgttcggat ttagggataa ggtagagagt cggagttttg cgttggtaat 11520
   ttcgtcgttt tgcggcgttc gtcgggattc gcggttaatt aggtacggtt ataggcggac 11580
   gttgggggat ttcggggatg gttttaggtt gtgttcgttt tttttatttt tcgttattat 11640
   agtattgatt ggcgtttacg ggtttcg 11667

<210> 18
<211> 1394
<212> DNA
<213> Artificial Sequence

<220>
<223> chemically treated genomic DNA (Homo sapiens)

<400> 18

cggaatttcg gttcgaagtc gagataggag attggatgcg aggttttttt agagttggtt 60
ttttttaaat aatttttaaa atttttagat tttaggggta cgtcgaaatt ttttaaagta 120
gtttaaagaa tataacgaga gttttaatat tttaggtggc ggcgcgttgg ttttttggag 180
cggggcggga cgcggtcgcg cggatttacg tgtataatcg cgcgggacgg ggttacgcgg 240
atttacgtgt ataatcgcgg gattttagcg ttagcgggat tttagcgtta gcgggatttt 300
agcgttagcg ggattttagc gttagcggga ttttagcgtt agcgggattt tagcgttagc 360
gggattttag cgttagcggg tttgtggttt agtggagcga gtggagcgtt ggcgatttga 420
gcggagattg cgttttggac gttttagttt agacgttaag ttatagttcg cgtagtagta 480
gtaaagggga aggggtagga gtcgggtata gttggattcg gaggtcgtga tttaggggaa 540
agcgtgggcg gtcgatttag ggtagttgcg gcggcgaggt aggtgggttt tttgtttttt 600
ggagtcgttt ttttttatat ttgttttcgg cgtttttagt agtttttatt ttggtttttc 660
gcggttattg cgggattcgg cgttgtcgtt agtttagtgg ggagtgaatt agcgtttttt 720
ttcgttttcg gttttttcga cggtacgagg aatttttgtt ttgttttata gattttcggt 780
tttcgtcgag tgcggtattg gagtttgttt cgttagggtt ttggaattag agaaagtcgt 840
tttttggtta tttgaagcgt cggattttta tagtgttttt tagtttgggc gggagcggcg 900
gttgcgtcgt tgaaggttgg ggtttttggt gcgaaaggga ggtagttgta gttttagttt 960
tattttagaa gcggttttcg tatcgttgcg gtgggcgttt tcgggtttcg atttcgttag 1020
cgtcgcgggg tagaggtatt tggagttcgt agggtttaga tttgggttgg aaaagtttcg 1080
ttgattgtag gtaagcgttc gggaggggcg gttaggcgaa gtttcggcgt tttattatat 1140
attttcgggt tttatgttag ttgtattcgc ggtattgggt aggaaatggt agggttgagg 1200
tcgattttag gagtataagg gagtttttta ttttttgttt atatttgtta tttttagttt 1260
tgtaatttat tttagatata tagaaagtaa gtaggattgg tggggagacg gagtttaata 1320
ggaatatttt ttagtagtga gtaggggttg tatgggacgc gggaggagtt tagaggaggc 1380
gcggagagtg ttcg 1394

<210> 19
<211> 1394
<212> DNA
<213> Artificial Sequence

<220>
<223> chemically treated genomic DNA (Homo sapiens)

<400> 19

cgggtatttt tcgcgttttt tttgagtttt tttcgcgttt tatatagttt ttgtttattg 60
ttggaaaata tttttgttaa gtttcgtttt tttattagtt ttgtttgttt tttgtgtgtt 120
tgggataggt tgtaaaattg gaggtgataa atgtgggtag gaaatggagg gtttttttat 180
atttttaggg tcggttttag ttttgttatt ttttgtttaa tatcgcggat gtaattggta 240
tgggattcgg aagtgtgtgg taaagcgtcg gggtttcgtt tggtcgtttt tttcggacgt 300
ttgtttgtag ttagcgaagt ttttttaatt taggtttggg ttttgcgagt tttaggtgtt 360
tttgtttcgc ggcgttggcg aagtcgaagt tcgagaacgt ttatcgtagc gatgcgaagg 420
tcgtttttgg ggtggggttg aggttgtagt tgtttttttt tcgtattaag gattttaatt 480
tttagcgacg tagtcgtcgt tttcgtttag gttgggaggt attgtaggga ttcgacgttt 540
taggtggtta aagagcgatt ttttttgatt ttagggtttt ggcggggtag gttttagtat 600
cgtattcggc ggaggtcgaa ggtttgtggg gtaggatagg agtttttcgt gtcgtcggaa 660
gggtcgagga cgaaggaggg cgttaattta ttttttattg ggttggcggt aacgtcgaat 720
ttcgtagtga tcgcggaggg ttaaggtgaa aattgttggg ggcgtcgagg gtaggtgtgg 780
ggaggggcgg ttttagggag taaggagttt atttgtttcg tcgtcgtagt tgttttgggt 840
cgatcgttta cgtttttttt tgggttacga ttttcggatt taattgtgtt cggtttttgt 900
tttttttttt ttgttgttgt tgcgcgggtt gtaatttgac gtttaggttg gggcgtttag 960
ggcgtagttt tcgtttaggt cgttagcgtt ttattcgttt tattgggtta tagattcgtt 1020
ggcgttgggg tttcgttggc gttggggttt cgttggcgtt ggggtttcgt tggcgttggg 1080
gtttcgttgg cgttggggtt tcgttggcgt tggggtttcg ttggcgttgg ggtttcgcgg 1140
ttgtgtacgt gagttcgcgt ggtttcgttt cgcgcggttg tgtacgtgag ttcgcgcggt 1200
cgcgtttcgt ttcgttttag ggagttagcg cgtcgttatt tggatgttta ggattttcgt 1260
tgtgtttttt ggattgtttt gggggatttc ggcgtatttt taggatttag gagttttgga 1320
agttgtttga gagaaattag ttttgggagg gtttcgtatt tagttttttg tttcggtttc 1380
ggatcggggt ttcg 1394

<210> 20
<211> 6357
<212> DNA
<213> Artificial Sequence

<220>
<223> chemically treated genomic DNA (Homo sapiens)

<400> 20

gtgtggagat tgggaaggtg ataaggtgaa ggtaattgaa ggaagagtcg agggggatat 60
ggggaaggat tttgttttat tttttttaag ttgaattatt gttttttgaa ggtcggtttt 120
tggagaaatt aaagggtttt tgtgtgatat agttatgtta tatataaata gaattttgaa 180
gtttattaat ttttgaggtt aagtaagagg gaatgtaggg gttaaggtag aagagaaatt 240
aaaattttag agcgttgagt aaagatgtta attagagaaa gagaaattta tttgcgatgt 300
taattaataa gcggttaatt aaaacggtat tttgagtgtt aattaatcgt tttattaagt 360
tatagttatt attggaataa attgaaattt tttcgtttcg ttttttgttt ttggtgtagg 420
cggggtcgcg tttttagata tcgtgagagg ttttggggcg cggaggttgg gggtagtttc 480
ggttagtttt tttagttttt tttaggttta tagaatacgt tattggataa gtgtttaagt 540
agcgattttt ggtttagata tatcgttcgg ggagtaagta gttgcgtcga agaataattt 600
atttagtagt agttaatatc gacgtttttt tttagaaaga gttttcgtaa agcgggggga 660
tgtgatttgt gggtttttag taggggtagg aggtagttta gttcgagagg gggcgtttta 720
gggtttggat tttgtatttt tattttttgg aatatattta acgttttatt ttgaaaattt 780
tgtttaggtt ttggttttgg tgtcgtttag taattaggaa agagttggat ttgtttttag 840
gtagtaagaa taggattgtt agttttttgt ggttttgttt ttcgaggttt tatgagaagg 900
ggatgggggt gtaagaaggg aagagtgagg tggtgtgttg ggcgtcgggg acgaggacgt 960
acgttagtta agacgtgttt tttatttagt ttacgcgcgt ttttttattt ttttggtttt 1020
ttaaaatcgg taagagaatt aagatttcga atttttattt tgaggagttt ttcgtatttt 1080
ttaattgtta aatttttgtt ttttattaaa ttttcggggg agaaatattt ggtaataaga 1140
agggattgtg aatttaaatg ttaattgagt gggttttttt ttcgtagttt tatttgtttg 1200
gtagtttttg ttgaaattaa atatattcgg agcgtttagt gtaatatttt tggggtgtcg 1260
agtagaagcg tagtaaagag agattttagc ggatttttgt ttggtttgtt ttttatcgat 1320
ttttatagaa aaaaagagaa tgttattgga agaagttttt ttgcgtggtg ggcgatgtgt 1380
gggtggggga tttgtggtat ggtttacggt gttgtttttg tgtttgcgat gatatacgta 1440
tgttttgagt tgtgggttcg tttttttgga ggtgcgttcg atcgtatttg ttggtgggtt 1500
tgagcgtgtt tggggtgttt taggagaatt gagagaacgg tttttacgtg taaagtttta 1560
aagtattaat atttttatta tattattatt atttaatata ataatatttg ttcggttagc 1620

ggtattaatt aggttatatt aaaatcgtag tgtgttttta atggtgcgta atgtgtttat 1680
atttatattt ttttttttga ggatgggcgg ttgtaggttg gtaggggagg agagataggt 1740
aagcggcggg ttggattagg gcgtgacgtt ttttattacg tatataaata tatatagttt 1800
attggatgtt tgtcgggtgg gagtcgtaat tttcgcgcgg tcgatggggt ttttcgttgc 1860
gtattcggtt ttgcgtcgag tattttgtag ttttttttcg cgatacggcg ttttgaattc 1920
ggcggattga ttttgttttt tttttttttt ttgtgtgtgt ttgcgtttaa ttggttaggt 1980
ttttaagatt tgggagggtt ggtgtgaaag aattaaaata tttttaattg gagttttttc 2040
gtcgagaatt ggaggtttcg ttttttagtt cggcgttttt aggatttttt ttttagaggg 2100
aatttttttt agaaatttta gggtgggttt gtaaaagacg ttttcgtaga gtaggtttcg 2160
ttagggtttt ttttttgttt ttggtgttag cggtcggttc gggcgtttcg tagatttcgg 2220
cgaggtagat gttaagttcg gagagtgttt ttttcgtagg cgtcgtggcg agattatttt 2280
gaatatgtaa tatatttgta acgtgcgtcg aggtgtgatg tgtgtgttga aataggggga 2340
tgggggaatt cgaagtcgga ttgggaaggc gggggggagg cgtatagaat ttataatgta 2400
tttcgtaatt taataatttg aatatttatt tattaaaagt tgttgcgtga tatttatatt 2460
gagttattag tttttgtttt taattcgggc gaaaacgatt gtattgtcga gttatggttg 2520
tagcgtatgg ggacgttgtt gttcgcggtc ggatagagtt tattagttat aacgcggaag 2580
gtttttgtat ttttttgggg gcgggaggaa agtattgtta gttttgtttg ggggtcgagg 2640
gtaataagta tcgagttttt cgttttacgt agggttagtt gtttagttta gcgaagtttt 2700
tgtgatttgg tgcgtgtttt tcgttttttt tttttattta aagaagtaaa tttttttattt 2760
atttttttta attcgatcgt ttagagttgt tgtttttttt ttgttagatt tttttttttc 2820
gattagtttg agtatacgat tagaattgtt tagagagtag gaagtatatt gattttagtt 2880
tgttttgttt atagataggt tttgataagg ttgttagaat agtcggagag gtttatataa 2940
ttatttaatt attaaaattg ttagttaggc gggacgcgga ttcgcgtttc gggttgcgtt 3000
aggtatttta gtattgggtc gcgcgcgtga ttgatcggtg ttgatagtat cgtaaaataa 3060
ttacggcgaa ttttttgatg tgtgatttta ttttaagttt atgttttaga gaggtaatcg 3120
gagaatgaga agggttagtg ttatttcgga ttatttggaa tttgcgagaa agggtaaaat 3180
gggggaagga gtttcgagga aaacgggaga gatgggggtg tagagagaga gggaagaaga 3240
aagcgagtta tggattgttg gagggattgt aagtaattcg ttaaattgtg taagtgattt 3300
tttttagagt tagtatatgg tagattgatt ttgtttaacg tcggttttag ttatatttaa 3360
aatgatttag cggttattat tgcgattggt ttaggaattg ataggtagtt ttaggcgtaa 3420
ggagtataga ttttgtttat cggagatgtg ttcgtaattg ttgttaaata tagttaagta 3480
aatattatta gcgaagagtt ttgttaagag aaatgttaat ttaataaata tgtttttttt 3540
tttcgttttt cgtatggttg tttgcgtttt ttttagaggt tttttttttt gtttttttgt 3600
tgtttgggtt agacgtttta ggtatggtgt tgattttcgt tattttggag tttcgagttg 3660
agtttcgggt agaagatgat aggttagtcg tggggtaagg aggtcgcgga aacgcggaac 3720
ggtttcgggg agacggaagc gtttaatgag atttattttg tagttcgggt ttagtttatt 3780
tttttcggag attgtcgcgg ttttcgaatt cgggtttagg tttttatgtt tcggcggtta 3840
gaggacgttg cggggattat tggggagttg tttttagtta gttttttgtt ttacgtcgga 3900
ggttttggcg cggttttttt ttcgaattag attggcgatt ttgggttagg ttttaaggat 3960
cgtttcggtt ttttcggttt tgcggggaga atttgaggaa tcgagtttaa gatagtcgat 4020
ttaggttgtt tttatttaga ttttgcgttt tcgattcgtt ggagtgaatt tgatattgtt 4080
aggttttttt ttatggtatg gagtgaatga agagggttat agattttttt atttagtata 4140
gtttttcggt aggttttgga aatttatagg gagtagaagt atagtatttt ttgaatcgtt 4200
tttttttttt gggtttgtgg ttatttgaag gtagagtttt gtgtttttaa gatagtaggt 4260
tttcggttaa gtttggagtt tggggtttta atatatttat atagggttgg tattatcgtt 4320
tttttggatt aaaggtaggt ttttatattt tttttaaagg aatagaagga aggaaaagga 4380
aattaatacg ggttatgttt agatagtagg tttatggtaa ttttttatag ttatagagtt 4440
ttaattcgag tattttttta gagaggaaaa atttaaaaaa tttttaaaag ggggaaagtt 4500
gggtagatta tagtatttat tttttatgtt taggtagaaa aattaattta gagggagtaa 4560
aggggtaaga aatatgaaga gatttttttt gggagttgag gagtatttta gtttataatt 4620
tggttaaagg agaaagttat tggttttttt ttttgataga ggcgtgttat ttattttttt 4680
agggaatatg atggtttata aatgaagagg ttagtttttt tgtagttttt ttttatagag 4740
tgtaaaatat atatcgtttt tattagtgtt tgggatgtaa agaattttgt ttatttaaaa 4800
gagatattgt atttttaaag ttaaatagta ttaatgtatg tggcgaatta agtaggtaaa 4860
taatttatat atggttgttg tatttgaagg aattatttat ttttatgtat agtaaattga 4920
agaaataatg gtattaatga gttttgtaaa atgtaattgt gaataatgaa agataatatt 4980
gtattttgta atagaaagaa taaaggtgaa ataattagtt agtaaagagg aaaagaaagc 5040
gagtaatgat taaatgatta aaagttggta gagtgaattt aatgttattg ttagacgtag 5100
ttatttattt ataagtgaaa gttaggtttt aagtatagtg taattatagt tggggttgtt 5160
agtttgatat taatgtagtt agtagaaatt ttttaattgg ttttagagga gaaagtgaat 5220
tagaaaatat attaatattt taaaaaagta tattttgttt aattttttta ttttcgaata 5280
atatttgaag attaaaatgt tttaggtata agaatttaaa tgagtaattt tgtttttgaa 5340
ggaaacggtt aatgagatag aaaatagatt aaagggaaat tattagtgga tgagagatat 5400

tgataggttt gttttgttga ttggttggtt tgttatttgt agtttgtgtt ttttagtttt 5460
acgttatgag ttaagttgat aatatgaaaa gatttataaa cgtgtagtta gaagttatag 5520
tttattattt ggaaatttaa atgtaagggg agggggtggt agagaaggta tcggcgaggt 5580
tgggagggag aggtgtgtat cgagggagga ggaggaggag gaaggggagg agggaaagga 5640
ggaggaggag gagaaaagaa gttttatttt tttggtataa aatcggttat attagagaat 5700
aataataagt tattatttgt tataaatgtg ttatggattg ttaaaaaatg tagtttcgaa 5760
tcgataatat tgttcgaatt gaagatagta ataaaatgtt taaagttgcg gatgtaattt 5820
tatatgcgtt cgggttgatg tgatatgatc gtattaggga aataaagtta agtgtagtta 5880
ggatttgtta gtatagtggt ttttgatgga atagttgagg tatatatcgt tcgtggtatg 5940
gatttcgggg tcgaacgttt acgattaaga tttttgtttt tttgaaatga aatagaaata 6000
ggggagttgt aggaaaatcg aatcgcgttt agggttagga gtaagatagg agtttttagc 6060
gaagatttga atatttagaa ttggaacggg taattagtag atagttagga aaaaataaat 6120
aaataaataa aaaagtttgg atggattttt gtaaataatt attaagaaaa ataaaaatga 6180
atttttttat tagtttgttt tggaggtagt tagaaataaa taattaaagt aagagaggat 6240
gaagatttaa ataaaataat tatgtgtatt attaaaataa ttatatatgt ttgtatagat 6300
acgtatatat taaggaacgt aatgggggtt tttcgtatag ttttaggaga tgtagga 6357

<210> 21
<211> 6357
<212> DNA
<213> Artificial Sequence

<220>
<223> chemically treated genomic DNA (Homo sapiens)

<400> 21

ttttgtattt tttgggattg tgcgaggagt ttttattacg ttttttggtg tatacgtgtt 60
tgtataaata tatatgatta ttttaatgat gtatataatt attttattta aatttttatt 120
tttttttgtt ttggttgttt gtttttgatt atttttaagg taggttaata agaaggttta 180
tttttatttt ttttaatgat tgtttataga ggtttattta ggttttttta tttatttatt 240
tatttttttt tggttatttg ttaattattc gttttagttt tgaatgttta gattttcgtt 300
gaaagttttt gttttgtttt tgattttaag cgcgattcgg tttttttgta gtttttttat 360
ttttatttta ttttaaaagg gtaaaagttt tggtcgtgag cgttcggttt cggagtttat 420
gttacgggcg atgtgtgttt tagttgtttt attaaaagtt attgtattaa tagattttga 480
ttgtatttag ttttgttttt ttgatacggt tatattatat taattcggac gtatgtgaaa 540
ttatattcgt aattttaagt attttgttgt tatttttagt tcgaataatg ttgtcgattc 600
gggattatat tttttggtag tttatagtat atttatgata gataatagtt tattattgtt 660
ttttgatatg gtcgatttta tgttaaagaa tgagggtttt tttttttttt tttttttttt 720
tttttttttt tttttttttt tttttttttt tttttcgatg tatatttttt ttttttaatt 780
tcgtcgatgt tttttttgtt attttttttt tttgtatttg aatttttaga taataggttg 840
tgatttttgg ttgtacgttt atgggttttt ttatgttatt aatttagttt atagcgtgga 900
attaaagaat atagattgta agtgataggt tagttagtta gtaaggtaag tttgttagta 960
ttttttattt attaatgatt tttttttagt ttattttttg ttttattggt cgtttttttt 1020
aaaaataaaa ttgtttattt aaatttttat gtttggggta ttttggtttt taaatattgt 1080
tcgaaagtga aaggattagg taaaatatgt ttttttaaaa tgttaatata ttttttggtt 1140
tatttttttt tttgaggtta attaggaaat ttttgttggt tgtattaatg ttaaattgat 1200
aattttagtt ataattatat tgtgtttgaa atttaatttt tatttgtggg tagatggttg 1260
cgtttggtag tgatattgaa tttattttgt tagtttttga ttatttaatt attgttcgtt 1320
tttttttttt ttttgttagt tgattatttt atttttattt tttttgttgt aaaatgtagt 1380
gttgtttttt attatttata gttgtatttt gtaaggttta ttagtgttat tgttttttta 1440
atttgttgtg tatgagaatg gatggttttt ttaagtgtag taattatatg taagttgttt 1500
atttatttga ttcgttatat atattggtat tgtttgattt taaaaatgta gtattttttt 1560
taaatagata gggtttttta tattttaaat attgatgaag gcggtgtgtg ttttatattt 1620
tgtagaaaaa agttgtagga gggttagttt ttttatttgt gaattattat gttttttggg 1680
aagatagatg atacgttttt attaaaggag gaggttagtg attttttttt ttgattaaat 1740
tataaattag ggtgtttttt agttttagaa ggggattttt ttatattttt tatttttttg 1800
ttttttttgg gttagttttt ttgtttaggt atgaagaatg ggtgttatga tttatttagt 1860
tttttttttt ttaaaagttt tttgggtttt ttttttttgg gaagatattc ggattaggat 1920
tttatggttg tgaagaattg ttataggttt attatttgaa tataattcgt gttggttttt 1980
tttttttttt ttttgttttt ttaaaaagga tataggagtt tgtttttagt ttaaggaaac 2040
ggtgatgtta attttgtgta aatgtgttgg ggttttaggt tttaaatttg atcgaaaatt 2100
tattgttttg gaggtataga gttttgtttt taaatggtta taggtttagg gagagggagc 2160

ggtttagaaa atattgtgtt tttgtttttt gtggattttt agggtttgtc gagggattgt 2220
gttgggtaag gggatttatg gtttttttta tttattttat gttatgagag agaatttggt 2280
agtgttagat ttattttagc gggtcgggga cgtagggttt gggtgaaaat agtttaggtc 2340
ggttattttg gattcggttt tttagatttt tttcgtaaag tcggagaggt cggggcggtt 2400
tttggggttt ggtttagagt cgttagttta gttcgggaaa gaagtcgcgt taggattttc 2460
ggcgtggggt agagagttga ttgagggtag ttttttagtg gttttcgtaa cgttttttgg 2520
tcgtcgggat atgaagattt aggttcgggt tcgagggtcg cggtaatttt cgaggaaggt 2580
gggttggatt cgggttgtag ggtgaatttt attgggcgtt ttcgtttttt cgaagtcgtt 2640
tcgcgttttc gcggtttttt tgttttacgg ttggtttgtt attttttgtt cgaggtttag 2700
ttcggggttt taaggtggcg ggagttagta ttatgtttgg gacgtttgat ttaagtagta 2760
aaggagtagg aaggagaatt tttggaggaa gcgtaggtag ttatgcggag ggcggggagg 2820
aaaagtatat ttattggatt ggtatttttt ttaatagagt ttttcgttaa tgatatttat 2880
ttaattgtat ttgatagtag ttacgaatat attttcggta aataggattt atattttttg 2940
cgtttaaaat tgtttgttag tttttaagtt aatcgtagta ataatcgttg gattatttta 3000
aatgtggtta aaatcgacgt tggataaaat taatttgtta tatgttggtt ttgaaggaaa 3060
ttatttgtat agtttgacga attgtttgta gtttttttag taatttataa ttcgtttttt 3120
tttttttttt ttttttgtat ttttattttt ttcgtttttt tcggagtttt tttttttatt 3180
ttattttttt tcgtagattt taggtaattc gaaatggtat tgattttttt tatttttcga 3240
ttattttttt gaagtatgaa tttgggataa aattatatat tagaaaattc gtcgtaatta 3300
ttttgcggtg ttattagtat cgattaatta cgcgcgcggt ttagtgttgg aatgtttagc 3360
gtagttcggg acgcggattc gcgtttcgtt tgattgatag ttttggtaat taagtgattg 3420
tatagatttt ttcggttgtt ttaataattt tgttagggtt tgtttgtgga tagaataagt 3480
tgaaattaat gtgttttttg ttttttgagt agttttgatc gtgtatttag attgatcggg 3540
gaggaggaat ttgataaaag gaaaatagta gttttaaacg atcggattag ggggagtagg 3600
tagaaagttt atttttttga tggggaggga agagcgagag atacgtatta gattataaga 3660
gtttcgttga gttgggtagt tggttttgcg tggagcgaga ggttcggtgt ttgttatttt 3720
cggtttttag gtaggattgg tagtattttt ttttcgtttt taagggggtg tagaggtttt 3780
tcgcgttgta gttgatgggt tttgttcggt cgcggatagt agcgttttta tacgttgtag 3840
ttataattcg gtagtataat cgttttcgtt cggattagag gtagagattg gtggtttagt 3900
gtaaatgtta cgtagtagtt tttaataaat gaatgtttag attgttagat tgcgaagtat 3960
attgtgagtt ttgtgcgttt ttttttcgtt tttttaattc ggtttcgaat ttttttattt 4020
ttttatttta gtatatatat tatatttcgg cgtacgttat aaatatgtta tatatttaga 4080
gtgatttcgt tacggcgttt gcgggagggg tatttttcga gtttaatatt tatttcgtcg 4140
aggtttgcgg ggcgttcggg tcgatcgttg gtattaggaa taggaaaaag attttgacgg 4200
gatttgtttt gcggaagcgt tttttataag tttattttgg aatttttgaa agaaattttt 4260
tttgggaaga gggttttgaa agcgtcgaat taggaggcgg gatttttagt tttcggcgga 4320
ggggttttag ttaagagtat tttaattttt ttatattagt ttttttaaat tttaaaaatt 4380
taattaattg aacgtaaata tatataaaag gggaagggga agtaaaatta attcgtcgag 4440
tttaaagcgt cgtgtcgcgg gaggaggttg tagggtgttc ggcgtagggt cgagtgcgta 4500
gcggagggtt ttatcgatcg cgcggagatt gcggttttta ttcggtagat atttagtggg 4560
ttgtgtatgt ttgtgtgcgt ggtggggggc gttacgtttt aatttagttc gtcgtttgtt 4620
tgtttttttt tttttattag tttgtagtcg tttattttta gagagaaaaa tgtgagtgtg 4680
agtatattac gtattattag ggatatatta cggttttaat gtggtttaat tagtgtcgtt 4740
aatcgaataa atattattat attgaataat gataatatga tgaaaatatt aatgttttgg 4800
aattttgtac gtgggagtcg tttttttagt ttttttggga tattttaagt acgtttagat 4860
ttattagtag atgcggtcgg gcgtattttt aggaaggcga gtttatagtt taagatatac 4920
gtgtgttatc gtaggtatag aaataatatc gtgggttatg ttataagttt tttatttata 4980
tatcgtttat tacgtaaaag agtttttttt aatggtattt tttttttttt tgtaagagtc 5040
ggtaaaaagt aaattagata ggagttcgtt agggtttttt tttattgcgt ttttattcgg 5100
tattttaaga atgttgtatt gggcgtttcg agtgtgtttg gttttaatag aggttgttag 5160
gtaggtggag ttgcggaaaa aggatttatt taattagtat ttaaatttat agtttttttt 5220
tattgttaaa tgtttttttt tcgggaattt ggtgaaaagt aggaatttaa taattaggaa 5280
atgcggaagg ttttttaaaa tagggattcg aaattttaat ttttttatcg attttggagg 5340
gttagggggg tggggaagcg cgcgtgggtt gggtgggagg tacgttttgg ttggcgtgcg 5400
ttttcgtttt cgacgtttag tatattattt tatttttttt tttttgtatt tttatttttt 5460
ttttatggag tttcgggaga tagagttata ggaggttggt agttttgttt ttgttgtttg 5520
aaggtaggtt tagttttttt ttggttgttg agcggtatta gggttagggt ttaagtaggg 5580
tttttagaat gaggcgttgg gtgtgtttta ggaaataggg atgtaggatt taggttttag 5640
agcgtttttt ttcgggttga attgtttttt atttttgttg ggggtttata ggttatattt 5700
tttcgttttg cgggagtttt ttttaggagg aaacgtcggt gttaattgtt gttgaatgag 5760
ttgtttttcg acgtaattat ttatttttcg ggcggtgtgt ttggattaga agtcgttgtt 5820
taggtatttg tttagtggcg tattttgtag atttgggaga gattgagaaa gttgatcgag 5880
gttgttttta attttcgcgt tttaaggttt tttacggtat ttgggaacgc ggtttcgttt 5940

gtattaaagg tagaaaacgg ggcggggagg ttttaatttg ttttagtgat ggttgtaatt 6000
taataaggcg attgattagt atttaaagtg tcgttttaat tagtcgtttg ttaattaata 6060
tcgtaaatga attttttttt ttttgattgg tatttttgtt tagcgttttg aggttttggt 6120
tttttttttg ttttggtttt tatatttttt tttatttagt tttaggagtt gataggtttt 6180
agagttttgt ttatgtatga tatggttgtg ttatataggg gttttttaat ttttttagga 6240
gtcggttttt aaaggataat ggtttaattt aggaggggtg aaataaaatt tttttttatg 6300
tttttttcgg tttttttttt aattgttttt attttgttat ttttttaatt tttatat 6357

<210> 22
<211> 9739
<212> DNA
<213> Artificial Sequence

<220>
<223> chemically treated genomic DNA (Homo sapiens)

<400> 22

atggaagtat agaaatgtat ttaataaata tgtaatagaa ggataattaa tttaatatta 60
aagtgattag agagttaatg ttagggtttt atgggtatgt tgatatttta gaatagagag 120
gattgaaggt tagagatttt gggtaatttg tatattaaga atgtttttaa gttattgggg 180
ggaaagatag agtttttttt gttttatata aaggggtaga tttaaaatta tagtttttta 240
aaatttttaa aaatttttgt aagatttgaa gagagttttg ttttaaattt tagtgtatat 300
aatataaata tatattatta atatttataa taagagattg gaattaaagt gtatattatt 360
ttttaaagtt ttttaaaatt gtttttaatt ataaaaaggt aaattaaaaa ttttttttat 420
gttatagtag tttaaaaggt aaataatgga agtagaaagg agatgtatat ttgttgaaat 480
ttgttatgta ttaggtattt tatataattt attttatttt gattttata ataattggta 540
aggtgtgtgt tgtaggatat taagaggtta tagattttag gaggttaaga aatgatttaa 600
aattttatat ttaatagttg gtatagtaaa gatttgaagt taagaatatt tgtttttaaa 660
aattagttgt atattatgat aatatatttt aaataaagat gtataaaaag tatttggttt 720
tttatagata ttatataaaa tatgggataa tattagtgat atatttaaat ttagatatta 780
gaaagaagtt ttttttaatt tattttgtaa ttgtaattta gttgtttatt ttataggttt 840
ttatagtaat aattaaatat tttttttttt gttttatatg tgtttttaaa ttgttattaa 900
agatttttgg ttagtagttt ttatattttt tggtgttttg ttgttaatgt ttggtaaata 960
tgggtagtta aggaaaatgt agatggtgtt atggaatata aattatgata atatatggta 1020
tatatttagt tttaataaag tgtgtttttt aatttttggg tgttgtttta ttattttttt 1080
ataagattat ttatttttag ggaaggttta gtttttaaaa ttagggtaat aggtttatat 1140
gtgttatatg gttatttaga tgtatagatt atgtgtttta atatttttaa gtaagtgtat 1200
ttagtatatg ttttttagta ttattatgtg tatttatgaa ttgttttgtt tagatggtaa 1260
gttttttgag gataagtgtt atgtttgaaa ttttatttta ttaattgtgt atggtagagt 1320
aataatgtta tttattttgt atatagagat atataattta taaattgttt ttttaggtaa 1380
attttttaat tatattgtta tattgtgata attgttatat tttttagatg aggtttatat 1440
aggttaagta attagttaga attataaatt aataggtaat gttttttttt tgtttataag 1500
agaaagtttt ttttgttttg taatatttta ttgttttttt tttttaaggt ttttattata 1560
aaggttaatg tttattggtt ttttaataaa agtaattatt atttagtatt tagtatatat 1620
atgtaaggta ttgttttaag ggttttataa atattaattt atttaatttt tttaattatt 1680
ttatgagata gatattattt ttatttatat tttatagata aggaaattga gggatagaga 1740
gattaatttg tttaaaatta gaggattggt aaatagtagt agtgaaattt gaatttagta 1800
gtgaagtata gttttatagt gtgtgttttt aaatatatat tattttgttt gaataagagt 1860
ttataaagat ttttatttag gatatatgag gagttgttta gtaagaaaag aagtatgttt 1920
attttttttt atttgatgta tttgattata ttttaaaatt tttttttatg ttaatagaat 1980
tttgtgtatt atattaaatt tttttgtatt gaaggtatat gtaatggtta ttttgattga 2040
tatgtaaaat atttaaaata aaatattttg agatttgagg attttttgtt ttatgaatta 2100
gatattaaaa aaaaaattgt ttaggtatta gaagtgttat atatttatat ttgtaagttt 2160
tttttgggat ttttagattt tttatataag attttttttt gtttgaaata ttttagtgtt 2220
tatttaatgt ttgagtttga aaattattta atttttttta atttttttat ttttagttat 2280
tttgttttta ggtatagggt agggtaagta tttgttttta atatgatgtt taatattttt 2340
tatgttttta tttttgttat tttgaaaata tgttagtaaa tatttaaata tattgtaaag 2400
atttaaaatt tggggtgtgt atatgtatat taataggagt tatgaattat gtatattgta 2460
ggtttatata aattagattt tgaagaataa gtattatagt aattaagtgg gttataataa 2520
gatattttat gtgaaatgta aaaattattt tgaataaatt atttgtaagt taatattttt 2580
attagaatga tatatttatt atttttgggt ttgtgatatt gttttaatgt attttggtta 2640
attgttttaa tggtatatta agaaattatt ttaggttttt tttttttgtt ggaatttgta 2700

gatttttatt tttgtttgaa ttaaaataat ataatattaa aaataatttt ttatttttta 2760
atgtataata ttattttttt ttgtttagaa atatttaatt aaagtaggat ataattttag 2820
taattatttt tatatagaat gatttttaaa ttaatagtta gttgtatgat taaaaattgg 2880
gagattgata attaaaataa ttttaaatgt tttgtttatg tgatgaaaat gagtgatttt 2940
ttaatttttt tatatatatt aaattgattt tatagatttt ttgtattggt gtttaatata 3000
gttaagtgtt tgaggttatg taaaataaat aattttgaga ttttattgta aatgtttgta 3060
atttatgatg taaattgatt tgtgaagaaa aaataggatt ttatgttgtt gaagttaaag 3120
aggtattttt tagaaattaa aatagttttg aaatttgagt attgtattat attaaagtat 3180
tattatttga ggatttaaaa aagttataaa tttgggaaaa aatttaaaat agatgtatat 3240
tagtttagtt ttaagtaaag gtattgattt tattatttta ttgtgttttt tgtattatag 3300
ggtttttaat attaatgatg ttttaaataa ttttttttaa tttagttttt tttagttatt 3360
gattttgtaa tttgggaagg ttttgtatgt ttaaaagatt tggggagtag aggatggggg 3420
tgttttttat taattatatt aggattattg agttttgatt taaattataa gtatgttggg 3480
ttttatttaa atttaaagtg aattttttag attttttttt gggatggttt ggggaattat 3540
gagtttggaa tttttgttta tatattgagg tgtttgtttt agagtttgga tattggtatt 3600
ttgggagagt aggttttgtg ggagtttgga tttgaagggt gagattttat agggttaagg 3660
aaagtggttt ttgttttttg ttagttttgg gggagtagat gtaagaggag gtaagggtgt 3720
tgtgagtttt ttggatgtat tggttttata ggttgtgttt gagtggagta ttgtgaatgg 3780
ggttaagaaa ttttggtttt ttttgttgga tttggttgtt tttgtgggtt tttttgttta 3840
ttgtgttttt gttgtggttt gatttttgtg ggtttttgtg ttgaatttat ttggttttta 3900
ttgtatggga tatttttgat ttatttatgt tgtgttattg agtttttgta ttgatatttg 3960
gtgtttttgt tagtagggtt tggatgtatt gttttttttg attttgggtt ttttttgtgt 4020
tttgttgttt ggggtagatt ggttttgaga gggagttatt attttttttg ttttagggtt 4080
tttagggttt gaatttgtgt tgggatttgg gttaggatta gggtttggag tttggagttt 4140
gtttgttagg atttttggtt ttggtgttga ttggagtttg ttggaggtta taggattatg 4200
gtggatggtt tggttgtttt agggtgtatt atgtttgtag gtagatgttt attattaaaa 4260
attattgatg tttattaatt aggagatttg taaggtttgt gtattgaata tggtttaaat 4320
tttgtttagt ggtttttgaa agttaaaaga aagaaattta ttgtttatgt ttatgttgag 4380
gaatagtttt gaatatgagt taagatttgg gagagggtta agtgggggtg gtggggaata 4440
ttgttggagg atgtggggtt ttggtagggg ttttatgtat tttttagtat gagggtgggg 4500
ggtttagggg gatgtttagg atttttttag tttttgtgga aggttttgtt attataagag 4560
tttgtgtggg aaggaaagtt tttgttttgg atatggttag ggttgagttt ttaaagtttt 4620
taattatttt tatataggta gtatttttgg gttttattag tgggattttt tttagaaggg 4680
ggttatgata tggggagaga gagtttttta ttatttttaa ggttaggttt tttttaaatt 4740
tgattttggt ttttattttt taaatttaat taatgaagaa gttgttttag ttaattttaa 4800
ataggagtgt tagatgggga attttttttt tatagtgttt tggtttgttt gtttttgtgg 4860
tttttttttt gttttgaagt tagtatttgt tttgttttag agagggttaa tgaattggga 4920
ttgattgttg attatgttat attaaattta ggttgatttt gttttgtagg attttttttt 4980
tttttttttt aaaagtgttt ttagataaag attggaatta tagtaaaatg aataatgaga 5040
gtttattttg gggaaggaag ttattttatt ttatgttatt ttatttttta tttgtttttt 5100
tattaatttg gtataatttt gtttttatgt tgggtggata aaatggaggg ggttggtgga 5160
aatgtgttta gtgttaatga tagttataga gttatttttt ttattgtttt ttgattgtgg 5220
tatgtaagag tagaggtaag tgttttttta agttggtata tttgagagag tgatatgtat 5280
taattaaaag ggaaagatta ttttggatat tttaatagta ataggaataa agatgatttg 5340
aattttatta aatgaaatga tatttttatt taattgattg taaagtgttt ttaattaata 5400
atttggtatt tatttaaaag gtttaataga attggaggtg atatattttg tattgataat 5460
atgttatgag ataatatttt ttgtttaaaa taatttaata tattagaaaa aaatatgtta 5520
aatataaata attttgtttt tatggagaat aaatagttat aaagtttaat tgtatattaa 5580
agtttagtta gaagagtttt tttttttttt tgtaaattag aaggtaaaat aaaaataaaa 5640
ataaaatttt ataaatttag gtttgttagt gaattgggat atagattggt ttagtttaag 5700
gtattttagt ttaaatagtt atatgaattg aaataaataa ttaaaagaaa aaaaaaaata 5760
aaaaagaaat aaataaataa aaaaaaaagg ttatttgaat agatatattt ttgtgagata 5820
aaatgtaaat gttgaaagtt agtttatata ttatagttaa aataaatttt gtttgtgtgt 5880
attaatatat tttatataaa tttagtgttt gtatttagtt gggtgttatt taaggtgaat 5940
ttgatgggat agagaggggg aaataaatta ttgtttttaa ataggatttt aggaaattaa 6000
taagaaggaa tataagaaaa ggggaatggt gggaattaat attgattgag tgtttattgt 6060
gtttggtata gtgaggtgtt gtatttttat tattttattt ggtggttttg tttttttttt 6120
ttttttttta tatattttgt ttatttttgt ttttttagga taatgagtgt aatttttttt 6180
ttttttgatt tttttttttt gttttttagg attaaagata gggtaaatat atatatatat 6240
atatatatat atatatatat atatatatgg taaatatatg atatatatat atggatatat 6300
atatattaat ttttagatat ttttggtatt gttttttata gtgaagatga aaatggagtg 6360
agtgtatggg agataagggg gtgggaggag atatattatt aatggaatat aagtaatatg 6420
taaattggaa tttattgtta gtagtttaga tagttttgtt ataataattt tttagagaaa 6480

ttattgtttg tggtattttt ttgtgttaat attatttttt tttttttttg ttttaatatt 6540
tttttttttt ttgttttttt atatatggtg ggtttaaatt taaagtattt gatttaatgt 6600
aaaaggaggt ttgttttttt tattaaattt tttgtgttaa tgtgaatttt gtagaaaaag 6660
gttagtttta aaatttgtga attttttagt tgtgtatttt ttttatttta gttttttaaa 6720
taagtatatt tttttttttt gttttgtttt tttagtgtat ttttaaaaat atatatttaa 6780
gttggtaatt ttttttagtt ttttgagaat tgtttgggtt gtttttttat atttttaaat 6840
gtataaaata ttttaatttt aaaatttgtt tttgtttttt ggttttttaa ttttagtttt 6900
tgttaaatag tagttaagaa ataagttttt tttttttttt ttttttttgt ttgttttttg 6960
atttttttgt ttgtttattt ttttattgtt aataagattt ttttttttat gtaagagttt 7020
attgttgtag ttttgtggtg agttaaattt tgtggtttta gtattttttt gtttagtttt 7080
tttttagatt tttttaaatt tgtttttata aaatttaatt tttaggtttt gagtaggaaa 7140
atgggtagga gttatggagt ttgtgtgttt tgtgagattt ttggttttgt gtggagtttg 7200
gttttttgag tttaagatgt gataggggat gagggatggt tagtgaggtg ggaagagggt 7260
tggtttttga ggttttaaag gggtaatgga gaagtagtgg ggtgtggagg gtgtgtaggt 7320
tgagtgttgt gggataggtg tgatattggt gttggtgttg gtgttataga tttagggttg 7380
tggtgtgttt tttggttttt agtttgagat tggtgatgtt ggagtttttg ttggtggttt 7440
tggtggttgt tgtggttgtt attattgagg tggtggaagt tgaatttgtg gttagtgtgg 7500
tgagtggtag tttgaagggt ggtgttggga atattatgta gggtgtgtgt gtggttaggt 7560
gtggatgtag gtggtggtgt gtgtgtgtta tagtgttgtt tagttgtagt tgtgtttgaa 7620
tttgaaagga taagggtgtt atgttgtaat gattatttta gggtgataat agaatagaaa 7680
tagagtatta atggtgttta gtttggttag agagagagtt gggttgtgtt tgggtatggg 7740
gagagggtat ttggttgtgt attttgtttt gatagtgatt tttttagttt gttgttgaat 7800
ttggggtttt atatggatat tttagtgttt tttgggtttt tgaaatttgt ttagggtagg 7860
tttatttttt tggattttgg gaattttttt ttgtaggaat gtggttgttt tttttgggaa 7920
ttgggttgaa atggtatatt ggaaagattg tgggtgagga tttttatttt ttttttttga 7980
tgttgttatg tattttttat tttattatta aaataatgga gtattaataa gtaaattatg 8040
taatttttta atgttattta gggaagttat tttattttta ttataatagg tataattgtg 8100
agttggtatt tttaattttt attgttataa taattattta gggattttgt taaaatttag 8160
attttgattt gtaaggttta ggttggggtt tgagattttg aatttttaat aatttttag 8220
atggtgttgt tgttgttggt ttaaagatta ttttttgagt agtaaaggtt tttttttaaa 8280
gttatttttt tttttttttt tagttatttt tttttgtttt taaagttaag tattaggatg 8340
ttagagtttt gtggtattaa gagagttaag atttttttaa tattgatttt taatttatgg 8400
ttatatttat ttgttttttt ggtttaagtt attggagatt gagagttaat ttatgatatt 8460
ataggtatgt taagtagggg ttttggttga ggatagggaa ggttagatat tggttgggaa 8520
aggattttgt ttttaggttt ggaaagtggg gagtttagag tattgggatt ttttaggatt 8580
aaattattgt tgtaggttgt tttttaaaa tgtattttta aaggtttttt tatggttaag 8640
ggattgtaaa ggtagggtag ttttgggaag attaagattt ttttttttat tagagatgaa 8700
gttttgtttg tagaaataga tttaaaaata aaagaatgaa aaaataaaag tatgagttgg 8760
gagtatttgt gtttattttt tttttgttag agttatttaa tgtgattaag aggtagaaaa 8820
tatttagaaa agtatttaga gggttgtttt agaaatattt taggattaat ttttgtattg 8880
gatttgataa aattttaatt aaaaggtttt tttttttttt ttttttaagg gtaattttta 8940
gtgtattttt aaaggattag ttattgtttt taggtttttt tagggatttt tatgaggata 9000
gaagggatta ggtatttagg tttttaatat tgtataggtt tttaaagggt agtgggttta 9060
ttatattatt ttaaaatgat tttttaatta tgtagatatt gatatttggg tttagagata 9120
ggtgatgttt aatagtttaa gtaaatattt aggtattttt atatttaaaa atagtttgaa 9180
attaggttgt atttgtttgt tgaaatggta tttttaaagt atttatgttg atataaggtg 9240
tgattttata agttttaaat tggttggtgg tttttatgag aatatttgta aaaagtataa 9300
gagaaaaata atgtgaggtt aatgtttgta gtatttttta gggttttaaa gggtataaga 9360
tagaatgaat ttttttgaaa atggattatt atttttttaa attgtattta aaatttaaat 9420
aaatgtaatt tatttttaaa ttttaggatt ttattaatag tttttgaaga ttattaagtt 9480
aagaattatt gttatttttg aggttttttt ttttttgtta ttgtaatagt aatatttatt 9540
attttttttt ttgtttagat tattaaatgt tttttgtata agaagtatat ttttatggag 9600
ttgatttttt tgttttttat atttagtttt ttgattttga aattaaattt ataggttgga 9660
gggggaaaaa aaataaaatt tagatgttat gattaaaaat ttttttaaat tataaaagta 9720
taaagagaaa agagttgtg 9739

<210> 23
<211> 9739
<212> DNA
<213> Artificial Sequence

<220>
<223> chemically treated genomic DNA (Homo sapiens)

<400> 23

tatgattttt ttttttttgt atttttgtaa tttaaaaaaa tttttagtta taatatttag 60
gttttatttt tttttttttt ttaatttata ggtttggttt taaaattgaa gagttaaatg 120
tagaaaataa gaaaattaat tttataaagg tatgtttttt atataaggaa tatttgatag 180
tttgagtaaa gagagaagta gtgaatatta ttattataat gatagaaaaa aaggaatttt 240
aaaaatgata atagtttttg atttaataat ttttaaaggt tgttaatgga gttttaaagt 300
ttgggagtaa gttatattta tttaaatttt ggatgtagtt tagaaagata atagtttatt 360
tttaaaggaa tttgttttgt tttgtatttt ttggaatttt gaaaaatgtt ataaatgtta 420
attttatatt attttttttt tgtatttttt ataggtgttt ttataggggt tgttagttag 480
tttgaagttt gtagagttgt attttatgtt aatgtaggtg ttttaaggat gttattttag 540
taggtaagta tagtttagtt ttaagttgtt tttaaatatg aaaatatttg aatgtttgtt 600
tgaattgttg aatattattt gtttttgagt ttagatgtta atgtttgtat agttggagaa 660
ttattttgag atgatatagt ggatttatta ttttttaaaa atttatatgg tattaaaggt 720
ttgaatgttt agtttttttt gtttttatgg gaatttttga aggggtttgg gggtagtaat 780
tggtttttta aaaatatatt aaagattgtt tttagagaga gaggaaggaa aagttttttg 840
attaagattt tgttaaattt aatatagagg ttagttttaa ggtattttta aggtaatttt 900
ttagatattt ttttaggtat tttttatttt ttagttatat taaataattt tgatagaaga 960
aaaataaata taagtatttt tagtttatat ttttgttttt ttgttttttt gtttttaaat 1020
ttgtttttgt aaataaagtt ttatttttgg taaagagaga agttttggtt tttttagggt 1080
tgttttgttt ttgtaatttt ttagttataa agaaattttt aagagtatat tttaaaagga 1140
tagtttgtag taatgattta attttgggaa attttagtgt tttgaatttt ttatttttta 1200
agtttgaggg tgaaattttt ttttaattag tgtttgattt tttttatttt tagttaggat 1260
ttttgtttgg tatgtttgtg atgttatgaa ttagttttta gtttttagtg atttggattg 1320
gggagataga taggtgtgat tatgaattga gagttggtgt tagggagatt ttagtttttt 1380
tggtgttata ggattttggt attttgatgt ttggttttgg aaataagaga gaatgattga 1440
agaaagaggg aggggtgatt ttaaaaggag atttttatta tttaaaaggt aatttttggg 1500
ttagtagtaa taatattgtt tgggagattg ttagaaattt agaattttag gttttaattt 1560
agattttgtg aattagaatt tgaattttaa tgagattttt gggtaattgt tatggtaata 1620
aaggttagaa gtattgattt ataattgtgt ttattgtggt gaagatggag taattttttt 1680
gaatgatatt aaaaagttgt gtagtttgtt tgttgatatt ttgttgtttt ggtagtgggg 1740
taagagatgt atggtagtat taagagaaga gggtaagggt ttttatttgt agttttttta 1800
atatattatt ttagtttagt ttttagggga gatggttata tttttgtaga agggggtttt 1860
tggggtttgg ggaaataagt ttattttaag tgagttttgg agatttaggg gatattggag 1920
tatttatgtg gaattttaga tttagtaata ggttgagaag gttattgttg gaataagatg 1980
tatagttaaa tgtttttttt ttgtgtttaa atataattta attttttttt tggttaagtt 2040
ggatgttgtt aatgttttgt ttttattttg ttgttatttt aggatagtta ttgtaatgtg 2100
atgtttttgt ttttttaggt ttaggtgtag ttgtagttgg atagtgttgt ggtgtatgtg 2160
tattattatt tgtatttgta tttggttgtg tatgtgtttt atatgatgtt tttagtattg 2220
ttttttggat tgttgtttgt tatgttggtt gtggatttgg tttttgttgt tttggtagtg 2280
gtggttgtag tagttgttaa gattattagt aagaatttta gtattgttga ttttagattg 2340
aaagttaaaa agtatgttgt agttttgggt ttgtgatgtt aatgttagta ttaatgttgt 2400
gtttgttttg tggtatttag tttgtatgtt ttttgtgttt tgttgttttt ttgttatttt 2460
tttgagattt tgggagttgg tttttttttt gttttattga ttattttttg ttttttattg 2520
tattttggat ttggaaagtt agattttatg taggattagg gattttatga ggtatgtagg 2580
ttttgtggtt tttgtttgtt tttttatttg agggtttaga attgggtttt gtaggagtgg 2640
gtttggggga gtttggagag agattggata ggggagtgtt ggaattgtgg agtttggttt 2700
attgtaaagt tgtaatgatg gatttttgta tagaaaaaaa aattttgtta ataatgaaaa 2760
aatgagtaaa taaaaaaatt gaaagataaa tgggagagaa aaagaggaag ggaatttatt 2820
ttttaattgt tatttggtag aagttgaaat tggagaatta aggagtaaaa ataaatttta 2880
aaattaaagt attttatata tttaaaaata tggaaaaata atttagatga tttttgagag 2940
attgggggga gttattaatt taaatgtgtg tttttaaaaa tgtgttaaga aggtaaagta 3000
gaaagaagag gtatatttat ttaaaaaatt aagatgaaaa aagtgtgtag ttgggaagtt 3060
tataggtttt gaaattgatt tttttttgtg aagtttatgt taatatgaga aatttgatga 3120
gagaggtggg ttttttttta tgttgaatta gatgttttga gtttaaattt attatgtatg 3180
gaagagtaag aaaagagaaa atattaaaat gaggagagag aaaaataata ttaatataaa 3240
aaaatgttat agataatgat ttttttgaga aattattatg gtaaaattgt ttggattgtt 3300
gatagtaaat tttggtttgt atgttatttg tattttattg atggtgtgtt tttttttatt 3360
tttttatttt ttatgtattt attttatttt tatttttatt atgaaaaata atattaaaag 3420
tatttggaaa ttgatatata tatatttata tatatatatt atatatttgt tatatatata 3480
tatatatata tatatatata tatatatata tatttgtttt gtttttgatt ttggggaata 3540
aaagaaaaaa gttagaaagg gaaaaaatta tatttattgt tttaagaaga tagaggtggg 3600

tagaatatgt ggggaaagga aaaagaaaat aagattatta aatgaaataa tgaaggtata 3660
gtgttttgtt gtgttagata tagtaggtgt ttaattagta ttagttttta ttattttttt 3720
tttttgtgt tttttttgt tggtttttg aagtttatt tgaagatagt ggtttatttt 3780
ttttttttta ttttgttaaa tttattttaa ataatattta gttagatata ggtattaggt 3840
ttgtgtaaga tatgttgata tatatgaata aagtttattt tgattataat gtgtggattg 3900
atttttaata tttgtatttt attttataaa ggtgtattta tttaagtaat tttttttttt 3960
tgtttgtttg tttttttttt gttttttttt tttttttggt tgtttgtttt aatttatgta 4020
gttatttaaa ttgggatatt ttggattaag ttagtttgta ttttaatttg ttagtaagtt 4080
taagtttgtg gggttttgtt tttgtttttg ttttattttt taatttataa gaaagaggaa 4140
aagttttttt aattgaattt tggtatgtgg ttgagttttg taattatttg ttttttatga 4200
aaataaaatt atttatattt gatatatttt tttttagtgt attaagttat tttaaataaa 4260
agatgttatt ttatgatgtg ttgttagtat aaaatgtgtt gtttttaatt ttgttaaatt 4320
ttttaaataa gtgttaagtt attaattgaa gatattttgt gattaattga atgaaaatat 4380
tgttttattt gatggggttt gagttatttt tgtttttgtt attattaaaa tatttgggat 4440
agtttttttt ttttgattaa tgtgtattat ttttttgaat atattaattt ggaaaagtgt 4500
ttgtttttgt ttttatgtgt tgtagttaag gggtagtaag aagggtggtt ttgtggttgt 4560
tgttagtgtt gagtgtattt ttgttggttt tttttatttt atttatttag tatagaaata 4620
gggttatatt aaattaatga aagaatagat aagaagtaaa ataatatagg atagagtaat 4680
tttttttttt aagatggatt tttgttattt gttttgttat aattttagtt tttatttggg 4740
gatatttttg ggaggaagga aggagggatt ttgtggagtg aaattaattt gggtttggta 4800
tggtataatt gataattagt tttagtttgt tgattttttt tggagtgggg taggtgttga 4860
ttttggagtg ggaaaagagt tgtaaaggtg ggtaggttag ggtattgtgg agggaggatt 4920
ttttatttga tatttttgtt tagggttggt tgaagtagtt tttttattgg ttaaatttag 4980
aaggtggaag ttagaattgg gtttaggaaa agtttggttt tgaaaataat gaggagtttt 5040
ttttttttat gttgtggttt ttttttggaa gaaattttat tagtaaagtt taaaggtgtt 5100
gtttgtgtgg ggatagttgg agattttgaa gatttggttt tgattatgtt taaggtagga 5160
attttttttt ttgtgtgggt ttttgtgatg atgggatttt ttgtaaagat tgaaagagtt 5220
ttgagtgttt tttgggattt ttttattttg tgttggaggg tgtgtaaaat ttttgttaaa 5280
gttttatatt ttttagtagt gttttttatt atttttattt ggtttttttt taggttttag 5340
tttgtattta aagttgtttt ttagtataga tgtgggtgat aggttttttt tttttaattt 5400
ttaaagatta ttgaatagga tttgggttgt atttagtgtg taggttttgt gggttttttg 5460
gttgatgaat gttggtagtt tttaataata aatatttgtt tgtgggtatg gtatgttttg 5520
aagtagttag gttatttgtt gtggttttgt ggtttttagt gagttttagt tggtgttggg 5580
gttgggggtt ttaataggta ggttttaagt tttaaatttt aattttaatt tagattttaa 5640
tatgggtttg gattttggag attttggagt aggggagatg gtggtttttt tttggggtta 5700
gtttgtttta agtagtggag tgtgggggaa gtttgaggtt aaaggaggtg gtgtgtttag 5760
gttttgttgg tggaggtgtt gggtattggt atagaggttt agtgatgtgg tgtgggtggg 5820
ttgggaatgt tttgtgtgat aggagttagg tgggtttggt gtggagattt gtgggagttg 5880
ggttgtggtg ggagtgtggt aggtggagag gtttgtggag gtagttaggt ttggtgagaa 5940
aggttaaaat tttttggttt tatttgtagt gttttatttg ggtatggttt gtgggattag 6000
tgtatttggg gagtttgtgg tgtttttgtt tttttttgtg tttgtttttt taagattaat 6060
ggaggataga ggttgttttt tttggttttg tggagttttg ttttttgggt ttagattttt 6120
gtaaggtttg tttttttgga atgttagtgt ttaaattttg gggtaggtgt tttggtgtgt 6180
gaataagagt tttaaatttg tagttttttg aattatttta agaaggggtt tgaggaattt 6240
attttaaatt taaataaggt ttaatatgtt tgtagtttgg gttgaaattt aataatttta 6300
atataattaa taaaggatat ttttattttt tgttttttaa atttttgggg tatgtaaaat 6360
ttttttgagt tgtaaaatta atgattggga aaaattgagt taaaagagat tgtttaagat 6420
attattagtg ttaaggattt tatgatataa gagatatagt aagatagtaa gattggtgtt 6480
tttgtttgga gttgaattga tgtatattta ttttgagttt ttttttagat ttataatttt 6540
tttgggtttt tagatggtaa tattttagta taatgtaatg tttaaatttt ggaattattt 6600
tgatttttga aaaatgtttt tttggtttta gtgatatagg atttgttttt ttttttataa 6660
attagtttat attataaatt gtaaatattt gtaataagat tttaagattg tttattttat 6720
ataattttaa gtatttgatt atgttaaata ttagtataga aaatttgtga aattagttta 6780
atgtgtgtga gggaattaaa ggattattta tttttattat ataaataaag tatttaagat 6840
tgttttgatt gttagttttt taatttttag ttatataatt gattattaat ttaagagtta 6900
ttttgtgtaa aaataattat tgaggttata ttttgtttta attaaatatt tttgggtaga 6960
agaaaatggt attgtatatt gggaagtggg ggattgtttt taatattata ttattttagt 7020
ttaaatggaa ataaaagttt gtaaatttta atggggaaaa aaggtttgaa atggtttttt 7080
aatatgttat taaaatagtt agttaaaata tattaaagta atattataaa tttagaaata 7140
ataaatatgt tattttaatg agaatgttaa tttatagata atttatttaa ggtagttttt 7200
atattttata tggaatgttt tattatgatt tgtttgatta ttgtgatgtt tgttttttaa 7260
aatttaattt atataggttt gtaatatata taatttataa tttttgttga tatatgtata 7320
tatattttaa attttaaatt tttatagtat gtttggatgt ttattagtat gtttttaaga 7380

tgatgaaaat aaaaatataa aaagtgttaa atattatatt gaaagtaagt gtttgttttg 7440
ttttatgttt gaaggtaaag tagttgaaaa tgaaaaaatt aaaaaggatt agataatttt 7500
taagtttagg tattgagtga gtattgaaat attttagata aaaaaaaatt ttgtataaga 7560
aatttgaaaa ttttaagaaa gatttataga tgtgagtgta taatattttt gatgtttaga 7620
taattttttt tttggtgttt aatttatgaa ataaaaaatt tttgagtttt ggggtatttt 7680
gttttgggta ttttatatgt tagttaaggt aattattata tatgtttttg gtgtaggaaa 7740
gtttagtata atatgtggag ttttattggt atgaggaaag gttttagaat ataattaaat 7800
atattaaata gggaggagta aatatgtttt tttttttgtt gaataatttt ttatatgttt 7860
tgagtaagga tttttgtaga tttttattta agtagaatag tgtatgttta agagtatata 7920
ttgtggagtt atgttttatt gttggattta aattttattg ttattgttta ttagtttttt 7980
gattttggat aagttaattt ttttgttttt tagttttttt atttgtaaaa tgtggataaa 8040
aatagtgttt attttatgga atggttagga ggattaaatg agttaatatt tgtaaaattt 8100
ttagaataat gttttgtata tgtatattaa gtattaaata ataattattt ttattaaaag 8160
gttaatagat attgattttt gtaataaaaa ttttaaaaga aaaaaataat aaagtgttgt 8220
aagataagaa aagttttttt ttgtgggtaa gaagaaaata ttgtttgtta gtttgtgatt 8280
ttggttaatt gtttagtttg tgtgagtttt atttagaaga tgtgataatt gttataatgt 8340
agtagtgtag ttaggaggtt tatttagaag agtaatttat aggttgtgtg tttttatatg 8400
taagatagat aatattgtta ttttgttatg tatagttaat aaggtgagat tttaggtatg 8460
gtatttgttt ttgaggagtt tattatttgg gtaaaatagt ttataaatat atatgatagt 8520
gttaggaagt atgtgttaaa tgtatttgtt taaaggtatt aggatatata atttgtgtat 8580
ttgaatggtt atataatata tgtgggtttg ttgttttggt tttggaaatt aaattttttt 8640
tggaaatgag tagttttata aaggaatggt gaagtgatat ttagaggtta agaagtgtat 8700
tttgttagga ttaaatgtat attatatgtt gttgtggttt gtgttttata gtattatttg 8760
tatttttttt ggttgtttat gtttattgag tattagtagt aaaatgttaa aagatataaa 8820
ggttgttgat taaggatttt taatagtagt ttgaaaatat atataaaata aagaaaggaa 8880
tgtttaatta ttattatagg gatttgtaag atgaataatt gagttataat tatagagtaa 8940
attaagaagg attttttttt aatgtttaaa tttaggtata ttattaatat tgttttatat 9000
tttatataat atttatgaag agttaagtat tttttatata tttttatttg ggatgtgttg 9060
ttatagtata tggttaattt ttagaggtag atattttgga ttttaaattt ttgttatatt 9120
aattattaga tgtaaagttt taggttattt tttagttttt taaaatttat gattttttag 9180
tgttttgtag tatatatttt gttaattatt gtgaggatta aataaaaata agttatgtaa 9240
ggtgtttggt atataatagg ttttaataaa tgtatatttt ttttttattt ttattattta 9300
ttttttaaat tattgtaata tagagaggat ttttaattta tttttttgtg attagaagta 9360
gttttgaaaa attttgggag gtggtgtgta ttttaatttt aattttttat tgtaagtatt 9420
gataatgtgt gtttatattg tgtgtattgg agtttggaat aaagtttttt ttaaattttg 9480
taaaaatttt tagagatttt gagggattgt gattttagat ttgttttttt gtgtagaata 9540
gaaaggattt tatttttttt tttgataatt tgaggatgtt tttaatgtgt aggttattta 9600
gagtttttga tttttagttt tttttatttt ggaatgttag tatatttatg gagttttggt 9660
attaattttt tagttatttt gatattagat tgattgtttt tttgttgtat atttattgaa 9720
tatattttta tgtttttgt 9739

<210> 24
<211> 4313
<212> DNA
<213> Artificial Sequence

<220>
<223> chemically treated genomic DNA (Homo sapiens)

<400> 24
   gggtttgatt ttttgagatt tggggaggat ttttggtaga tgtgtgttta gttagaatat 60
   ttggtaagga tttttttaat gaagaaaaag tggaggaatt tagttttagt gagaagaggt 120
   ttttttattt tgttttagat atattggata gagggtatat tttgattaga gttatgttta 180
   gtggttagga ggttagttta gtattttttt ttttattatt tttgttttgg gtgggggggt 240
   aatttttttg ggagtagttg tgggaattgt tgttttttat tttagtttag ttagtatttt 300
   gaagtttgta ggggaaggat agtatgtggg atggatattg gggaaggagt tttgtaaggt 360
   tagggtgtaa tttttaggtt ttaggtggtt tggtaggtta tgttgttttg gagatgtttg 420
   ttagattttt taagtttatt tagggtttgg tagtaatttg ttggttgttt ttgtgggggt 480
   ttgggttgtt gagtatggtg tagttgttta gggttaatta gttttagggt gtttgtgtta 540
   ggttgtggtt tttttgtttt ttttgtattg agggtattta tggtgtgtaa atgtttttgt 600
   atttttagag ttgttttatt ggatgttttt aggaatttat atatttttat aaaaatgtat 660
   tttaaatgat ggataggtga gtttggggta ataatgggtg tttggtgggt agataagagt 720
   aaatgggaag gagtttgagg gaggaggggg aagagaagag gaaatagaat ttttagttgg 780
   atattttgat aatagttgga aggaaagttt agaaaagatg aagagagagg aggggagaaa 840
   ttaattgggg tttttatttt tgttgttgga tttttaattt ttgttttaaa tgggttttgt 900
   tttttggtaa aattagttta aaggatttta aaataaagaa aatgagatga ttggtttggg 960
   agttttttaa ttagagtaga gaagttagag gggggtgggt gatttggttt tgaagtttta 1020
   gttgaatagt tatttttttt ttttttggta aaaaggattt ttttagaatt tttgaggttt 1080
   ttggattttt ttttttgtaa atggagttgt atattgtatt tttttgtttt tttggattgt 1140
   taagtatgtt ttatgagggt tgttgttttt gggtggaatg tggttgtatg tatgtgtttt 1200
   tttgtatatg tatatatatg tatatttata ataagtgttt gtaggaggag tgttttgtgt 1260
   gttagttttg tgtttaagat aggaagttgt tgggttattg agttaaatgg gagtgatatt 1320
   attttttttt attagtaagg aaagtggatt ataaaagttt ttttgtattt tggtagttta 1380
   tttaatatta tttatgtatt ttgtgtaagg aattgtggga ttttgtttta tggtaaataa 1440
   tatggaaatt ttaaaaatag tgattttttt gtgtgtgttt atttatgtgt tttggggtga 1500
   tttggtgggg ttgttgttgg gtgatttata tttttgaatt gtgaagtgat agggaaagtg 1560
   tgggtgagtg taggagatgt ggttgggggt ttttttgggt ttttgggttt ttgtatttgg 1620
   agtgggggat gtggttgttt taaggggagg aggggtggtg ggttgttttt gttatttagt 1680
   ggtggttgga gtgttatgtg ggtgtgtggt gttgtggtta ttggtttgag gtatgtgttt 1740
   aggagattgg tttgtgatgt tatttgaggg ggttttgtta aaaataagaa taaaaattta 1800
   gagtgaaagt gttttaggtt gtgttgagtg gtttggaaat ttttgagttt gtgtggaggt 1860
   tgaggtggtg agggtggtgg atggttgggg agtgtgggtg gtttagtttg gtttggttgg 1920
   gttttggttt tgtgtttttt atttatgtga tttgggttgt ggagttttgt ggggtttggt 1980
   gggggtgtgg ttgtatgttg gtggggtgtt ttggtttgta gtggggtggt ggttgtgagg 2040
   agggggtttt tatgtgtgtg tgggtggtgg tgggtgtgtt gattgtgggt gtttggtatt 2100
   tttgagggtt ggttagggtg tgtgggtggg gatggttggg tggtggtggt ggttggagtt 2160
   ggtttgggtg ggtgtgagtg ttggggaatg tgttgtttgt atgtgtgtag tttttgtttt 2220
   gggtggttta ggtggtggtg ttggagtttg aggtggttgg atgtggagag gagtggggag 2280
   tttgggaggt ggtttgtgtt tttgttggat tattgtgatt gtttagattt tggttgtgtg 2340
   gtgaagttga ggatttggtt ttgttgaatt ttttattgtt tgggtgagtg gggtggtttg 2400
   tggtgttttt aatttagttt gtggatttaa aggtggtttt gtgttgagtg tggttggtga 2460
   tttgtaggat tttagttttg gttgtggttg ttgtgtatgt ttttggaaga tttggtgggg 2520
   tgggggtgtg ggggtttttg tgtgtgttgt gggagggttg aaggttgatt tggaagggtg 2580
   tttttggaga attagtgtgg gatttattgt gaatagtatg gaggagaatg attttaagtt 2640
   tggtgaagta gtggtggtgg tggagggata gtggtagttg gaatttagtt ttggtggtgg 2700
   tttgggtggt ggtggtggta gtagtttggg tgaagtggat attgggtgtt ggtgggtttt 2760
   gatgttgttt gtggttttgt aggtgtttgg taattattag tatttgtatt gtattattaa 2820
   tttttttatt gataatattt tgtggtttga gtttggttgg tgaaaggatg tggggatttg 2880
   ttgtgtgggt gtgggaggag gaaggggtgg tggagttggt ggtgaaggtg gtgtgagtgg 2940
   tgtggaggga ggtggtggtg tgggtggttt ggagtagttt ttgggtttgg gtttttgaga 3000
   gttttggtag aatttgttat gtgtgtttgg tgtgggtggg ttgtttttag ttgttggtag 3060
   tgattttttg ggtgatgggg aaggtggttt taagatgttt ttgttgtatg gtggtgttaa 3120
   gaaaggtggt gattttggtg gttttttgga tgggttgttt aaggtttgtg gtttgggtgg 3180
   tggtgatttg ttggtgagtt tggatttgga tagtttgtaa gttggtgtta atttgggtgt 3240
   gtagtttatg ttttggttgg tgtgggttta ttgtatgtgt tatttggatt ggtttttttt 3300
   aggtgagttt gtggggatta tgtgttttgg tttgttgtgg ggaggtttgt ggagttgggg 3360
   ggtggtgttg gtgtgggaat ttattgggag gaaaatattt tgaatttttt ttgtgtatat 3420
   gtataaagat ttatgtgata ttgtgtgaag ttgatgttgg tttgggtagt ggttaggagt 3480
   ttagtggtag gattgatttg ttagggggta tagatttttt aggattgtag aaggattttt 3540
   tttttttttt ttgttttttt tttttttttt tttttttttg tttttttttt ttgtttttta 3600
   ttttgttttg gtgtattttt ttttagtttt tagtttatgt tttttttatt gtagtttttt 3660
   ttggtgggaa tgtggtggtt ggaagatggg tttggaagtg tatattttta tttttttttt 3720
   tatgattttt taatttaggt taggttgggg atgtatgttt tagtttattt tagatttgtt 3780
   ttattatttg gttattttgg ttgtgtttgg ggaagaaaag gtgaggtttt ttgttgtttt 3840
   gttttttgtt ttttgggttt gtgttgattg gtgggattta ggaggatgta tatagggaag 3900
   gaggaaaata aaggtgtttt ttttttttgg ttttattttg tttgttagtg ttagtttgta 3960
   gtggtggggt ttagtttttt ttttgtatat agtgaggata agggaggtag ttgttttttt 4020
   tggtatttgt tatttttaaa tagaaaggat ttttttttag ggttttttgg gggttgttga 4080
   tgggaaagag gtagtatttg taggggtttt gtagagatgt tggatatatt tttttataga 4140
   tttgtgattt taaaaaatta agtttatgtt tttgtagaaa ttattaattg tattttatgt 4200
   gggtttgtgg ttgggaattg ttattagaag tggattgttt gattttgagt tggtagtgga 4260
   tttttgttgt ttttaaattt ttaattattt tgtgggggtt atttgtttag att 4313

<210> 25
<211> 4313

<212> DNA
<213> Artificial Sequence

<220>
<223> chemically treated genomic DNA (Homo sapiens)

<400> 25

gatttgggta aatgattttt gtaaaatagt tgagggtttg ggggtagtgg ggatttgttg 60
ttagtttggg gttaaatagt ttatttttaa tggtggtttt tagttgtaga tttgtatgga 120
atgtagttga tgatttttat aaggatatgg atttagtttt ttaaaattgt agatttatga 180
aaaaatatat ttagtatttt tgtagggttt ttgtgaatgt tgtttttttt ttattagtag 240
tttttaggaa attttgagag aaggtttttt ttatttgggg atggtaggtg ttgggaggga 300
tggttgtttt ttttgttttt gttgtgtgta ggaagggggt tgagttttat tattgtgggt 360
tggtgttggt aaataaagtg gagttaaggg gaaagggtgt ttttattttt tttttttttt 420
gtgtgtattt ttttgagttt tattggttag tgtaggtttg aggggtagaa ggtagagtgg 480
taaagggttt tgtttttttt tttttgagta tagttgggat aattggatgg taggataagt 540
ttggggtggg ttggggtatg tgtttttggt ttggtttgag ttggaagatt gtagggaagg 600
ggatgagagt gtgtattttt gggtttattt tttagttatt atgtttttat taaaaaaaat 660
tatagtgggg gagatatggg ttaggggttg ggaagagatg tgttaaggtg gggtggaaga 720
tagagagggg agatagggag agaggaagga gagagagaga tagagaaaga aagaaggttt 780
tttttgtggt tttaagaagt ttgtattttt tagtgaatta gttttgttgt tggatttttg 840
gttgttgttt gggttggtgt tagttttata taatgttgtg tgagtttttg tgtgtgtgtg 900
tgggggaggt ttgagatgtt ttttttttgg taagtttttg tgttagtatt gttttttagt 960
tttgtgggtt tttttgtggt gagttgggat gtgtggtttt tgtgggttta tttgaagaag 1020
gttggtttga gtagtgtgta tagtagattt atgttggtta gagtatgggt tgtgtgttta 1080
ggttggtgtt ggtttgtgag ttgtttgagt ttgagtttat tgataggttg ttgttgttta 1140
agttgtgggt tttgagtgat ttgtttaggg ggttgttggg gttgttgttt tttttggtgt 1200
tattgtgtag tgagagtgtt ttggagttgt tttttttgtt atttggagag ttgttgttgg 1260
tggttgggag tggtttgttt gtgttgggtg tatatggtgg gttttgttgg ggtttttggg 1320
agtttgagtt taagagttgt tttgagttgt ttgtgttgtt gttttttttt gtattgtttg 1380
tgttgttttt gttgttggtt ttgttgtttt tttttttttt tgtgtttgta tagtaggttt 1440
ttgtgttttt ttgttggttg aatttgggtt gtaggatgtt gttgatgaag aagttggtga 1500
tgtggtgtgg gtgttggtgg ttgttgggtg tttgtaggat tgtgggtagt attagagttt 1560
gttggtgttt ggtgtttgtt ttgtttgggt tgttattgtt gttgttgttt gagttgttgt 1620
tggggttgga ttttggttgt tgttgttttt ttattgttgt tgttgttttg ttaggtttgg 1680
ggttattttt ttttatgttg tttatagtaa attttatatt ggttttttgg ggatgttttt 1740
ttaaattagt ttttggtttt tttgtggtgt atatggagat ttttgtgttt ttattttgtt 1800
gagttttttg agggtgtgtg tggtggttgt ggttagggtt gaggttttat aagttgttgg 1860
ttgtgtttgg tgtggagtta tttttgaatt tatgaattgg gttagaaata ttatgagttg 1920
ttttgtttgt ttagatgatg agagatttaa tagagttaag tttttgattt tgttgtgtag 1980
ttgggggttta gatagttgta gtggtttggt ggggatgtgg gttgtttttt gggtttttttg 2040
tttttttttg tgtttggttg ttttgggttt tggtgttgtt gtttgggttg tttggggtga 2100
gagttgtgtg tatgtaggta gtgtgttttt tggtgtttat gtttgtttgg gttggttttg 2160
gttgttgttg ttgtttggtt gtttttgttt gtatgtttta gttggttttt gagggtgttg 2220
ggtgtttgtg gttagtgtgt ttgttattgt ttgtatgtat atggaggttt tttttttgtg 2280
gttgttgttt tgttgtgggt tggggtgttt tattggtgtg tggttgtgtt tttgttgagt 2340
tttgtagagt tttgtggttt gagttgtatg ggtgagagat gtgaggttag ggtttggttg 2400
ggttgggttg ggttgtttgt gttttttggt tgtttgttgt ttttgttgtt ttggtttttg 2460
tgtgggtttg gaaattttta ggttatttgg tgtaatttga gatattttta ttttggattt 2520
ttgtttttat ttttaataga gtttttttga gtgatgttgt aggttggttt tttggatatg 2580
tgttttgggt taatggttgt ggtgttgtgt gtttatgtga tgttttggtt gttgttgggt 2640
gataggagta gtttgttgtt tttttttttt ttaaagtggt tgtgtttttt gttttgggtg 2700
tgggagttta ggaatttgga gagatttttg attgtgtttt ttgtgtttgt ttgtgttttt 2760
tttgttgttt tgtggtttag gggtgtgagt tatttggtga tagttttgtt aggttatttt 2820
ggggtgtgta ggtggatatg tataggaaga ttgttatttt taagattttt atattgttta 2880
ttgtggggtg aaattttata attttttgta taaaatgtat aaataatatt aaatgagttg 2940
ttgagatata aagggatttt tgtggtttgt tttttttgtt gatggagagg aatagtgtta 3000
tttttatttg atttggtaat ttggtagttt tttgttttaa atgtagagtt ggtgtgtagg 3060
atattttttt tgtagatatt tattgtaagt gtgtgtgtgt gtgtgtgtgt agggaggtgt 3120
gtgtatatgg ttgtatttta tttggggata gtgattttta tgaaatatgt ttagtgattt 3180
gaaagagtgg gggaatgtag tatgtggttt tatttgtgaa gggagaaatt taggagtttt 3240
ggaggtttta aaggaatttt ttttgttaag gagaggaggg gtgattgttt agttaagatt 3300

ttaaaattaa gttgtttgtt tttttttaat ttttttattt tgattgagga gtttttagat 3360
tggttatttt gttttttttg ttttaaaatt ttttaaatta attttgttgg agagtagggt 3420
ttatttgaaa tgagaattag gaatttaatg gtaagggtgg gagttttagt tggttttttt 3480
tttttttttt ttttattttt tttagatttt ttttttagtt gttgttagaa tgtttaattg 3540
gaagttttgt tttttttttt tttttttttt tttttttggg ttttttttta tttgtttttg 3600
tttatttatt aaatatttgt tgttatttta ggtttgtttg tttattattt aaaatgtatt 3660
tttatggaaa tgtgtgaatt tttggaaata tttgataggg tagttttggg ggtgtgggag 3720
tatttgtatg ttatgagtat ttttagtgtg ggggaggtgg ggaggttgta gtttggtatg 3780
gatattttag ggttgattgg ttttgggtag ttgtattgtg tttagtaatt tagatttttg 3840
tagaggtagt tagtaggttg ttgttaggtt ttgagtgaat ttggggagtt tggtaagtat 3900
ttttgaggta gtgtggtttg ttaggttatt tggggtttga aggttgtatt ttggttttgt 3960
gaagtttttt ttttagtgtt tattttatgt gttgtttttt ttttgtaggt tttagagtgt 4020
tggttgggtt gggatgagaa atgatagttt ttatagttgt ttttaggaaa attatttttt 4080
tatttaggat gggaatggtg ggggaggagg tgttgggttg gttttttggt tattggatgt 4140
ggttttggtt agagtgtgtt ttttgtttgg tgtgtttagg gtagggtggg gaagtttttt 4200
tttgttgggg ttggattttt ttattttttt tttattggag gagtttttat taaatgtttt 4260
ggttaggtat atatttgtta ggggtttttt ttgagtttta aaaaattaaa ttt 4313

<210> 26
<211> 16197
<212> DNA
<213> Artificial Sequence

<220>
<223> chemically treated genomic DNA (Homo sapiens)

<400> 26

ttttgtaggt ggagggggaa agggtttggg ggttggtgga ggatgtagga gtatggggga 60
gttgtggaaa agatgtggag gtagagttag taggttttgt taagggatag gaggtggttt 120
tagagagaaa atgagggatt agggatgttt tttggggggt ggttagttgg gtgattggga 180
gaaattggga aggtgtagat tagggtagta ggggtagtgg gattgggggt gttttgaagt 240
gaatatgtga aattgaaggt gtttgttagt tttttagtgg aggtatttag gaggtagttg 300
gaattggaag gaaatggtga tagttgttag tattgtagag gtggtggtgg tggtggttat 360
agttggattt ttttgggttg gtgggaagtg tggaggtgaa ggagtaggga gtagggggag 420
gtggagaagg aaattttagg tttatatttt tattttattt ttggttgtta tatttttagg 480
aagttttttt tgaggttggg atagagggtg gggatagttt agtttttttg agagagttta 540
tttttggagg tttgtggtgg gagttagggt gtggtggaga gggtttttta tttttttttt 600
agtaagtggg agggaggtgt tgggttgagg ttgtgttgag tttgagtttg ttgtttgggt 660
ggatttgttt tgtttaagtg tggaggggtg gaggtttggt tgggttgtag tgtggtgtgg 720
agtaggatgt tgttttgtgt tatggttatt ggagatgtat gtttattttt ttttttgggt 780
ttgttgattt tgttattttt tgtttttggt tgtttgtggg tgtttgttat ttagattttt 840
tttgtgtttt atgggatgta agtttttttt tagagtttgg ttttgtaaaa gaggtttgga 900
gtttttgtta tagatttttt ttttgggtta tggaggatga gaagggttat tgagttgagt 960
tgtaattttg tgtaattttt gatttttttt tttttttgtt tttatggtat tttattgttt 1020
tttttttgtt tttgaggttt tttagaaaat agtgtagaat tgtgtagatg tttaggagat 1080
gtgaagatgt tggagatgtt taggaggtga tggttatgtg aggagaattg tttttaggtg 1140
tgtttttgga atgtatggag attttttggt ggggaagggg ttggggtttg tgttatttag 1200
tgtttgttta ttgatttttt tttgaagagg gggtttaggg tttttgttga gggagtgggg 1260
aggtggtgtg ggtttttttt gggtatatat gggtgtttgt tttttttttt tttttttttt 1320
tttttgtatg gagagtggag agtggagagt ggagagtgat agggaggtag ttgaagattt 1380
gaattttgaa aggggagttg gtggtgaatg gtgaatgaga tagttattta ggaagtgagt 1440
ttagttagtt tgggaggtgg tggagattta tgtttggaag taattggatt aggttttaga 1500
atgtgattgt tttttgggtt ttgggggaga tgttaaggat gtgtaagtgg agggtgtgga 1560
gataattggg agttagagtt tttattattt gggttgggaa tgtttttggg tgttttgatg 1620
gggtggtggt gggggttggg ggaggttttt gagaattgtg tggtttgggg agagtttatt 1680
tattgttgag ttttgatata ggttttgaag ttgttgtagt ggtttagttt tttttttgtt 1740
tggttttgtg tagtgtggtg ttgtagagtt taggttgtgt ttttgttttg ttgtttagag 1800
tttattgtgg tggtttattg gattatgttg gtggggtgat tgtagttttt gatttgtgag 1860
ttgtaaagag ttttgaggtt tatttataaa tttgtgtttt tagttgtttt tttatgtatt 1920
tgagttatgt tttgggattt ggagagtttg ggtgttgggg tgttgtggag gaggtttggt 1980
ttttgttgtt ttttttattt tttagtttgt gagggatttg ggggaagggg gagtaagttt 2040
ttgttttgga agaaatgttt ggaattaagg agtttgattt ttggatttgg gtgtttgttg 2100

gatttaggtt tttatttttg ggttttttgg ttgaattaag ggttttgata gggtttgagg 2160
tatttgtatt tttaggagat aggagtttgg ttagggtgta ttattgggtt ttgttttgag 2220
ttagaggatg tagtgtagat atttattttt atattgtttt tttatagagt tttttttttt 2280
ttggggtgtt gttgggtata gggtaggttg ttaggaattt ttagtaaaat tatgtttgtt 2340
tagaggtttg tggttttttt agagggtgtt ggggaaagag aggggatttg atttttttga 2400
tttttggagg aaaagtgttt ggggtttttt agggatatag ttttggaagt ttattttatt 2460
ttagtttagg ttgtggtgag gtggggagga gagttaggag agggggagag gggttttgtg 2520
ttttgtagag gtttttaatt ttggaggaaa aagattggga tatatttaag tgagttaagg 2580
tttgagtttt atgtattttt atttatttgg ggtggtgtat agttagtttt tgttgggtgt 2640
gagtatttga ttaagggagt aagtggaatg aaaatttagt tgggggggtt tttattgata 2700
taattgtttt gtagttgagt ttttggattt ttgggagatg tggagagttt ggggttggtt 2760
tttgttttgt agagtagatt ggattgtttt aggtgtttgg aatgtgtttg tattttgttt 2820
tttggatttg tgggagattt gtgttttgta agtttttttt tttattttag tttgttttta 2880
tttatatttt gttggtgagt gtgttattgt gaggtgtttt tttttttggg aagggagttt 2940
ttttttgtgt agatttgtat tgtttttttt tttgtttggt tttgtttttt aggggtagtt 3000
tttgtagaaa ggagattttt ttttgggttg aagggttatt agtttgtagt tagtttagtt 3060
ttggattttg ggagatgttt attattttgt ggattttgat ttgaaatttt ttttggttgt 3120
ttattgtgga gagtgttttt gtagagaggt ttttaattga aggagtttgg gttttatatt 3180
ttgtttttaa gtttgagttt ttagggttgt tttgtatgag gtgtagatga atttgtgttt 3240
gtaaataaga tagaaaattt taagtgttgt ttgatttttt ttttttgggg aatttgtatt 3300
ttgttttggg agtgtgttta gtgttttagt attaaatttt ttggttgggg ttggtagagt 3360
ttagagtttt gttttttttt aggtgtggtt ttttaatatt tgtaatttaa atgttgtgtt 3420
gtggttaaaa ttagttttgg tagtgtgaat agagaattaa aagtaggtag tgaatgagaa 3480
tagtttgtat tttttttttt tggtagatgg ggaggtgtaa atttgaggaa atttagggt 3540
atttgtttta aatgtgggaa atttttgtgt gtattttgtt tttttttttt agtttgtgtt 3600
aatgttttaa atggtgttga gttgtttaat tttgttgtta ttatagaggt tgttgtgttt 3660
taggggatta attgatgtga gatatataaa,atttgtaat tttataatat aaattatagt 3720
atagtttttt tggagagggt tggaatattt gagtgagttt ttgagaggaa aagaggagtt 3780
ttttagagga gaaatagagt atttttataa tgtgttttaa ttgagaaatt ttgttttatt 3840
gagttttttt ttaagtggaa ttagaagtgt tgggatgaga gggaaaggat gggagtgtgt 3900
ttaaaggtgg atagtaggtt tttatttttg gtgggagtga gattggatgg tattttttgg 3960
aaaggtggtt tgggttttgg ataaggttag aggtaggagt ttatgatgta gagatgatat 4020
agtgtttttt tgtgtgtgag tttatgaagg ttattattga ggttttgtgt ttgtaaaagg 4080
ttgttatgtt ttatataagt ttttatattt aatataggga ttgattgggt atagggattt 4140
ttttatatta tatatgtaag tatgtatgtt aattaaagat gtttgtgtta aagaaatggt 4200
taattttgtt gaatttagag gaattgatta attatttaat taagtagagg aaatgtttga 4260
atttaatttg taatttagtt gtttttttat ataaaattat atatttttat ttatatttga 4320
tgaatgaaaa aagaaattag tttatgattt taatttaaat atatgttttt aaaaatatat 4380
tttttttagt ttagttagta tataaattaa ttgagttttt ttggttaagt atgatttagt 4440
tgtgatattt aagagtggga gtggttgttt agatattttt ttttttatgt gaaatttaga 4500
ttaatgagtt attatttaat aagttgtagg tagtttggtt gggttggatt tagatggttt 4560
gagttaaatt tagattgtat ttgtttaaaa ttttgtaaat ttttagttat gtaaatttta 4620
tttttaaaaa tgttgggaag ttattgtata agagtttaag ttatattagt ttttttgtga 4680
ttatttgtag tttttgggga aagaatagaa gaaaagaaaa tgttagtttt tgtgaggtgg 4740
ggttggtgtt ttaggggttt tttttgaata ttttgttttt tttttaaagg ttaaaaggaa 4800
ggtagtggat atatattaga atttttttta tttgtgaatg gttgtaaggt tggagaaggt 4860
ggttagtgta ttttaaggtt tattattttt ttttgtgttt ttttttttgt tttggtaggt 4920
ttagttagtt taagttttgg gtgttatttt ttaaattttt ttgttaaatt aattttattt 4980
atttgattgg attatttgag aggtgttatt tttttttggg ttgttgtgat tttgaggggg 5040
tatttttata agagtttagg tattaggtgg tgaaatagtt tgtgttttta aatttgtttt 5100
ttttagggtt tttgggagat tttagagtgt aggtttgttt ggggagtttt aggggtgggt 5160
tttgagtgga agtgggttta ttttttatag gagtttaaat tttataggaa taagaatagt 5220
agtaaaatag gataagagag taggtaggga gttgttaagg aaaggtgatt tttgggaagg 5280
taggttattt agaataaggt ttttttggtg ttggagagtt ggaaggggga gtgggtatag 5340
aggatttggt ttaggtgtgg gggttattag tataagtaga gttatttttt agattttttt 5400
ttagaagtag ttgtttttta gagaaattag gtgagggata gtttttgtat ttttatatgt 5460
tagttttgga gatttgttta tttgttttta gttgtttttt tttttgggtg agtttgggtt 5520
aagtattaag ttaggataga agggtggttt ttagttggtt tttggtttat tttgtttttt 5580
taatatttag ggagtttggg tttagtatag gtgttttttt agtgattggg gtagaattag 5640
gatgtgtaat gtgattgttt tttttttttt gttttttggt agagtttttg tttgtgttaa 5700
tttatttatt aggttttgtt tgtgttatgt gtgtttttgg taggtttggg gtggggaaag 5760
gtgaagtgtt gggtatgtga gggtttgtgt attttagttt ttatgatgtt tttggttttt 5820
tttaggtttg ttgttgttgt atttaatttt ttttttttgg gggttatttt gaagaagttt 5880

tagattttag atttagtttt ttagattttg tttttgagtt tgtttggtgg gtttgtaggt 5940
tggttttttt ttgtttagag gagagtgtag atatgtaatg tttgtttgtt ggttttgttt 6000
gttttatttt tgttttttgt gtttttttgt tttggttttt gtttataggt tgggttggaa 6060
tgttagtttt aggagttgat ggtggttttt tgtttgtttt ggggaaggtg ttgttttttt 6120
attggtttta attttttgtt tggtgtttgg ggtttggttg tggggtttgg tttttagttg 6180
agggtgtagg gttggttagg tttgttttgg ttgaggtgga gattttgttt ttagggattg 6240
ttgggtgttt ttgttttgtg agtaatgaga ttgttgtgag tgaatggttt ttattgagtt 6300
tattttttta agtgttatta tgttaagtta gagaagtgag gtgagtggag gggatgtaga 6360
ggggttggaa aagttatttt tttttggttt tgtttttaga taaaaatggg agtttggttt 6420
ggtatttggg tgtttggtgt ttttggggtg ttttattgag gttttgtttg taaaattagt 6480
gtttgtgttt tgaagtgtat ggtgtttgga agttgttttt ttgtttgttt tttttgaggt 6540
ttttttttgt gtagtgagtt tgagaaatat ggaggattgt tttttgtaag tgggtggttg 6600
tgggtgtttt ttgatttttg ggtgaagtta gagggaaaat ggggtttttg ggttagtgtt 6660
ttatttttta ttttgggaga aattaatgtt tggagagggt ttgtttgatt tgtatagaaa 6720
ggttgatttt gagggtggtg gttgtttggg ggagaaagtg gaggttttgg gtttgtggga 6780
gtgtggtagt tggggttggt attgtagagg agagatatgt tattgttttg tatttttaga 6840
aagtgtgagg tgttgtttta gttggggtag gtggttgagg ttggttttta tgtgtgtttt 6900
ttgaagtttt ttgaaatatt ttgtggagtt ttgtgtgtat agaatttagt attgtttagt 6960
ttttgggtag tttaattttt tgtagtttta ttttagtttt tttggttttt tgaatggtga 7020
ttttttttat tgttttttta gagttgtttt gtgtttgggt ttattttggg gggtttggta 7080
tttttagtt atttttttt ttatttattt ttttttttt tattttttt tttattttt 7140
attttttttt tttttaggag agtgatttat gaattttttt ttttttagtt aattttaggg 7200
tttagtttgt agattttgtg agggtaggtt tttgtttatt ggtttgttag gtgttttgga 7260
ggtgatgttt tgttttttag agtttttgtt gtagttatga attggggttt gggttgtagg 7320
aaagtatagg gttgaagttt agtgttttgg ggttatttat attgaggtag ttagaggtaa 7380
agagttttaa gaatttagaa aatatttttt aggaagttgt ttaattggtt tttatgggat 7440
aggtggagtt attaatttgg gatggttttg taggaattaa agagtttagg gttttttttt 7500
tttttaatat tatgtttagg agatttagag ttgttggatt tttttttttt tgattggtga 7560
ttattagagt ttttagagtt gtagaaaatt ttttttttaa aaaattaagt aagtgttaat 7620
aagatttttt ataaattttt attagtttta tttttttggg gggtaggtag attgtggggt 7680
ttgatttttt gagatttggg gaggattttt ggtagatgtg tgtttagtta gaatatttgg 7740
taaggatttt tttaatgaag aaaaagtgga ggaatttagt tttagtgaga agaggttttt 7800
ttattttgtt ttagatatat tggatagagg gtatattttg attagagtta tgtttagtgg 7860
ttaggaggtt agtttagtat tttttttttt attatttttg ttttgggtgg gggggtaatt 7920
tttttgggag tagttgtggg aattgttgtt ttttatttta gtttagttag tattttgaag 7980
tttgtagggg aaggatagta tgtgggatgg atattgggga aggagttttg taaggttagg 8040
gtgtaatttt taggttttag gtggtttggt aggttatgtt gttttggaga tgtttgttag 8100
attttttaag tttatttagg gtttggtagt aatttgttgg ttgtttttgt gggggtttgg 8160
gttgttgagt atggtgtagt tgtttagggt taattagttt tagggtgttt gtgttaggtt 8220
gtggtttttt tgtttttttt gtattgaggg tatttatggt gtgtaaatgt ttttgtattt 8280
ttagagttgt tttattggat gtttttagga atttatatat ttttataaaa atgtatttta 8340
aatgatggat aggtgagttt ggggtaataa tgggtgtttg gtgggtagat aagagtaaat 8400
gggaaggagt ttgagggagg agggggaaga gaagaggaaa tagaattttt agttggatat 8460
tttgataata gttggaagga aagtttagaa aagatgaaga gagaggaggg gagaaattaa 8520
ttggggtttt tatttttgtt gttggatttt taatttttgt tttaaatggg ttttgttttt 8580
tggtaaaatt agtttaaagg attttaaaat aaagaaaatg agatgattgg tttgggagtt 8640
ttttaattag agtagagaag ttagaggggg gtgggtgatt tggttttgaa gttttagttg 8700
aatagttatt tttttttttt ttggtaaaaa ggattttttt agaatttttg aggtttttgg 8760
attttttttt ttgtaaatgg agttgtatat tgtatttttt tgtttttttg gattgttaag 8820
tatgttttat gagggttgtt gtttttgggt ggaatgtggt tgtatgtatg tgtttttttg 8880
tatatgtata tatatgtata tttataataa gtgtttgtag gaggagtgtt ttgtgtgtta 8940
gttttgtgtt taagatagga agttgttggg ttattgagtt aaatgggagt gatattattt 9000
ttttttatta gtaaggaaag tggattataa aagttttttt gtattttggt agtttattta 9060
atattattta tgtattttgt gtaaggaatt gtgggatttt gttttatggt aaataatatg 9120
gaaattttaa aaatagtgat ttttttgtgt gtgtttattt atgtgttttg gggtgatttg 9180
gtggggttgt tgttgggtga tttatatttt tgaattgtga agtgataggg aaagtgtggg 9240
tgagtgtagg agatgtggtt gggggttttt ttgggttttt gggtttttgt atttggagtg 9300
ggggatgtgg ttgttttaag gggaggaggg gtggtgggtt gtttttgtta tttagtggtg 9360
gttggagtgt tatgtgggtg tgtggtgttg tggttattgg tttgaggtat gtgtttagga 9420
gattggtttg tgatgttatt tgagggggtt ttgttaaaaa taagaataaa aatttagagt 9480
gaaagtgttt taggttgtgt tgagtggttt ggaaattttt gagtttgtgt ggaggttgag 9540
gtggtgaggg tggtggatgg ttggggagtg tgggtggttt agtttggttt ggttgggttt 9600
tggttttgtg ttttttattt atgtgatttg ggttgtggag ttttgtgggg tttggtgggg 9660

gtgtggttgt atgttggtgg ggtgttttgg tttgtagtgg ggtggtggtt gtgaggaggg 9720
ggtttttatg tgtgtgtggg tggtggtggg tgtgttgatt gtgggtgttt ggtatttttg 9780
agggttggtt agggtgtgtg ggtggggatg gttgggtggt ggtggtggtt ggagttggtt 9840
tgggtgggtg tgagtgttgg ggaatgtgtt gtttgtatgt gtgtagtttt tgttttgggt 9900
ggtttaggtg gtggtgttgg agtttgaggt ggttggatgt ggagaggagt ggggagtttg 9960
ggaggtggtt tgtgtttttg ttggattatt gtgattgttt agattttggt tgtgtggtga 10020
agttgaggat ttggttttgt tgaatttttt attgtttggg tgagtggggt ggtttgtggt 10080
gtttttaatt tagtttgtgg atttaaaggt ggttttgtgt tgagtgtggt tggtgatttg 10140
taggatttta gttttggttg tggttgttgt gtatgttttt ggaagatttg gtggggtggg 10200
ggtgtggggg tttttgtgtg tgttgtggga gggttgaagg ttgatttgga agggtgtttt 10260
tggagaatta gtgtgggatt tattgtgaat agtatggagg agaatgattt taagtttggt 10320
gaagtagtgg tggtggtgga gggatagtgg tagttggaat ttagttttgg tggtggtttg 10380
ggtggtggtg gtggtagtag tttgggtgaa gtggatattg ggtgttggtg ggttttgatg 10440
ttgtttgtgg ttttgtaggt gtttggtaat tattagtatt tgtattgtat tattaatttt 10500
tttattgata atattttgtg gtttgagttt ggttggtgaa aggatgtggg gatttgttgt 10560
gtgggtgtgg gaggaggaag gggtggtgga gttggtggtg aaggtggtgt gagtggtgtg 10620
gagggaggtg gtggtgtggg tggtttggag tagtttttgg gtttgggttt ttgagagttt 10680
tggtagaatt tgttatgtgt gtttggtgtg ggtgggttgt ttttagttgt tggtagtgat 10740
tttttgggtg atggggaagg tggttttaag atgtttttgt tgtatggtgg tgttaagaaa 10800
ggtggtgatt ttggtggttt tttggatggg ttgtttaagg tttgtggttt gggtggtggt 10860
gatttgttgg tgagtttgga tttggatagt ttgtaagttg gtgttaattt gggtgtgtag 10920
tttatgtttt ggttggtgtg ggtttattgt atgtgttatt tggattggtt ttttttaggt 10980
gagtttgtgg ggattatgtg ttttggtttg ttgtggggag gtttgtggag ttggggggtg 11040
gtgttggtgt gggaatttat tgggaggaaa atattttgaa ttttttttgt gtatatgtat 11100
aaagatttat gtgatattgt gtgaagttga tgttggtttg ggtagtggtt aggagtttag 11160
tggtaggatt gatttgttag ggggtataga ttttttagga ttgtagaagg gatttttttt 11220
ttttttttgt tttttttttt tttttttttt tttttgtttt ttttttttgt tttttatttt 11280
gttttggtgt atttttttt agttttagt ttatgtttt tttattgtag tttttttttgg 11340
tgggaatgtg gtggttggaa gatgggtttg gaagtgtata tttttatttt ttttttatg 11400
attttttaat ttaggttagg ttggggatgt atgttttagt ttattttaga tttgttttat 11460
tatttggtta ttttggttgt gtttggggaa gaaaaggtga ggttttttgt tgttttgttt 11520
tttgtttttt gggtttgtgt tgattggtgg gatttaggag gatgtatata gggaaggagg 11580
aaaataaagg tgtttttttt ttttggtttt attttgtttg ttagtgttag tttgtagtgg 11640
tggggtttag tttttttttt gtatatagtg aggataaggg aggtagttgt tttttttggt 11700
atttgttatt tttaaataga aaggattttt ttttagggtt ttttgggggt tgttgatggg 11760
aaagaggtag tatttgtagg ggttttgtag agatgttgga tatatttttt tatagatttg 11820
tgattttaaa aaattaagtt tatgtttttg tagaaattat taattgtatt ttatgtgggt 11880
ttgtggttgg gaattgttat tagaagtgga ttgtttgatt ttgagttggt agtggatttt 11940
tgttgttttt aaatttttaa ttattttgtg ggggttattt gtttagatta tagtaggagt 12000
gagttaattt ttgggttgtt attttgtaga attatgtgtg tatatttttg atgaaattta 12060
gattttttag ttagatttga aatttgtttt attgtttttg tttttttttt ttgttaatat 12120
ttaattaata tataggttta taatgttggg tgaggagatt tggttgggtt ttgtgtggtg 12180
tgggagtttg ttgagttagt ttttaatggt ttgggagttg ggtagtattg tttggtttgg 12240
tttggtttgg tttagtttag tttagtttaa gttgtttatt tttatgggtt ttaaaatatt 12300
tttgtaagat aatgtttttg ttttttggtt tttttgaaag aaaggggaga gagagttttt 12360
ttggggaggt ttgattttgt ttttgagatt tttaagtatt tgttttttga aagaaaatta 12420
agaaaaaaat ttaaaaatta ttattttagg gaaatttatt gttataaaat ggtgtttttt 12480
tgtgggttgt tttatgagtg tattaataag agttttagga ttagaagagt ttgggggtag 12540
agttttgggg aagggagtgg ttggaaattt agatagagat gggttttggg agtaggaggt 12600
tggggttttt tttggagttt tgtgttttat tttttattat tgttttggag ggttaatttt 12660
atttttaaat ttgtatttat ttttattaaa gttaggttta ttggtttgga gttttgggtg 12720
tgagtaagat aggtattgag tgtgtatgtg tgtatggggt gggtgtttaa gtatagggtg 12780
tgtgttttta tgggtggtga gtttgtttat gggttgtttt aaaagttgtt tttggtgttt 12840
ttgaggtggt gtttatagat tttttttttt taggtttgtt ttttggagag agtataagat 12900
ttatttggtt atgagggagt gtttggtatt tattttgggt ttttagtttg ttttttattt 12960
tttgttgggt atagttttag tattttagtt gatttttttg atttgggtag ggtgtagttt 13020
tagggttttt aaggagattt atattttttt tttttttagt gtgtttggta gttttttggt 13080
tttgaagggt ggggggtttt tagttttttt tagttatagg gatttgtgat gaagttgggg 13140
ttagatgttt tttaaagttg atttatatat tgtataaatt gaaatttaga ggtgaggtta 13200
ttattttttg ttagtggttt tgtttttttt tttttttata gggaatgtta gggggttgag 13260
ttttttatta ttaaaaagaa attgatgata tttttttttt tttgtttttt tttttttgtt 13320
ttttttttat ggatagtagg ttttagaagt tttatagtga ttttgtttaa aatttggggt 13380
aggtttatag ggagaaggtt aggttaggtt tataagtttg aattttagtt gggaggtata 13440

gtggggaggg ttagaagtgg atttggataa ggttagttgg gttattttgt tgtttatagt 13500
gaagtagttt tatgtttggg gaaagggtgg tgtagttaat atttttgtag agttaggttt 13560
ttttttttgg ataggaaatt tgggagattt ttagtgggtg aaggatttat ttattgtgag 13620
tagtttagtg ttttttttta ttaaggaggg aagtatatgt attgattttt ttttaaagga 13680
atgaatttgg gtttatagag tttttggttg ggagttatag aggagtttgg gtggaggtag 13740
atattttggg ttttttttgt ttttagggtt tatttgtttt tgatttttat agtttttggt 13800
attttgtggg gtatttttat gagggttttt attatagttt ttagggtgtt ttttgttttt 13860
gtgattgttt tgtagttttt tttagttttt tttttttttt ttttttttag tatttattgt 13920
tatttttgtt tttgaataga gagttttaga aaggattagg aaaaattagg ttagaaagtg 13980
tggggagttt tgtttatatt taggagtttt attttatttt agagattttt tatttgtggt 14040
tagtttgttt attaggtttg ttttatagtt ttatttatat tatatgtagt ttttttttat 14100
taagtggtgg aggtttgtgt tgagtttatg tttagtttga agtttagttt tatatttggt 14160
ggtttagttt tgagtggttt tgggtgagtt attttttttt tggggtttta gaatttttat 14220
ttggtgttta ttgggtggta tatttttggg taatttgatt tttttttgtt tattgtattt 14280
atttaggttt taggttttga aaattaaaga agaagaattt gaataaagag gataagtggt 14340
tgtgtatggt ttttattgtt gagtagttgt agaggtttaa ggttgagttt tagattaata 14400
ggtatttgat ggagtagtgg tgttagagtt tggtgtagga gttgagtttt aatgagttat 14460
agattaagat ttggttttag aataagtgtg ttaagattaa gaaggttatg ggtaataaga 14520
atatgttggt tgtgtatttt atggtatagg gtttgtataa ttattttatt atagttaagg 14580
agggtaagtt ggatagtgag tagggtgggg ggtatggagg ttaggtttta gtttgtgtta 14640
aataatgtaa taatttaaaa ttataaaggg ttagtgtata aagattatat tagtattaat 14700
agtgaaaata ttgtgtatta gttaaggttt tgaaatattt tatgtatata ttatttatag 14760
gtggtataaa atttaaaata tttgattata aaatattttt ttgagttttt tgtgtttatg 14820
agattatgtt aattttatgg gttttttttt tttttgtgaa gggggttgtt tagggtttta 14880
ttttttttta attttttaag ttttattata tgatattgga tattttttta ttattttaaa 14940
agaagaaaaa attaaaataa tttgttgaag tttaaagatt ttttattgtt gtattttata 15000
taattgtgaa ttgaataaat agttttattt tggtttatga tttttgttat tttgtttgtg 15060
ttggtttggt gaggatagta ggagggggttt atatttaag tttggattag ttattttaag 15120
gttttgggga gtttagggga tttggtggga gagaggggat ttttagggtt tttgggttag 15180
ttttgggatt tggttttggg aagtagttta gtgtatttta ggtttgtttt gggaagttgg 15240
ttttatgttt attagtagtt gtttaggttt gtagttttat ttggtttttt ttttttattt 15300
ttttgtattt aattttttt ttttttttt ttttttttt ttttttttt ttttttttt 15360
tttgtttttt ttttttttt ttttttttt ttttttttt ttttttttt ttttttttt 15420
tttttttttt tttttttttt attaagggtt taattgtgtg tatatattgt ttgtgtttgt 15480
ggtttgtgtt gttgttttta gttttattgt agttttgttg taggtttaat ttttttgttt 15540
tgggtattgt ttttatgtag aagtgttttg aggttttggg gttaaaggtt tggggtgtgt 15600
ggtttaaagt ttaagagtgg tggggtgatt ttttttttgg tttggtttta ggaatttttt 15660
gtgattttat tagttattat gggtgttagt tagggtttta gaaatgaggt tatggtttat 15720
tgtttttggg tgggtagaag gttttgtaga gggagatggt attatttatt tttttttttt 15780
ttttttttt tttttattt ttttttttt tttttttatt ttttttttt tttggagtgg 15840
ttgtttttgt tatagagaat atttttttaa gataaatatg tgtgtttata tatatgtttg 15900
tatgtatgtg aatatatata tatatatata tatattaggt gtgtttgagt ttatagtttt 15960
gaaatatgtg gttattttgt tttttaaaag aatttagaat tttttaggat ttagaagaag 16020
gaagaaagtg tgtaaataat tattttttat tattattttt tgtttttttt tgttttttaa 16080
aatatatatt ttatttttga aggtgtggta tagtgtaaat taaatatatt taatatattt 16140
tttattaagt atttatatat gtatataaat aaatatatta tttatatata atgttat 16197

<210> 27
<211> 16197
<212> DNA
<213> Artificial Sequence

<220>
<223> chemically treated genomic DNA (Homo sapiens)

<400> 27

gtggtgttat atatagataa tgtgtttgtt tatatatata tataggtatt tggtgggaaa 60
tatattgaat atatttaatt tatattgtat tatattttta aaaataaaat gtatatttta 120
aaaaataaga aaagataaaa agtgatgata agaaatgatt atttatatat tttttttttt 180
tttttagatt ttggaggatt ttgagttttt ttgaaagata aggtagttat atgttttaga 240
attgtggatt taaatatgtt tggtgtgtgt gtgtgtgtgt gtgtgtttat atgtatgtag 300
atatatgtgt aaatatatat atttattttg gaagaatgtt ttttatagta gaagtagtta 360

ttttaagaaa agaaaaaaat aaaggaaaaa aagaaaaaaa tagggaagaa aagaaaaagg 420
aaaggaagat agatgatgtt attttttttt atagagtttt ttgtttgttt agaaatagtg 480
agttatggtt ttatttttgg gattttggtt ggtatttatg atggttggtg gagttatagg 540
aaatttttgg ggttaagtta aaaggagggt tgttttattg tttttgggtt ttaggttata 600
tattttaggt ttttagtttt agaattttga agtgtttttg tatggaggta gtgtttaggg 660
taggagggtt aggtttgtgg taggattgtg gtgggattgg ggatagtgat atagattata 720
gatgtagatg atgtatgtat atggttgggt ttttggtgag gaggaggagg aaagagaagg 780
aggaggagga aggaaggagg aggaggagaa gaaaaagaag aagaaaggag gagtaggagg 840
aaggaggaag gaggaagagg aggaaaaagg agaaggagga gggagttagg tgtaggaggg 900
tgaggagagg gagttgggtg aggttgtggg tttgggtggt tgttggtgag tatggagttg 960
attttttaga gtaggtttgg ggtatgttgg gttgtttttt agggttaaat tttagaattg 1020
gtttaaggat tttggaagtt tttttttttt tattaggttt tttaagtttt ttaaggtttt 1080
gaggtggttg gtttaggttt gaggtgtggg ttttttttgt tgtttttatt aagttaatat 1140
aaataaagtg gtagaagtta tagattaaat aggagttatt tatttggttt atagttgtgt 1200
gaaatgtagt aataaaaaat tttggatttt tagtaagttg ttttaatttt tttttttttt 1260
ggaataataa aaaagtgttt aatgttatat aatggagttt aggggattaa aaaaaggtga 1320
aattttaagt agtttttttt gtaaaaaaga aaaaaattta taaaattagt ataattttat 1380
aaatataaaa aatttaaaaa aatattttat agttagatat tttggatttt atattatttg 1440
taaatgatat atatatagaa tattttagaa ttttagttaa tatataatat ttttattatt 1500
aatgttggta taatttttat atattggttt tttatgattt taaattattg tattgtttag 1560
tgtggattga gatttggttt ttatgttttt tgttttattt gttgtttgat ttgttttttt 1620
tggttgtggt ggagtggttg tataagtttt gtgttatgag gtgtatggtt agtgtgtttt 1680
tgttgtttgt ggtttttttg attttggtgt gtttgttttg gaattaaatt ttgatttgtg 1740
atttgttgag gtttagtttt tgtgttaggt tttggtgttg ttgttttgtt aggtatttgt 1800
tggtttggaa tttggttttg agtttttgta gttgtttggt ggtaaaggtt gtgtgtggtt 1860
gtttgttttt tttgtttggg tttttttttt ttggtttttg agatttggga tttgggtaga 1920
tatggtggat agagagaagt taggttattt agaggtgtgt tatttaatag gtattgggta 1980
aggattttgg ggttttaaga gggaagtgat ttgtttaagg ttatttgagg ttgaattgtt 2040
agatgtgggg ttagattttg gattgggtat gggtttaatg tggattttta ttatttggtg 2100
agggaggatt gtgtgtgatg taagtgggat tatggggtag gtttagtggg taggttgatt 2160
ataaatgagg ggtttttaga taaaagtaaa atttttggat gtaagtaaaa ttttttatat 2220
tttttggttt ggtttttttt agtttttttt gaggtttttt gtttaggaat agggatggtg 2280
gtaggtgttg agagagaggg gaggagagaa gattgggaaa ggttgtaggg tggttataga 2340
agtaggaggt gttttgaggg ttatgatggg gatttttatg ggatattttt atagggtgtt 2400
aagggttgtg ggaattaggg gtaggtgggt tttgaggata ggaggggttt agagtgtttg 2460
tttttattta agttttttta tgatttttag ttaagggttt tgtggattta agtttatttt 2520
tttaaggaag agttagtgta tgtatttttt tttttggtgg ggaggggtat tgagttattt 2580
atagtgggtg agttttttat ttattggaaa ttttttaggt tttttgttta ggagagggga 2640
tttggtttta taagggtgtt gattgtatta tttttttttt agatatggga ttgttttatt 2700
gtgggtagta gggtagttta gttgattttg tttaggttta tttttgattt tttttattgt 2760
gttttttaat tgggatttag atttatgaat ttgatttggt tttttttttg tggatttgtt 2820
ttaggttttg gatagggttg ttgtaaggtt tttaggattt gttatttatg gggaaagggt 2880
agagggagga gagtagaagg agggaagtgt tattagtttt tttttggtga taagaggttt 2940
aattttttgg tgttttttgt gggggaagaa gggggtaagg ttattggtag ggagtggtga 3000
ttttgttttt gggttttaat ttgtgtggtg tatgaattgg ttttagggag tatttggttt 3060
tagttttatt ataggttttt gtggttggag aaggttgggg gttttttatt ttttagggtt 3120
ggagagttgt tgggtatatt gaggagaaga ggaatgtgga ttttttttgga ggttttggga 3180
ttgtattttg tttaggttag gagagttaat tagggtgttg gggttgtgtt tagtaagagg 3240
tgggggatga attggaggtt tagggtgaat gttaagtatt tttttatggt taagtgagtt 3300
ttgtgttttt tttaggagat aggtttggag aaagaggatt tgtgggtatt attttaggag 3360
tattaagggt agtttttaaa gtaatttata ggtaggttta ttatttatga gagtatatat 3420
tttatatttg ggtatttatt ttatgtatat gtatatattt agtgtttgtt ttgtttatgt 3480
ttagggtttt aggttagtgg gtttggtttt ggtaggggta ggtgtagatt tggagatggg 3540
gttggttttt tggaatggtg gtgggggatg gggtataagg ttttaggaga ggttttagtt 3600
ttttgttttt agagtttatt tttgtttggg tttttaatta tttttttttt tagaatttta 3660
tttttaaatt tttttggttt tggggttttt gttaatgtat ttatgaagta gtttgtaaaa 3720
ggatattatt ttatggtaat aaattttttt aaaataataa tttttaggtt ttttttttga 3780
ttttttttta aaagataaat atttaggagt tttagaaata gaattaaatt tttttaagaa 3840
aatttttttt tttttttttt ttggagaaat taaagaatag aaatattatt ttgtagggat 3900
attttaaagt ttatgaagat aggtgatttg ggttgggttg agttgggttg ggttaggttg 3960
ggttgggtgg tgttgtttag tttttgggtt gttgggggtt ggtttagtga atttttgtgt 4020
tgtataaagt ttggttgagt ttttttgttt ggtattgtga gtttgtatgt taattaaata 4080
ttaataaagg aggaagatga gaataatgga gtaaatttta gatttagttg agaaatttga 4140

attttattag aaatatgtat gtatagtttt gtgggatggt ggtttaaggg ttggtttatt 4200
tttgttgtga tttgggtaaa tgatttttgt aaaatagttg agggtttggg ggtagtgggg 4260
atttgttgtt agtttggggt taaatagttt atttttaatg gtggttttta gttgtagatt 4320
tgtatggaat gtagttgatg atttttataa ggatatggat ttagtttttt aaaattgtag 4380
atttatgaaa aaatatattt agtatttttg tagggttttt gtgaatgttg tttttttttt 4440
attagtagtt tttaggaaat tttgagagaa ggtttttttt atttggggat ggtaggtgtt 4500
gggagggatg gttgtttttt ttgtttttgt tgtgtgtagg aagggggttg agttttatta 4560
ttgtgggttg gtgttggtaa ataaagtgga gttaagggga aagggtgttt ttattttttt 4620
ttttttttgt gtgtattttt ttgagtttta ttggttagtg taggtttgag gggtagaagg 4680
tagagtggta aagggttttg tttttttttt tttgagtata gttgggataa ttggatggta 4740
ggataagttt ggggtgggtt ggggtatgtg tttttggttt ggtttgagtt ggaagattgt 4800
agggaagggg atgagagtgt gtatttttgg gtttattttt tagttattat gtttttatta 4860
aaaaaaatta tagtggggga gatatgggtt aggggttggg aagagatgtg ttaaggtggg 4920
gtggaagata gagaggggag atagggagag aggaaggaga gagagagata gagaaagaaa 4980
gaaggttttt tttgtggttt taagaagttt gtatttttta gtgaattagt tttgttgttg 5040
gatttttggt tgttgtttgg gttggtgtta gttttatata atgttgtgtg agtttttgtg 5100
tgtgtgtgtg ggggaggttt gagatgtttt ttttttggta agtttttgtg ttagtattgt 5160
tttttagttt tgtgggtttt tttgtggtga gttgggatgt gtggtttttg tgggtttatt 5220
tgaagaaggt tggtttgagt agtgtgtata gtagatttat gttggttaga gtatgggttg 5280
tgtgtttagg ttggtgttgg tttgtgagtt gtttgagttt gagtttattg ataggttgtt 5340
gttgtttaag ttgtgggttt tgagtgattt gtttaggggg ttgttggggt tgttgttttt 5400
tttggtgtta ttgtgtagtg agagtgtttt ggagttgttt tttttgttat ttggagagtt 5460
gttgttggtg gttgggagtg gtttgtttgt gttgggtgta tatggtgggt tttgttgggg 5520
tttttgggag tttgagttta agagttgttt tgagttgttt gtgttgttgt ttttttttgt 5580
attgtttgtg ttgtttttgt tgttggtttt gttgtttttt tttttttttg tgtttgtata 5640
gtaggttttt gtgttttttt gttggttgaa tttgggttgt aggatgttgt tgatgaagaa 5700
gttggtgatg tggtgtgggt gttggtggtt gttgggtgtt tgtaggattg tgggtagtat 5760
tagagtttgt tggtgtttgg tgtttgtttt gtttgggttg ttattgttgt tgttgtttga 5820
gttgttgttg gggttggatt ttggttgttg ttgttttttt attgttgttg ttgttttgtt 5880
aggtttgggg ttattttttt ttatgttgtt tatagtaaat tttatattgg ttttttgggg 5940
atgttttttt aaattagttt ttggtttttt tgtggtgtat atggagattt ttgtgttttt 6000
atttgttgga gtttttgag ggtgtgtgtg gtggttgtgg ttagggttga ggtttataa 6060
gttgttggtt gtgtttggtg tggagttatt tttgaattta tgaattgggt tagaaatatt 6120
atgagttgtt ttgtttgttt agatgatgag agatttaata gagttaagtt tttgattttg 6180
ttgtgtagtt ggggtttaga tagttgtagt ggtttggtgg ggatgtgggt tgttttttgg 6240
gttttttgtt tttttttgtg tttggttgtt ttgggttttg gtgttgttgt ttgggttgtt 6300
tggggtgaga gttgtgtgta tgtaggtagt gtgttttttg gtgtttatgt ttgtttgggt 6360
tggttttggt tgttgttgtt gtttggttgt ttttgtttgt atgttttagt tggtttttga 6420
gggtgttggg tgtttgtggt tagtgtgttt gttattgttt gtatgtatat ggaggttttt 6480
tttttgtggt tgttgttttg ttgtgggttg gggtgtttta ttggtgtgtg gttgtgtttt 6540
tgttgagttt tgtagagttt tgtggtttga gttgtatggg tgagagatgt gaggttaggg 6600
tttggttggg ttgggttggg ttgtttgtgt tttttggttg tttgttgttt ttgttgtttt 6660
ggtttttgtg tgggtttgga aatttttagg ttatttggtg taatttgaga tatttttatt 6720
ttggattttt gtttttattt ttaatagagt ttttttgagt gatgttgtag gttggttttt 6780
tggatatgtg ttttgggtta atggttgtgg tgttgtgtgt ttatgtgatg ttttggttgt 6840
tgttgggtga taggagtagt ttgttgtttt tttttttttt aaagtggttg tgttttttgt 6900
tttgggtgtg ggagtttagg aatttggaga gatttttgat tgtgtttttt gtgtttgttt 6960
gtgttttttt tgttgttttg tggtttaggg gtgtgagtta tttggtgata gttttgttag 7020
gttattttgg ggtgtgtagg tggatatgta taggaagatt gttattttta agatttttat 7080
attgtttatt gtggggtgaa attttataat tttttgtata aaatgtataa ataatattaa 7140
atgagttgtt gagatataaa gggatttttg tggtttgttt tttttgttga tggagaggaa 7200
tagtgttatt tttatttgat ttggtaattt ggtagttttt tgttttaaat gtagagttgg 7260
tgtgtaggat attttttttg tagatattta ttgtaagtgt gtgtgtgtgt gtgtgtgtag 7320
ggaggtgtgt gtatatggtt gtattttatt tggggatagt gatttttatg aaatatgttt 7380
agtgatttga aagagtgggg gaatgtagta tgtggtttta tttgtgaagg gagaaattta 7440
ggagttttgg aggttttaaa ggaatttttt ttgttaagga gaggaggggt gattgtttag 7500
ttaagatttt aaaattaagt tgtttgtttt tttttaattt ttttattttg attgaggagt 7560
ttttagattg gttattttgt tttttttgtt ttaaaatttt ttaaattaat tttgttggag 7620
agtagggttt atttgaaatg agaattagga atttaatggt aagggtggga gttttagttg 7680
gttttttttt tttttttttt ttattttttt tagatttttt ttttagttgt tgttagaatg 7740
tttaattgga agttttgttt tttttttttt tttttttttt tttttgggtt tttttttatt 7800
tgtttttgtt tatttattaa atatttgttg ttattttagg tttgtttgtt tattatttaa 7860
aatgtatttt tatggaaatg tgtgaatttt tggaaatatt tgatagggta gttttggggg 7920

tgtgggagta tttgtatgtt atgagtattt ttagtgtggg ggaggtgggg aggttgtagt 7980
ttggtatgga tattttaggg ttgattggtt ttgggtagtt gtattgtgtt tagtaattta 8040
gatttttgta gaggtagtta gtaggttgtt gttaggtttt gagtgaattt ggggagtttg 8100
gtaagtattt ttgaggtagt gtggtttgtt aggttatttg gggtttgaag gttgtatttt 8160
ggttttgtga agtttttttt ttagtgttta ttttatgtgt tgtttttttt ttgtaggttt 8220
tagagtgttg gttgggttgg gatgagaaat gatagttttt atagttgttt ttaggaaaat 8280
tattttttta tttaggatgg gaatggtggg ggaggaggtg ttgggttggt tttttggtta 8340
ttggatgtgg ttttggttag agtgtgtttt ttgtttggtg tgtttagggt agggtgggga 8400
agtttttttt tgttggggtt ggattttttt attttttttt tattggagga gtttttatta 8460
aatgttttgg ttaggtatat atttgttagg ggtttttttt gagttttaaa aaattaaatt 8520
ttatagttta tttgtttttt gggaaaatag ggttggtgag agtttgtggg aagttttatt 8580
gatgtttatt tggttttttg gggggagaat tttttgtaat tttggaaatt ttgatgatta 8640
ttagttagga aagggagggt ttaatgattt tgggtttttt gaatatgata ttggggggag 8700
gaggggtttt gagttttttg gtttttgtaa aattatttta ggttaatggt tttatttgtt 8760
ttatgaaagt taattaggtg gttttttgag aagtattttt tgaatttttg ggattttttg 8820
tttttgattg ttttagtata aatggtttta agatgttggg ttttagtttt gtgttttttt 8880
gtgatttaga ttttaatttg tggttgtaat agagattttg ggaagtagag tgttattttt 8940
agggtgtttg gtgggttggt gggtagagat ttgtttttgt agggtttgtg ggttgggttt 9000
tggggttggt tggaggaaga ggaatttgtg ggttgttttt ttggaaagga ggaaggtgag 9060
aggtggaggg aggaatagga gaaagagaaa taaatgaaga gagagataat tagagggtat 9120
tgggtttttt agagtggatt taaatatgaa ataattttgg gaaagtggta agaggggtta 9180
ttgtttaaag ggttggggaa gttgggatgg ggttgtagaa agttgagttg tttaggggtt 9240
gggtggtgtt gagttttata tatatggaat tttgtaaggt gttttaggaa gttttgagaa 9300
gtgtgtatga ggattggttt tggttgtttg ttttagttgg gatggtgttt tgtgtttttt 9360
ggggatgtgg ggtagtggtg tgtttttttt ttgtgatgtt agttttggtt gttgtgtttt 9420
tgtgagttta gagtttttgt tttttttttt gaatgattgt tgtttttaag gttggttttt 9480
ttgtgtggat tagatgagtt ttttttggat attagttttt tttgggatgg aaagtggggt 9540
attgatttgg gaattttgtt ttttttttag ttttatttag gaattggaga gtgtttgtga 9600
ttatttgttt gtggaggatg gttttttata ttttttaggt ttgttgtata aagaggggtt 9660
ttggagaggg tgagtaggaa agtagttttt agatgttgtg tgttttagga tatggatgtt 9720
agttttgtaa ataaaatttt agtgaagtgt tttggagata ttgggtgttt ggatgttgag 9780
ttaggttttt gttttatttt gaaaatgagg ttagagaagg gtgatttttt tggttttttt 9840
gtgttttttt tatttgtttt gtttttttag tttgatgtgg tgatatttgg aaaaatggat 9900
ttaatgaaag ttgtttgttt gtggtggttt tgttatttgt ggggtagaga tgtttaataa 9960
tttttgagag tgagattttt attttagtta aagtaggttt ggttagtttt gtgtttttgg 10020
ttgggagttg agttttgtag ttaagtttta ggtgttgggt ggggaattgg ggttgatagg 10080
agagtaatat tttttttggg gtgagtagag agttattgtt ggtttttggg gttgatgttt 10140
tgatttggtt tgtagataaa ggttgaaatg aaggggtgtg gggagtaggg gtggggtggg 10200
tggggttaat gggtggatat tgtgtgtttg tgtttttttt tgggtgaagg aggattggtt 10260
tataggttta ttgggtgagt ttggggatgg ggtttgggag gttgggttta gggtttgaga 10320
tttttttaga ataatttttg gaaggagggg gttaaatgtg gtagtggtgg gtttgaaaag 10380
ggttaggagt gttgtaggaa ttggggtgta taggtttttg tgtgtttagt gttttgtttt 10440
tttttgtttt aggtttgtta ggggtgtgtg tggtgtgggt agagtttggt gggtgggttg 10500
gtgtggatag aagttttgtt ggggagtggg gagggggggg tagttgtatt gtatgttttg 10560
gttttgtttt gattgttgga gaagtgtttg tgttgagttt aagtttttta ggtgttgggg 10620
aagtagagtg ggttaggagt tgattgggga ttgttttttt gttttgattt gatatttagt 10680
ttaggtttgt ttggggaggg gggtggttgg gggtaggtgg gtagattttt agagttggtg 10740
tgtaaagatg tagaaattgt tttttatttg gtttttttgg aaagtagttg tttttgaagg 10800
agaatttgaa ggatggtttt atttgtgtta gtaattttta tatttaaatt aggttttttg 10860
tgtttgtttt tttttttggt tttttagtgt taggggagtt ttatttgggg tgatttgttt 10920
ttttaagaat tgtttttttt tggtagtttt ttgtttgttt ttttgttttg ttttattgtt 10980
gtttttattt ttataaaatt tggatttttg tggaaggtgg gtttattttt atttaaggtt 11040
tatttttgag attttttaga tagatttgta ttttggagtt ttttgggagt tttgggaggg 11100
gtaggtttgg gagtatagat tgttttatta tttaatgttt ggatttttat gaaaatgttt 11160
ttttaaaatt atagtaattt agaagaaagt ggtatttttt aaataattta gttaaataag 11220
taaaattagt ttggtaaagg aatttgaaga atggtattta aggtttgagt tggttaagtt 11280
tattaaaata ggaaggaaaa tatagaaaag gataatgagt tttgaagtat attagttatt 11340
ttttttagtt ttgtgattat ttatagatgg aagaaatttt agtatgtatt tattgttttt 11400
tttttgattt ttggaaagaa aataagatgt ttaaaaggaa tttttgaaat attagtttta 11460
ttttatagag gttgatattt tttttttttt tatttttttt ttaaaggtta taagtaatta 11520
tagagaggtt agtgtagttt aaatttttat atagtgattt tttagtattt ttggaaatag 11580
gatttgtgta gttagaggtt tatagaattt taggtaagta taatttagat ttaatttaaa 11640
ttatttggat ttagtttagt taaattattt atagtttgtt agatgataat ttattagttt 11700

aggttttatg tgaggaagaa aatatttaaa taattatttt tgtttttaaa tattataatt 11760
gaattatgtt taattaggag aatttaatta gtttatatat tggttaagtt gggggaaata 11820
tatttttaaa aatatatgtt taagttaaag ttatgaattg attttttttt ttatttatta 11880
aatataaata aagatatata gttttatata aaaaggtaat taaattgtga attaaattta 11940
aatatttttt ttatttagtt aaatgattga ttagtttttt tgagtttaat aaagttggtt 12000
gtttttttag tatgagtatt tttaattgat atatatgttt atatgtatgg tatgggggga 12060
tttttgtatt taattaattt ttgtgttgag tatagaagtt tgtgtgaagt gtggtgattt 12120
tttataagta taaagtttta gtagtgattt ttgtggattt atatgtggag gggtattgtg 12180
ttatttttgt attatggatt tttgttttta gttttgttta aggtttaaat tatttttttg 12240
ggggatgttg tttagtttta tttttattag ggatggggat ttgttgttta tttttggatg 12300
tatttttatt tttttttttt tattttagta tttttggttt tatttaaaga aaagtttagt 12360
agaataagat tttttagtta gggtatatta tagaagtgtt ttgttttttt ttttaaaaat 12420
tttttttttt tttttggaaa tttatttaaa tattttagtt ttttttagaa aggttgtgtt 12480
atagtttatg ttatggagtt gtagggtttt gtgtgtttta tattagttaa ttttttaaga 12540
tataatagtt tttgtgatga tggtaaagtt gagtaatttg atattatttg gagtattaat 12600
ataggttggg aggaagaaat gggatatgtg tgaagatttt ttatgtttgg agtgaatgtt 12660
ttagaatttt ttgggattta tatttttttg tttattagga aggagaaatg tgagttgttt 12720
ttatttattg tttgttttta gttttttgtt tgtattgtta gggttaattt tggttgtggt 12780
gtaatatttg ggttatagat gttagggggt tgtgtttagg gggaagtggg attttgagtt 12840
ttgttagttt tggttagaaa gtttggtatt aaggtgttgg gtatgttttt aggataaagt 12900
gtgggttttt taaaagagga aggttgggtg gtatttaggg ttttttgttt tgtttgtaga 12960
tataagttta tttgtatttt gtgtaaagta gttttgaaag tttaggtttg aagataaaat 13020
gtggagttta ggtttttttg attgaaaatt tttttatgag gatatttttt gtagtgggtg 13080
gttagaaagg attttggatt aagatttgta gagtggtgag tattttttag ggtttggggt 13140
tgaattgatt gtaggttggt agttttttgg tttggaggag aatttttttt ttgtagaaat 13200
tatttttgga aaataaaatt gagtgaaaag aagggtaatg tgggtttgtg tggaagggag 13260
tttttttttt ggggaaggag gtgttttgtg gtggtatatt tgttggtggg atgtaggtag 13320
gggtagatta gggtgagaag ggggatttgt ggaatatggg ttttttgtgg gtttgaggga 13380
tagggtgtag gtgtatttta ggtatttagg gtagtttagt ttgttttgtg gagtgggagt 13440
tggttttagg ttttttatat tttttaggaa tttagaagtt tgattgtggg atagttgtgt 13500
tgatggggat ttttttagtt ggatttttat tttatttgtt tttttgattg agtgtttatg 13560
tttggtagga gttggttgtg tgttgttttg ggtgggtgga ggtgtgtggg gtttgggttt 13620
tggtttgttt gggtgtgttt tagttttttt tttttagaat taaaggtttt tgtagggtgt 13680
agagtttttt tttttttttt ttaatttttt tttttatttt gttgtggttt aagttaagat 13740
gaggtgagtt tttagggttg tgtttttagg aggttttgag tatttttttt ttgagggttg 13800
aagaaattag gttttttttt tttttttagt gttttttgag gaagttgtga atttttgagt 13860
gagtgtggtt ttattgggga tttttagtga tttgttttgt gtttagtgat gttttaaggg 13920
gagaggagtt ttgtggaggg gtggtgtggg ggtgggtgtt tatattatat tttttggttt 13980
ggagtaggat ttggtgatgt gttttggttg agtttttgtt ttttaggaat gtgggtgttt 14040
tgggttttgt tgggattttt ggtttgattg gagggtttgg gagtgggagt ttggatttaa 14100
taggtatttg gatttaggag ttagattttt tggttttgaa tgtttttttt ggagtgaaag 14160
tttgtttttt ttttttttaa gttttttgtg ggttgggagg tgaagggagt gatagaagtt 14220
gggttttttt tgtagtgttt aatgttttgg gttttttggg ttttgggatg tggtttgggt 14280
gtgtggaggg atggttgggg gtgtagattt gtgaatgaat tttggagttt tttgtagttt 14340
atagattaga ggttgtgatt gttttattga tgtggtttaa tgagttgttg taataagttt 14400
tgagtgatga aatggaggta tggtttggat tttgtggtat tgtgttgtgt agggttggat 14460
gggggagggg ttgagttatt gtagtaattt taaagtttgt gttggggttt agtaatgggt 14520
gggttttttt tgggttgtat ggtttttaag ggtttttttt gatttttatt gttattttgt 14580
tggaatgttt agaggtgttt ttagtttaag tgatggagat tttgattttt agttgttttt 14640
gtgttttttg tttgtgtgtt tttggtgttt tttttgggat ttggggggtg attatatttt 14700
gagatttaat ttggttattt ttgggtgtgg gtttttattg ttttttgggt tggttggatt 14760
tgttttttaa gtagttgttt tatttattat ttgttgttag tttttttttt aaaatttaag 14820
tttttggttg tttttttgtt gttttttgtt ttttgttttt tgttttttgt gtgaggggag 14880
gggaggggga ggaaggagtg gatatttatg tgtgtttggg aggggtttgt attgtttttt 14940
tgttttttta gtagaggttt tgggtttttt ttttagaggg aaattggtga gtagatatta 15000
agtgatgtga gttttggttt tttttttgtt gggggatttt tgtgtgtttt agaagtgtat 15060
ttggaagtaa ttttttttgt gtaattgttg ttttttaagt atttttagta tttttatatt 15120
ttttaaatat ttgtataatt ttgtgttgtt ttttggagga ttttggagat ggggagggga 15180
tgatggggtg ttgtgggggt agggaaggag agaggttagg ggttatgtag gattatggtt 15240
tggtttggtg atttttttta ttttttgtgg tttagggagg ggatttgtag tgagggtttt 15300
agattttttt tgtaaaattg aattttgggg gagggtttgt gttttatgaa gtatggggaa 15360
ggtttaaatg atgaatattt atgagtggtt gggagtggaa gatggtagga ttgatgggtt 15420
tgggagggaa gatgggtgtg tgtttttagt gattatggta tgaggtggtg ttttgttttg 15480

tattatgtta taatttaatt aggtttttgt ttttttgtgt ttgaataggg tgggtttatt 15540
tgggtggtga gtttgagttt agtgtagttt tagtttggtg tttttttttt gtttgttggg 15600
gaagaggtgg gaaatttttt ttgttgtgtt ttggttttta ttgtaggttt ttgagaataa 15660
atttttttag ggagattgaa ttgtttttat tttttgtttt agttttaaga gggatttttt 15720
gaaaatatgg tagttaagaa tagggtgaaa atgtaagttt gaggtttttt tttttatttt 15780
tttttgtttt ttgtttttt attttatat tttttattag tttagagggg tttagttgta 15840
attattatta ttgttatttt tatggtgttg atgattgtta ttattttttt ttagttttag 15900
ttgttttttg gatgttttta ttggaaggtt gatgagtatt tttaatttta tgtgtttatt 15960
ttagagtatt tttgattttg ttatttttgt tgttttgatt tgtgtttttt tagttttttt 16020
tagttattta gttggttatt ttttaggaga tatttttgat tttttatttt ttttttagag 16080
ttattttttg ttttttagta aggtttgttg gttttgtttt tatgtttttt ttatagtttt 16140
tttatatttt tgtgtttttt attagttttt aagttttttt tttttttatt tgtaggg 16197

<210> 28
<211> 2609
<212> DNA
<213> Artificial Sequence

<220>
<223> chemically treated genomic DNA (Homo sapiens)

<400> 28

tgtagattag agatgattat agattttttt tagtgtggat taaagggatt gaattgaatg 60
ttttagttta atgatttaat ttttgttata tttataggga tgtgaatttt gattttataa 120
gtaggtgtgt atgtgtattt agatatttat ataaagttgt gtggagggat gaaaagatta 180
attatttgat tgatgaggat aggtttgatt tttttgatta ttttagtgtg ttagtgtata 240
tttttggttg ggtttagtgt tttaagaaat tttggaattt tagttgttaa tttttgtttt 300
tttattatga ttttttaaag atattttatt tgtttattgg ggtgaagaga aatgggatta 360
ggtgttaggg tggtgggatt ttgtttaggg ttttgatttt gtgttagggt tttagattag 420
ttggtttttg aaggtttttt tatttatttt atataagagg aaataaagat tttttagttt 480
aaggttttag ggttgttttt tgattttggt tagtttgtag gaagaggaaa taataaaata 540
aaggaattgt taatttgttg ggtattatat tttttagttt taatttttga ttttggattg 600
ttagggttat tttttttatg ttgattttgt tttttttaaa tgaaaatatg ttaataaaag 660
tatattttgg atataaaatt taagtatgta ttttttttgg ggaggttaga gttgaggtgt 720
attttggaag atgagaattt tgtttttatg aattgggtaa tatttaggta ttgttaggta 780
ttttgataga ttttttagat attttttttt ttttttttta tatttttttt ttattaaaat 840
agttattgtt ttgaaattta ttagaataat gatgttttaa aaataaaggt gtagtaagta 900
tttttttttt tgttgttgtg ggttgaatta tggatgtttg tgggttgttt agttttgatg 960
gtttgtaggg ggtgtgtgtt gtagttgtag tatagtttgg ttatttttag aaagggagtt 1020
gaatggaggg aagtagggag tgtggagggt ttgaggtttg tagataagga gaggtgtatt 1080
ttgggatttg ggttttttgt tgttataata taattgtgtt attgttggta ttgtttgatt 1140
taagtgttgg tggtaagtgg tgatgttggg gttgggtttt tagtaattgt tgtgttttgg 1200
gttaggttgg ttgttttagt tattgggatt tttttttgga tgtttttagg gtgataggtg 1260
ttgtatttat tgatgggata gttgtatatt tttgaatagg tagtggagtt tgtttttggt 1320
aggtatttta gttgtgttga tattaaggtt gttatataat agttattagt tttttttaag 1380
ttttagaagt aggttttttt tgttttagtt atagtggttt tagttgttgt tatgagttta 1440
ttttttattt ttaagtgtat tttttttttt ttatttgggt ttatgtttgt tatatagaga 1500
gaattatata gggggaatta tggttttata tttttgaggg gatagatatt ggttgtgaga 1560
taggtattat gtagagtttt ttggtgattg tttgtaggag tgagattttt tttggttttg 1620
tagttggtta ggtgtgtgtg tgtgagggtt tttagttgat tattgggatg tattgttatt 1680
ttttggtttg gtgggttttg ggattttttg gtttttgtag gaggttattt taggtttttg 1740
gaggaggtgt ttttagttgg tggtgttttt tgttttgggt ttagaggtgg atatggttgg 1800
ttgtgttttg ttggtttttt gtttgtgttg tagtgggttg ggagtagttg tgtgatatta 1860
gatttatagt gttaagatgt gaagtgtgag gaaattgttg tgtttgattt tttttttttt 1920
aatttttgga tttaggaatg tttttagttt ttgtgtttta tatgtttagt tttggttttt 1980
tttttggttt agaagttttt aaggattgaa gggttttgtt tagggttttg tatttttgtt 2040
tgatttttat ggtttagaaa agtaggggga tatttgaaat gttattttgg gataaatatt 2100
aataaaaaag taaatggatt tgtgtagggg ttagttatta attaattagg ttgtaaggtt 2160
atttagggta aatatagttt aggttgggtt gggtgagatt tttttatggt tgttttttga 2220
ttgaattttt ttttttttgg tttaggtttt ttttaggttg ttttggggta aatattggat 2280
gggaaggggg tgttgttaat ttttttgtgg ggtttggagg tttttttgtt tttaagtttt 2340
gtagggtagg gttggagtgt ttaatattta tttttgtttg aattttgggt ttgtgtgttg 2400

tttttttttg ggtttgtggt gttgtgtttg ttgttgggtg tgtgttgttg tttttttttg 2460
agttggtagt ttttgttgtg tggttgaagt tttttggaat ttttaattgg aaattaattt 2520
tggttttgat agatgtttat gttagaggtg tgttatttat ttatattttt tgttttattt 2580
tggaggagat gtggtgagaa ttttgttgt 2609

<210> 29
<211> 2609
<212> DNA
<213> Artificial Sequence

<220>
<223> chemically treated genomic DNA (Homo sapiens)

<400> 29
   gtgatagggt ttttgttgtg ttttttttgg gatgaggtgg ggggtgtggg tgggtggtgt 60
   gtttttgatg tgggtgttta ttaagattaa gattagtttt taattaaaga ttttagaggg 120
   ttttggttat atagtaggag ttgttggttt ggaaagagat agtgatatat atttaatagt 180
   aagtgtagta ttgtggattt aggggaaggt ggtgtgtagg tttgaggttt gggtgggggt 240
   gggtgttggg tattttgatt ttgttttgtg ggatttggag gtgaggaaat ttttaaattt 300
   tgtgggggag ttggtggtgt tttttttttg tttggtgttt gttttagaat gatttaagaa 360
   ggatttgagt taagggggga ggggtttggt tggggggtgg ttatgagaaa gttttgttta 420
   gtttaatttg ggttgtgttt gttttgagtg gttttgtggt ttggttgatt gataattggt 480
   ttttgtataa gtttatttgt ttttttgtta atatttattt tggggtgata ttttaaatgt 540
   ttttttgttt ttttgggtta tgggagttag gtaggagtgt ggggttttag gtagagtttt 600
   ttaatttttg aagatttttg ggttgggaga gaagttagga ttgggtgtgt gggatgtagg 660
   agttggaagt gtttttgggt ttaagagttg ggggagaagg agttaagtgt agtggttttt 720
   ttgtgttttg tgttttggtg ttgtgggttt ggtgttgtgt agttgttttt agtttgttgt 780
   ggtgtgaata aagggttagt agggtgtgat tgattgtgtt tgtttttgag tttggggtga 840
   ggggtgttgt tagttgggag tgtttttttt gaaggtttag gatggttttt tatggagatt 900
   aaggagtttt agagtttgtt aggttgaaaa gtggtagtgt attttggtag ttaattgggg 960
   atttttatat atatatattt agttgattgt aaaattaaaa aaggttttgt ttttgtggat 1020
   agttattaga aggttttgtg tggtgtttgt tttatggttg gtgtttgttt ttttgagggt 1080
   gtaggattat agtttttttt gtgtagtttt ttttgtgtaa tgggtataaa tttgggtgag 1140
   gagagggagg tgtgtttggg gataagaagt gggtttgtgg tgatgattgg aattgttatg 1200
   gttggggtag gggaaattta tttttggggt ttaagggggg ttgatggtta ttgtgtggtg 1260
   attttggtgt tagtgtagtt aggatgtttg ttagagatga attttattat ttgtttggag 1320
   gtatgtggtt attttgttaa tgggtgtagt atttattgtt ttggaagtat ttggagaggg 1380
   attttggtag ttggggtgat tagtttgatt tagggtatag tggttgttag agatttagtt 1440
   ttgatattgt tgtttattat tgatatttgg gttggatagt gttaataata gtatggttgt 1500
   gttgtagtag tagaggattt aaattttagg atgtgttttt ttttatttgt aagttttgag 1560
   ttttttgtgt tttttgtttt tttttatttg gttttttttt tgggggtagt tgggttgtgt 1620
   tgtggttgtg gtgtgtgttt tttgtgggtt gttggggttg ggtgatttgt gagtgtttgt 1680
   ggtttagttt gtggtagtga agaaagggat gtttgttgtg tttttgtttt tagaatgttg 1740
   ttgttttagt gaattttaaa ataataatta ttttgatagg aagaaagtgt gaaggaggaa 1800
   gagaaggata tttagagggt ttgttggggt atttaataat gtttgggtgt tatttaattt 1860
   atgaaaataa aatttttatt ttttgaagta tattttagtt ttaatttttt taaaaaagat 1920
   gtgtatttgg attttgtatt tgaagtatgt ttttgttggt gtgtttttat ttaagaaaag 1980
   tggggttagt gtagagaagg tgattttggt ggtttgaagt tggaggttgg agttgaggga 2040
   tataatattt ggtggattgg tggttttttt gttttgttgt tttttttttt tgtaaattga 2100
   ttggagttag gaggtagttt tgaggttttg agttgagaag tttttgtttt tttttgtgtg 2160
   gggtgggtga aagggttttt ggaggttgat tggtttggag ttttggtgtg gagttaggat 2220
   tttggatagg gttttattgt tttgatgttt ggttttattt ttttttgttt tggtaggtag 2280
   atagaatgtt tttgggaagt tatagtaaga aaataaaaat taatagttaa agttttgaag 2340
   ttttttaggg tgttaggttt agttgggaat atatattggt atattgaggt aattgaaaag 2400
   attaaattta tttttgttga ttgggtggtt gatttttttg tttttttatg tggttttgtg 2460
   tgagtatttg agtgtatgtg tatatttgtt tataaaattg gaatttgtgt ttttgtgggt 2520
   gtgatagggg ttggattatt aagttaaaat gtttaattta gtttttttga tttgtgttgg 2580
   ggaaaattta tagttatttt taatttatg 2609

<210> 30
<211> 11667
<212> DNA

<213> Artificial Sequence

<220>
<223> chemically treated genomic DNA (Homo sapiens)

<400> 30

tgggatttgt gggtgttaat taatgttatg gtggtggaga gtaaagggga tgaatatagt 60
ttggggttat ttttggagtt ttttagtgtt tgtttgtggt tgtgtttggt tggttgtgga 120
ttttggtggg tgttgtaaag tggtgggatt gttagtgtag agttttggtt ttttgttttg 180
tttttgggtt tgagtattgg agtttttggt gtttgtgggg agaagttttg gattgagaaa 240
tatgggaggg ttttgttagt ggttgtaggt gtggtagtta ttttggggat ttagtgagaa 300
tggggttgtt tggttttgtg tgaatttttt atgtgggtgt agtttttgag ttgttgaggg 360
aggtggtggt aatgttgttt agtgttagta gagggtagtt ttgaggttgt gagtttgaat 420
ggtgattttg ttaaattgtg ggtttttttt tagtttttgg attttgtggg gtagaggtgg 480
ttttggagtt tagagattag tgattttagt ttgtaggagt ttggtgtaga ggtttaaggg 540
tatttttggg atgtggttaa gttatagttt ttaggtagtt ttattttgtg atggtaaggg 600
tttagagggt ggagggggat tagatgtttt aggaggggtt agaaagttaa tgtatatagg 660
gaatttgttt ttaagtaata gattttaagt atgtggaaat tttttaaatg atgttggtga 720
gagttatgat agtttttgta tttttggtga gggaagttgg aggttagtag tgggatggtt 780
tttgggtgtg tgggagatag agggattagt gatttttgtt gggggtagag ggattgtgga 840
ttaaggattt agatatattt aattggggat tttagttttg attgtggtta tttaattggt 900
gttgaatttg agtaatttat tatattgttt tagtttttga ttaattattt ttgtaaaatg 960
ggtattgtgg gttttgagtt ttgtaagggt gttagggatt tgagatagta ggaatttttt 1020
tattgtttta gtaattttgg gtttttgggt ttgtaggtgg gttgaaagga tttttttatt 1080
gtatgtttgt ttggtgtggt tgttttagag attgaggttt gttaggtttt gtaatttagt 1140
tgtgtgtggt tagttatggt tttggaattg ttgggattgt tttagtggta ttttggttag 1200
tttttgattt tttgttttgg gtattagggt tatttagttt tagaagatag tgtttattag 1260
tataggaatt gagattttat atttggtgta gtgggttttt taggaagttt ggtgaagagt 1320
taaggtttgt tggatttgag gttgtttggg gtgttaaata gatttattta tgagtatatt 1380
tggttaatat atttaagttt aatagtgggt gggatttttg gttggttttg atttttttta 1440
tagtgtgtgt aaatgggtat ttattagatt tttttgtttt gtttttttat ttgtaaattt 1500
tgaaaggaga gtattttttg gggtgatttt gaaaattatg ttagaattga aaaggtttgt 1560
gtaaatgtga ggtgtaatga tgatttatta gaagtagtta gttttgtatg tgtttgtagg 1620
atagatgtta gaatgtttta taaatgtgtg tatatatata ttatgtatgt gtgaaggtgt 1680
atatttgttt ttagatattt gataaatgta tatatgtatg tgtgtatttt tggttgagag 1740
taaaggggtt aatatagggt ttatatagga tattattttg tagttttgtg tgtgtatagt 1800
atatatgtga gtatgattgt ttgtgtttgg agatattgta tggtattgga ggagttgttt 1860
agttgttggt gttatttggg aagtagggtt ttgggtgttg ttttggagtg tgggaaagtt 1920
tggatttggg tttgttggtt agtgttttgg tgttgttggg tttttttatt ttttatttgt 1980
tggttggtga gtggttttag ttttaagatt tttgattttg ttggtgaggg gagggagtga 2040
gaaggagtgt gttttgggtt taagaggttg tagggtttta ttttttaggt ttgttgtttt 2100
ttttttattt ttttaggtta tatttttatt taaaataaag aagggtgttt gatttattta 2160
ggttagggaa gttggttttt gggagttttt tgtttttata agttatggtg aagggaggtg 2220
aagttagggt ttgttatggt ttgggagaaa atgtggttgt agggtttttt tgggttgtag 2280
tgttggtttg gggttttaag attgttaagg tgtgtgtggg aggtgtgtag tgtggttatt 2340
gaagagtttt ttattattgt attggtaggt tgttgggttt gttttttttt tatgttttta 2400
agggttttga gttgtgtagt atttgtatta tttagaagtg tttgtggaga aaatgatttt 2460
aggtgtaagt ttaaggttgt tgtttgtaaa ggtaatgttg tggagattgg gttttatagt 2520
gatttgggtt tttaagttat tgaaagttat agggtagggt tataaatatt tttttgtttt 2580
ttttgtagaa ttgtgtggtt gataggagag tgttgtgtag aaaatttgag gttgggtgtt 2640
ggaggagttt ttgtggtttg gagaaggtat agaggtgttt ttgagaggtg tagttggaat 2700
aggtgatgta tgggtttgga tttggttggt taattgagtt tagtggttgt gaaaattaga 2760
gttgttatag aggttgagta tgattttatt tttggggatt gggtttggtg gggagttatt 2820
gtttttaatg tttttagaga ttttaagtag gataatataa tgttggtagg agtaaggtgt 2880
aaggaattta gtggtagaag ttttttgggg gggtggggaa aggtagattt gtgttttgtt 2940
tgagtttggg ggtggggata gtttttggtt ttgtagtttt tgttttgggg attagttggg 3000
tttatttaat ttttttatat ggggttgaaa ggggttaatg ggatggtttt gttgtttttt 3060
ttttgggatt ttatagggtt ttgatttggt ttttattttt gtatggttag tagttgtggg 3120
ggttttagga aggaggataa aggtttggtg ttattgtggg tgggggtttg gttttttggt 3180
tttttgttta tatttatagt ttttagtggt tgttggggga agatttttaa aaatatgtgt 3240
tgaatttttt tttttttttt tttagaaata aataaataaa aaaggtaaaa ggtgaatttt 3300
tttttatttt ttgtttatag agattaaagt tttttgggat tttgtttttt tttttttttt 3360

gattgtttag aaatatttgt ttttttttgt atatagagga aaatgtgggg aaaggttttt 3420
taaaatttgg ttttatatta ttattattat taaggataat tgggtaggtt gaggttttaa 3480
tgtggatgat ttgagttggt tttgtgttgg ggttttgtag ttattgtttt gtgtgtttag 3540
tatttttggg ggtgattagg gtttttgtgt ttttgtttgt tgtttggtag ttgagagtat 3600
tttgtgttta gattggttga tttatttttt tttgaatttt gtttagagtt ggtaaggggg 3660
atttagtttg tgttttaaga tttgggtttg tagtgttgtt aataggtttg gggatatgag 3720
gtgttttagg ttggggtttt tttggttgtt ggtttttttt gtttttattt tgttggtggt 3780
gttttggttg tttgtaattg atttaatttg ttttttgtgt ttgtttttta ggttttttgt 3840
ttttttataa aggtttaggg gagttttgtt tataggttga ttttgtaatt tttggtttgg 3900
tggttatttt ttgttttttt gaaaaagaaa aggaaaaaaa aaaaaaaaaa gaaaaaatta 3960
attagttgag ggaggtgtgt gaggattgga ggtgttagtt ggatagttta tgagtaggtt 4020
tttgggttgg tgttgttttg tgggttagta tggttttttt tagagaaaat tttttaaatg 4080
tgtgaagatt gttttggggg aagtgagagg gaggttggag gagttttggg tggggtttta 4140
gtgtttatta gttgtgtttt tagggtttgg gtgtttgttg taatggtaat tgtgtgagtt 4200
ttatttttat ggttaagggg ttagggtagg gtggatgtaa ttgtgtgtgt ttggttttgg 4260
aaggtgtttg tagggggtgt ttgtggttag ttgggttagg aagttaaatt ttagaaagtt 4320
attattaaag gttttttttt tttttttttt tttttttttt ttttttgttt tttttttttt 4380
tttttttttt ttaatagggg aagggaaaaa atattattga atatttatta taaattgggt 4440
atttaaaaat tagaaaattt aattatttta ataattttga tatatgtatg tatttttgtt 4500
ttatagagga gaatattgaa atttgaatgg gtattttgtt taaggttata tagttgttaa 4560
agaggatatt aggttttaat ttagtttttt ttgattaaat aggttaggtt tttgttttat 4620
gttgtatagg ttatttttgt gaagtgataa gattttagaa tatgtttttt atagtttagt 4680
attaagtttg tgtggtaaat agtttgtgta gtagtagttt ttgtggtata gaaataaata 4740
aaaaaggtaa aaggtatagt ttagattgtg tttttttttt tttttgggag gttttttttt 4800
attatttttg ggaggttttt ttttattatg tagttttttt gagtattagt ggtagttagg 4860
tttagttgtt gttaagtttt ttttaaaaat tttggaggtt atttgtttta tagatatttg 4920
gaagattttt ggttggtttt tttttttttt aggatttgag attttttaaa tgtaatgtaa 4980
ttttgggttt ttagatttaa gatatttttt ttatgatagt tgggagtttt ttttttagaa 5040
agtggaaagt tgttaagatt gtttggtttt aagaattttt aatttttggt tgttttgaag 5100
gtatggggtt tttagtttta tttttttttt atttttttta tgggttttat agtttatttt 5160
aagaagtagg ggttgttata tagtggtggt aggtttgatg ggtagggttt tagggttatt 5220
attaggttag atttgtagaa tggggatatt gtagggatgt tgagtttgat taagatgttt 5280
tttggtttta gatttttatt tagaagttag gagtttggtt tggtgatttt gtttatgaag 5340
tttggtttag atttttataa agggaagagt ttagttgaga gtaaattatt ttgggttaga 5400
tttagatttt tggtttatat tttgtttgtt tggttgtatg aagtttgggt ttggttttaa 5460
tttttatttg ttgttagggg gttttttgta gttttatttt tagttttggt ttttaggaaa 5520
ttttggtaaa ggttggatgt atttaatttt ggatgaaagg tttagggttt tttgtttatt 5580
taagttttag atttggagta gtgaattatt tattgatgta agttggaaat aggatgttaa 5640
tattttttgg agtagggtat aggattattt ttagaatttt gtaggttttt tttttatagt 5700
tttttggtgt atgtatttta atggtgaaga tttttttagg ttttttttga gaataggata 5760
tttttttttg ttttttttaa attaatttat ttattttgat tgggtaattt tagtgaggtg 5820
gaggtgattt taagaatttt tgtgtaaatg tgaggagagg gtttatattg ttagtagtgt 5880
gtgagatgtt tatatttatt atatatataa tttttatgta agaatgattt attatttagg 5940
atatttagaa attggttggg tatttgtatt aaattttatt tatattgtat ttatatattg 6000
gttaatatat atatatatat ttatatggtt tagttattta ggttagattg tatatgttaa 6060
ggtataattg ttatataatt taaatatttt tgtttttagg aattgtttaa attagttttt 6120
ataatagaaa tttaaaagtg tgaagaggga gagaagagaa aggaaatgag agatgaagaa 6180
agtttgttat tgaattaaat ttttggtttt ggggaaaatg attaaattta agagttttgg 6240
agaaattttg agggttggag ttagatagat tttttttgtg ggtatttatg ggaagtgggg 6300
gtatgatttg gttttttttt tttatttttt ttaatttttg tgtagattta ggaaattttt 6360
attgattgtt ttattttgtt ttttttattt ggtgaggttg ttgttttttt tttagttttg 6420
ggtgtttttt gttttttagt ttttggggag gagataggtt ggttttttat tgttggtttt 6480
ggttaatttt tttgttttat tggaggtggg ttgttatttg tttattaggg tttggttttg 6540
gaagtttttt ttttttgttt tttttgtttg agatggtagt tgggggagtg ttgatagtta 6600
gttttagaat agggagtttt tgtttgatga agtaaaggtt ttgtttatat tattttattt 6660
tttagtaagt taggaatata tatttataaa tgtatatatt attaatgagg tatgaatgtt 6720
atatatattg tggttttagt aagtattttt taagtgttag ttatatgatt attataatgt 6780
taaaaatata tgtttatttt gtaggtgaat gatagatata tatatttttt attttgttat 6840
taatatataa ttgatattgt atatatatat ttgtagtaga tatagattat ataattattt 6900
gtttatgggt taaattttta tgttagataa agtataaata gatatgtata tagtaggttg 6960
ttttttgttt tgtaattttt tttagttaga tagtggagta gttgggtatt aaaggtattt 7020
agatattagg tttgattagt ttttagggtt ttttgtttta gttgagtttt tttttttatt 7080
ttgtttttgt tggtttttgg taagttttag ttttagtttt gaggtgatgt tttttgagaa 7140

tttttgtggt ttggtttttt gttttgtagt tttttgtagg atgtttgtgg ggggtggggg 7200
gtattttgag tagaagttat ttgggtttaa ttatatataa taggatatta ttttgaggtt 7260
tttattgttt gattattgga gggttgaagt gagaggtttg taggtttatt tttgtggtgt 7320
aaaattgttt ttggtttatt agttggtttg gatttgttgt tatattgttg gttaggtttt 7380
gagatagatg gggggggggt agtgagggtt gggtgtgggg tttagtttta gttgagtagt 7440
agtttgtaag aattggtgga tttattttat tagaagttgg aagttattta ttttattaga 7500
agagtgaaag ttgattattt tttaatttgt ttgggagaat tagattgtaa ttaggttaaa 7560
tgtttgtgtg tttgtgtgga agtatggatt ttaatttgtt atttggggat tttagtaaag 7620
gaagagagag aaagaaaaaa gattaaattt ttgtagttgt gaagtatgtt taatttttat 7680
tgaaattttt aaatttttta tttatatttt aaggtaaatg aattttgatt attaaaaata 7740
ataaagtagt tattttttat atttgtatag aggttagtag tttgtagtgt atttttataa 7800
ttagaatttt atttattaat gtttggaata aatttttggt tgttatagtt gatttaaaat 7860
tgaaaagttt gagaataata ataaaaatat agttagttta tatatttagt ataattgtgt 7920
atgaggtaga atataaattt gaataggtat aaaaataaat taagttttat ttatagttgt 7980
gttttgtgaa tgaattatgg tagaaaaagt ttttttgttg atgttaaata gttgtaatgg 8040
gttagtgatg atatagaaaa taaataattt taggaaaata aagtagtagt gatagttata 8100
aatgtttgtt attttggttg tttttaattt ttgtttaatt tattttgaaa aagataaaat 8160
tttgttgaaa attttttttt tttaattagt tgttgaatta aggagtgata gaaataaaga 8220
agtttgtgtt aaaagaagga atataaaatt tatttgggaa ttgataaggt aaaaaataaa 8280
aataaaataa aaaaatataa taaaagtgtt ttagtttttt ttagaaaatt tggtttttta 8340
aataataagt tatagtataa ttaggtttat ggtttttggg ttgggttggt tttgggttat 8400
aagattgttt aggttggttg ttgttgggtt ttatattttt tttttttaag gtgggagaag 8460
taggaggttt gaaaaataaa aagtagggag gatgagttgt tttagtagtg tgggttaggt 8520
aggtagtgat ttttttgttt ttaggatttg tattgaaaga tttggggata tttgtttttg 8580
attttttttt ttagataggg agagggtgaa atttttttaa ttggtagatg aaattatttt 8640
ttattggata tattttttta attttgaata tagttttttg aaggtttaat aaaaatttta 8700
atgtaaattt tttagaagag aagatgagga atattaaagt ttttgatttt tatatttgga 8760
tttagtaaat tttaaggtta taatattttt ttggttgttt ggtgaatttt tttttttttt 8820
ttttttttta aatatttttt taaaaagttt tattgatttt ttaaataaaa tattagttag 8880
ttgttggggt ttggaggtgt ttgaagtgat gggattgata gtggaggaaa tgtagttttt 8940
ttttgtgttt gtttgtggtg taaattgagt ataggttgtg gagttaggtt gttttggttt 9000
ttgttgggtt ttaatttttg ttttgtttag tgggttttgt ggtaagtggt ttttgaatag 9060
ttttaagagg gtttgtggag taaatatatg tattggtttg tttttttttt gggtagtgtg 9120
gttttgttat tagttttgtt tggtgtgttt agttatggat tgtatggtag ttgggtgggg 9180
aatgtggaga gtgagtgtat tgatttgtga gagaaggtta agaggtttgt gttgttgatg 9240
tttggttgta tttttgtttt gggttttttt tgtggtgaat ttgggtagga gatgttgggg 9300
ttttggaaag agatgagttt agtagaaagt gtgtagagag gtagttttag gttaggggag 9360
tgtaaggtta tagaggttag ggaggtgagt ataggaggat ataaattgag gggataaaga 9420
ggagtgatag gagtttagga aagtgaaaaa gtatagaggg atttgggtgg ttggttttag 9480
aggtgggttt agagggtgtg aggttaggtt ggtggtggtg ttgttggttg tgattggggt 9540
tggtgttgtg tgtttttgta tttttgtatt tgtttgtatt ggtatgtggt gggtttttag 9600
agattgaggt gtgaatgtag tgtgttggtt ttgttgttgt ggttttagta agagtaatgt 9660
attatggtga gtttgggttg ttgggtataa gtgaattgta ggtttggtgt attggtgggt 9720
gtgggtgttg ggggtgtggt ggtggttggg ttggtagggt tgagttggtt tggtggtggt 9780
gtggtggttt tgtggtgtgg tggggtaggt ggtggtgtgg tggttgtgtg tagatggtgt 9840
gtgtgtggat gtgggtgggg gtgtggtgga ggtgggggtg tggttggtgg gttttttagg 9900
ttattgtatg agtttattat gttggggggt agtgttatgt tttgtatgtg tgtgtatggt 9960
ttgtatgagg tggttgggtt tgttagtttt ttgattatag tggttgtgtt agggttattg 10020
gggtttgtgg ttgtagttgt agttgttgta gttgttgtgg ttgttgttat ttggtaggag 10080
gtatagggta tgggtgaggg aggttgtggt agtggttatg agttgttgag gaagttagat 10140
tgtaggtatt tggggggtgt taggtagttg tagttgttgg ttttggtgtt tgttatgttg 10200
tatttgtttg tggtgttttt ggttttgaag agttttttgt tgggttggaa gtgtgtgggt 10260
ggtggttgga agggtttttt tatgtggtgg tggtgttggt agttgttttt tttgaatatg 10320
tttttgtagg ttgggtttag tgtttagtag ttgtttttgt gtttgttgtt gtttttgtgt 10380
ggtattttga tgaagtattt gttgaggttg aggttgtggt ggatgttatt ttgttagttt 10440
tttttatttt ttttgtagaa tgggaatttt gtgatgatgt attggtagat gttggatagt 10500
gtgagttttt tttttgtgtt tttgtggatt gttatggtga tgagtgttat gtatgagtat 10560
gggggttttt gtgttgggtt tggttttttt ggggttgttt tgttgttatt tttattgttt 10620
ttgtttgggt ttggtggtgg tttttttggt tttttgattg tgtgattggt ttttggggtt 10680
agtagggttt ttgttgtgtt tttgggtttg gggtagttgg ttattatgat aaagttggtg 10740
tgttgtggtt gggttgtttt tgttttttgt tttaggtgtt ggtgtggtaa agagttgggg 10800
tgtatgagtt tgtttatggt taagttttaa atttttggag attgtggatg ttgtttgtgt 10860
ttgtttgttg gaggtttgtt gttgtttttt tttttttttt tttttttttt agggagtggt 10920

tggtgggagt ggagtttagt ttttggttat ggggagtttg tttaatagag aggggttttg 10980
gttttgttgt ttttttttgt ttaggttagt ttttgttttg gtgggttttt ttttttgtgt 11040
tttttttttt tttttgtttt ttggtttttt gaagtatgat ttgtgttttt ggtggagttg 11100
ttttttggag tttttagtgt gttaggagtt ttgttttgtt ttgatttgta tgggttttat 11160
tgagttttgt ttgtgttagg tgtttttgtt tttatagtgg ggtggttagt tgtgtatggg 11220
tgagtttatt tttaagttat tttttgtaaa tgttttgtat agtttggatt ggtttgtttt 11280
tgtttagtga gttttagggg tttagttgat agttaggttt atgtgttttt gaaatttgtt 11340
ggtatttgtt ttgtgggttg ggttgggaga tgatgaggat tttggtgggg tttgtttgta 11400
tttggttaaa gtttaggaag tttgggtttt agtgaggaaa ggtgttttaa gtttttttgt 11460
ggtttttagg tgaaagaaaa tgattttttt gttttgttgt ttgttgttgt tttgaggttg 11520
aatttttagt ttggggttgg ggaggggtga gatggtgagg gggttggatg gggtagggtg 11580
gggagagttg ttttgaggtt ttgggaaagt tagtttagaa atgggtgtga ttgtatgaag 11640
aagttttggt ttggtttgtt ttttgtg 11667

<210> 31
<211> 11667
<212> DNA
<213> Artificial Sequence

<220>
<223> chemically treated genomic DNA (Homo sapiens)

<400> 31

tgtgagggat aggttaggtt gaggtttttt tgtatagtta tatttgtttt tgggttgatt 60
tttttaaagt tttagagtag ttttttttat tttattttgt ttagtttttt tgttgttttg 120
ttttttttta gttttgagtt agaagtttag ttttaagatg gtagtaaatg gtagagtaaa 180
ggagttgttt ttttttattt gaaagttgtg aggaggtttg gagtgttttt ttttgttggg 240
gtttgagttt tttgggtttt ggttgggtgt gggtagattt tattggggtt tttgttattt 300
tttagtttag tttgtagagt gagtattggt agattttaag ggtgtgtgag tttggttgtt 360
ggttgggttt ttgaggtttg ttgggtgggg gtaggttggt ttaggttgtg tggggtgttt 420
ataaaaagtg atttggagat gaatttgttt gtgtgtggtt ggttgttttg ttataggggt 480
gaaggtgttt gatgtaagtg gaatttggtg gagtttatat gaattagaat agagtgaggt 540
ttttggtgta ttagggattt taggaggtag ttttgttaga gatgtgggtt gtgttttggg 600
aaattggggg gtggggggag gggaagagtg tagaaaagaa aatttattaa ggtggggatt 660
ggtttgagtg ggagggggtg gtgaggttgg agtttttttt tgttgggtgg atttttatg 720
gttagaggtt gagttttatt tttgttggtt gttttttagg ggaaggggaa ggagagggga 780
gagtagtgat aggtttttag taagtaagtg tgggtggtat ttgtagtttt tagaagtttg 840
agatttggtt gtaagtggat ttgtgtgttt taattttttg ttgtgttagt gtttggagtg 900
gagagtagag gtggtttggt tgtggtgtgt tggttttgtt atgatggtta gttattttga 960
gtttgaggat gtggtggggg ttttgttggt tttagagatt ggttgtatag ttaaggagtt 1020
agaagggttg ttgttgagtt taggtaaggg tggtgggggt ggtggtggga tagttttgga 1080
gaagttggat ttggtgtaga agtttttgta tttgtatgtg gtgtttattg ttatggtgat 1140
ttgtgagagt gtggagaaga ggtttatgtt gtttggtatt tattagtata ttattgtgaa 1200
gtttttgttt tatgagaaga ataagaaggg ttggtaaaat agtatttgtt ataattttag 1260
ttttaatgag tgttttatta aggtgttgtg tgagggtggt ggtgagtgta agggtaatta 1320
ttggatgttg gatttggttt gtgaagatat gtttgagaag ggtaattatt ggtgttgttg 1380
ttgtatgaag aggttttttt ggttgttgtt tgtgtatttt tagtttggta aggggttttt 1440
tggggttgga ggtgttgtag gtgggtgtgg tgtggtgggt gttggggttg atggttatgg 1500
ttatttggtg ttttttaagt atttgtagtt tggttttttt aataatttgt ggttgttatt 1560
gtagtttttt ttatttatgt tttatgtttt ttgttagatg gtggtagttg tagtggttgt 1620
agtagttgtg gttgtagttg tgggttttgg tagttttggt gtggttgttg tggttaaggg 1680
gttggtgggt ttggttgttt tgtatgggtt gtatatatgt gtgtagagta tggtgttgtt 1740
ttttggtgta gtgaatttgt ataatggttt gggaggtttg ttggttgtat tttgtttttt 1800
gttgtatttt tatttgtatt tgtatgtata ttatttgtat gtggttgttg tattgttgtt 1860
tgttttattg tattatgggg ttgttgtgtt gttgttgggt tagtttagtt ttgttagttt 1920
agttattgtt gtgtttttgg tgtttgtgtt tattagtgtg ttgggtttgt agtttgtttg 1980
tgtttggtag tttgagtttg ttatgatgta ttgtttttat tgggattatg atagtaagat 2040
tggtgtgttg tatttgtgtt ttgatttttg agagtttatt gtatgttggt gtagatggat 2100
gtgaggatgt agggatgtgt gatgttggtt ttggttgtag ttgatgatgt tgttgttagt 2160
ttgattttat attttttggg tttgtttttg gagttagtgt ttagggtttt tttgtgtttt 2220
tttgtttttt taagtttttg ttgttttttt ttgttttttt agtttatgtt tttttgtgtt 2280
tatttttttg atttttgtga ttttgtattt ttttggtttg aagttgtttt tttgtgtgtt 2340

ttttattggg tttgtttttt tttggagttt tagtgttttt tgtttaaatt tattgtggaa 2400
agggtttggg gtggaggtgt gattgggtgt tggtagtgta gattttttgg ttttttttta 2460
taggttggtg tgtttgtttt ttgtgttttt tgtttgattg ttgtgtagtt tatggttaga 2520
tgtgttggat aggattgatg gtgggattgt gttgtttgag aaagggatgg attaatatgt 2580
gtgtttgttt tgtgaatttt tttgaagttg tttagaagtt gtttgttgtg gggtttatta 2640
ggtggggtgg gggttgggat ttagtgggag ttggggtagt ttggttttat ggtttgtatt 2700
tggtttatat tgtgggtggg tgtggaggga ggttgtgttt tttttgttat tagttttgtt 2760
gttttgggta tttttgggtt ttggtggttg gttaatgttt tgtttgaaag attggtggaa 2820
ttttttaaga gagtatttaa aaaaaaaaaa aggaaaaaaa atttattggg taattgggga 2880
agtattgtgg ttttggagtt tgttaaattt aaatatgaaa attaaaagtt ttagtatttt 2940
ttattttttt ttttggaaga tttgtgttag agtttttgtt gggtttttaa aaagttgtgt 3000
ttagagttag gagaatatat ttaataaaag atggttttgt ttattaattg gggaagtttt 3060
attttttttt tatttgaaga aaaaaattaa aaataaatgt ttttggattt tttgatgtaa 3120
gttttggagg tagggagatt attgtttgtt tggtttatgt tgttgggatg gtttgttttt 3180
tttgtttttt gttttttaaa ttttttgttt tttttatttt gggaaggaga aatgtgaaat 3240
ttggtagtgg ttgatttagg tggttttgtg gtttggagtt ggtttggttt gaaaattata 3300
gatttggttg tattgtagtt tgttgtttgg gggattaaat tttttagaga gaattagagt 3360
atttttgttg tgtttttttg ttttgttttt gttttttgtt ttgttgattt ttgaataaat 3420
tttgtgtttt tttttttaat atggattttt ttatttttgt tgttttttag tttagtagtt 3480
agttaaaaga ggaagatttt tagtaaaatt ttattttttt tagaatgagt taaataaaga 3540
ttgagagtaa ttggggtggt aggtgtttgt ggttgttgtt gttgttttgt ttttttggga 3600
ttgtttgttt tttgtgttat tattggtttg ttgtggttgt ttaatgttag tagaaagatt 3660
ttttttgttg tggtttgttt ataaggtgtg attgtaggta ggatttggtt tatttttgta 3720
tttatttaag tttgtatttt attttatata tagttgtatt aggtgtatgg gttggttgtg 3780
tttttattat tgtttttaaa ttttttgatt ttgagttaat tataatagtt aagagtttgt 3840
tttaaatatt aatagataaa attttggttg tggaaatgtg ttgtaaattg ttaatttttg 3900
tataaatgtg agaagtgatt gttttattat ttttaatagt tagaatttat ttattttgag 3960
atatgggtag gagatttgga ggttttagta aggattgaat atattttgtg gttgtagaaa 4020
tttagttttt tttttttttt tttttttttt tattgagatt tttaaataat gaattaagat 4080
ttatgttttt gtgtaggtat ataggtgttt gatttggttg tagtttggtt tttttaggtg 4140
gattaggggg tggttggttt ttattttttt gatgggatag atgatttttg atttttgata 4200
ggatagattt gttggttttt gtgggttgtt gtttagttga gattagattt tgtatttagt 4260
ttttattgtt ttttttttgt ttattttggg gtttggttag taatgtggtg gtaggtttag 4320
gttggttggt gggttaggag tggttttgtg ttatgggaat gagtttgtag gttttttatt 4380
ttagtttttt agtggttagg tggtaggaat tttagggtgg tattttgtta tgtatagttg 4440
ggtttaagtg gtttttgttt agaatgtttt ttgtttttta taggtatttt gtgagaggtt 4500
ataaggtagg agattaagtt atggggattt ttagggggta ttattttaag attgggattg 4560
gagtttatta ggaattggta ggagtaggat ggagagggag gtttagttgg agtaggaagt 4620
tttagaggtt ggttaggttt ggtgtttggg tgtttttggt gtttggttgt tttattattt 4680
ggttaagggg agttgtagaa tagaaagtag tttgttgtgt gtatgtttgt ttgtgttttg 4740
tttagtgtga aaatttagtt tatgggtaag taattgtgta gtttgtattt gttgtaaatg 4800
tgtgtgtata gtgttagttg tgtattggtg atagggtggg aggtgtgtgt gtttgttatt 4860
tgtttgtaga gtgggtatat atttttggta ttgtaataat tatatggtta atatttagga 4920
agtgtttatt gggattatag tgtgtgtggt atttatattt tattggtgat gtgtgtattt 4980
gtgggtgtgt atttttggtt tgttgaaaaa taaagtaata tggatagagt ttttgtttta 5040
ttaggtagag gttttttgtt ttgaagttgg ttgttagtat ttttttaatt gttgttttaa 5100
gtagagaaag taagggaggg aagtttttag gattaagttt tggtgggtag gtagtagttt 5160
atttttagtg agatggagaa gttggttggg gttagtaatg aaggattagt ttgttttttt 5220
tttgagaatt gaaaggtaaa aggtatttaa ggttgggaag agagtagtag ttttattaag 5280
taaggaagat aaaatgaagt aattagtgag ggttttttgg gtttatatag ggattgaagg 5340
aggtgagaga agggagttaa attatatttt atttttttat ggatgtttat aggaaaaatt 5400
tgtttaattt tagtttttgg gattttttta aagtttttaa gtttggttat tttttttagg 5460
gttgaaagtt tagtttaatg gtaggttttt tttatttttt attttttttt tttttttttt 5520
ttttttgtat ttttgagttt ttattataga agttgattta agtaattttt gagggtaaaa 5580
gtatttgagt tatatagtaa ttatgtttta gtatatatag tttggtttag atggttgggt 5640
tatgtgagtg tgtgtatata tattggttag tgtgtaaata taatgtaaat agagtttaat 5700
ataaatattt agttaatttt tgaatgtttt gggtaatgag ttgtttttgt atgaagatta 5760
tgtgtgtggt gggtgtaaat attttatata ttgttgatag tatgagtttt ttttttatat 5820
ttatatagaa atttttagag ttatttttat tttattaaaa ttgtttagtt agagtaagtg 5880
ggttggtttg gagaaagtag ggggaggtgt tttgttttta agagggattt ggagaaattt 5940
ttgttattga agtatatgta ttaagggatt gtgggaaagg gatttatgga gttttggaga 6000
tggttttgta ttttatttta gagggtattg atgttttgtt tttagtttgt attagtaaat 6060
agtttattgt tttagatttg aggtttagat gagtaagagg ttttggattt tttatttgag 6120

gttaggtgta tttagttttt gttagggttt tttggaagtt aaggttggag atggggttgt 6180
aaggagtttt ttggtggtgg gtggaaattg gggttagatt taggttttat gtagttagat 6240
aagtagaata tgggttagga atttgagttt ggtttaagat agtttgtttt taattgagtt 6300
ttttttttta tgaaggtttg ggttaggttt tatggatagg attattggat tagatttttg 6360
gtttttgggt gggggtttaa ggttaggaaa tattttgatt aggtttagta tttttgtagt 6420
gtttttattt tgtagattta atttggtggt gattttgggg ttttgtttgt taggtttatt 6480
attattgtgt ggtagttttt atttttggga gtagattgtg ggatttatag aaaagatggg 6540
gaaaggatgg gattaagagt tttatgtttt tgggataatt aggaattaag gatttttaaa 6600
attgagtagt tttagtaatt ttttattttt tggagagagg atttttagtt gttataaaaa 6660
gagtgttttg ggtttaggaa tttaaaatta tattatattt gaggaatttt aggttttaag 6720
aagagaagag attaattaga ggttttttag gtatttgtga ggtaaatggt ttttgaaatt 6780
tttaaaggag atttgatagt agttgagttt agttgttatt gatgtttaag ggagttgtgt 6840
agtaagggag agttttttag gagtagtaag ggagagtttt ttaggagaag aaggaagtat 6900
agtttgagtt gtgttttttg ttttttttgt ttgtttttgt attatagaaa ttgttgttgt 6960
ataggttgtt tgttgtatag gtttggtgtt gagttgtgaa aagtatgttt tgggattttg 7020
ttattttgtg gaggtggttt gtgtagtatg aggtaaagat ttggtttgtt tagttaggaa 7080
gaattgggtt ggagtttggt gtttttttta atagttatgt aattttgggt aaaatattta 7140
tttaaatttt agtgtttttt tttataaaat agaaatatgt gtgtgtatta gaattattaa 7200
aataattaaa ttttttaatt tttaagtgtt tagtttatag tagatgttta ataatgtttt 7260
tttttttttt ttattaaaaa aaaaaaagga aggaaggaag taaaaaagga aagaagaaag 7320
gaagggggaa aaaaaatttt tagtaataat tttttaggat ttgatttttt aatttagttg 7380
gttataggta ttttttgtga gtattttttg gggttaggtg tatgtgattg tatttatttt 7440
gtttagtttt tttggttgtg ggagtgaggt ttatgtggtt gttgttgtag tgaatattta 7500
agttttgaag gtatagttgg tgggtgttga gatttgtttt ggggtttttt taattttttt 7560
tttgtttttt ttaaagtggt ttttatatgt ttaggaggtt ttttttgaga aaggttgtgt 7620
tgatttgtga agtggtattg atttagggat ttatttatag gttgtttggt tggtgttttt. 7680
agtttttatg tgtttttttt gattggttga tttttttttt tttttttttt tttttttttt 7740
tttttttaga aaagtagaag gtagttattg ggttagaggt tgtagggtta gtttgtgggt 7800
gaggtttttt taggtttttg tggagaaatg ggaaattgga ggggtaaatg taggaagtgg 7860
gttgggttaa ttgtgggtga ttgaggtgtt gttagtgggt ggggagtgag aggggttagt 7920
agttgggaag atttggtttt ggagtgtttt gtgtttttgg gtttgttggt ggtgttgtaa 7980
gtttaggttt tggggtgtga gttaagtttt ttttgttagt tttaaataaa atttggggga 8040
gaatgagttg gttagtttgg gtatagggtg tttttgattg ttgggtggtg ggtggaagtg 8100
taggggtttt gattgttttt agaggtgttg agtgtatagg gtagtggttg tagagttttg 8160
gtgtgaggtt aatttggatt atttatgttg ggattttagt ttgtttggtt gtttttagta 8220
ataataatag tatgaaattg agtttaaaaa aatttttttt tgtatttttt tttatgtata 8280
agggagaata agtattttta ggtagttaaa gaaaaaaaaa gggtaggatt ttaggaaatt 8340
ttaattttta tggatgagga gtgaagggga atttgttttt tgtttttttt gtttgtttgt 8400
ttttagaaga gaaaaaaagg aaatttgata tatattttta aaaatttttt tttaataatt 8460
gttaaaggtt gtgagtgtga gtagagagtt aggaaattga gtttttgttt gtggtgatat 8520
tgggtttttg tttttttttt tgaagttttt gtgattgttg attgtgtaga gataaaggtt 8580
gggttagggt tttgtggaat tttgagggag gggtggtggg gttgttttat tggttttttt 8640
taattttatg taggggggtt aggtgggttt agttgatttt tggaataggg gttataaggt 8700
taggggttgt ttttattttt aggtttagat gaggtatgga tttgtttttt tttatttttt 8760
tgaaaggttt ttgttgttaa attttttgta ttttgttttt attagtatta tattgtttta 8820
tttaaagttt ttggaggtgt tagggatagt ggttttttat tggatttgat ttttagaaat 8880
ggaattgtgt ttaatttttg tggtgatttt ggtttttgtg attgttgggt ttggttggtt 8940
ggttagattt gaatttgtgt attgtttgtt ttagttgtgt tttttagggg tgtttttgtg 9000
ttttttttgg gttgtggaga tttttttagt gtttggtttt gaattttttg tgtaatgttt 9060
ttttgttagt tgtatggttt tgtagagaag gtgggagagt gtttgtgatt ttgttttgta 9120
gtttttggtg atttaaaaat ttaggttgtt gtggaattta gtttttatgg tattgttttt 9180
gtaggtagta gttttgggtt tatatttgag gttgtttttt ttatagatat ttttgggtga 9240
tgtgagtgtt gtgtagttta gaatttttgg aagtatgaga ggaggatgag tttggtggtt 9300
tgttggtgtg gtgatggagg gttttttggt agttatattg tgtgtttttt atatatattt 9360
taatagtttt ggggttttgg gttagtgttg tagtttaaag agattttgta gttgtatttt 9420
tttttaggtt gtggtaaatt ttggttttgt ttttttttat tatggtttgt gagggtagga 9480
ggtttttggg ggttgatttt tttgatttga atgagttagg tatttttttt tattttaagt 9540
gggggtgtga tttgaggaga taaaggaagg atgatagatt tggggagtgg ggttttgtag 9600
ttttttgggt ttggagtgta tttttttttg tttttttttt ttattggtag gattgggggt 9660
tttgaaattg gagttgttta ttggttggtg gatgagagat aaaggagttt ggtggtgttg 9720
aagtgttgat tagtagattt ggatttggat ttttttgtgt tttagagtag tatttgaaat 9780
tttatttttt aggtggtatt gatggttgag tagttttttt ggtgttatat ggtgttttta 9840
ggtatagata gttgtgtttg tgtgtgtatt gtgtatgtat aagattgtag gatgatgttt 9900

tgtatgagtt ttgtattagt ttttttgttt ttagttagag gtatatgtat atgtgtgtgt 9960
atttgttaag tgtttaggga taggtgtgta tttttatata tatgtgatgt gtatgtgtat 10020
atatttgtag aatgttttag tatttatttt ataggtatgt atagggttgg ttgtttttaa 10080
taagttatta ttatattttg tatttatata ggttttttta gttttggtat gatttttaga 10140
attattttgg gaaatatttt ttttttaaag tttatagatg aggaaataag taaaaagaat 10200
ttaatgggta tttgtttgta tatattgtga gagagattga ggttagttaa agattttgtt 10260
tattgttgaa tttgggtgtg ttggttgaat gtgtttatag atgggtttgt ttagtgtttt 10320
aggtgatttt aggtttggtg ggttttggtt ttttgttagg ttttttggga gatttattgt 10380
gttgggtgtg gggttttaat ttttgtgttg ataagtatta ttttttggag ttgggtgatt 10440
ttagtgttta gggtgggaag ttgggagttg gttggagtgt tgttgaggtg attttggtgg 10500
ttttaaggtt atggttgatt atgtgtaatt gggttgtaaa gtttggtggg ttttggtttt 10560
taaagtagtt atgttaggta aatgtgtgat gaaaggattt ttttagttta tttataaatt 10620
taggaattta gggttgttgg agtagtgagg aaatttttgt tgttttgaat ttttagtgtt 10680
tttatgagat ttggagttta taatgtttat tttatagaag tgattagttg gaggttagag 10740
tggtatagtg agttgtttaa gtttaatgtt agttgagtgg ttatagttgg gattggaatt 10800
tttaattaag tgtgtttgga tttttggttt atagtttttt tgtttttagt aaggattgtt 10860
ggtttttttg ttttttgtgt gtttaggagt tattttgtta ttagtttttg attttttttg 10920
ttaagaatgt gaaaattatt atagttttta ttagtattat ttaaaaggtt tttatatgtt 10980
tggaatttgt tgtttgaaaa taaatttttt gtgtgtgttg gttttttaat tttttttaag 11040
gtatttggtt tttttttatt ttttaagttt ttgttgttgt agagtgaggt tgtttaaggg 11100
ttgtagtttg attatatttt gggggtgttt ttgggttttt gtgttgggtt tttgtagatt 11160
gaggttgttg gtttttgggt tttaaggttg tttttatttt gtagaatttg ggagttggaa 11220
gggagtttgt ggtttggtga ggttgttgtt tgggtttatg gttttgaggt tgttttttgt 11280
tggtattggg tgatattatt attgtttttt ttggtggttt aggagttgta tttatgtgag 11340
gggtttgtgt agagttaggt gattttattt ttattgggtt tttagagtgg ttgttgtatt 11400
tgtagttatt gatgagattt ttttgtattt tttaatttga gatttttttt tgtagatatt 11460
agaggttttg gtgtttggat ttagggataa ggtagagagt tggagttttg tgttggtaat 11520
tttgttgttt tgtggtgttt gttgggattt gtggttaatt aggtatggtt ataggtggat 11580
gttgggggat tttggggatg gttttaggtt gtgtttgttt tttttatttt ttgttattat 11640
agtattgatt ggtgtttatg ggttttg 11667

<210> 32
<211> 1394
<212> DNA
<213> Artificial Sequence

<220>
<223> chemically treated genomic DNA (Homo sapiens)

<400> 32

tggaattttg gtttgaagtt gagataggag attggatgtg aggttttttt agagttggtt 60
ttttttaaat aatttttaaa atttttagat tttaggggta tgttgaaatt ttttaaagta 120
gtttaaagaa tataatgaga gttttaatat tttaggtggt ggtgtgttgg ttttttggag 180
tggggtggga tgtggttgtg tggatttatg tgtataattg tgtgggatgg ggttatgtgg 240
atttatgtgt ataattgtgg gattttagtg ttagtgggat tttagtgtta gtgggatttt 300
agtgttagtg ggattttagt gttagtggga ttttagtgtt agtgggattt tagtgttagt 360
gggattttag tgttagtggg tttgtggttt agtggagtga gtggagtgtt ggtgatttga 420
gtggagattg tgttttggat gttttagttt agatgttaag ttatagtttg tgtagtagta 480
gtaaagggga aggggtagga gttgggtata gttggatttg gaggttgtga tttaggggaa 540
agtgtgggtg gttgatttag ggtagttgtg gtggtgaggt aggtgggttt tttgtttttt 600
ggagttgttt ttttttatat ttgtttttgg tgtttttagt agtttttatt ttggtttttt 660
gtggttattg tgggatttgg tgttgttgtt agtttagtgg ggagtgaatt agtgtttttt 720
tttgtttttg gtttttttga tggtatgagg aatttttgtt ttgttttata gatttttggt 780
ttttgttgag tgtggtattg gagtttgttt tgttagggtt ttggaattag agaaagttgt 840
tttttggtta tttgaagtgt tggattttta tagtgttttt tagtttgggt gggagtggtg 900
gttgtgttgt tgaaggttgg ggtttttggt gtgaaaggga ggtagttgta gttttagttt 960
tattttagaa gtggtttttg tattgttgtg gtgggtgttt ttgggttttg attttgttag 1020
tgttgtgggg tagaggtatt tggagtttgt agggtttaga tttgggttgg aaaagttttg 1080
ttgattgtag gtaagtgttt gggaggggtg gttaggtgaa gttttggtgt tttattatat 1140
atttttgggt tttatgttag ttgtatttgt ggtattgggt aggaaatggt agggttgagg 1200
ttgattttag gagtataagg gagtttttta ttttttgttt atatttgtta tttttagttt 1260
tgtaatttat tttagatata tagaaagtaa gtaggattgg tggggagatg gagtttaata 1320

ggaatatttt ttagtagtga gtaggggttg tatgggatgt gggaggagtt tagaggaggt 1380
gtggagagtg tttg 1394

<210> 33
<211> 1394
<212> DNA
<213> Artificial Sequence

<220>
<223> chemically treated genomic DNA (Homo sapiens)

<400> 33

tgggtatttt ttgtgttttt tttgagtttt ttttgtgttt tatatagttt ttgtttattg 60
ttggaaaata tttttgttaa gttttgtttt tttattagtt ttgtttgttt tttgtgtgtt 120
tgggataggt tgtaaaattg gaggtgataa atgtgggtag gaaatggagg gtttttttat 180
atttttaggg ttggttttag ttttgttatt ttttgtttaa tattgtggat gtaattggta 240
tgggatttgg aagtgtgtgg taaagtgttg gggttttgtt tggttgtttt ttttggatgt 300
ttgtttgtag ttagtgaagt ttttttaatt taggtttggg ttttgtgagt tttaggtgtt 360
tttgttttgt ggtgttggtg aagttgaagt ttgagaatgt ttattgtagt gatgtgaagg 420
ttgtttttgg ggtggggttg aggttgtagt tgtttttttt ttgtattaag gattttaatt 480
tttagtgatg tagttgttgt ttttgtttag gttgggaggt attgtaggga tttgatgttt 540
taggtggtta aagagtgatt ttttttgatt ttagggtttt ggtggggtag gttttagtat 600
tgtatttggt ggaggttgaa ggtttgtggg gtaggatagg agttttttgt gttgttggaa 660
gggttgagga tgaaggaggg tgttaattta ttttttattg ggttggtggt aatgttgaat 720
tttgtagtga ttgtggaggg ttaaggtgaa aattgttggg ggtgttgagg gtaggtgtgg 780
ggaggggtgg ttttagggag taaggagttt atttgttttg ttgttgtagt tgttttgggt 840
tgattgttta tgtttttttt tgggttatga tttttggatt taattgtgtt tggtttttgt 900
tttttttttt ttgttgttgt tgtgtgggtt gtaatttgat gtttaggttg gggtgtttag 960
ggtgtagttt ttgtttaggt tgttagtgtt ttatttgttt tattgggtta tagatttgtt 1020
ggtgttgggg ttttgttggt gttggggttt tgttggtgtt ggggttttgt tggtgttggg 1080
gttttgttgg tgttggggtt ttgttggtgt tggggttttg ttggtgttgg ggttttgtgg 1140
ttgtgtatgt gagtttgtgt ggttttgttt tgtgtggttg tgtatgtgag tttgtgtggt 1200
tgtgttttgt tttgttttag ggagttagtg tgttgttatt tgggatgtta ggatttttgt 1260
tgtgtttttt ggattgtttt gggggatttt ggtgtatttt taggatttag gagttttgga 1320
agttgtttga gagaaattag ttttgggagg gttttgtatt tagttttttg ttttggtttt 1380
ggattggggt tttg 1394

<210> 34
<211> 6357
<212> DNA
<213> Artificial Sequence

<220>
<223> chemically treated genomic DNA (Homo sapiens)

<400> 34

gtgtggagat tgggaaggtg ataaggtgaa ggtaattgaa ggaagagttg agggggatat 60
ggggaaggat tttgttttat tttttttaag ttgaattatt gttttttgaa ggttggtttt 120
tggagaaatt aaagggtttt tgtgtgatat agttatgtta tatataaata gaattttgaa 180
gtttattaat ttttgaggtt aagtaagagg gaatgtaggg gttaaggtag aagagaaatt 240
aaaattttag agtgttgagt aaagatgtta attagagaaa gagaaattta tttgtgatgt 300
taattaataa gtggttaatt aaaatggtat tttgagtgtt aattaattgt tttattaagt 360
tatagttatt attggaataa attgaaattt ttttgttttg ttttttgttt ttggtgtagg 420
tggggttgtg tttttagata ttgtgagagg ttttggggtg tggaggttgg gggtagtttt 480
ggttagtttt tttagttttt tttaggttta tagaatatgt tattggataa gtgtttaagt 540
agtgattttt ggtttagata tattgtttgg ggagtaagta gttgtgttga agaataattt 600
atttagtagt agttaatatt gatgtttttt tttagaaaga gtttttgtaa agtgggggga 660
tgtgatttgt gggtttttag taggggtagg aggtagttta gtttgagagg gggtgtttta 720
gggtttggat tttgtatttt tattttttgg aatatattta atgttttatt ttgaaaattt 780
tgtttaggtt ttggttttgg tgttgtttag taattaggaa agagttggat ttgtttttag 840
gtagtaagaa taggattgtt agtttttttgt ggtttgtttt tttgaggttt tatgagaagg 900

ggatgggggt gtaagaaggg aagagtgagg tggtgtgttg ggtgttgggg atgaggatgt 960
atgttagtta agatgtgttt tttatttagt ttatgtgtgt ttttttattt ttttggtttt 1020
ttaaaattgg taagagaatt aagattttga atttttattt tgaggagttt tttgtatttt 1080
ttaattgtta aatttttgtt ttttattaaa tttttggggg agaaatattt ggtaataaga 1140
agggattgtg aatttaaatg ttaattgagt gggttttttt tttgtagttt tatttgtttg 1200
gtagtttttg ttgaaattaa atatatttgg agtgtttagt gtaatatttt tggggtgttg 1260
agtagaagtg tagtaaagag agattttagt ggatttttgt ttggtttgtt ttttattgat 1320
ttttatagaa aaaaagagaa tgttattgga agaagttttt ttgtgtggtg ggtgatgtgt 1380
gggtggggga tttgtggtat ggtttatggt gttgtttttg tgtttgtgat gatatatgta 1440
tgttttgagt tgtgggtttg tttttttgga ggtgtgtttg attgtatttg ttggtgggtt 1500
tgagtgtgtt tggggtgttt taggagaatt gagagaatgg tttttatgtg taaagtttta 1560
aagtattaat atttttatta tattattatt atttaatata ataatatttg tttggttagt 1620
ggtattaatt aggttatatt aaaattgtag tgtgttttta atggtgtgta atgtgtttat 1680
atttatattt ttttttttga ggatgggtgg ttgtaggttg gtaggggagg agagataggt 1740
aagtggtggg ttggattagg gtgtgatgtt ttttattatg tatataaata tatatagttt 1800
attggatgtt tgttgggtgg gagttgtaat ttttgtgtgg ttgatggggt tttttgttgt 1860
gtatttggtt ttgtgttgag tattttgtag tttttttttg tgatatggtg ttttgaattt 1920
ggtggattga ttttgttttt tttttttttt ttgtgtgtgt ttgtgtttaa ttggttaggt 1980
ttttaagatt tgggagggtt ggtgtgaaag aattaaaata tttttaattg gagttttttt 2040
gttgagaatt ggaggtttg tttttagtt tggtgttttt aggatttttt ttttagaggg 2100
aatttttttt agaaatttta gggtgggttt gtaaaagatg tttttgtaga gtaggttttg 2160
ttagggtttt ttttttgttt ttggtgttag tggttggttt gggtgttttg tagattttgg 2220
tgaggtagat gttaagtttg gagagtgttt tttttgtagg tgttgtggtg agattatttt 2280
gaatatgtaa tatatttgta atgtgtgttg aggtgtgatg tgtgtgttga aataggggga 2340
tgggggaatt tgaagttgga ttgggaaggt gggggggagg tgtatagaat ttataatgta 2400
ttttgtaatt taataatttg aatatttatt tattaaaagt tgttgtgtga tatttatatt 2460
gagttattag tttttgtttt taatttgggt gaaaatgatt gtattgttga gttatggttg 2520
tagtgtatgg ggatgttgtt gtttgtggtt ggatagagtt tattagttat aatgtggaag 2580
gtttttgtat ttttttgggg gtgggaggaa agtattgtta gttttgtttg ggggttgagg 2640
gtaataagta ttgagttttt tgttttatgt agggttagtt gtttagttta gtgaagtttt 2700
tgtgatttgg tgtgtgtttt ttgttttttt ttttttatta aagaagtaaa ttttttattt 2760
atttttttta atttgattgt ttagagttgt tgtttttttt ttgttagatt tttttttttt 2820
gattagtttg agtatatgat tagaattgtt tagagagtag gaagtatatt gattttagtt 2880
tgttttgttt atagataggt tttgataagg ttgttagaat agttggagag gtttatataa 2940
ttatttaatt attaaaattg ttagttaggt gggatgtgga tttgtgtttt gggttgtgtt 3000
aggtatttta gtattgggtt gtgtgtgtga ttgattggtg ttgatagtat tgtaaaataa 3060
ttatggtgaa ttttttgatg tgtgatttta ttttaagttt atgttttaga gaggtaattg 3120
gagaatgaga agggttagtg ttattttgga ttatttggaa tttgtgagaa agggtaaaat 3180
gggggaagga gttttgagga aaatgggaga gatgggggtg tagagagaga gggaagaaga 3240
aagtgagtta tggattgttg gagggattgt aagtaatttg ttaaattgtg taagtgattt 3300
tttttagagt tagtatatgg tagattgatt ttgtttaatg ttggttttag ttatatttaa 3360
aatgatttag tggttattat tgtgattggt ttaggaattg ataggtagtt ttaggtgtaa 3420
ggagtataga ttttgtttat tggagatgtg tttgtaattg ttgttaaata tagttaagta 3480
aatattatta gtgaagagtt ttgttaagag aaatgttaat ttaataaata tgtttttttt 3540
ttttgttttt tgtatggttg tttgtgtttt ttttagaggt tttttttttt gtttttttgt 3600
tgtttgggtt agatgtttta ggtatggtgt tgatttttgt tattttggag ttttgagttg 3660
agttttgggt agaagatgat aggttagttg tggggtaagg aggttgtgga aatgtggaat 3720
ggttttgggg agatggaagt gtttaatgag atttattttg tagtttgggt ttagtttatt 3780
ttttttggag attgttgtgg tttttgaatt tgggtttagg tttttatgtt ttggtggtta 3840
gaggatgttg tggggattat tggggagttg tttttagtta gttttttgtt ttatgttgga 3900
ggttttggtg tggttttttt tttgaattag attggtgatt ttgggttagg ttttaaggat 3960
tgttttggtt tttttggttt tgtggggaga atttgaggaa ttgagtttaa gatagttgat 4020
ttaggttgtt tttatttaga ttttgtgttt ttgatttgtt ggagtgaatt tgatattgtt 4080
aggttttttt ttatggtatg gagtgaatga agagggttat agattttttt atttagtata 4140
gttttttggt aggttttgga aatttatagg gagtagaagt atagtatttt ttgaattgtt 4200
tttttttttt gggtttgtgg ttatttgaag gtagagtttt gtgtttttaa gatagtaggt 4260
ttttggttaa gtttggagtt tggggtttta atatatttat atagggttgg tattattgtt 4320
tttttggatt aaaggtaggt ttttatattt tttttaaagg aatagaagga aggaaaagga 4380
aattaatatg ggttatgttt agatagtagg tttatggtaa ttttttatag ttatagagtt 4440
ttaatttgag tattttttta gagaggaaaa atttaaaaaa tttttaaaag ggggaaagtt 4500
gggtagatta tagtatttat tttttatgtt taggtagaaa aattaattta gagggagtaa 4560
aggggtaaga aatatgaaga gatttttttt gggagttgag gagtatttta gtttataatt 4620
tggttaaagg agaaagttat tggttttttt ttttgataga ggtgtgttat ttattttttt 4680

agggaatatg atggtttata aatgaagagg ttagtttttt tgtagttttt ttttatagag 4740
tgtaaaatat atattgtttt tattagtgtt tgggatgtaa agaattttgt ttatttaaaa 4800
gagatattgt atttttaaag ttaaatagta ttaatgtatg tggtgaatta agtaggtaaa 4860
taatttatat atggttgttg tatttgaagg aattatttat ttttatgtat agtaaattga 4920
agaaataatg gtattaatga gttttgtaaa atgtaattgt gaataatgaa agataatatt 4980
gtattttgta atagaaagaa taaaggtgaa ataattagtt agtaaagagg aaaagaaagt 5040
gagtaatgat taaatgatta aaagttggta gagtgaattt aatgttattg ttagatgtag 5100
ttatttattt ataagtgaaa gttaggtttt aagtatagtg taattatagt tggggttgtt 5160
agtttgatat taatgtagtt agtagaaatt ttttaattgg ttttagagga gaaagtgaat 5220
tagaaaatat attaatattt taaaaaagta tattttgttt aattttttta tttttgaata 5280
atatttgaag attaaaatgt tttaggtata agaatttaaa tgagtaattt tgtttttgaa 5340
ggaaatggtt aatgagatag aaaatagatt aaagggaaat tattagtgga tgagagatat 5400
tgataggttt gttttgttga ttggttggtt tgttatttgt agtttgtgtt ttttagtttt 5460
atgttatgag ttaagttgat aatatgaaaa gatttataaa tgtgtagtta gaagttatag 5520
tttattattt ggaaatttaa atgtaagggg agggggtggt agagaaggta ttggtgaggt 5580
tgggagggag aggtgtgtat tgagggagga ggaggaggag gaaggggagg agggaaagga 5640
ggaggaggag gagaaaagaa gtttttattt tttggtataa aattggttat attagagaat 5700
aataataagt tattatttgt tataaatgtg ttatggattg ttaaaaaatg tagttttgaa 5760
ttgataatat tgtttgaatt gaagatagta ataaaatgtt taaagttgtg gatgtaattt 5820
tatatgtgtt tgggttgatg tgatatgatt gtattaggga aataaagtta agtgtagtta 5880
ggatttgtta gtatagtggt ttttgatgga atagttgagg tatatattgt ttgtggtatg 5940
gattttgggg ttgaatgttt atgattaaga tttttgtttt tttgaaatga aatagaaata 6000
ggggagttgt aggaaaattg aattgtgttt agggttagga gtaagatagg agtttttagt 6060
gaagatttga atatttagaa ttggaatggg taattagtag atagttagga aaaaataaat 6120
aaataaataa aaaagtttgg atggattttt gtaaataatt attaagaaaa ataaaaatga 6180
atttttttat tagtttgttt tggaggtagt tagaaataaa taattaaagt aagagaggat 6240
gaagatttaa ataaaataat tatgtgtatt attaaaataa ttatatatgt ttgtatagat 6300
atgtatatat taaggaatgt aatgggggtt ttttgtatag ttttaggaga tgtagga 6357

<210> 35
<211> 6357
<212> DNA
<213> Artificial Sequence

<220>
<223> chemically treated genomic DNA (Homo sapiens)

<400> 35

ttttgtattt tttgggattg tgtgaggagt ttttattatg ttttttggtg tatatgtgtt 60
tgtataaata tatatgatta ttttaatgat gtatataatt attttattta aatttttatt 120
tttttttgtt ttggttgttt gtttttgatt atttttaagg taggttaata agaaggttta 180
tttttatttt ttttaatgat tgtttataga ggtttattta ggttttttta tttatttatt 240
tatttttttt tggttatttg ttaattattt gttttagttt tgaatgttta gatttttgtt 300
gaaagttttt gttttgtttt tgattttaag tgtgatttgg tttttttgta gtttttttat 360
ttttatttta ttttaaaagg gtaaaagttt tggttgtgag tgtttggttt tggagtttat 420
gttatgggtg atgtgtgttt tagttgtttt attaaaagtt attgtattaa tagattttga 480
ttgtatttag ttttgttttt ttgatatggt tatattatat taatttggat gtatgtgaaa 540
ttatatttgt aattttaagt attttgttgt tatttttagt ttgaataatg ttgttgattt 600
gggattatat tttttggtag tttatagtat atttatgata gataatagtt tattattgtt 660
ttttgatatg gttgatttta tgttaaagaa tgagggtttt tttttttttt tttttttttt 720
tttttttttt tttttttttt tttttttttt ttttttgatg tatatttttt ttttttaatt 780
ttgttgatgt tttttttgtt attttttttt tttgtatttg aatttttaga taataggttg 840
tgatttttgg ttgtatgttt atgggttttt ttatgttatt aatttagttt atagtgtgga 900
attaaagaat atagattgta agtgataggt tagttagtta gtaaggtaag tttgttagta 960
ttttttattt attaatgatt tttttttagt ttattttttg ttttattggt tgtttttttt 1020
aaaaataaaa ttgtttattt aaatttttat gtttggggta ttttggtttt taaatattgt 1080
ttgaaagtga aaggattagg taaaatatgt ttttttaaaa tgttaatata ttttttggtt 1140
tatttttttt tttgaggtta attaggaaat ttttgttggt tgtattaatg ttaaattgat 1200
aattttagtt ataattatat tgtgtttgaa atttaatttt tatttgtggg tagatggttg 1260
tgtttggtag tgatattgaa tttattttgt tagtttttga ttatttaatt attgtttgtt 1320
tttttttttt ttttgttagt tgattatttt atttttattt tttttgttgt aaaatgtagt 1380
gttgtttttt attatttata gttgtatttt gtaaggttta ttagtgttat tgttttttta 1440

atttgttgtg tatgagaatg gatggttttt ttaagtgtag taattatatg taagttgttt 1500
atttatttga tttgttatat atattggtat tgtttgattt taaaaatgta gtattttttt 1560
taaatagata gggtttttta tattttaaat attgatgaag gtggtgtgtg ttttatattt 1620
tgtagaaaaa agttgtagga gggttagttt ttttatttgt gaattattat gttttttggg 1680
aagatagatg atatgttttt attaaaggag gaggttagtg attttttttt ttgattaaat 1740
tataaattag ggtgtttttt agtttttaga ggggattttt ttatattttt tatttttttg 1800
ttttttttgg gttagttttt ttgtttaggt atgaagaatg ggtgttatga tttatttagt 1860
tttttttttt ttaaaagttt tttgggtttt ttttttttgg gaagatattt ggattaggat 1920
tttatggttg tgaagaattg ttataggttt attatttgaa tataatttgt gttggttttt 1980
tttttttttt ttttgttttt ttaaaaagga tataggagtt tgtttttagt ttaaggaaat 2040
ggtgatgtta atttgtgtta aatgtgttgg ggttttaggt tttaaatttg attgaaaatt 2100
tattgttttg gaggtataga gttttgtttt taaatggtta taggtttagg gagagggagt 2160
ggtttagaaa atattgtgtt tttgtttttt gtggattttt agggtttgtt gagggattgt 2220
gttgggtaag ggatttatgg gtttttttta tttattttat gttatgagag agaatttggt 2280
agtgttagat ttattttagt gggttgggga tgtagggttt gggtgaaaat agtttaggtt 2340
ggttattttg gatttggttt tttagatttt ttttgtaaag ttggagaggt tggggtggtt 2400
tttggggttt ggtttagagt tgttagttta gtttgggaaa gaagttgtgt taggattttt 2460
ggtgtggggt agagagttga ttgagggtag ttttttagtg gtttttgtaa tgttttttgg 2520
ttgttgggat atgaagattt aggtttgggt ttgagggttg tggtaatttt tgaggaaggt 2580
gggttggatt tgggttgtag ggtgaatttt attgggtgtt tttgtttttt tgaagttgtt 2640
ttgtgttttt gtggtttttt tgttttatgg ttggtttgtt attttttgtt tgaggtttag 2700
tttggggttt taaggtggtg ggagttagta ttatgtttgg gatgtttgat ttaagtagta 2760
aaggagtagg aaggagaatt tttggaggaa gtgtaggtag ttatgtggag ggtggggagg 2820
aaaagtatat ttattggatt ggtatttttt ttaatagagt tttttgttaa tgatatttat 2880
ttaattgtat ttgatagtag ttatgaatat atttttggta aataggattt atattttttg 2940
tgtttaaaat tgtttgttag tttttaagtt aattgtagta ataattgttg gattatttta 3000
aatgtggtta aaattgatgt tggataaaat taatttgtta tatgttggtt ttgaaggaaa 3060
ttatttgtat agtttgatga attgtttgta gtttttttag taatttataa tttgtttttt 3120
tttttttttt ttttttgtat ttttattttt tttgtttttt ttggagtttt tttttttatt 3180
ttattttttt ttgtagattt taggtaattt gaaatggtat tgattttttt tattttttga 3240
ttattttttt gaagtatgaa tttgggataa aattatatat tagaaaattt gttgtaatta 3300
ttttgtggtg ttattagtat tgattaatta tgtgtgtggt ttagtgttgg aatgtttagt 3360
gtagtttggg atgtggattt gtgttttgtt tgattgatag ttttggtaat taagtgattg 3420
tatagatttt tttggttgtt ttaataattt tgttagggtt tgtttgtgga tagaataagt 3480
tgaaattaat gtgttttttg ttttttgagt agttttgatt gtgtatttag attgattggg 3540
gaggaggaat ttgataaaag gaaaatagta gttttaaatg attggattag ggggagtagg 3600
tagaaagttt atttttttga tggggaggga agagtgagag atatgtatta gattataaga 3660
gttttgttga gttgggtagt tggttttgtg tggagtgaga ggtttggtgt ttgttatttt 3720
tggtttttag gtaggattgg tagtattttt tttttgtttt taagggggtg tagaggtttt 3780
ttgtgttgta gttgatgggt tttgtttggt tgtggatagt agtgttttta tatgttgtag 3840
ttataatttg gtagtataat tgtttttgtt tggattagag gtagagattg gtggtttagt 3900
gtaaatgtta tgtagtagtt tttaataaat gaatgtttag attgttagat tgtgaagtat 3960
attgtgagtt ttgtgtgttt tttttttgtt tttttaattt ggttttgaat ttttttattt 4020
ttttatttta gtatatatat tatattttgg tgtatgttat aaatatgtta tatatttaga 4080
gtgattttgt tatggtgttt gtgggagggg tattttttga gtttaatatt tattttgttg 4140
aggtttgtgg ggtgtttggg ttgattgttg gtattaggaa taggaaaaag attttgatgg 4200
gatttgtttt gtggaagtgt tttttataag tttattttgg aatttttgaa agaaattttt 4260
tttgggaaga gggttttgaa agtgttgaat taggaggtgg gatttttagt ttttggtgga 4320
ggggttttag ttaagagtat tttaattttt ttatattagt ttttttaaat tttaaaaatt 4380
taattaattg aatgtaaata tatataaaag ggggaaggga agtaaaatta atttgttgag 4440
tttaaagtgt tgtgttgtgg gaggaggttg tagggtgttt ggtgtagggt tgagtgtgta 4500
gtggagggtt ttattgattg tgtggagatt gtggttttta tttggtagat atttagtggg 4560
ttgtgtatgt ttgtgtgtgt ggtggggggt gttatgtttt aatttagttt gttgtttgtt 4620
tgtttttttt tttttattag tttgtagttg tttattttta gagagaaaaa tgtgagtgtg 4680
agtatattat gtattattag ggatatatta tggttttaat gtggtttaat tagtgttgtt 4740
aattgaataa atattattat attgaataat gataatatga tgaaaatatt aatgttttgg 4800
aattttgtat gtgggagttg tttttttagt ttttttggga tattttaagt atgtttagat 4860
ttattagtag atgtggttgg gtgtattttt aggaaggtga gtttatagtt taagatatat 4920
gtgtgttatt gtaggtatag aaataatatt gtgggttatg ttataagttt tttatttata 4980
tattgtttat tatgtaaaag agtttttttt aatggtattt tttttttttt tgtaagagtt 5040
ggtaaaaagt aaattagata ggagtttgtt agggtttttt tttattgtgt ttttatttgg 5100
tattttaaga atgttgtatt gggtgttttg agtgtgtttg gttttaatag aggttgttag 5160
gtaggtggag ttgtggaaaa aggatttatt taattagtat ttaaatttat agtttttttt 5220

tattgttaaa tgtttttttt ttgggaattt ggtgaaaagt aggaatttaa taattaggaa 5280
atgtggaagg tttttaaaaa tagggatttg aaattttaat ttttttattg attttggagg 5340
gttagggggg tggggaagtg tgtgtgggtt gggtgggagg tatgttttgg ttggtgtgtg 5400
tttttgtttt tgatgtttag tatattattt tatttttttt tttttgtatt tttatttttt 5460
ttttatggag ttttgggaga tagagttata ggaggttggt agttttgttt ttgttgtttg 5520
aaggtaggtt tagttttttt ttggttgttg agtggtatta gggttagggt ttaagtaggg 5580
tttttagaat gaggtgttgg gtgtgtttta ggaaataggg atgtaggatt taggttttag 5640
agtgtttttt tttgggttga attgtttttt atttttgttg ggggtttata ggttatattt 5700
ttttgttttg tgggagtttt ttttaggagg aaatgttggt gttaattgtt gttgaatgag 5760
ttgttttttg atgtaattat ttattttttg ggtggtgtgt ttggattaga agttgttgtt 5820
taggtatttg tttagtggtg tattttgtag atttgggaga gattgagaaa gttgattgag 5880
gttgttttta atttttgtgt tttaaggttt tttatggtat ttgggaatgt ggttttgttt 5940
gtattaaagg tagaaaatgg ggtggggagg ttttaatttg ttttagtgat ggttgtaatt 6000
taataaggtg attgattagt atttaaagtg ttgttttaat tagttgtttg ttaattaata 6060
ttgtaaatga attttttttt ttttgattgg tatttttgtt tagtgttttg aggttttggt 6120
tttttttttg ttttggtttt tatatttttt tttatttagt tttaggagtt gataggtttt 6180
agagttttgt ttatgtatga tatggttgtg ttatataggg gttttttaat ttttttagga 6240
gttggttttt aaaggataat ggtttaattt aggaggggtg aaataaaatt tttttttatg 6300
tttttttttgg tttttttttt aattgttttt atttgttat tttttaatt tttatat 6357

<210> 36
<211> 167
<212> DNA
<213> Artificial Sequence

<220>
<223> ONECUT 1 MSP-Amplicon

<400> 36

gttttgaaat ttattagaat aacgacgttt taaaaataaa ggcgtagtaa gtattttttt 60
tttcgttgtc gcgggttgaa ttacggacgt tcgcgggtcg tttagtttcg acggttcgta 120
gggggcgcgc gtcgtagtcg tagtatagtt cggttatttt tagaaag 167

<210> 37
<211> 139
<212> DNA
<213> Artificial Sequence

<220>
<223> TFAP2E (76256) MSP-Amplicon (on Bis-1)

<400> 37

tttagaagcg gttttcgtat cgttgcggtg ggcgttttcg ggtttcgatt tcgttagcgt 60
cgcggggtag aggtatttgg agttcgtagg gtttagattt gggttggaaa agtttcgttg 120
attgtaggta agcgttcgg 139

<210> 38
<211> 97
<212> DNA
<213> Artificial Sequence

<220>
<223> BARHL2 TSP PCR Amplicon

<400> 38

attgtttgtt agtttttaag ttaatcgtag taataatcgt tggattattt taaatgtggt 60
taaaatcgac gttggataaa attaatttgt tatatgt 97

<210> 39
<211> 163

<212> DNA
<213> Artificial Sequence

<220>
<223> FOXL2 HM-Amplicon

<400> 39

ccaagacctg ggcttgcagc gccgccaaca ggcccgggga cacgaggcgc tccaggccgg 60
ggtcttcccg gctgctggcc cctctcgctc cccacccgct ggcggcgcct cggtcgcccg 120
caattgaccc aacccgcttc ctgcgtttgc ccctcaggtt tcc 163

<210> 40
<211> 95
<212> DNA
<213> Artificial Sequence

<220>
<223> TFAP2E HM-Amplicon

<400> 40

aaacccaaac ctaaattaaa aaaacttcgc taactacaaa caaacgtccg aaaaaaacga 60
ccaaacgaaa ccccgacgct ttaccacaca cttcc 95

<210> 41
<211> 29
<212> DNA
<213> Artificial Sequence

<220>
<223> ONECUT 1 3658891.1Forward

<400> 41

gttttgaaat ttattagaat aacgacgtt 29

<210> 42
<211> 31
<212> DNA
<213> Artificial Sequence

<220>
<223> ONECUT 13658891.1Reverse

<400> 42

ctttctaaaa ataaccgaac tatactacga c 31

<210> 43
<211> 21
<212> DNA
<213> Artificial Sequence

<220>
<223> ONECUT 13658891.1Probe

<400> 43

tacggacgtt cgcgggtcgt t 21

<210> 44
<211> 20

<212> DNA
<213> Artificial Sequence

<220>
<223> FOXL2 17389-Forward

<400> 44

ccaaaaccta aacttacaac                                                    20

<210> 45
<211> 16
<212> DNA
<213> Artificial Sequence

<220>
<223> FOXL217389-Reverse

<400> 45

gagaggggtt agtagt                                                        16

<210> 46
<211> 31
<212> DNA
<213> Artificial Sequence

<220>
<223> FOXL2 17389-Forward Blocker

<400> 46

tacaacacca ccaacaaacc caaaaacaca a                                       31

<210> 47
<211> 23
<212> DNA
<213> Artificial Sequence

<220>
<223> FOXL2 17389-Reverse Blocker

<400> 47

ttgggaagat tttggtttgg agt                                                23

<210> 48
<211> 39
<212> DNA
<213> Artificial Sequence

<220>
<223> FOXL2 17389-SC-CH3 (methylation specific Scorpion probe

<400> 48

ccgccgaaaa cacgaaacgg cgggagaggg gttagtagt                               39

<210> 49
<211> 49
<212> DNA
<213> Artificial Sequence

<220>
<223> FOXL2 17389-SC-total (Scorpion probe specific for total DNA)

<400> 49

cccgggaaga ttttggtttg gagcccgggc caaaacctaa acttacaac 49

<210> 50
<211> 20
<212> DNA
<213> Artificial Sequence

<220>
<223> FOXL2 17389-Taqman probe specific for total DNA

<400> 50

ctccaaacca aaatcttccc 20

<210> 51
<211> 19
<212> DNA
<213> Artificial Sequence

<220>
<223> FOXL2 17389-Taqman probe specific for methylated DNA

<400> 51

ccgaaaacac gaaacgctc 19

<210> 52
<211> 21
<212> DNA
<213> Artificial Sequence

<220>
<223> TFAP2E MSPepsilon-G2 (forward Primer) MSP-Amplicon (on Bis-1)

<400> 52

tttagaagcg gttttcgtat c 21

<210> 53
<211> 20
<212> DNA
<213> Artificial Sequence

<220>
<223> TFAP2E MSPepsilon-C1 (reverse Primer) MSP-Amplicon (on Bis-1)

<400> 53

ccgaacgctt acctacaatc 20

<210> 54
<211> 22
<212> DNA
<213> Artificial Sequence

<220>
<223> TFAP2E MSPepsilon-P1 (Probe) MSP-Amplicon (on Bis-1)

<400> 54

ttgcggtggg cgttttcggg tt 22

<210> 55
<211> 20
<212> DNA
<213> Artificial Sequence

<220>
<223> TFAP2E HM 76256-21 (forward Primer) (on Bis-2)

<400> 55

aaacccaaac ctaaattaaa 20

<210> 56
<211> 17
<212> DNA
<213> Artificial Sequence

<220>
<223> TFAP2E 76256-22 (reverse Primer) (on Bis-2)

<400> 56

ggaagtgtgt ggtaaag 17

<210> 57
<211> 30
<212> DNA
<213> Artificial Sequence

<220>
<223> TFAP2E 76256.2B5 (Blocker) (on Bis-2)

<400> 57

gtaaagtgtt ggggttttgt ttggttgttt 30

<210> 58
<211> 26
<212> DNA
<213> Artificial Sequence

<220>
<223> TFAP2E 76256-28dS (Probe) (on Bis-2)

<400> 58

aaaaacttcg ctaactacaa acaaac 26

<210> 59
<211> 25
<212> DNA
<213> Artificial Sequence

<220>
<223> BARHL2 Primer

<400> 59

attgtttgtt agtttttaag ttaat 25

<210> 60
<211> 27
<212> DNA
<213> Artificial Sequence

<220>
<223> BARHL2 Primer

<400> 60

acatataaca aatatatttt atccaac                                          27

<210> 61
<211> 26
<212> DNA
<213> Artificial Sequence

<220>
<223> BARHL2 TAQMAN probe

<400> 61

ttggattatt ttaaatgtgg ttaaaa                                           26